(19) Europäisches Patentamt
European Patent Office
Office européen des brevets

(11) EP 1 710 233 A1

(12) EUROPEAN PATENT APPLICATION
published in accordance with Art. 158(3) EPC

(43) Date of publication:
11.10.2006 Bulletin 2006/41

(21) Application number: 05704221.0

(22) Date of filing: 21.01.2005

(51) Int Cl.:
*C07D 201/00* (2006.01)

(86) International application number:
PCT/JP2005/001168

(87) International publication number:
WO 2005/073210 (11.08.2005 Gazette 2005/32)

(84) Designated Contracting States:
AT BE BG CH CY CZ DE DK EE ES FI FR GB GR HU IE IS IT LI LT LU MC NL PL PT RO SE SI SK TR

(30) Priority: 28.01.2004 JP 2004019235

(71) Applicant: Kissei Pharmaceutical Co., Ltd.
Matsumoto-shi, Nagano 399-8710 (JP)

(72) Inventors:
- NAKAMURA, T.
  Kissei Pharmac. Co. Ltd,
  Centr. Res.
  Minamiazumi-gun, Nagano 399-8404 (JP)
- SHIOHARA, H.
  Kissei Pharmac. Co. Ltd.,Centr. Res.
  Minamiazumi-gun, Nagano 399-8304 (JP)
- TERAO, Y.
  Kissei Pharmac. Co. Ltd.,
  Centr. Resrch.
  Minamiazumi-gun, Naagano 399-8304 (JP)
- NAKAYAMA, S.
  Kissei Pharmac. Co. Ltd.,
  Centr. Res.
  Minamiazumi-gun, Nagano 399-8304 (JP)
- MIYAZAWA, K.
  Kissei Pharmac. Co. Ltd. Centr. Res.
  Minamiazumi-gun, Nagano 399-8304 (JP)
- OHNOTA, H.
  Kissei Parmach. Co. Ltd. Centr. Resrch.
  Minamiazumi-gun, Nagano399-8304 (JP)

(74) Representative: Hayes, Adrian Chetwynd
Boult Wade Tennant,
Verulam Gardens
70 Gray's Inn Road
London WC1X 8BT (GB)

# (54) NOVEL BENZOFURAN DERIVATIVE, MEDICINAL COMPOSITION CONTAINING THE SAME, AND USES OF THESE

(57) The present invention provides compounds represented by general formula (I):

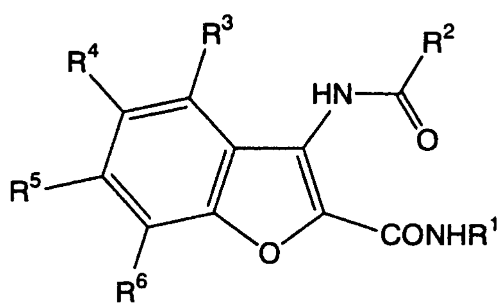

(I)

or pharmaceutical acceptable salts thereof, wherein $R^1$ is hydrogen or lower alkyl; $R^2$ is lower alkyl, halo-lower alkyl, cycloalkyl, heterocycloalkyl, aryl, aralkyl, arylalkenyl, aryloxy-lower alkyl, heteroaryl, heteroaryl-lower alkyl, etc; $R^3$, $R^4$, $R^5$ and $R^6$ are each hydrogen, halogen, cyano, lower alkyl, halo-lower alkyl, lower alkoxy, hydroxy, aryl, etc; provided that at least one of $R^3$, $R^4$, $R^5$ and $R^6$ is other than hydrogen. Compound (I) of the present invention shows a potent adenosine $A_{2A}$ receptor antagonistic activity, and are useful for treating or preventing a disease mediated by adenosine $A_{2A}$ receptors such as motor function disorders, depression, anxiety disorders, cognitive function disorders, cerebral

EP 1 710 233 A1

ischemia disorders, restless legs syndrome and the like.

## Description

### TECHNICAL FIELD

The present invention relates to novel benzofuran derivatives, which exhibit adenosine $A_{2A}$ receptor antagonistic activities, pharmaceutical compositions containing the same, and their uses.

### BACKGROUND ART

Adenosine is an endogenous purine nucleoside, and has a wide variety of regulatory functions and physiological effects. Four distinct adenosine receptor subtypes are known as $A_1$, $A_{2A}$, $A_{2B}$ and $A_3$ receptors. Most of the actions of adenosine are mediated by the interaction with its membrane receptors: $A_1$, $A_{2A}$, $A_{2B}$ and $A_3$ receptors which belong to G-protein coupled receptor families.

Distribution and function of adenosine $A_{2A}$ receptors in the central nervous system are well known. It has been reported that adenosine $A_{2A}$ receptors are involved in the regulation of cholinergic, GABA-nergic and glutamate neurons. Moreover, it has been reported that adenosine $A_{2A}$ receptors are associated functionally with dopamine $D_2$ receptors, and blockade of adenosine $A_{2A}$ receptors increases the binding affinity of dopamine to dopamine $D_2$ receptors (see nonpatent literature 1). Parkinson's disease is well known as a disorder relevant to impairment of dopamine neuron. Parkinson's disease is a progressive neurodegenerative disease, which usually affects elderly patients and is characterized by impairment in coordinated motor function such as rest tremor, rigidity, akinesia, postural instability and the like. It is thought that Parkinson's disease results from deficiency of dopamine in the striatum, which is caused by degeneration of dopamine neuron in the substantia nigra. Adenosine $A_{2A}$ receptors are abundantly present in the striatum which is playing a crucial role in the regulation of coordinated motor function. As discussed above, there is an opposing relationship between adenosine $A_{2A}$ receptors and dopamine D2 receptors. Accordingly, it is expected that agents selectively antagonizing adenosine $A_{2A}$ receptors are useful for treating motor function disorders such as Parkinson' s disease, Huntington's disease, Wilson's disease and the like (see nonpatent literatures 2 to 4). It has been reported that blockade of adenosine $A_{2A}$ receptors results in antidepressing, anxiolytic or neuroprotective actions. Accordingly antagonists of adenosine $A_{2A}$ receptors are expected to be useful for treating depression, anxiety, cognitive function disorders such as Alzheimer disease and the like (see nonpatent literatures 5 and 6, patent literature 1) . It has also been reported that blockade of adenosine $A_{2A}$ receptors ameliorates disorders after cerebral ischemia and reduces the volume of cerebral infarction. Accordingly, antagonists of adenosine $A_{2A}$ receptors are expected to be useful for treating cerebral ischemia disorders such as cerebral apoplexy, brain lesion after cerebral vasospasm and the like (see nonpatent literature 7). Antagonists of adenosine $A_{2A}$ receptors are also expected to be useful for treating restless legs syndrome (see patent literature 2).

Sangapure S.S. and Agasimundin Y.S. et al disclose benzofuran derivatives of the following general formula:

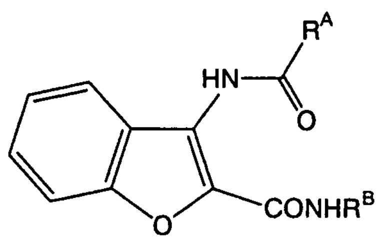

wherein $R^A$ is a phenyl group, unsubstituted or substituted with halogen, lower alkyl, lower alkoxy or carboxy, a benzyl group or a lower alkyl group; $R^B$ is a hydrogen atom or a lower alkyl group, as an intermediate for preparing benzofuro [3,2-d]pyrimidine derivatives (see nonpatent literatures 8 to 11). However, with regard to biological activities of the benzofuran derivatives , there is no description at all.

Basavaraj P. et al disclose 1-acetylaminonaphtho-[2,1-b]furan-2-carboxamide and 1-benzoylaminonaphtho [2,1-b]-furan-2-carboxamide as an intermediate for preparing 4-oxonaphtho[2,1-b]furo[3,2-d]pyrimidine derivatives which are useful as an antimicrobial agent, anthelmintic agent or anti-inflammatory agent (see nonpatent literature 12). However, with regard to biological activities of the naphtho [2,1-b]furan derivatives, there is no description at all.

3-Benzoylamino-5-chlorobenzofuran-2-carboxamide, 5-chloro-3-[2-(3,4-diethoxyphenyl)acetylamino]benzofuran-2-carboxamide, 5-bromo-3-[2-(3,4-diethoxyphenyl)acetylamino]-benzofuran-2-carboxamide, 5-chloro-3-(2-chloroacetylamino)-benzofuran-2-carboxamide, and 3-acetylamino-5-chlorobenzofuran-2-carboxamide are registered in Chemical Abstract (see nonpatent literatures 13 to 17). However, with regard to biological activities of these compounds, there is no description at all.

Oota T. et al disclose benzofuran derivatives represented by the following general formula:

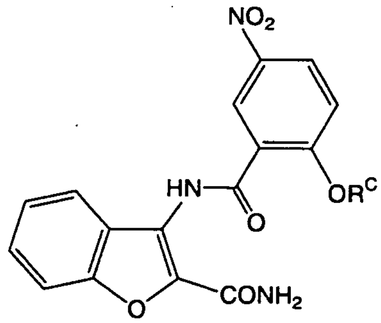

wherein $R^c$ is a lower alkyl group, as an intermediate for preparing benzofuro[3,2-d]pyrimidine-4-one derivatives which are useful as a phosphodiesterase inhibitor specific to cyclic GMP (see patent literature 3). However, with regard to biological activities of the benzofuran derivatives, there is no description at all.
Nonpatent literature:


1. Ferre S. et al, "Proc. Natl. Acad. Sci. U.S.A.", 1991, vol.88, p.7238-7241
2. Ferre S. et al, "Neurosci. Lett.", 1991, vol.130, p.162-164
3. Mandhane S.N. et al, "Eur. J. Pharmacol.", 1997, vol.328, p.135-141
4. Varani K. et al, "The FASEB Journal", 2003, vol.17, p.2148-2150
5. EL. Yacoubi M. et al, "British J. Pharmacol.", 2001, vol. 134, p.68-77
6. Dall'Igna O. et al, "British J. Pharmacol.", 2003, vol.138, p.1207-1209
7. Phillis J.W. et al, "Brain Res.", 1995, vol.705, p.79-84 8. Sangapure S. S. et al, "Indian J. Chem.", 1978, vol.16B, p.627-629
9. Agasimundin Y.S. et al, "Indian J. Chem.", 1981, vol.20B, p.114-117
10. Agasimundin Y.S. et al, "Indian J. Chem.", 1993, vol.32B, p.965-968
11. Agasimundin Y.S. et al, "Indian J. Heterocyclic Chem.", 1994, vol.3, p.247-252
12. Basavaraj P. et al, "Indian J. Heterocyclic Chem.", 2002, vol.12, p.89-94
13. "Chemical Abstract", Registry Number 340017-67-6
14. "Chemical Abstract", Registry Number 663931-40-6
15. "Chemical Abstract", Registry Number 633288-47-8
16. "Chemical Abstract", Registry Number 397881-00-4
17. "Chemical Abstract", Registry Number 332375-01-6


Patent literature:


1. International Publication No. WO2004/108137 pamphlet
2. International Publication No. WO2004/019949 pamphlet
3. Japanese Patent Laid Open No. 1995/267961


## DISCLOSURE OF THE INVENTION

The present inventors have intensively investigated a novel compound having adenosine $A_{2A}$ receptor antagonistic activities, and found surprisingly that benzofuran derivatives represented by general formula (I) show a potent adenosine $A_{2A}$ receptor antagonistic activity, and are useful for treating or preventing a disease mediated by adenosine $A_{2A}$ receptors. Based on these findings, the present invention has been accomplished.

The present invention therefore provides a compound represented by general formula (I):

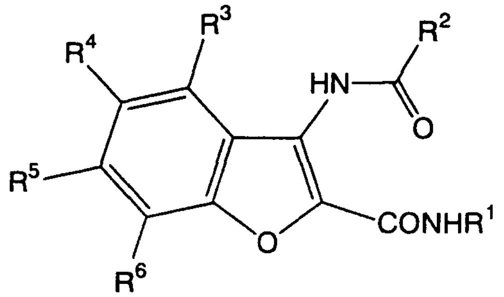

(I)

a prodrug thereof, or a pharmaceutically acceptable salt thereof, wherein
$R^1$ is a hydrogen atom or a lower alkyl group;
$R^2$ is:

a) a lower alkyl group,
b) a halo-lower alkyl group,
c) a hydroxy-lower alkyl group,
d) a cycloalkyl group,
e) an aryl-cycloalkyl group,
f) a heterocycloalkyl group,
g) an aryl group, unsubstituted or substituted with 1 to 5 substituents selected from the group consisting of $X^1$, $X^2$, $X^3$, $X^4$ and $X^5$,
h) an aralkyl group, wherein the ring of the aralkyl group is unsubstitutedor substituted with 1 to 5 substituents selected from the group consisting of $X^1$, $X^2$, $X^3$, $X^4$ and $X^5$,
i) an aryl-alkenyl group, wherein the ring of the aryl-alkenyl group is unsubstituted or substituted with 1 to 5 substituents selected from the group consisting of $X^1$, $X^2$, $X^3$, $X^4$ and $X^5$,
j) a lower alkyl group substituted with a group selected from a lower alkoxy group or a lower acyloxy group,
k) an aryloxy-lower alkyl group, wherein the ring of the aryloxy-lower alkyl group is unsubstituted or substituted with 1 to 5 substituents selected from the group consisting of $X^1$, $X^2$, $X^3$, $X^4$ and $X^5$,
1) an aralkyloxy-lower alkyl group, wherein the ring of the aralkyloxy-lower alkyl group is unsubstituted or substituted with 1 to 5 substituents selected from the group consisting of $X^1$, $X^2$, $X^3$, $X^4$ and $X^5$,
m) an arylsulfanyl-lower alkyl group, wherein the ring of the arylsulfanyl-lower alkyl group is unsubstituted or substituted with 1 to 5 substituents selected from the group consisting of $X^1$, $X^2$, $X^3$, $X^4$ and $X^5$,
n) a heteroaryl group, unsubstituted or substituted with 1 to 3 substituents selected from the group consisting of $X^6$, $X^7$ and $X^8$, or
o) a heteroaryl-lower alkyl group, wherein the ring of the heteroaryl-lower alkyl group is unsubstituted or substituted with 1 to 3 substituents selected from the group consisting of $X^6$ , $X^7$ and $X^8$;

$X^1$, $X^2$, $X^3$, $X^4$ and $X^5$ are each independently:

a) a halogen atom,
b) a lower alkyl group,
c) a halo-lower alkyl group,
d) a cycloalkyl group,
e) a lower alkoxy group,
f) a halo-lower alkoxy group,
g) a cycloalkyloxy group,
h) a heterocycloalkyloxy group,
i) a lower alkoxy-lower alkoxy group,
j) a hydroxy-lower alkyl group,
k) a hydroxyl group,
1) a carboxy group,
m) a lower alkoxycarbonyl group,
n) an aralkyloxycarbonyl group,
o) a lower acyl group,
p) a cyano group,

q) $-A^1-NR^{20}R^{21}$,
r) $-A^2-SR^{22}$,
s) $-SO_2NR^{23}R^{24}$,
t) a phenyl group, unsubstituted or substituted with 1 to 3 substituents selected independently from the group consisting of a halogen atom, a lower alkyl group, a halo-lower alkyl group, a lower alkoxy group, a halo-lower alkoxy group and a di(lower alkyl)amino-lower alkyl group,
u) a phenoxy group, unsubstituted or substituted with 1 to 3 substituents selected independently from the group consisting of a halogen atom, a lower alkyl group, a halo-lower alkyl group, a lower alkoxy group, a halo-lower alkoxy group and a di(lower alkyl)amino-lower alkyl group,
v) a heteroaryl group, unsubstituted or substituted with 1 to 3 substituents selected independently from the group consisting of a halogen atom, a lower alkyl group, a halo-lower alkyl group, a lower alkoxy group, a halo-lower alkoxy group and a di(lower alkyl)amino-lower alkyl group,
w) a heteroaryloxy group, unsubstituted or substituted with 1 to 3 substituents selected independently from the group consisting of a halogen atom, a lower alkyl group, a halo-lower alkyl group, a lower alkoxy group, a halo-lower alkoxy group and a di(lower alkyl)amino-lower alkyl group, or
x) a lower alkoxy group substituted with a group selected form an aryl group or a heteroaryl group, or
when two of $X^1$, $X^2$, $X^3$, $X^4$ and $X^5$ are adjacent each other, they are bonded together to form a group represented by $-O\ (CH_2)_mO-$, $-O\ (CH_2)_n-$ or $-(CH_2)_p-$;
$R^{20}$ and $R^{21}$ are each independently a hydrogen atom, a lower alkyl group, a cycloalkyl group, a heterocycloalkyl group, a bridged cyclic hydrocarbon group, a heteroaryl-lower alkyl group, a hydroxy-lower alkyl group, a lower alkoxy-lower alkyl group, a lower acyl group, a lower alkoxycarbonyl group, an aralkyloxycarbonyl group or a di (lower alkyl)amino-lower alkyl group, or
$R^{20}$ and $R^{21}$, taken together with the nitrogen atom to which they are bonded, form a cyclic amino group, wherein the cyclic amino group is unsubstituted or substituted with one or two substituents selected independently from the group consisting of:

a) a lower alky group,
b) a cycloalkyl group,
c) a phenyl group, unsubstituted or substituted with 1 to 3 substituents selected independently from the group consisting of a halogen atom, a lower alkyl group, a halo-lower alkyl group, a lower alkoxy group and a halo-lower alkoxy group,
d) an aralkyl group, wherein the ring of the aralkyl group is unsubstituted or substituted with 1 to 3 substituents selected independently from the group consisting of a halogen atom, a lower alkyl group, a halo-lower alkyl group, a lower alkoxy group and a halo-lower alkoxy group, or adjacent ring-carbon atoms of the aralkyl group are substituted with $-O-(CH_2)_m-O-$,
e) a heteroaryl group,
f) a heteroaryl-lower alkyl group,
g) a lower alkyl group substituted with a group selected from a hydroxyl group, a lower alkoxy group, a carboxy group, an aralkyloxycarbonyl group, a cyclic aminocarbonyl group or a di(lower alkyl)amino group,
h) a hydroxyl group,
i) an oxo group,
j) a lower alkoxycarbonyl group,
k) an aralkyloxycarbonyl group,
1) a carbamoyl group,
m) a lower acyl group,
n) a benzoyl group,
o) a di(lower alkyl)amino group, and
p) a diphenylmethylene group;

$A^1$ is a bond, a $C_{1-3}$-alkylene group or a carbonyl group;
$A^2$ is a bond or a $C_{1-3}$-alkylene group;
$R^{22}$ is:

a) a lower alkyl group,
b) a phenyl group, unsubstituted or substituted with 1 to 3 substituents selected independently from the group consisting of a halogen atom, a lower alkyl group, a halo-lower alkyl group, a lower alkoxy group and a halo-lower alkoxy group,
c) a heteroaryl group, unsubstituted or substituted with 1 to 3 substituents selected independently from the group consisting of a halogen atom, a lower alkyl group, a halo-lower alkyl group, a lower alkoxy group and a halo-lower

alkoxy group, or
d) a di(lower alkyl)amino-lower alkyl group;
$R^{23}$ and $R^{24}$ are each independently a hydrogen atom or a lower alkyl group, or
$R^{23}$ and $R^{24}$, taken together with the nitrogen atom to which they are bonded, form a cyclic amino group, wherein the cyclic amino group is unsubstituted or substituted with a group selected from a lower alkyl group or an aralkyl group;
m is 1 or 2;
n is 2 or 3;
p is 3 or 4;

$X^6$, $X^7$ and $X^8$ are each independently:

a) a halogen atom,
b) a lower alkyl group,
c) a halo-lower alkyl group,
d) a hydroxy-lower alkyl group,
e) a cycloalkyl group,
f) a heterocycloalkyl-lower alkyl group,
g) a lower alkoxy group,
h) a halo-lower alkoxy group,
i) a lower acyl group,
j) a carboxy group,
k) $-A^1-NR^{20}R^{21}$,
1) $-A^2-SR^{22}$,
m) $-SO_2NR^{23}R^{24}$,
n) a phenyl group, unsubstituted or substituted with 1 to 3 substituents selected independently from the group consisting of a halogen atom, a lower alkyl group, a halo-lower alkyl group, a lower alkoxy group, a halo-lower alkoxy group and a di(lower alkyl)amino-lower alkyl group,
o) a phenoxy group, unsubstituted or substituted with 1 to 3 substituents selected independently from the group consisting of a halogen atom, a lower alkyl group, a halo-lower alkyl group, a lower alkoxy group, a halo-lower alkoxy group and a di(lower alkyl)amino-lower alkyl group,
p) an aralkyl group, wherein the ring of the aralkyl group is unsubstituted or substituted with 1 to 3 substituents selected independently from the group consisting of a halogen atom, a lower alkyl group, a halo-lower alkyl group, a lower alkoxy group, a halo-lower alkoxy group and a di (lower alkyl) amino-lower alkyl group,
q) a heteroaryl group, unsubstituted or substituted with 1 to 3 substituents selected independently from the group consisting of a halogen atom, a lower alkyl group, a halo-lower alkyl group, a lower alkoxy group, a halo-lower alkoxy group and a di(lower alkyl)amino-lower alkyl group,
r) a heteroaryloxy group, unsubstituted or substituted with 1 to 3 substituents selected independently from the group consisting of a halogen atom, a lower alkyl group, a halo-lower alkyl group, a lower alkoxy group, a halo-lower alkoxy group and a di(lower alkyl)amino-lower alkyl group, or
s) an aralkyloxy group;

$R^3$, $R^4$, $R^5$ and $R^6$ are each independently:

a) a hydrogen atom,
b) a halogen atom,
c) a lower alkyl group,
d) a halo-lower alkyl group,
e) a lower alkoxy group,
f) a halo-lower alkoxy group,
g) a hydroxyl group,
h) a cyano group,
i) an aryl group, unsubstituted or substituted with 1 to 3 substituents selected independently from the group consisting of a halogen atom, a lower alkyl group, a halo-lower alkyl group, a lower alkoxy group and a halo-lower alkoxy group,
j.) an aralkyloxy group, wherein the ring of the aralkyloxy group is unsubstituted or substituted with 1 to 3 substituents selected independently from the group consisting of a halogen atom, a lower alkyl group, a halo-lower alkyl group, a lower alkoxy group and a halo-lower alkoxy group,
k) a di(lower alkyl)amino group,

l) a lower alkylsulfanyl group, or
m) a nitro group, or
when two of $R^3$, $R^4$, $R^5$ and $R^6$ are adjacent each other, they are bonded together to form a group represented by -CH=CH-CH=CH-, provided that at least one of $R^3$, $R^4$, $R^5$ and $R^6$ is other than a hydrogen atom; with the proviso that the following compounds are excluded:

(1) 1-acetylaminonaphtho[2,1-b]furan-2-carboxamide,
(2) 1-benzoylaminonaphtho[2,1-b]furan-2-carboxamide,
(3) 3-benzoylamino-5-chlorobenzofuran-2-carboxamide,
(4) 5-chloro-3-[2-(3,4-diethoxyphenyl)acetylamino]-benzofuran-2-carboxamide,
(5) 5-bromo-3-[2-(3,4-diethoxyphenyl)acetylamino]-benzofuran-2-carboxamide,
(6) 5-chloro-3-(2-chloroacetylamino)benzofuran-2-carboxamide, and
(7) 3-acetylamino-5-chlorobenzofuran-2-carboxamide.

In another aspect, the present invention provides a pharmaceutical composition which comprises, as an active ingredient, a compound represented by general formula (I) or a pharmaceutically acceptable salt thereof.

In still another aspect, the present invention provides a therapeutic or prophylactic agent for a disease mediated by adenosine $A_{2A}$ receptors, which comprises a compound represented by general formula (I) or a pharmaceutically acceptable salt thereof.

In still another aspect, the present invention provides a pharmaceutical combination comprising a compound represented by general formula (I) or a pharmaceutically acceptable salt thereof and at least one selected from anti-Parkinson drugs, antidepressants, drugs for cognitive function disorders and drugs for cerebral ischemia disorders other than adenosine $A_{2A}$ receptor antagonists.

In still another aspect, the present invention provides a use of a compound represented by general formula (I) or a pharmaceutically acceptable salt thereof for the manufacture of a medicament for treating or preventing a disease mediated by adenosine $A_{2A}$ receptors.

In still another aspect, the present invention provides a method for treating or preventing a disease mediated by adenosine $A_{2A}$ receptors, which comprises administering an effective amount of a compound represented by general formula (I) or a pharmaceutically acceptable salt thereof.

The invention is described using the terms defined below unless otherwise specified.

The term "halogen atom" refers to a fluorine, chlorine, bromine or iodine atom. Preferred halogen atoms for $X^1$, $X^2$, $X^3$, $X^4$ and $X^5$ are a fluorine, chlorine or bromine atom, more preferably a fluorine or chlorine atom and most preferably a fluorine atom. Preferred halogen atoms for $X^6$, $X^7$ and $X^8$ are a fluorine, chlorine or bromine atom and most preferably a chlorine atom. Preferred halogen atoms for $R^3$ are a fluorine atom. Preferred halogen atoms for $R^4$ are a chlorine or fluorine atom, and more preferably a fluorine atom. Preferred halogen atoms for $R^5$ and $R^6$ are a chlorine or fluorine atom.

The term "lower alkyl group" refers to a straight chained or branched alkyl group having 1 to 6 carbon atoms such as a methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl group and the like. Preferred lower alkyl groups for $R^1$, $R^3$, $R^4$, $R^5$ and $R^6$ are a $C_{1-4}$ alkyl group, more preferably a methyl, ethyl or isopropyl group, and most preferably a methyl group. Preferred lower alkyl groups for $R^2$ are a $C_{2-4}$ alkyl group, and more preferably an ethyl, isopropyl or tert-butyl group.

The term "halo-lower alkyl group" refers to a lower alkyl group substituted with the same or different 1 to 3 halogen atoms such as a fluoromethyl, chloromethyl, bromomethyl, 3-bromopropyl, 4-bromobutyl, 5-bromopentyl, difluoromethyl, trifluoromethyl, 2,2,2-trifluoroethyl group and the like. Preferred halo-lower alkyl groups for $R^2$ are a 4-bromobutyl or 5-bromopentyl group. Preferred halo-lower alkyl groups for $R^4$, $R^5$ and $R^6$ are a trifluoromethyl group. Preferred halo-lower alkyl groups for $X^1$, $X^2$, $X^3$, $X^4$, $X^5$, $X^6$, $X^7$ and $X^8$ are a chloromethyl or trifluoromethyl group.

The term "hydroxy-lower alkyl group" refers to a lower alkyl group substituted with a hydroxyl group such as a hydroxymethyl, 1-hydroxyethyl, 2-hydroxyethyl, 2-hydroxy-2-methylpropyl, 3-hydroxypropyl group and the like.

The term "cycloalkyl group" refers to a 3- to 7-membered saturated cyclic hydrocarbon such as a cyclopropyl, cyclobutyl, cyclopentyl, cyclohexyl and cycloheptyl group. Preferred cycloalkyl groups for $R^2$ are a cyclopropyl or cyclobutyl group, and more preferably a cyclopropyl group.

The term "bridged cyclic hydrocarbon group" refers to a 5- to 7-membered bridged saturated cyclic hydrocarbon having 7 to 10 carbon atoms such as a bicyclo[2.2.1]heptane-2-yl, adamantan-1-yl group and the like.

The term "cycloalkyloxy group" refers to a group represented by (cycloalkyl)-O- such as a cyclopropyloxy, cyclobutyloxy, cyclopentyloxy, cyclohexyloxy group and the like.

The term "aryl-cycloalkyl group" refers to a cycloalkyl group substituted with an aryl group such as a 2-phenylcyclopropyl, 2-phenylcyclopentyl, 3-phenylcyclopentyl group and the like.

The term "heterocycloalkyl group" refers to a 4- to 7-membered saturated heterocyclic group having -NH-, -O- or -S-as a member of the ring such as a tetrahydrofuryl, tetrahydrothienyl, tetrahydropyranyl, pyrrolidin-2-yl, pyrrolidin-3-yl, piperidin-2-yl, piperidin-3-yl, piperidin-4-yl group and the like. The heterocycloalkyl group may be optionally substituted with one or two lower alkyl or aralkyl groups such as a N-methylpiperidin-4-yl, N-benzylpiperidin-4-yl, N-phenethylpiperidin-4-yl group and the like.

The term "heterocycloalkyloxy group" refers to a group represented by (heterocycloalkyl)-O- such as a tetrahydropyran-4-yloxy, piperidin-4-yloxy, N-methylpiperidin-4-yloxy, N-benzylpiperidin-4-yloxy, N-phenethylpiperidin-4-yloxy group and the like.

The term "heterocycloalkyl-lower alkyl group" refers to a lower alkyl group substituted with a heterocycloalkyl group such as a N-methylpiperidin-4-ylmethyl, N-isopropylpiperidin-4-ylmethyl, N-benzylpiperidin-4-ylmethyl, N-phenethylpiperidin-4-ylmethyl group and the like.

The term "alkenyl group" refers to an unsatutated hydrocarbon having 2 to 6 carbon atoms, which may be straight chained or branched and contains at least one double bond such as a vinyl, allyl group and the like.

The term "aryl group" refers to an aromatic hydrocarbon group having 6 to 10 carbon atoms such as a phenyl, 1-naphtyl and 2-naphthyl group, preferably a phenyl group.

The term "aralkyl group" refers to a lower alkyl group substituted with an aryl group such as a benzyl, phenethyl, 1-phenylethyl, 3-phenylpropyl, 4-phenylbutyl, naphthylmethyl group and the like, pref erably a benzyl , phenethyl, 1-phenylethyl, 3-phenylpropyl or 4-phenylbutyl group.

The term "aryl-alkenyl group" refers to an alkenyl group substituted with an aryl group such as a stylyl, cinnamyl group and the like.

The term "aryloxy group" refers to a group represented by (aryl)-O- such as a phenoxy, 1-naphthyloxy, 2-naphthyloxy group and the like, preferably a phenoxy group.

The term "aralkyloxy group" refers to a group represented by (aralkyl)-O- such as a benzyloxy, phenethyloxy, 1-phenylethoxy, 3-phenylpropoxy group and the like, preferably a benzyloxy group.

The term "aryloxy-lower alkyl group" refers to a lower alkyl group substituted with an aryloxy group such as a phenoxymethyl, 1-phenoxyethyl, 2-phenoxyethyl, 1-methyl-1-phenoxyethyl, 3-phenoxypropyl, naphthyloxymethyl group and the like, preferably a phenoxymethyl, 1-phenoxyethyl, 2-phenoxyethyl or 3-phenoxypropyl group.

The term "aralkyloxy-lower alkyl group" refers to a lower alkyl group substituted with an aralkyloxy group such as a benzyloxymethyl, 2-benzyloxyethyl, phenethyloxymethyl, naphthyloxymethyl group and the like, preferably a benzyoxymethyl group.

The term "lower alkylsulfanyl group" refers to a group represented by (lower alkyl)-S- such as a methylsulfanyl, ethylsulfanyl, propylsulfanyl, isopropylsulfanyl, butylsulfanyl group and the like.

The term "arylsulfanyl group refers to a group represented by (aryl)-S- such as a phenylsulfanyl, 1-naphthylsulfanyl, 2-naphthylsulfanyl group and the like, preferably a phenylsulfanyl group.

The term "arylsulfanyl-lower alkyl group" refers to a lower alkyl group substituted with an arylsulfanyl group such as a phenylsulfanylmethyl, 1-phenylsulfanylethyl, 2-phenylsulfanylethyl, 1-methyl-1-phenylsulfanylethyl, 3-phenylsulfanylpropyl, naphthylsulfanylmethyl group and the like, preferably a phenylsulfanylmethyl group.

The term "lower alkoxy group" refers to a straight chained or branched alkoxy group having 1 to 6 carbon atoms such as a methoxy, ethoxy, propoxy, isopropoxy, butoxy isobutoxy, sec-butoxy, tert-butoxy, pentyloxy, hexyloxy group and the like. Preferred lower alkoxy groups for $R^3$, $R^4$, $R^5$, $R^6$, $X^6$, $X^7$ and $X^8$ are a $C_{1-3}$ alkoxy group, more preferably a methoxy or ethoxy group, and most preferably a methoxy group. Preferred alkoxy groups for $X^1$, $X^2$, $X^3$ , $X^4$ and $X^5$ are a $C_{1-4}$ alkoxy group, and more preferably a methoxy, ethoxy, propoxy or isopropoxy group.

The term "halo-lower alkoxy group" refers to a lower alkoxy group substituted with the same or different 1 to 3 halogen atoms such as a difluoromethoxy, trifluoromethoxy, 2,2,2-trifluoroethoxy group and the like.

The term "lower alkoxy-lower alkyl group" refers to a lower alkyl group substituted with a lower alkoxy group such as a methoxymethyl, 2-methoxyethyl, ethoxymetyl group and the like, preferably a methoxymethyl group.

The term "lower alkoxy-lower alkoxy group" refers to a lower alkoxy group substituted with a lower alkoxy group such as a 2-methoxyethoxy, 2-ethoxyethoxy, 3-methoxypropoxy, 4-methoxybutoxy group and the like.

The term "lower acyl group" refers to a group represented by H-CO- or (lower alkyl) -CO- such as a formyl, acetyl, propionyl, butyryl, isobutyryl, valeryl, isovaleryl, pivaloyl group and the like, preferably an acetyl group.

The term "lower acyloxy group" refers to a group represented by (lower acyl)-O-such as an acetyloxy, propionyloxy, isobutyryloxy, pivaloyloxy group and the like, preferably an acetyloxy group.

The term "lower alkoxycarbonyl group" refers to a group represented by (lower alkoxy)-CO- such as a methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl group and the like.

The term "aralkyloxycarbonyl group" refers to a group represented by (aralkyloxy)-CO- such as a benzyloxycarbonyl group and the like.

The term "di(lower alkyl)amino group" refers to an amino group substituted with two lower alkyl groups such

as a dimethylamino, diethylamino, dipropylamino, diisopropylamino group and the like, preferably a dimethylamino or diethylamino group.

The term "di(lower alkyl)amino-lower alkyl group" refers to a lower alkyl group substituted with a di(lower alkyl) amino group such as a dimethylaminomethyl, 2-dimethylaminoethyl, 3-dimethylaminopropyl, diethylaminomethyl, 2-diethylaminoethyl group, preferably a 2-dimethylaminoethyl group.

The term "cyclic amino group" refers to a 5- to 8-membered cyclic amine which may contain -NH-, -O- or -S- as a member of the ring. Examples of cyclic amino group include a 1-pyrrolidyl, piperidino, piperazino, morpholino, thiomorpholino, azepan-1-yl group and the like, preferably a 1-pyrrolidyl, piperidino, morpholino, piperazino or azepan-1-yl group. Adjacent ring carbon atoms of the cyclic amino group may be optionally condensed with a benzene or cycloalkyl ring. Such condensed cyclic amino groups include a indolin-1-yl, 1,2,3,4-tetrahydroquinolin-1-yl, octahydroisoindol-2-yl group and the like. A ring carbon of the cyclic amino group may form a spiro ring together with a 1, 2 -ethylenedioxy group such as 1,4-dioxa-8-azaspiro[4,5]dec-8-yl group and the like.

The term "cyclic aminocarbonyl group" refers to a group represented by (cyclic amino)-CO- such as pyrrolidinocarbonyl, piperidinocarbonyl, morpholinocarbonyl, piperazinocarbonyl group and the like.

The term "heteroaryl group" refers to a 5- or 6-membered monocyclic aromatic heterocycle having 1 to 5 carbon atoms and 1 to 4 heteroatoms selected independently from the group consisting of an oxygen, nitrogen and sulfur atom, or a 8- to 10-membered bicyclic aromatic heterocycle having 1 to 9 carbon atoms and 1 to 4 heteroatoms selected independently from the group consisting of an oxygen, nitrogen and sulfur atom, provided that said heterocycles do not include adjacent oxygen and/or sulfur atoms. Examples of monocyclic heteroaryl groups include pyrrolyl, furyl, thienyl, imidazolyl, pyrazolyl, oxazolyl, isoxazolyl, 1,2,4-oxadiazolyl, tetrazolyl, thiazolyl, isothiazolyl, 1,2,3-thiadiazolyl, triazolyl, pyridyl, pyrazinyl, pyrimidyl and pyridazinyl, preferably furyl, thienyl, isoxazolyl or pyridyl, and more preferably furyl. Examples of bicyclic heteroaryl groups include indolyl, indazolyl, benzofuranyl, benzothienyl, benzothiazolyl, quinolyl, isoquinolyl, phthalazinyl, benzimidazolyl, benzoxazolyl and the like, and preferably benzofuranyl. The heterocycles include all position isomers such as 2-pyridyl, 3-pyridyl or 4-pyridyl.

The term "heteroaryl-lower alkyl group" refers to a lower alkyl group substituted with a heteroaryl group such as a 2-furylmethyl, 3-furylmethyl, 2-thienylmethyl, 3-thienylmethyl, 3-pyridylmethyl, 3-pyridylethyl, 3-benzofurylmethyl, 3-benzothienylmethyl group and the like.

The term "$C_{1-3}$ alkylene group" refers to a bivalent and straight chained saturated hydrocarbon chain having 1 to 3 carbon atoms, which may be optionally substituted with 1 to 3 methyl groups. Examples of $C_{1-3}$ alkylene groups include $-CH_2-$, $-CH_2CH_2-$, $-CH(CH_3)-$, $-CH(CH_3)CH_2-$, $-CH_2CH(CH_3)-$, $-C(CH_3)_2-$, $-C(CH_3)_2CH_2-$, $-CH_2C(CH_3)_2-$, $-CH_2CH_2CH_2-$, $-C(CH_3)_2CH_2CH_2-$, $-C(CH_3)_2CH_2CH(CH_3)-$and the like, preferably $-CH_2-$, $-CH_2CH_2-$ or $-CH_2CH_2CH_2-$, and more preferably $-CH_2-$.

In the case where a compound represented by general formula (I) contains one or more asymmetric carbons, then all stereoisomers in the R- or S-configuration at each of asymmetric carbons and their mixture are contemplated within the scope of the present invention. In such cases, racemic compounds, racemic mixtures, individual enantiomers and mixtures of diastereomers are also contemplated within the scope of the present invention. In the case where a compound represented by general formula (I) exists in one or more geometrical isomers, then all geometrical isomers such as cis isomer, trans isomer and the mixture thereof are also contemplated within the scope of the present invention. A compound represented by general formula (I) may form a solvate with a pharmaceutically acceptable solvent such as water, ethanol and the like.

Compounds represented by general formula (I) may exist in the form of salts. Examples of such salts include acid addition salts formed with mineral acids such as hydrochloric acid, hydrobromic acid, hydroiodic acid, sulfuric acid, nitric acid, phosphoric acid and the like; acid addition salts formed with organic acids such as formic acid, acetic acid, trifluoroacetic acid, methanesulfonic acid, benzenesulfonic acid, p-toluenesulfonic acid, propionic acid, citric acid, succinic acid, tartaric acid, fumaric acid, butyric acid, oxalic acid, malonic acid, maleic acid, lactic acid, malic acid, carbonic acid, glutamic acid, aspartic acid and the like; basic salts formed with inorganic bases such as sodium, potassium, calcium and the like; basic salts formed with organic bases such as triethylamine, piperidine, morpholine, lysine and the like.

The term "prodrug" as used herein refers to a compound which can be converted into a compound represented by general formula (I) in vivo. Such prodrugs are also contemplated within the scope of the present invention. Various forms of prodrugs are well known in the art.

In the case where a compound represented by formula (I) contains a carboxylic acid as a functional group, then a prodrug may include an ester formed by the replacement of the hydrogen atom of the carboxylic acid group with the following groups: lower alkyl, lower alkanoyloxymethyl, 1-(lower alkanoyloxy)ethyl, 1-methyl-1-(lower alkanoyloxy) ethyl, lower alkoxycarbonyloxymethyl, 1-(lower alkoxycarbonyloxy)ethyl, 1-methyl-1-(lower alkoxycarbonyloxy)ethyl, N-(alkoxycarbonyl)aminomethyl, 1-(N-(lower alkoxycarbonyl)amino)ethyl, 3-phthalidyl, 4-crotonolactonyl, gamma-butyrolacton-4-yl, N,N-di(lower alkyl)amino-lower alkyl such as β-dimethylaminoethyl, carbamoyl-lower alkyl, N,N-di(lower alkyl)carbamoyl-lower alkyl, piperidino-lower alkyl, pyrrolidino-lower alkyl or morpholino-lower alkyl.

In the case where a compound represented by formula (I) contains a hydroxyl group, then a prodrug may include a compound formed by the replacement of the hydrogen atom of the hydroxyl group with the following groups: a lower acyl group such as an acetyl, propionyl, butyryl, isobutyryl, pivaloyl group and the like; a lower alkoxycarbonyl group such as a methoxycarbonyl, ethoxycarbonyl, propoxycarbonyl, isopropoxycarbonyl, tert-butoxycarbonyl group and the like; or a succinoyl group.

In the case where a compound represented by formula (I) contains an amino group such as -NH or $-NH_2$, then a prodrug may include a compound formed by the replacement of the hydrogen atom of the amino group with the following groups: a lower acyl group such as an acetyl, propionyl, butyryl, isobutyryl, pivaloyl group and the like; or a lower alkoxycarbonyl group such as a methoxycarbonyl, ethoxycarbonyl, propoxycarbonyl, isopropoxycarbonyl, tert-butoxycarbonyl group and the like.

The prodrug compounds described above may be prepared from compounds represented by general formula (I) according to known methods as described in T.W. Green and P.G.H. Wuts, "Protective Groups in Organic Synthesis" the third edition and references described therein.

In an embodiment of a compound represented by general formula (I) of the present invention,
$R^1$ is preferably a hydrogen atom;
$R^2$ is preferably:

a) a lower alkyl group,
b) a cycloalkyl group,
c) an aryl group, unsubstituted or substituted with 1 to 5 substituents selected from the group consisting of $X^1$, $X^2$, $X^3$, $X^4$ and $X^5$,
d) an aralkyl group, wherein the ring of the aralkyl group is unsubstituted or substituted with 1 to 5 substituents selected from the group consisting of $X^1$, $X^2$, $X^3$, $X^4$ and $X^5$,
e) a lower alkoxy-lower alkyl group,
f) an aryloxy-lower alkyl group, wherein the ring of the aryloxy-lower alkyl group is unsubstituted or substituted with 1 to 5 substituents selected from the group consisting of $X^1$, $X^2$, $X^3$, $X^4$ and $X^5$,
g) an aralkyloxy-lower alkyl group, wherein the ring of the aralkyloxy-lower alkyl group is unsubstituted or substituted with 1 to 5 substituents selected from the group consisting of $X^1$, $X^2$, $X^3$, $X^4$ and $X^5$, or
h) a heteroaryl group, unsubstituted or substituted with 1 to 3 substituents selected from the group consisting of $X^6$, $X^7$ and $X^8$; and

$R^2$ is more preferably:

a) a cycloalkyl group,
b) an aryl group, unsubstituted or substituted with 1 to 5 substituents selected from the group consisting of $X^1$, $X^2$, $X^3$, $X^4$ and $X^5$, or
c) a heteroaryl group, unsubstituted or substituted with 1 to 3 substituents selected from the group consisting of $X^6$, $X^7$ and $X^8$;

preferred $X^1$, $X^2$, $X^3$, $X^4$ and $X^5$ are each independently:

a) a halogen atom,
b) a lower alkyl group,
c) a lower alkoxy group,
d) a halo-lower alkoxy group,
e) a heterocycloalkyloxy group,
f) a hydroxyl group,
g) $-A^1-NR^{20}R^{21}$,
h) $-A^2-SR^{22}$,
i) $-SO_2NR^{23}R^{24}$,
j) a heteroaryl group, unsubstituted or substituted with 1 to 3 substituents selected independently from the group consisting of a halogen atom, a lower alkyl group, a halo-lower alkyl group, a lower alkoxy group, a halo-lower alkoxy group and di(lower alkyl)amino-lower alkyl group, or

when two of $X^1$, $X^2$, $X^3$, $X^4$ and $X^5$ are adjacent each other, they are bonded together to form $-OCH_2O-$;
preferred $X^6$, $X^7$ and $X^8$ are each independently:

a) a halogen atom,

b) a lower alkyl group,
c) a hydroxy-lower alkyl group,
d) a cycloalkyl group,
e) a heterocycloalkyl-lower alkyl group,
f) $-A^1-NR^{20}R^{21}$,
g) $-SO_2NR^{23}R^{24}$,
h) a phenyl group, unsubstituted or substituted with 1 to 3 substituents selected independently from the group consisting of a halogen atom, a lower alkyl group, a halo-lower alkyl group, a lower alkoxy group, a halo-lower alkoxy group and a di(lower alkyl)amino-lower alkyl group, or
i) a phenoxy group, unsubstituted or substituted with 1 to 3 substituents selected independently from the group consisting of a halogen atom, a lower alkyl group, a halo-lower alkyl group, a lower alkoxy group, a halo-lower alkoxy group and a di(lower alkyl)amino-lower alkyl group;

$R^3$ is preferably a hydrogen atom; and
preferred $R^4$, $R^5$ and $R^6$ are each independently:

a) a hydrogen atom,
b) a halogen atom,
c) a lower alkyl group,
d) a halo-lower alkyl group, or
e) a lower alkoxy group, provided that at least one of $R^4$, $R^5$ and $R^6$ is other than a hydrogen atom.

In a preferable embodiment of the present invention,
$R^1$ is a hydrogen atom.
In a more preferable embodiment of the present invention,
$R^1$ is a hydrogen atom, and
$R^3$ is a hydrogen atom.
In an even more preferable embodiment of the present invention,
$R^1$ is a hydrogen atom,
$R^2$ is:

a) a lower alkyl group,
b) a cycloalkyl group,
c) an aryl group, unsubstituted or substituted with 1 to 5 substituents selected from the group consisting of $X^1$, $X^2$, $X^3$, $X^4$ and $X^5$,
d) an aralkyl group, wherein the ring of the aralkyl group is unsubstituted or substituted with 1 to 5 substituents selected from the group consisting of $X^1$, $X^2$, $X^3$, $X^4$ and $X^5$,
e) a lower alkoxy-lower alkyl group,
f) an aryloxy-lower alkyl group, wherein the ring of the aryloxy-lower alkyl group is unsubstituted or substituted with 1 to 5 substituents selected from the group consisting of $X^1$, $X^2$, $X^3$, $X^4$ and $X^5$,
g) an aralkyloxy-lower alkyl group, wherein the ring of the aralkyloxy-lower alkyl group is unsubstituted or substituted with 1 to 5 substituents selected from the group consisting of $X^1$, $X^2$, $X^3$, $X^4$ and $X^5$, or
h) a heteroaryl group, unsubstituted or substituted with 1 to 3 substituents selected from the group consisting of $X^6$, $X^7$ and $X^8$, and

$R^3$ is a hydrogen atom.
In an even more preferable embodiment of the present invention,
$R^1$ is a hydrogen atom,
$R^2$ is:

a) a lower alkyl group,
b) a cycloalkyl group,
c) an aryl group, unsubstituted or substituted with 1 to 5 substituents selected from the group consisting of $X^1$, $X^2$, $X^3$, $X^4$ and $X^5$,
d) an aralkyl group, wherein the ring of the aralkyl group is unsubstituted or substituted with 1 to 5 substituents selected from the group consisting of $X^1$, $X^2$, $X^3$, $X^4$ and $X^5$,
e) a lower alkoxy-lower alkyl group,
f) an aryloxy-lower alkyl group, wherein the ring of the aryloxy-lower alkyl group is unsubstituted or substituted with

1 to 5 substituents selected from the group consisting of $X^1$, $X^2$, $X^3$, $X^4$ and $X^5$,
g) an aralkyloxy-lower alkyl group, wherein the ring of the aralkyloxy-lower alkyl group is unsubstituted or substituted with 1 to 5 substituents selected from the group consisting of $X^1$, $X^2$, $X^3$, $X^4$ and $X^5$, or
h) a heteroaryl group, unsubstituted or substituted with 1 to 3 substituents selected from the group consisting of $X^6$, $X^7$ and $X^8$,

$R^3$ is a hydrogen atom, and
$R^4$, $R^5$ and $R^6$ are each independently:

a) a hydrogen atom,
b) a halogen atom,
c) a lower alkyl group,
d) a halo-lower alkyl group, or
e) a lower alkoxy group, provided that at least one of $R^4$, $R^5$ and $R^6$ is other than a hydrogen atom.

In a still more preferable embodiment of the present invention,
$R^1$ is a hydrogen atom,
$R^2$ is:

a) a cycloalkyl group,
b) an aryl group, unsubstituted or substituted with 1 to 5 substituents selected from the group consisting of $X^1$, $X^2$, $X^3$, $X^4$ and $X^5$, or
c) a heteroaryl group, unsubstituted or substituted with 1 to 3 substituents selected from the group consisting of $X^6$, $X^7$ and $X^8$,

$R^3$ is a hydrogen atom, and
$R^4$, $R^5$ and $R^6$ are each independently:

a) a hydrogen atom,
b) a halogen atom,
c) a lower alkyl group,
d) a halo-lower alkyl group, or
e) a lower alkoxy group, provided that at least one of $R^4$, $R^5$ and $R^6$ is other than a hydrogen atom.

In a still more preferable embodiment of the present invention,
$R^1$ is a hydrogen atom,
$R^2$ is:

a) a cycloalkyl group,
b) an aryl group, unsubstituted or substituted with 1 to 5 substituents selected from the group consisting of $X^1$, $X^2$, $X^3$, $X^4$ and $X^5$, or
c) a heteroaryl group, unsubstituted or substituted with 1 to 3 substituents selected from the group consisting of $X^6$, $X^7$ and $X^8$,

$R^3$ is a hydrogen atom,
$R^4$, $R^5$ and $R^6$ are each independently:

a) a hydrogen atom,
b) a halogen atom,
c) a lower alkyl group,
d) a halo-lower alkyl group, or
e) a lower alkoxy group, provided that at least one of $R^4$, $R^5$ and $R^6$ is other than a hydrogen atom,

$X^1$, $X^2$, $X^3$, $X^4$ and $X^5$ are each independently:

a) a halogen atom,
b) a lower alkyl group,
c) a lower alkoxy group,

d) a halo-lower alkoxy group,
e) a heterocycloalkyloxy group,
f) a hydroxyl group,
g) $-A^1-NR^{20}R^{21}$,
h) $-A^2-SR^{22}$,
i) $-SO_2NR^{23}R^{24}$,
j) a heteroaryl group, unsubstituted or substituted with 1 to 3 substituents selected independently from the group consisting of a halogen atom, a lower alkyl group, a halo-lower alkyl group, a lower alkoxy group, a halo-lower alkoxy group and di(lower alkyl)amino-lower alkyl group, or

when two of $X^1$, $X^2$, $X^3$, $X^4$ and $X^5$ are adjacent each other, they are bonded together to form $-OCH_2O-$; and
$X^6$, $X^7$ and $X^8$ are each independently:

a) a halogen atom,
b) a lower alkyl group,
c) a hydroxy-lower alkyl group,
d) a cycloalkyl group,
e) a heterocycloalkyl-lower alkyl group,
f) $-A^1-NR^{20}R^{21}$,
g) $-SO_2NR^{23}R^{24}$,
h) a phenyl group, unsubstituted or substituted with 1 to 3 substituents selected independently from the group consisting of a halogen atom, a lower alkyl group, a halo-lower alkyl group, a lower alkoxy group, a halo-lower alkoxy group and a di (lower alkyl)amino-lower alkyl group, or

i) a phenoxy group, unsubstituted or substituted with 1 to 3 substituents selected independently from the group consisting of a halogen atom, a lower alkyl group, a halo-lower alkyl group, a lower alkoxy group, a halo-lower alkoxy group and a di(lower alkyl)amino-lower alkyl group.

Specific examples of preferred embodiments of the present invention are compounds selected form the group consisting of:

(1) 3-cyclopropanecarbonylamino-5-fluorobenzofuran-2-carboxamide;
(2) 5-chloro-3-cyclopropanecarbonylaminobenzofuran-2-carboxamide;
(3) 3-(3-fluorobenzoylamino)-6-methoxybenzofuran-2-carboxamide;
(4) 3-(4-fluorobenzoylamino)-6-methoxybenzofuran-2-carboxamide;
(5) 5-fluoro-3-(3-methylbenzoylamino)benzofuran-2-carboxamide;
(6) 3-(benzo[1,3]dioxole-5-carbonyl)amino-6-fluoro-benzofuran-2-carboxamide;
(7) 5-chloro-3-(furan-2-carbonyl)aminobenzofuran-2-carboxamide;
(8) 5,7-difluoro-3-(furan-2-carbonyl)aminobenzofuran-2-carboxamide;
(9) 5,7-difluoro-3-(5-methylfuran-2-carbonyl)aminobenzofuran-2-carboxamide;
(10) 3-(5-ethylfuran-2-carbonyl)amino-5-fluorobenzofuran-2-carboxamide;
(11) 3-(5-ethylfuran-2-carbonyl)amino-5,7-difluorobenzofuran-2-carboxamide;
(12) 6-methoxy-3-(5-phenylfuran-2-carbonyl)aminobenzofuran-2-carboxamide;
(13) 6-fluoro-3-(6-phenoxypyridine-3-carbonyl)aminobenzofuran-2-carboxamide;
(14) 6-methoxy-3-(2-methoxyacetylamino)benzofuran-2-carboxamide;
(15) 3-[2-(4-chlorophenoxy)acetylamino]-5-fluorobenzofuran-2-carboxamide;
(16) 3-(2-benzyloxyacetylamino)-5-fluorobenzofuran-2-carboxamide;
(17) 6-chloro-3-cyclopropanecarbonylaminobenzofuran-2-carboxamide;
(18) 3-cyclopropanecarbonylamino-5,7-difluorobenzofuran-2-carboxamide;
(19) 7-chloro-3-cyclopropanecarbonylamino-5-fluorobenzofuran-2-carboxamide;
(20) 3-cyclopropanecarbonylamino-5-fluoro-7-methoxybenzofuran-2-carboxamide;
(21) 3-cyclobutanecarbonylamino-5,7-difluorobenzofuran-2-carboxamide;
(22) 5-fluoro-7-methoxy-3-(4-methoxybenzoylamino)-benzofuran-2-carboxamide;
(23) 5,7-difluoro-3-phenylacetylaminobenzofuran-2-carboxamide;
(24) 5,7-difluoro-3-[3-(4-methylpiperazine-1-carbonyl)-benzoylamino]benzofuran-2-carboxamide;
(25) 6-methoxy-3-[3-(4-phenylpiperazin-1-ylmethyl)benzoylamino]benzofuran-2-carboxamide;
(26) 6-methoxy-3-[4-(1-methyl-1H-imidazol-2-ylsulfanylmethyl)benzoylamino]benzofuran-2-carboxamide;
(27) 3-[5-(4-benzylpiperazin-1-ylmethyl)furan-2-carbonyl]-amino-5,7-difluorobenzofuran-2-carboxamide;

(28) 3-[5-(4-benzo[1,3]dioxol-5-ylmethylpiperazin-1-ylmethyl)furan-2-carbonyl]amino-5,7-difluorobenzofuran-2-carboxamide;

(29) tert-butyl 4-[5-(2-carbamoyl-5,7-difluorobenzofuran-3-ylcarbamoyl)furan-2-ylmethyl]piperazine-1-carboxylate, and

(30) 5-fluoro-3-[5-(1-hydroxyethyl)furan-2-carbonyl]aminobenzofuran-2-carboxamide, or a pharmaceutically acceptable salt thereof.

A compound represented by general formula (I) can be prepared by methods as illustrated in schemes 1 to 4.

Scheme 1

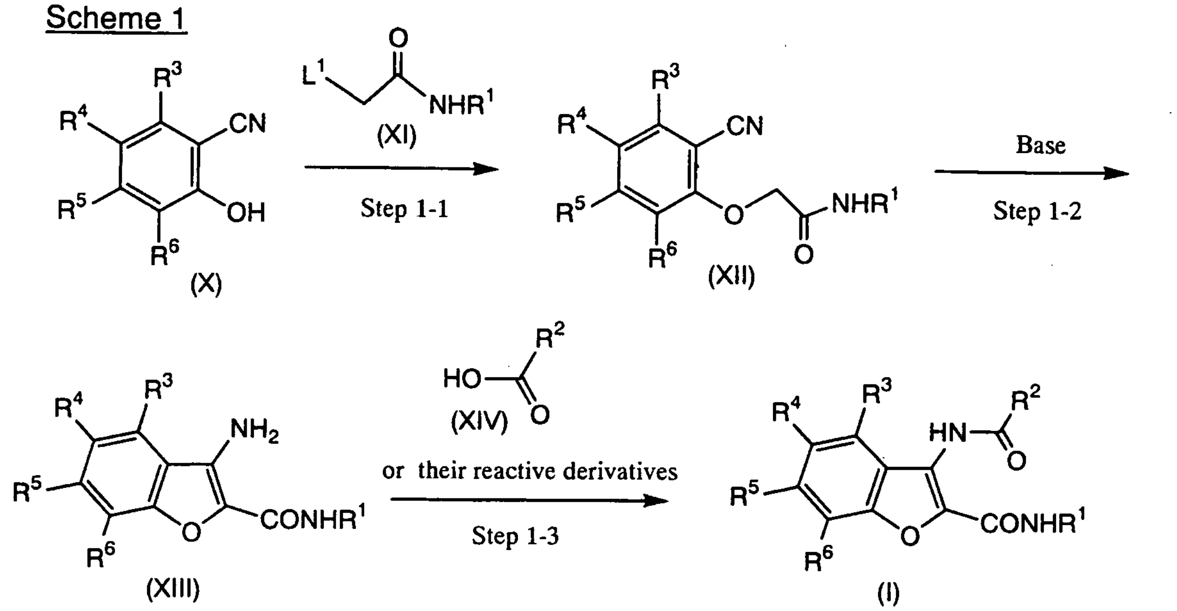


wherein $R^1$, $R^2$, $R^3$, $R^4$, $R^5$ and $R^6$ are as defined above, $L^1$ is a leaving group such as a chlorine, bromine or iodine atom, a methanesulfonyloxy, p-toluenesulfonyloxy group or the like.

(Step 1-1)

Condensation of 2-hydroxybenzonitrile derivative (X) with compound (XI) in the presence of a base in an inert solvent provides compound (XII). The solvents employed in the condensation reaction include ethanol, acetonitrile, N, N-dimethylformamide, dimethylsulfoxide, tetrahydrofuran, 1-methyl-2-pyrrolidone or their mixed solvents. The bases include potassium carbonate, sodium carbonate, cesium carbonate, triethylamine, N,N-diisopropylethylamine or the like. The reaction is carried out ordinarily at 0°C to room temperature. The reaction time varies depending on the starting materials employed, the solvent, the reaction temperature or the like, but is usually 1 to 24 hours.

(Step 1-2)

Thereafter, cyclization of the compound (XII) in the presence of a base in an inert solvent provides compound (XIII). The solvents employed in the reaction include ethanol, acetonitrile, N,N-dimethylformamide, dimethylsulfoxide, tetrahydrofuran, 1-methyl-2-pyrrolidone or their mixed solvents. The bases include potassium carbonate, sodium carbonate, cesium carbonate, triethylamine, N,N-diisopropylethylamine or the like. The reaction is carried out ordinarily at room temperature to reflux temperature. The reaction time varies depending on the starting materials employed, the solvent, the reaction temperature or the like, but is usually 1 to 24 hours.

Alternatively, the compound (XIII) can be prepared from compound (X) by conducting steps 1-1 and 1-2 without isolating compound (XII).

(Step 1-3)

The compound (XIII) is condensed with carboxylic acid (XIV) in the presence of a condensing agent such as dicyclohexylcarbodiimide, 1-(3-dimethylaminopropyl)-3-ethylcarbodiimide hydrochloride, ethyl cyanophosphate, diphenylphosphoryl azide or the like in an inert solvent to afford compound (I).

Alternatively, the compound (I) can be prepared by converting carboxylic acid (XIV) into their reactive derivatives such as acid halide, acid anhydride, mixed acid anhydride, 4-nitrophenyl ester, 2,5-dioxapyrrolidine ester or the like, followed by condensing with compound (XIII) in the presence or absence of a base. Solvents employed in the reaction include acetonitrile, N,N-dimethylformamide, tetrahydrofuran, methylene chloride or their mixed solvents. The bases include potassium carbonate, triethylamine, N,N-diisopropylethylamine, pyridine, N-methylmorpholine, N,N-dimethylaniline or the like. The reaction is carried out ordinarily at -20°C to reflux temperature. The reaction time varies depending on the starting materials employed, the solvent, the reaction temperature or the like, but is usually 15 minutes to 24 hours.

Among compounds represented by general formula (I), compound (Ia) wherein $R^1$ is a hydrogen atom can be also prepared by methods as illustrated in scheme 2.

Scheme 2

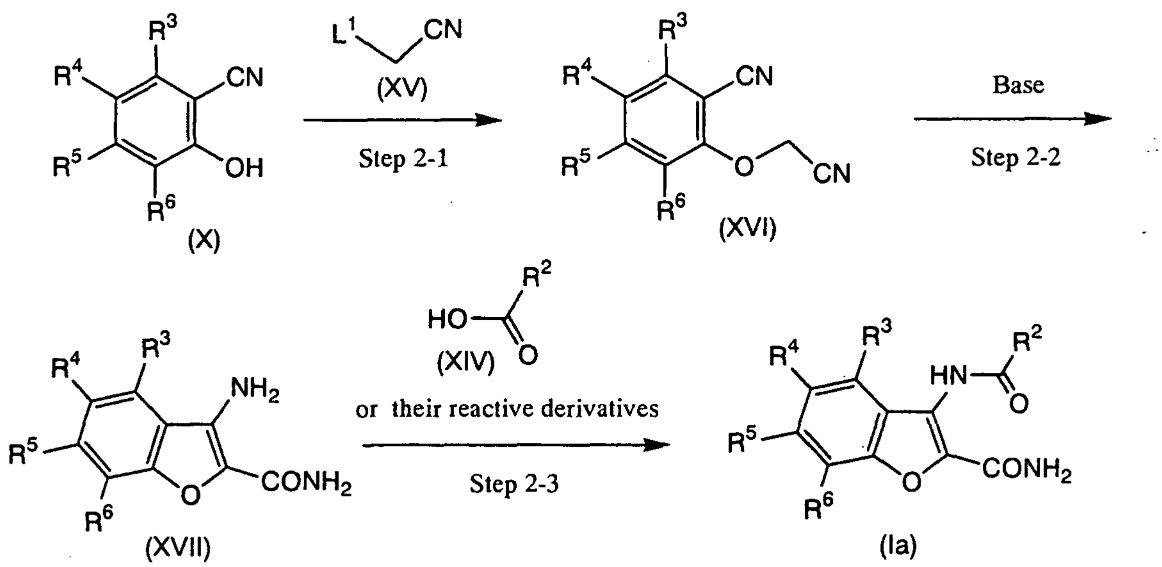


wherein $R^2$, $R^3$, $R^4$, $R^5$, $R^6$ and $L^1$ are as defined above.

(Step 2-1)

Condensation of 2-hydroxybenzonitrile derivative (X) with compound (XV) in the presence of a base in an inert solvent provides compound (XVI). The solvents employed in the condensation reaction include ethanol, acetonitrile, 1-methyl-2-pyrrolidone, N,N-dimethylformamide or the like. The bases include potassium carbonate, sodium carbonate, cesium carbonate, triethylamine, N,N-diisoproylethylamine or the like. The reaction is carried out ordinarily at 0° C to 80° C. The reaction time varies depending on the starting materials employed, the solvent, the reaction temperature or the like, but is usually 1 to 24 hours.

(Step 2-2)

Thereafter, the compound (XVI) is cyclized of in the presence of a base in an inert solvent to provide compound (XVII). The solvents employed in the reaction include alcohols such as methanol, ethanol, isopropanol or the like, and a mixed solvent of the alcohols and water. The bases include potassium hydroxide, sodium hydroxide or the like. The reaction is carried out ordinarily at room temperature to reflux temperature. The reaction time varies depending on the starting materials employed, the solvent, the reaction temperature or the like, but is usually 1 to 24 hours.

(Step 2-3)

The compound (XVII) is then condensed with carboxylic acid (XIV) according to procedures analogous to those as described in step 1-3 to afford compound (Ia).

Among compounds represented by general formula (I), compound (Ib) can be prepared by methods as illustrated

in scheme 3.

Scheme 3

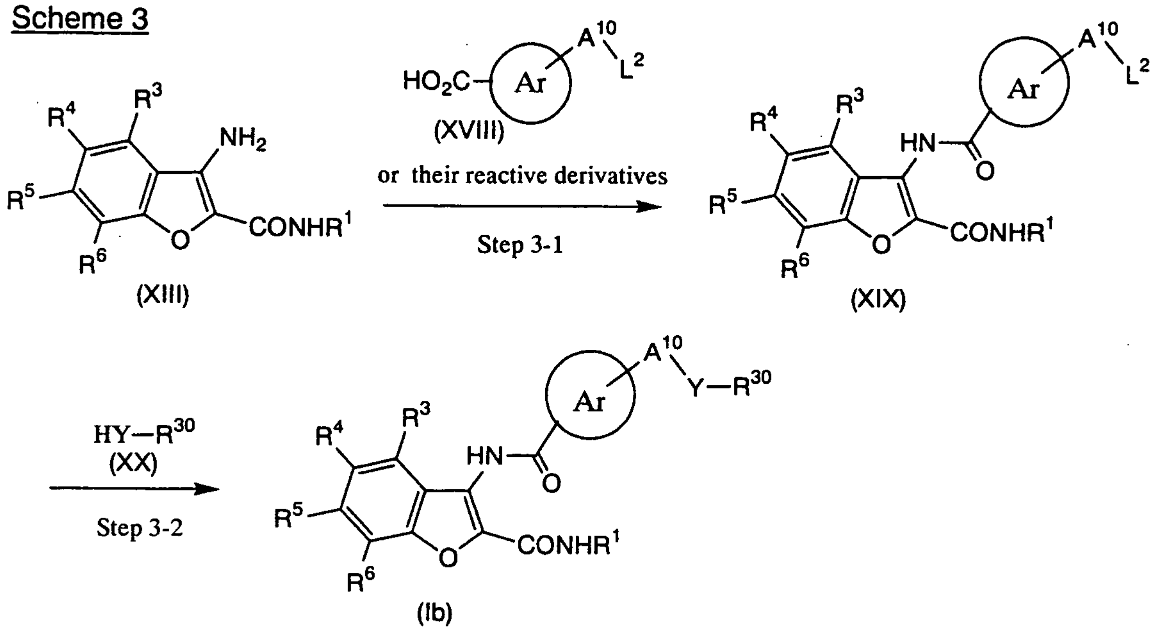


(XIII) (XIX) (Ib)

wherein $R^1$, $R^3$, $R^4$, $R^5$ and $R^6$ are as defined above; $A^{10}$ is a $C_{1-3}$ alkylene group; Ar is an aryl or heteroaryl group; $L^2$ is a leaving group such as a chlorine, bromine or iodine atom, a methanesulfonyloxy, p-toluenesulfonyloxy group or the like; Y is -N($R^{20}$)- or -S-; when Y is -N($R^{20}$)-, $R^{30}$ is the same as $R^{21}$, and when Y is -S-, $R^{30}$ is the same as $R^{22}$.

(Step 3-1)

Compound (XIII) is condensed with compound (XVIII) according to procedures analogous to those as described in step 1-3 to afford compound (XIX).

(Step 3-2)

Thereafter, the compound (XIX) is treated with compound (XX) in the presence or absence of a base in an inert solvent to afford compound (Ib). The solvents employed in the reaction include ethanol, isopropanol, acetonitrile, N,N-dimethylformamide, tetrahydrofuran, methylene chloride, 1-methyl-2-pyrrolidone or their mixed solvents. The bases include potassium carbonate, triethylamine, N,N-diisopropylethylamine, pyridine, N-methylmorpholine, N,N-dimethylaniline or the like. The reaction is carried out ordinarily at -20°C to reflux temperature. The reaction time varies depending on the starting materials employed, the solvent, the reaction temperature or the like, but is usually 15 minutes to 24 hours.

Among compounds represented by general formula (I), compound (Ic) can be prepared by methods as illustrated in scheme 4.

Scheme 4

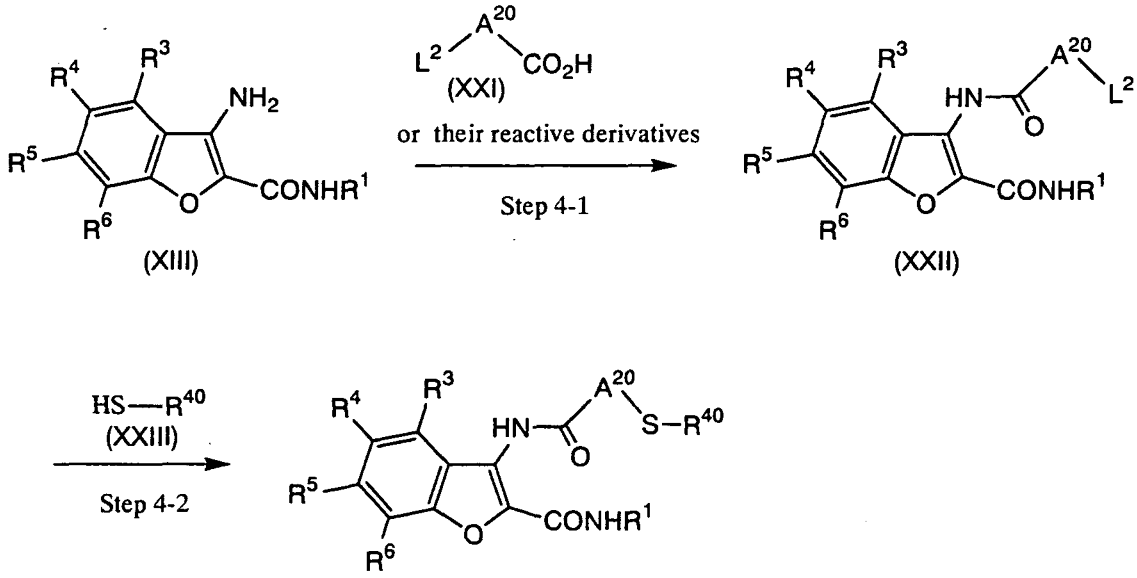


(XIII) (XXII) (Ic)

wherein $R^1$, $R^3$, $R^4$, $R^5$, $R^6$ and $L^2$ are as defined above; $A^{20}$ a is $C_{1-6}$ alkylene group; $R^{40}$ is an aryl group, unsubstituted or substituted with 1 to 5 substituents selected from the group consisting of $X^1$, $X^2$, $X^3$, $X^4$ and $X^5$; and $X^1$, $X^2$, $X^3$, $X^4$ and $X^5$ are as defined above.

(Step 4-1)

Compound (XIII) is condensed with compound (XXI) according to procedures analogous to those as described in step 1-3 to afford compound (XXII).

(Step 4-2)

Thereafter, the compound (XXII) is treated with compound (XXIII) in the presence of a base in an inert solvent to afford compound (Ic). The solvents employed in the reaction include ethanol, isopropanol, acetonitrile, N,N-dimethylformamide, tetrahydrofuran, methylene chloride, 1-methyl-2-pyrrolidone, or their mixed solvents. The bases include potassium carbonate, triethylamine, N,N-diisopropylethylamine, pyridine, N-methylmorpholine, N,N-dimethylaniline or the like. The reaction is carried out ordinarily at -20° C to reflux temperature. The reaction time varies depending on the starting materials employed, the solvent, the reaction temperature or the like, but is usually 15 minutes to 24 hours.

Among the starting materials employed in scheme 1, compound (X) can be prepared by methods as illustrated in scheme 5 or 6.

Scheme 5

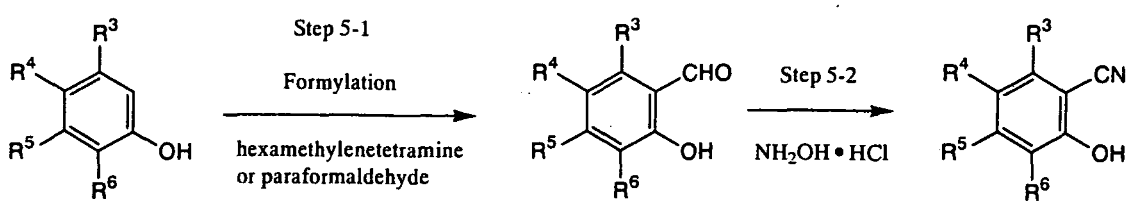


(XXIV) (XXV) (X)

wherein $R^3$, $R^4$, $R^5$ and $R^6$ are as defined above.

(Step 5-1)

Phenol derivative (XXIV) is condensed with hexamethylenetetramine in a solvent of trifluoroacetic acid to afford an imine compound. Hydrolysis of the imine compound using an acid provides compound (XXV). The condensation reaction is carried out ordinarily at room temperature to reflux temperature , and the reaction time varies depending on the starting materials employed, the solvent, the reaction temperature or the like, but is usually 1 to 144 hours. The acids employed in the hydrolysis reaction include an aqueous solution of sulfuric acid or the like. The hydrolysis reaction is carried out ordinarily at room temperature to reflux temperature, and the reaction time is usually 15 minutes to 12 hours.
Alternatively, the compound (XXV) can be prepared by condensing phenol derivative (XXIV) with paraformaldehyde in the presence of magnesium chloride and a base in an inert solvent such as acetonitrile or the like. The bases employed in the reaction include triethylamine, N,N-diisopropylethylamine or the like. The condensation reaction is carried out ordinarily at room temperature to reflux temperature. The reaction time varies depending on the startingmaterials employed, the solvent, the reaction temperature or the like, but is usually 0.5 to 48 hours.

(Step 5-2)

Thereafter, the compound (XXV) is treated with hydroxylamine hydrochloride in a suitable solvent such as 1-methyl-2-pyrrolidone, formic acid or the like to afford compound (X). The reaction is carried out ordinarily at room temperature to reflux temperature. The reaction time varies depending on the starting materials employed, the solvent, the reaction temperature or the like, but is usually 1 to 144 hours.

Scheme 6

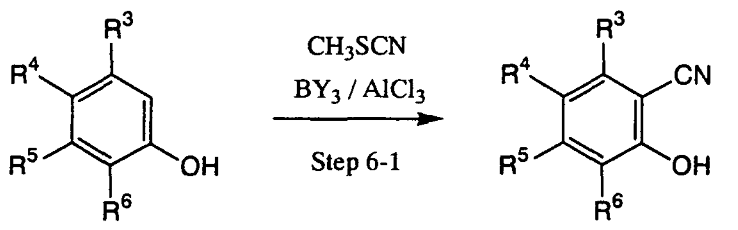


(XXIV) (X)

wherein $R^3$, $R^4$, $R^5$ and $R^6$ are as defined above; and Y is a chlorine or bromine atom.

(Step 6-1)

Compound (X) can be also prepared by treating phenol derivative (XXIV) with methylthiocyanic acid in the presence of Lewis acid in an inert solvent . The solvents employed in the reaction include dichloromethane, 1,2-dichloroethane, or the like. Lewis acids include boron trihalides such as boron trichloride or boron tribromide/aluminum chloride or the like. The reaction is carried out ordinarily at 0˚C to reflux temperature. The reaction time varies depending on the starting materials employed, the solvent, the reaction temperature or the like, but is usually 1 to 24 hours.
The forementioned schemes are exemplary for preparing compounds represented by general formula (I) of the present invention and synthetic intermediates thereof. Those skilled in the art will appreciate that various changes or modifications of the forementioned schemes may be made without departing from the scope of the invention.
Compounds represented by general formula (I) of the present invention and intermediates for preparing the compounds of the present invention can be isolated or purified, if required, according to conventional isolation or purification techniques well known to those in the art, such as solvent extraction, crystallization, recrystallization, chromatography, preparative high performance liquid chromatography or the like.
Compounds of general formula (I) prepared in the above-mentioned schemes exhibit excellent adenosine $A_{2A}$ receptor antagonistic activities, and are accordingly useful as a therapeutic or prophylactic agent for motor function disorders such as Parkinson's disease, Huntington's disease or Wilson's disease and the like; depression; an anxiety disorder; cognitive function disorders such as Alzheimer disease and the like; cerebral ischemia disorders such as cerebral apoplexy, brain lesion after cerebral vasospasm and the like; restless legs syndrome and the like.
Compounds of general formula (I) can be used, if required, in combination with anti-Parkinson drugs, antidepressants, drugs for cognitive function disorders and drugs for cerebral ischemia disorders other than adenosine $A_{2A}$ receptor antagonists.

Examples of anti-Parkinson drugs which may be used in combination with compounds of general formula (I) include levodopa, levodopa/carbidopa, lovodopa/benserazide, droxidopa, melevodopa, threodops; dopamine $D_2$ receptor agonists such as cabergoline, bromocriptine mesylate, terguride, talipexole hydrochloride, ropinirole hydrochloride, pergolide mesylate, pramipexole hydrochloride, rotigotine and the like; anticholinergic agents such as profenamine, trihexyphenidyl hydrochloride, mazaticol hydrochloride, biperiden, piroheptine hydrochloride, methixene hydrochloride and the like; COMT (catechol O-methyl transferase) inhibitors such as tolcapone, entacapone and the like; NMDA antagonists such as budipine and the like; monoamine oxidase B inhibitors such as selegiline hydrochloride, rasagiline mesylate and the like; zonisamide; amantadine hydrochloride and the like.

Examples of antidepressants which may be used in combination with compounds of general formula (I) include selective serotonin reuptake inhibitors such as fluoxetine hydrochloride, sertraline hydrochloride, paroxetine hydrochloride, citalopram hydrobromide, fluvoxamine maleate and the like; selective noradrenalin reuptake inhibitors such as desipramine hydrochloride, amitriptyline hydrochloride, nortriptyline hydrochloride, reboxetine and the like; serotonin/noradrenalin reuptake inhibitors such as venlafaxine hydrochloride, bupropion hydrochloride, nefazodone hydrochloride, milnacipran hydrochloride and the like.

Examples of drugs for cognitive function disorders which may be used in combination with compounds of general formula (I) include acetylcholine esterase inhibitors such as tacrine, donepezil hydrochloride, rivastigmine tartrate, metrifonate, galantamine hydrobromide; memantine hydrochloride; aripiprazole; S-8510; AC-3933 and the like.

Examples of drugs for cerebral ischemia disorders which may be used in combination with compounds of general formula (I) include thrombolytic agents such as t-PA (tissue plasminogen activator), urokinase and the like; trombin inhibitors such as argatroban and the like; $TXA_2$ synthase inhibitors such as ozagrel sodium and the like; radical scavengers such as ebselen, edaravone, nicaraven and the like; 5-$HT_{1A}$ agonists such as SUN-N4057, BAYx3702 and the like; NMDA antagonists such as aptiganel hydrochloride and the like; AMPA antagonists such as S-1746 and the like; Rho kinase inhibitors such as fasudil and the like; src inhibitors and the like.

Various dosage forms of pharmaceutical compositions comprising a compound represented by general formula (I) or a pharmaceutically acceptable salt thereof, can be administered depending on their usages. Exemplary dosage forms include powders, granules, fine granules, dry syrups, tablets, capsules, injections, liquids, ointments, suppositories, poultices and the like, which are administered orally or parenterally.

Pharmaceutical compositions can be formulated by admixing, diluting or dissolving with appropriate pharmaceutical additives such as excipients, disintegrators, binders, lubricants, diluents, buffers, isotonic agents, preservatives, wetting agents, emulsifying agents, dispersing agents, stabilizing agents, solubilizing agents and the like, according to a conventional formulation procedure depending upon their dosage forms.

The dosage of a compound represented by general formula (I) or a pharmaceutically acceptable salt thereof is appropriately determined depending on the age, sex or body weight of the individual patient, the severity of the disease, the condition to be treated and the like. A typical dosage for oral administration is in the range of from about 1mg to about 5000 mg per day per adult human. A typical dosage for parenteral administration is in the range of from about 0.1mg to about 500 mg per day per adult human. The dosages may be administered in single or divided doses, for example one to several times daily.

A pharmaceutical combination comprising a compound represented by general formula (I) or a pharmaceutically acceptable salt thereof, and at least one selected from anti-Parkinson drugs, antidepressants, drugs for cognitive function disorders and drugs for cerebral ischemia disorders other than adenosine $A_{2A}$ receptor antagonists, can be administered as a single pharmaceutical composition comprising all of active ingredients, or as separately formulated pharmaceutical compositions each of which comprises a single active ingredient. Where separately formulated pharmaceutical compositions are used, the compositions may be administered separately, concurrently or at different intervals. Alternatively, where separately formulated pharmaceutical compositions are used, the compositions may be mixed together with an appropriate diluent, and administered simultaneously.

In a pharmaceutical combination comprising a compound represented by general formula (I) or a pharmaceutically acceptable salt thereof, and at least one selected from anti-Parkinson drugs, antidepressants, drugs for cognitive function disorders and drugs for cerebral ischemia disorders other than adenosine $A_{2A}$ receptor antagonists, the dosage of each active ingredient may be appropriately determined depending on the age, sex or body weight of the individual patient, the severity of the disease, administration time, dosage form, administration method, combination of active ingredients and the like.

Compounds represented by general formula (I) of the present invention exhibit potent adenosine $A_{2A}$ receptor antagonistic activities. Moreover, preferable compounds of the present invention show selective antagonistic activities on adenosine $A_{2A}$ receptors. Therefore, compounds of the present invention are useful for treating or preventing diseases mediated by adenosine $A_{2A}$ receptors such as motor function disorders, depression, an anxiety disorder, cognitive function disorders, cerebral ischemia disorder, restless legs syndrome and the like, and are especially useful for treating or preventing Parkinson's disease.

## BEST MODE FOR CARRYING OUT THE INVENTION

The following reference examples, examples and test examples illustrate the invention in further detail. It is to be understood, however, that they are not to be construed as limiting the scope of the invention in any way.

Reference Example 1-1

3-Bromo-5-fluoro-2-hydroxybenzaldehyde

To a solution of 2-bromo-4-fluorophenol (2g) in trifluoroacetic acid (10mL) was added hexamethylenetetramine (2.94g) at room temperature, and the resulting mixture was stirred under reflux for 20 hours. A 50% aqueous solution of sulfuric acid was added to the reaction mixture, and the mixture was stirred for further 4 hours at room temperature. The reaction mixture was extracted with ethyl acetate, and washed with water, 1mol/L hydrochloric acid and brine. The organic layer was dried over anhydrous magnesium sulfate, and concentrated under reduced pressure. The residue was purified by column chromatography on silica gel to give the title compound (1.93g).
$^1$H-NMR ($CDCl_3$) δ ppm: 7.25-7.30 (1H, m), 7.56-7.61 (1H, m), 9.83 (1H, s), 11.35 (1H, s)
Reference Examples 1-2 and 1-3 were prepared in a manner similar to those as described in Reference Example 1-1 using 2,4-difluorophenol and 4-fluoro-2-methoxyphenol instead of 2-bromo-4-fluorophenol.

Reference Example 1-2

3,5-Difluoro-2-hydroxybenzaldehyde
$^1$H-NMR ($CDCl_3$) δ ppm: 7.08-7.20 (2H, m), 9.88 (1H, d, J=1.9Hz), 10.72 (1H, s)

Reference Example 1-3

5-Fluoro-2-hydroxy-3-methoxybenzaldehyde
$^1$H-NMR ($CDCl_3$) δ ppm: 3.92 (3H, s), 6.85-6.90 (2H, m), 9.87 (1H, s), 10.80 (1H, brs)

Reference Example 2-1

2-Fluoro-6-hydroxybenzonitrile

Hydroxylamine hydrochloride (0.60g) was added to a solution of 2-fluoro-6-hydroxybenzaldehyde (1g) in 1-methyl-2-pyrrolidone (10ml), and the resulting mixture was stirred at 120˚ C for 2days. The reaction mixture was extracted with ethyl acetate, washed with water and brine. The organic layer was dried over anhydrous magnesium sulfate, and concentrated under reduced pressure. The residue was purified by column chromatography on silica gel to give the title compound (0.35g).
$^1$H-NMR ($CDCl_3$) δ ppm: 6.30-6.86 (3H, m), 7.36-7.50 (1H, m)
Reference Examples 2-2 to 2-20 were prepared in a manner similar to those as described in Reference Example 2-1 using the corresponding 2-hydroxybenzaldehydes instead of 2-fluoro-6-hydroxybenzaldehyde.

Reference example 2-2

5-Fluoro-2-hydroxybenzonitrile
$^1$H-NMR (DMSO-$d_6$) δ ppm: 6.95-7.05 (1H, m), 7.30-7.45 (1H, m), 7.50-7.65 (1H, m), 11.05 (1H, s)

Reference example 2-3

4-Fluoro-2-hydroxybenzonitrile
$^1$H-NMR ($CDCl_3$) δ ppm: 6.20-6.35 (1H, m), 6.65-6.80 (2H, m), 7.45-7.60 (1H, m)

Reference example 2-4

3,5-Difluoro-2-hydroxybenzonitrile
$^1$H-NMR (DMSO-$d_6$) δ ppm: 7.46-7.74 (2H, m), 11.42 (1H, brs)

Reference example 2-5

3-Bromo-5-fluoro-2-hydroxybenzonitrile
$^1$H-NMR(DMSO-$d_6$) δ ppm: 7.69-7.76 (1H, m), 7.89-7.96 (1H, m), 11.01(1H, brs)

Reference example 2-6

2-Hydoroxy-5-methylbenzonitrile
$^1$H-NMR ($CDCl_3$) δ ppm: 2.29 (3H, s), 6.89 (1H, d, J=8.4Hz), 7.24-7.32 (2H, m)

Reference example 2-7

2-Hydroxy-3-methylbenzonitrile
$^1$H-NMR ($CDCl_3$) δ ppm: 2.28 (3H, s), 5.87 (1H, brs), 6.86-6.94 (1H, m), 7.30-7.40 (2H, m)

Reference example 2-8

1-Hydroxynaphthalene-2-carbonitrile
$^1$H-NMR ($CDCl_3$) δ ppm: 7.35-7.85 (5H, m), 8.21-8.38 (1H, m)

Reference example 2-9

2-Hydroxy-5-methoxybenzonitrile
$^1$H-NMR ($CDCl_3$)δ ppm: 3.78 (3H, s), 5.77 (1H, brs), 6.88-6.98 (2H, m), 7.02-7.08 (1H, m)

Reference example 2-10

2-Hydroxy-4-methoxybenzonitrile
$^1$H-NMR ($CDCl_3$) δ ppm: 3.94 (3H, s), 6.27 (1H, brs), 6.86-7.16 (3H, m)

Reference example 2-11

4-Benzyloxy-2-hydroxybenzonitrile
$^1$H-NMR ($CDCl_3$) δ ppm: 5.08 (2H, s), 6.54-6.64 (2H, m), 7.30-7.48 (6H, m)

Reference example 2-12

2-Hydroxy-3-methoxybenzonitrile
$^1$H-NMR ($CDCl_3$) δ ppm: 3.83 (3H, s), 6.46-6.58 (2H, m), 7.40 (1H, d, J=8.8Hz)

Reference example 2-13

3-Ethoxy-2-hydroxybenzonitrile
$^1$H-NMR ($CDCl_3$) δ ppm: 1.48 (3H, t, J=6.9Hz), 4.15 (2H, q, J=6.9Hz), 6.27 (1H, s), 6.80-7.15 (3H, m)

Reference example 2-14

4-Diethylamino-2-hydroxybenzonitrile
$^1$H-NMR (DMSO-$d_6$) δ ppm: 1.06-1.13 (6H, m), 3.28-3.37 (4H, m), 6.13-6.26 (2H, m), 7.25 (1H, d, J=8.8Hz), 10.35 (1H, s)

Reference example 2-15

4,5-Difluoro-2-hydroxybenzonitrile
$^1$H-NMR (DMSO-$d_6$) δ ppm: 6.90-7.00 (1H, m), 7.85-7.95 (1H, m), 11.57 (1H, brs)

Reference example 2-16

3-Chloro-5-fluoro-2-hydroxybenzonitrile

$^1$H-NMR ($CDCl_3$) δ ppm: 7.18-7.25 (1H, m), 7.34-7.39 (1H, m)

Reference example 2-17

5-Fluoro-2-hydroxy-3-methoxybenzonitrile
$^1$H-NMR ($CDCl_3$) δ ppm: 3.94 (3H, s), 6.04 (1H, s), 6.75-6.85 (2H, m)

Reference example 2-18

4-Ethyl-2-hydroxybenzonitrile
$^1$H-NMR ($CDCl_3$) δ ppm: 1.25 (3H, t, J=7.6Hz), 2.67 (2H, q, J=7.6Hz), 6.78-6.92 (2H, m), 7.40-7.50 (1H, m)

Reference example 2-19

2-Hydroxy-4-isopropylbenzonitrile
$^1$H-NMR ($CDCl_3$) δ ppm: 1.24 (6H, d, J=6.9Hz), 2.75-3.00 (1H, m), 6.35 (1H, brs), 6.80-6.95 (2H, m), 7.30-7.50 (1H, m)

Reference example 2-20

4-Ethoxy-2-hydroxybenzonitrile
$^1$H-NMR ($CDCl_3$) δ ppm: 1.42 (3H, t, J=6.9Hz), 4.04 (2H, q, J=6.9Hz), 6.40-6.60 (2H, m), 7.30-7.45 (1H, m)

Reference example 3-1

4-Chloro-2-hydroxybenzonitrile

To a solution of 3-chlorophenol(2.86mL) in dichloroethane (48mL) were added aluminum chloride (4.00g) and boron tribromide (3.39mL) under ice-cooling, and methyl thiocyanate (2.46mL) was added to the reaction mixture, and stirred at room temperature until aluminum chloride was dissolved. Then, the reaction mixture was stirred and heated at 120˚C for 20 hours. After cooling, a 4mol/L aqueous solution of sodium hydroxide (99mL) was added to the reaction mixture, and the mixture was stirred at approximately 80˚ C for 30 minutes. The solution was washed with dichloromethane. The aqueous layer was acidified with 6mol/L hydrochloric acid , and extracted with diethyl ether. The organic layer was washed with brine and dried over anhydrous magnesium sulfate. The organic solvent was concentrated under reduced pressure. The residue was treated with hexane and collected by filtration to give the title compound (2.5g).
$^1$H-NMR ($CDCl_3$) δ ppm: 6.99 (1H, dd, J=1.8, 8.5Hz), 7.03 (1H, d, J=1.8Hz), 7.44 (1H, d, J=8.5Hz)

Reference, Example 3-2

3-Hydroxybiphenyl-4-carbonitrile

Reference Example 3-2 was prepared in a manner similar to those as described in Reference Example 3-1 using 3-hydroxybiphenyl instead of 3-chlorophenol.
$^1$H-NMR ($CDCl_3$) δ ppm: 6.10 (1H, brs), 7.18-7.28 (2H, m), 7.39-7.50 (3H, m), 7.54-7.60 (3H, m)

Reference example 4-1

3-Amino-4-fluorobenzofuran-2-carboxamide

To a solution of 2-fluoro-6-hydroxybenzonitrile (0.35g) in ethanol (20mL) were added potassium carbonate (0.54g) and bromoacetoamide (0.43g) successively at room temperature, and the resulting mixture was heated under reflux for 2 hours. Then, potassium hydroxide (0.29g) was added to the reaction mixture and heated under reflux for 12 hours. The reaction mixture was diluted with water, and the organic solvent was concentrated under reduced pressure. The resulting precipitate was collected by filtration to give the title compound (0.25g).
$^1$H-NMR ($CDCl_3$) δ ppm: 5.24 (2H, brs), 5.81 (2H, brs), 6.80-6.96 (1H, m), 7.10-7.42 (2H, m)

Reference Examples 4-2 to 4-27 were prepared in a manner similar to those as described in Reference Example 4-1 using the corresponding 2-hydroxybenzonitriles instead of 2-fluoro-6-hydroxybenzonitrile. These were illustrated in table 1.

Table 1

| Reference example | Structure | Reference example | Structure |
|---|---|---|---|
| 4-1 | 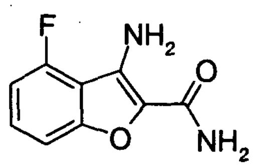 | 4-10 | 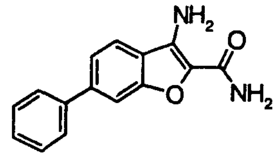 |
| 4-2 | 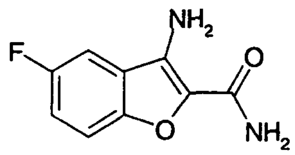 | 4-11 | 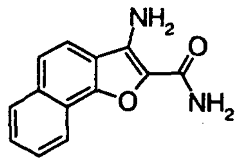 |
| 4-3 | 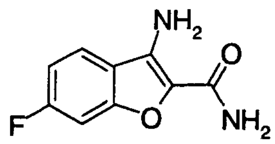 | 4-12 | 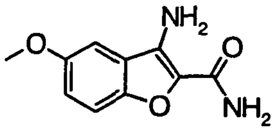 |
| 4-4 | 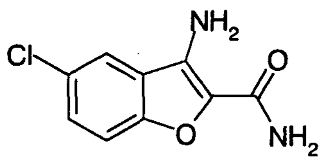 | 4-13 | 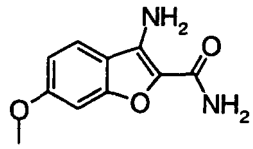 |
| 4-5 | 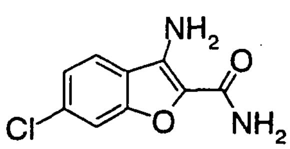 | 4-14 | 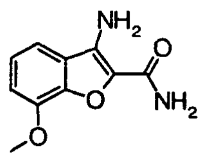 |
| 4-6 | 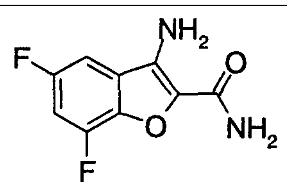 | 4-15 | 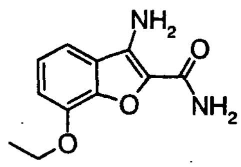 |
| 4-7 | 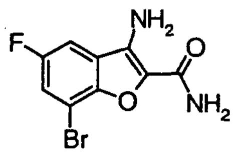 | 4-16 | 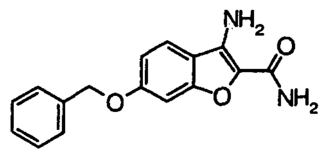 |
| 4-8 | 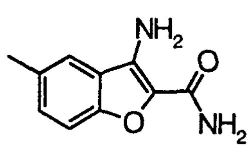 | 4-17 | 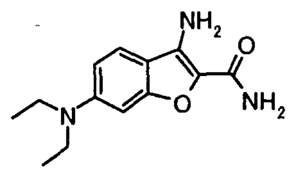 |
| 4-9 | 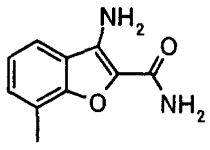 | 4-18 | 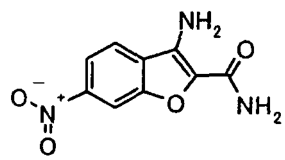 |

(continued)

| Reference example | Structure | Reference example | Structure |
|---|---|---|---|
| 4-19 | 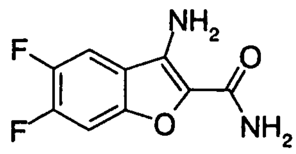 | 4-24 | 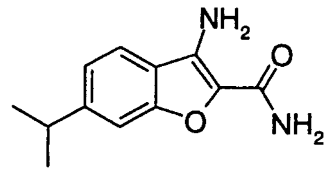 |
| 4-20 | 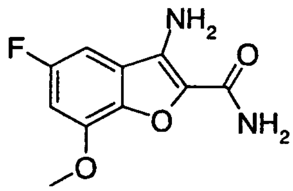 | 4-25 | 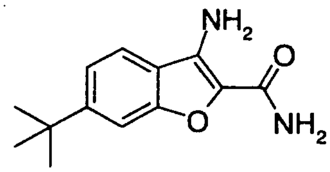 |
| 4-21 | 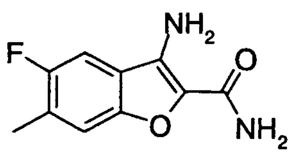 | 4-26 | 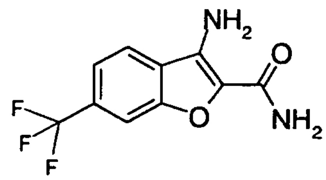 |
| 4-22 | 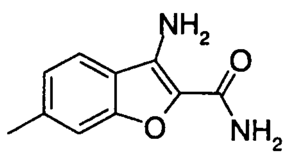 | 4-27 | 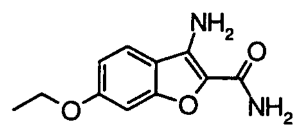 |
| 4-23 | 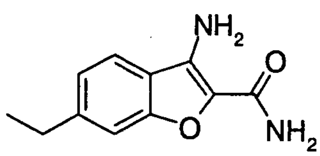 | | |

The physical data of reference examples 4-2 to 4-27 were shown as below.

Reference example 4-2

$^1$H-NMR (DMSO-$d_6$) δ ppm: 5.97 (2H, s), 7.08-7.72 (5H, m) Reference example 4-3
$^1$H-NMR ($CDCl_3$) δ ppm: 5.03 (2H, brs), 5. 71 (2H, brs), 6.96-7.14 (2H, m), 7.44-7.54 (1H, m)

Reference example 4-4

$^1$H-NMR (DMSO-$d_6$) δ ppm: 6.02 (2H, s), 7.12-7.50 (4H, m), 7.94-8.00 (1H, m)

Reference example 4-5

$^1$H-NMR (DMSO-$d_6$) δ ppm: 6.07 (2H, brs), 7.10-7.36 (3H,m), 7.48-7.58 (1H, m), 7.80-7.92 (1H, m)

Reference example 4-6

$^1$H-NMR (DMSO-$d_6$) δ ppm: 6.07 (2H, brs), 7.15-7.65 (4H, m) Reference example 4-7
$^1$H-NMR (DMSO-$d_6$) δ ppm: 6.06 (2H, brs), 7.29 (2H, s), 7.62-7.78 (2H, m)

Reference example 4-8

$^1$H-NMR (DMSO-$d_6$) δ ppm: 2.39 (3H, s), 5.92 (2H, s), 7.04-7.32 (4H, m), 7.57-7.65 (1H, m)

Reference example 4-9

$^1$H-NMR (DMSO-$d_6$) δ ppm: 2.44 (3H, s), 5.93 (2H, brs), 7.06-7.28 (4H, m), 7.63 (1H, d, J=7.6Hz)

Reference example 4-10

$^1$H-NMR (DMSO-$d_6$) δ ppm: 6.05 (2H, brs), 7.22 (2H, brs), 7.35-7.80 (7H, m), 7.92 (1H, d, J=8.2Hz)

Reference example 4-11

$^1$H-NMR (DMSO-$d_6$) δ ppm: 6.05 (2H, brs), 7.30 (2H, brs), 7.52-8.40 (6H, m)

Reference example 4-12

$^1$H-NMR (DMSO-$d_6$) δ ppm: 3.79 (3H, s), 5.90 (2H, s), 7.02 (1H, dd, J=2.7, 9.0Hz), 7.17 (2H, brs), 7.31 (1H, d, J=9.0Hz), 7.40 (1H, d, J=2.7Hz)

Reference example 4-13

$^1$H-NMR (DMSO-$d_6$) δ ppm: 3.93 (3H, s), 5.98 (2H, s), 6.97-7.44 (5H, m)

Reference example 4-14

$^1$H-NMR (DMSO-$d_6$) δ ppm: 3.81 (3H, s), 5.97 (2H, s), 6.83-6.95 (2H, m), 7.04 (2H, brs), 7.71 (1H, d, J=8.7Hz)

Reference example 4-15

$^1$H-NMR ($CDCl_3$) δ ppm: 1. 52 (3H, t, J=6.9Hz), 4.24 (2H, q, J=6.9Hz), 4.99 (2H, brs), 6.85-7.00 (1H, m), 7.05-7.20 (2H, m) Reference example 4-16

$^1$H-NMR (DMSO-$d_6$) δ ppm: 5.18 (2H, s), 5.99 (2H, s), 6.91-7.02 (2H, m), 7.08 (2H, brs), 7.30-7.52 (5H, m), 7.73 (1H, d, J=8.7Hz) Reference example 4-17

$^1$H-NMR (DMSO-$d_6$) δ ppm: 1.05-1.18 (6H, m), 3.28-3.52 (4H, m), 5.87 (2H, brs), 6.47 (1H, s), 6.60-6.72 (1H, m), 6.84 (2H, brs), 7.55 (1H, d, J=8.9Hz)

Reference example 4-18

$^1$H-NMR (DMSO-$d_6$) δ ppm: 6.21 (2H, s), 7.51 (2H, brs), 8.08-8.26 (3H, m)

Reference example 4-19

$^1$H-NMR (DMSO-$d_6$) δ ppm: 6.04 (2H, brs), 7.29 (2H, brs), 7.55-7.70 (1H, m), 7.85-7.95 (1H, m)

Reference example 4-20

$^1$H-NMR (DMSO-$d_6$) δ ppm: 3.94 (3H, s), 5.93 (2H, brs), 6.93-7.04 (1H, m), 7.13-7.45 (3H, m)

Reference example 4-21

$^1$H-NMR (DMSO-$d_6$) δ ppm: 2.31-2.36 (3H, m), 5.96 (2H, s), 7.21 (2H, brs), 7.30-7.37 (1H, m), 7.55-7.65 (1H, m)

Reference example 4-22

$^1$H-NMR (DMSO-$d_6$) δ ppm: 2.42 (3H, s), 5.98 (2H, s), 6.95-7.35 (4H, m), 7.68-7.74 (1H, m)

Reference example 4-23

$^1$H-NMR ($CDCl_3$) δ ppm: 1.28 (3H, t, J=7.6Hz), 2.77 (2H, q, J=7.6Hz), 4.99 (2H, brs), 7.06-7.22 (2H, m), 7.40-7.48 (1H, m)

Reference example 4-24

$^1$H-NMR ($CDCl_3$) δ ppm: 1.30 (6H, d, J=6.9Hz), 2.90-3.15 (1H, m), 4.99 (2H, brs), 5.71 (2H, brs), 7.05-7.30 (2H, m), 7.40-7.50 (1H, m)

Reference example 4-25

$^1$H-NMR ($CDCl_3$) δ ppm: 1.37 (9H, s), 4.99 (2H, brs), 7.28-7.50 (3H, m)

Reference example 4-26

$^1$H-NMR (DMSO-$d_6$) δ ppm: 6.16 (2H, s), 7.41 (2H, brs), 7.55-7.65 (1H, m), 7.73-7.80(1H, m), 8.05-8.15 (1H, m)

Reference example 4-27

$^1$H-NMR ($CDCl_3$) δ ppm: 1.46 (3H, t, J=6.9Hz), 4.07 (2H, q, J=6.9Hz), 4.99 (2H, brs), 6.80-6.90 (2H, m), 7.35-7.45 (1H, m)

Reference example 5-1

4-Phenoxybenzoyl chloride

To a suspension of 4-phenoxybenzoic acid (1.4g) in dichloromethane (15mL) was added N,N-dimethylformamide (0.05mL), and oxalyl chloride (1.2mL) was added dropwise to the mixture under ice-cooling. The resulting mixture was stirred at room temperature for 1 hour, and the reaction mixture was concentrated under reduced pressure to give the title compound (1.54g).
$^1$H-NMR ($CDCl_3$) δ ppm: 6.95-7.15 (4H, m), 7.20-7.30 (1H, m), 7.40-7.50 (2H, m), 8.05-8.15 (2H, m)
Reference examples 5-2 and 5-3 were prepared in a manner similar to those as described in reference example 5-1 using the corresponding carboxylic acids instead of 4-phenoxybenzoic acid.

Reference example 5-2

5-Ethylfuran-2-carbonyl chloride

$^1$H-NMR ($CDCl_3$) δ ppm: 1.30 (3H, t, J=7.5Hz), 2.76 (2H, q, J=7.5Hz), 6.20-6.30 (1H, m), 7.40-7.45 (1H, m)

Reference example 5-3

3-Bromo-4-fluorobenzoyl chloride

$^1$H-NMR ($CDCl_3$) δ ppm: 7.20-7.30 (1H, m), 8.05-8.15 (1H, m), 8.30-8.40 (1H, m)

Reference example 6-1

2-Carbamoyl-5-fluoro-7-hydroxybenzofuran-3-yl ammonium bromide

To a mixture of 3-amino-5-fluoro-7-methoxybenzofuran-2-carboxamide (4.44g) and dichloromethane (100mL) was added a 1 mol/L solution of boron tribromide in dichloromethane (100mL) under ice-cooling, and the resulting mixture was stirred at room temperature overnight. The reaction mixture was poured into methanol (200mL) under ice-cooling, and the mixture was stirred for another 1 hour. The solvent of the mixture was removed under reduced pressure. To the residue was added methanol, and the resulting precipitate was collected by filtration to give the title compound (4.0g).
$^1$H-NMR (DMSO-$d_6$) δ ppm: 6.62-6.70 (1H, m), 7.00-7.30 (3H, m)

Reference example 6-2

2-Carbamoyl-7-hydroxybenzofuran-3-yl ammonium bromide

Reference Example 6-2 was prepared in a manner similar to those as described in Reference Example 6-1 using 3-amino-7-methoxybenzofuran-2-carboxamide instead of 3-amino-5-fluoro-7-methoxybenzofuran-2-carboxamide.
$^1$H-NMR (DMSO-$d_6$) δ ppm: 6.60-7.50 (3H, m), 7.61(1H, d, J=8.5Hz)

Reference example 7-1

3-Amino-7-chloro-5-fluorobenzofuran-2-carboxamide

To a mixture of 3-chloro-5-fluoro-2-hydroxybenzonitrile (24.8g), potassium carbonate (60.1g) and 1-methyl-2-pyrrolidone (150mL) was added chloroacetonitrile (11.0mL) at room temperature, and the resulting mixture was stirred at 60° C for 5 hours. The reaction mixture was poured into ice/water, and the precipitate was collected by filtration. The solid was dissolved with ethyl acetate (250mL) and purified by column chromatography on aminopropylated silica gel (eluent: ethyl acetate) to give 3-chloro-2-cyanomethoxy-5-fluorobenzonitrile (23.88g).

To a solution of 3-chloro-2-cyanomethoxy-5-fluorobenzonitrile in ethanol (200mL) was added potassium hydroxide (9.5g), and the mixture was stirred under reflux overnight. Water (750mL) was added to the reaction mixture, and the resulting precipitate was collected by filtration. The solid was suspended in ethyl acetate (500mL) and purified by column chromatography on aminopropylated silica gel (eluent: ethyl acetate) to give the title compound (13.09g).
$^1$H-NMR (DMSO-$d_6$) δ ppm: 6.09 (2H, s), 7.34 (2H, brs), 7.55-7.61 (1H, m), 7.67-7.74 (1H, m)

Reference example 8-1

Methyl 5-[2-(4-methoxyphenyl)ethyl]furan-2-carboxylate

Sodium hydride (60%, 0.09g) was added to a mixture of (5-methoxycabonylfuran-2-ylmethyl) triphenylphosphonium chloride (0.823g), 4-methoxybenzaldehyde (0.257g) and N,N-dimethylformamide (10mL), and the resulting mixture was stirred at room temperature for 3 hours. Water and ethyl acetate were added to the reaction mixture, and the organic layer was separated. The organic layer was washed with 1mol/L hydrochloric acid, a 1mol/L aqueous solution of sodium hydroxide, 1mol/L hydrochloric acid, water and brine sucessively, and dried over anhydrous magnesium sulfate. The solvent was removed under reduced pressure, and the residue was purified by column chromatography on silica gel (eluent: hexane/ethyl acetate = 8/1) to give methyl 5-[2-(4-methoxyphenyl)vinyl]furan-2-carboxylate.
A mixture of methyl 5-[2-(4-methoxyphenyl)vinyl]furan-2-carboxylate, 10% palladium on carbon (50% wet, 0.01g) and ethyl acetate (30mL) was stirred at room temperature under hydrogen atmosphere for 1.5 hours. After the catalyst was removed by filtration, the solvent was removed under reduced pressure to give the title compound (0.344g).
$^1$H-NMR ($CDCl_3$) δ ppm: 2.90-3.02 (4H, m), 3.79 (3H, s), 3.89 (3H, s), 6.07 (1H, d, J=3.5Hz), 6.78-6.88 (2H, m), 7.05-7.15 (3H, m)

Reference examples 8-2 to 8-6 were prepared in a manner similar to those as described in Reference Example 8-1 using the corresponding aldehydes or ketones instead of 4-methoxybenzaldehyde.

Reference example 8-2

Methyl 5-[2-(3-methoxyphenyl)ethyl]furan-2-carboxylate

$^1$H-NMR ($CDCl_3$) δ ppm: 2.92-3.05 (4H, m), 3.79 (3H, s), 3.89 (3H, s), 6.10 (1H, d, J=3.5Hz), 6.70-6.82 (3H, m), 7.06-7.24 (2H, m)

Reference example 8-3

Methyl 5-[2-(2-methoxyphenyl)ethyl]furan-2-carboxylate

$^1$H-NMR ($CDCl_3$) δ ppm: 2.99 (4H, s), 3.82 (3H, s), 3.89 (3H, s), 6.09 (1H, d, J=3.5Hz), 6.81-6.91 (2H, m), 7.05-7.24 (3H, m)

Reference example 8-4

Methyl 5-(1-isopropylpiperidin-4-ylmethyl)furan-2-carboxylate

$^1$H-NMR ($CDCl_3$) δ ppm: 1.02 (6H, d, J=6.6Hz), 1.20-1.38 (2H, m), 1.62-1.77 (3H, m), 2.00-2.15 (2H, m), 2.56-2.90 (5H, m), 3.87 (3H, s), 6.12 (1H, d, J=3.5Hz), 7.09 (1H, d, J=3.5Hz)

Reference example 8-5

Methyl 5-(1-benzylpiperidin-4-ylmethyl)furan-2-carboxylate

$^1$H-NMR ($CDCl_3$) δ ppm: 1.22-1.38 (2H, m), 1.58-1.80 (3H, m), 1.87-2.00 (2H, m), 2.61 (2H, d, J=7.3Hz), 2.80-2.90 (2H, m), 3.48 (2H, s), 3.87 (3H, s), 6.11 (1H, d, J=3.5Hz), 7.09 (1H, d, J=3.5Hz), 7.20-7.40 (5H, m)

Reference example 8-6

Methyl 5-(1-phenethylpiperidin-4-ylmethyl)furan-2-carboxylate
MS (ESI, m/z) : 328 (M+H)+

Reference example 9-1

5-[2-(4-Methoxyphenyl)ethyl]furan-2-carboxylic acid

To a solution of methyl 5-[2-(4-methoxyphenyl)ethyl]-furan-2-carboxylate (0.344g) in ethanol (5mL) was added a 2mol/L aqueous solution of sodium hydroxide (1.3 5mL), and the resulting mixture was heated under reflux for 2 hours. To the reaction mixture were added water and diethyl ether, and the aqueous layer was separated. Then, the aqueous layer was acidified with 1mol/L hydrochloric acid (6mL). The acidified aqueous layer was extracted with ethyl acetate. The organic layer was washed with brine, and then dried over anhydrous magnesium sulfate. The solvent was removed under reduced pressure to give the title compound (0.296g).
$^1$H-NMR ($CDCl_3$) δ ppm: 2.90-3.07 (4H, m), 3.79 (3H, s), 6.12 (1H, d, J=3.5Hz), 6.80-6.86 (2H, m), 7.06-7.14 (2H, m), 7.23 (1H, d, J=3.5Hz)
Reference Examples 9-2 to 9-26 were prepared in a manner similar to those as described in Reference Example 9-1 using the corresponding carboxylate ester instead of methyl 5-[2-(4-methoxyphenyl)ethyl]furan-2-carboxylate.

Reference example 9-2

5-[2-(3-Methoxyphenyl)ethyl]furan-2-carboxylic acid
$^1$H-NMR ($CDCl_3$) δ ppm: 2.95-3.10 (4H, m), 3.79 (3H, s), 6.15 (1H, d, J=3.6Hz), 6.70-6.80 (3H, m), 7.18-7.25 (2H, m)

Reference example 9-3

5-[2-(2-Methoxyphenyl)ethyl]furan-2-carboxylic acid
$^1$H-NMR ($CDCl_3$) δ ppm: 3.01 (4H, s), 3.82 (3H, s), 6.14 (1H, d, J=3.4Hz), 6.80-6.93 (2H, m), 7.05-7.25 (3H, m)

Reference example 9-4

5-(1-Isopropylpiperidin-4-ylmethyl)furan-2-carboxylic acid
MS (ESI, m/z) : 252 (M+H)+

Reference example 9-5

5-(1-Benzylpiperidin-4-ylmethyl)furan-2-carboxylic acid
MS (ESI, m/z) : 300 (M+H)+

Reference example 9-6

5-(1-Phenethylpiperidin-4-ylmethyl)furan-2-carboxylic acid
MS (ESI, m/z) : 314 (M+H)+

Reference example 9-7

5-(Azepane-1-sulfonyl)furan-2-carboxylic acid
$^1$H-NMR ($CDCl_3$) δ ppm: 1.55-1.85 (8H, m), 3.40-3.50 (4H, m), 7.00-7.10 (1H, m), 7.30-7.35 (1H, m)

Reference example 9-8

5-(4-Methylpiperazine-1-sulfonyl)furan-2-carboxylic acid
$^1$H-NMR (DMSO-$d_6$) δ ppm: 2.26 (3H, s), 2.45-2.60 (4H, m), 3.05-3.20 (4H, m), 7.05-7.10 (1H, m), 7.20-7.25 (1H, m)
Reference example 9-9

3-Cyclohexyloxybenzoic acid

$^1$H-NMR ($CDCl_3$) δ ppm: 1.25-1.65 (6H, m), 1.75-2.10 (4H, m), 4.25-4.40 (1H, m), 7.10-7.20 (1H, m), 7.30-7.45 (1H, m), 7.55-7.75 (2H, m)

Reference example 9-10

3-(Tetrahydropyran-4-yloxy)benzoic acid

$^1$H-NMR ($CDCl_3$) δ ppm: 1.75-1.90 (2H, m), 1.95-2.10 (2H, m), 3.50-3.70 (2H, m), 3.95-4.05 (2H, m), 4.50-4.65 (1H, m), 7.10-7.25 (1H, m), 7.30-7.45 (1H, m), 7.60-7.75 (2H, m)

Reference example 9-11

4-Benzyloxy-3-morpholin-4-ylmethylbenzoic acid
$^1$H-NMR (DMSO-$d_6$) δ ppm: 2.25-2.50 (4H, m), 3.40-3.70 (4H, m), 3.59 (2H, brs), 5.22 (2H, s), 7.10-7.55 (6H, m), 7.80-8.00 (2H, m), 12.66 (1H, brs)

Reference example 9-12

4-Ethoxy-3-morpholin-4-ylmethylbenzoic acid hydrochloride
$^1$H-NMR ($CDCl_3$+MeOD-$d_4$) δ ppm: 1.50 (3H, t, J=7.0Hz), 2.85-3.05 (2H, m), 3.35-3.45 (2H, m), 3.90-4.00 (1H, m), 4.15-4.25 (4H, m), 4.34 (2H, s), 6.95-7.05 (1H, m), 8.10-8.25 (2H, m)

Reference example 9-13

6-(2-Fluorophenoxy)nicotinic acid

$^1$H-NMR ($CDCl_3$) δ ppm: 7.00-7.15 (1H, m), 7.15-7.30 (4H, m), 8.30-8.40 (1H, m), 8.86 (1H, d, J=2.1Hz)

Reference example 9-14

6-(3-Fluorophenoxy)nicotinic acid

$^1$H-NMR ($CDCl_3$) δ ppm: 6.90-7.05 (4H, m), 7.35-7.45 (1H, m), 8.30-8.40 (1H, m), 8.90 (1H, d, J=2.2Hz)

Reference example 9-15

6-(4-Fluorophenoxy)nicotinic acid

$^1$H-NMR ($CDCl_3$) δ ppm: 6.99 (1H, d, J=8.4Hz), 7.05-7.20 (4H, m), 8.30-8.40 (1H, m), 8.88 (1H, d, J=2.5Hz)

Reference example 9-16

6-(2,4-Difluorophenoxy)nicotinic acid

$^1$H-NMR ($CDCl_3$) δ ppm: 6.85-7.05 (2H, m), 7.08 (1H, d, J=9.0Hz), 7.15-7.30 (1H, m), 8.30-8.45 (1H, m), 8.83

(1H, d, J=2.2Hz)

Reference example 9-17

6-(2,4,6-Trifluorophenoxy)nicotinic acid

$^{1}$H-NMR ($CDCl_3$) δ ppm: 6.75-6.90 (2H, m), 7.10-7.20 (1H, m), 8.35-8.45 (1H, m), 8.80-8.85 (1H, m)

Reference example 9-18

6-(2-Isopropylphenoxy)nicotinic acid

$^{1}$H-NMR ($CDCl_3$) δ ppm: 1.19 (6H, d, J=7.0Hz), 3.00-3.15 (1H, m), 6.94 (1H, d, J=8.9Hz), 7.00-7.10 (1H, m), 7.20-7.30 (2H, m), 7.35-7.45 (1H, m), 8.25-8.35 (1H, m), 8.91 (1H, d, J=2.0Hz)

Reference example 9-19

6-(4-Isopropylphenoxy)nicotinic acid

$^{1}$H-NMR ($CDCl_3$) δ ppm: 1.27 (6H, d, J=6.9Hz), 2.85-3.00 (1H, m), 6.90-7.00 (1H, m), 7.05-7.15 (2H, m), 7.20-7.35 (2H, m), 8.25-8.35 (1H, m), 8.90-8.95 (1H, m)

Reference example 9-20

6-(4-Fluro-2-methylphenoxy)nicotinic acid

$^{1}$H-NMR ($CDCl_3$) δ ppm: 2.15 (3H, s), 6.90-7.10 (4H, m), 8.30-8.40 (1H, m), 8.87 (1H, d, J=2.0Hz)

Reference example 9-21

6-(2-Methoxyphenoxy)nicotinic acid

$^{1}$H-NMR ($CDCl_3$) δ ppm: 3.76 (3H, s), 6.95-7.10 (3H, m), 7.10-7.20 (1H, m), 7.20-7.30 (1H, m), 8.25-8.35 (1H, m), 8.87 (1H, d, J=1.9Hz)

Reference example 9-22

6-(3-Methoxyphenoxy)nicotinic acid

$^{1}$H-NMR ($CDCl_3$) δ ppm: 3.81 (3H, s), 6.65-6.85 (3H, m), 6.90-7.00 (1H, m), 7.34 (1H, t, J=8.2Hz), 8.25-8.40 (1H, m), 8.92 (1H, d, J=2.3Hz)

Reference example 9-23

6-(4-Fluoro-2-methoxyphenoxy)nicotinic acid

$^{1}$H-NMR ($CDCl_3$) δ ppm: 3.74 (3H, s), 6.65-6.80 (2H, m), 7.00 (1H, d, J=9.0Hz), 7.05-7.15 (1H, m), 8.25-8.35 (1H, m), 8.85 (1H, d, J=2.1Hz)

Reference example 9-24

6-(Pyridin-3-yloxy)nicotinic acid

$^{1}$H-NMR ($CDCl_3$) δ ppm: 7.33 (1H, d, J=8.4Hz), 7.85-7.95 (1H, m), 8.15-8.30 (1H, m), 8.30-8.45 (1H, m), 8.65-8.75 (2H, m), 8.87 (1H, d, J=2.1Hz)

Reference example 9-25

6-(3-Hydroxymethylphenoxy)nicotinic acid

$^1$H-NMR (DMSO-$d_6$) δ ppm: 4.52 (2H, brs), 5.25 (1H, brs), 7.00-7.25 (4H, m), 7.35-7.45 (1H, m), 8.25-8.35 (1H, m), 8.60-8.70 (1H, m), 13.17 (1H, brs)

Reference example 9-26

6-Phenethyloxynicotinic acid

$^1$H-NMR ($CDCl_3$) δ ppm: 3.11 (2H, t, J=7.1Hz), 4.62 (2H, t, J=7.1Hz), 6.70-6.85 (1H, m), 7.15-7.40 (5H, m), 8.15-8.25 (1H, m), 8.85-8.95 (1H, m)

Reference example 10-1

Benzyl 3-(4-methylpiperazine-1-carbonyl)benzoate

To a solution of 1-methylpiperaz.ine (0.656mL) in dichloromethane (5mL) was added a solution of benzyl 3-chlorocarbonylbenzoate (2.14g) in dichloromethane (5mL) and the resulting mixture was stirred at room temperature overnight. To the reaction mixture were added water and ethyl acetate, and the organic layer was separated. The organic layer was washed with a 1mol/L aqueous solution of sodium hydroxide, water and brine successively, and dried over anhydrous sodium sulfate. The solvent was removed under reduced pressure, and the residue was purified by column chromatography on aminopropylated silica gel (eluent: ethyl acetate) to give the title compound (1.7g).
$^1$H-NMR ($CDCl_3$) δ ppm: 2.24-2.60 (7H, m), 3.41 (2H, brs), 3.81 (2H, brs), 5.37 (2H, s), 7.30-7.64 (7H, m), 8.08-8.16 (2H, m)

Reference Example 10-2

Benzyl 3-(morpholin-4-carbonyl)benzoate

Reference example 10-2 was prepared in a manner similar to those as described in reference example 10-1 using morpholine instead of 1-methylpiperazine.
$^1$H-NMR ($CDCl_3$) δ ppm: 3.30-3.95 (8H, m), 5.38 (2H, s), 7.31-7.65 (7H, m), 8.07-8.18 (2H, m)

Reference example 11-1

3-(4-Methylpiperazine-1-carbonyl)benzoic acid

A mixture of benzyl 3-(4-methylpiperazine-1-carbonyl)-benzoate (1.7g) and 10% palladium on carbon (50%wet, 0.128g) in ethanol (50mL) was stirred at room temperature under hydrogen atmosphere for 3 days. To the reaction mixture was added a mixed solvent of trifluoroethanol (50mL) and dichloromethane (50ml), and the mixture was stirred at room temperature for 10 minutes. The catalyst was removed by filtation, and the filtrate was concentrated under reduced pressure to give the title compound (1.15g).
$^1$H-NMR (DMSO-$d_6$) δ ppm: 2.16-2.46 (7H, m), 3.00-3.80 (4H, m), 7.50-7.66 (2H, m), 7.83-8.06 (2H, m)

Reference example 11-2

3-(Morpholine-4-carbonyl)benzoic acid

Reference example 11-2 was prepared in a manner similar to those as described in reference example 11-1 using benzyl 3-(morpholine-4-carbonyl)benzoate instead of benzyl 3-(4-methylpiperazine-1-carbonyl)benzoate.
$^1$H-NMR (DMSO-$d_6$) δ ppm: 3.20-3.80 (8H, m), 7.55-7.70 (2H, m), 7.90-8.04 (2H, m), 13.13 (1H, brs)

Reference example 12-1

Methyl 5-(4-methylpiperazine-1-sulfonyl)furan-2-carboxylate

To a solution of methyl 5-chlorosulfonyl-2-furancarboxylate (0.414g) in tetrahydrofuran (5mL) was added 1-methylpiperazine (0.41mL) under ice-cooling, and the resulting mixture was stirred at room temperature for 1 day. To the reaction mixture were added a saturated aqueous solution of sodium carbonate and ethyl acetate. The organic layer was separated, washed with water and brine, and dried over anhydrous magnesium sulfate. The solvent was removed under reduced pressure to give the title compound (0.265g).
$^1$H-NMR ($CDCl_3$) δ ppm: 2.30 (3H, s), 2.40-2.55 (4H, m), 3.20-3.40 (4H, m), 3.92 (3H, s), 7.04 (1H, d, J=3.5Hz), 7.20 (1H, d, J=3.5Hz)

Reference Examples 12-2 to 12-4 were prepared in a manner similar to those as described in Reference Example 12-1 using the corresponding sulfonylchlorides and amines instead of methyl 5-chlorosulfonyl-2-furancarboxylate and 1-methylpiperazine.

Reference example 12-2

Methyl 5-(azepane-1-sulfonyl)furan-2-carboxylate

$^1$H-NMR ($CDCl_3$) δ ppm: 1.55-1.80 (8H, m), 3.35-3.45 (4H, m), 3.92 (3H, s), 6.95-7.05 (1H, m), 7.15-7.20 (1H, m)

Reference example 12-3

3-(4-Methylpiperazine-l-sulfonyl)benzoic acid hydrochloride $^1$H-NMR (DMSO-$d_6$) δ ppm: 2.73 (3H, s), 2.95-4.10 (8H, m), 7.75-7.95 (1H, m), 8.00-8.10 (1H, m), 8.15-8.35 (2H, m), 10.85 (1H, brs), 13.57 (1H, brs)

Reference example 12-4

3-(4-Benzylpiperazine-1-sulfonyl)benzoic acid

$^1$H-NMR (DMSO-$d_6$) δ ppm: 2.35-2.50 (4H, m), 2.80-3.00 (4H, m), 3.46 (2H, s), 7.15-7.40 (5H, m), 7.75-7.85 (1H, m), 7.90-8.00 (1H, m), 8.15-8.30 (2H, m), 13.54 (1H, brs)

Reference example 13-1

Methyl 3-cyclohexyloxybenzoate

To a solution of cyclohexanol (1.10mL) in tetrahydrofuran (10mL) was added triethylamine (1.50mL) under ice-cooling, and then methansulfonyl chloride (0.81mL) was added to the reaction mixture during 5 minutes.The resulting mixture was stirred at room temperature for 30 minutes. Insoluble materials were removed by filtration, and the filtrate was added to a mixture of methyl 3-hydroxybenzoate (1.52g), cesium carbonate (3.91g) and N,N-dimethylformamide (5mL) under stirring. The mixture was stirred at 60° C for 3 days. Water and diethyl ether were added to the reaction mixture, and the organic layer was separated, and washed with water, a saturated solution of sodium bicarbonate, and brine successively. After the organic layer was dried over anhydrous magnesium sulfate, the solvent was removed under reduced pressure. The residue was purified by column chromatography on silica gel (eluent: hexane/ethyl acetate = 3/1 - 1/1) to give the title compound (0.337g).
$^1$H-NMR ($CDCl_3$) δ ppm: 1.20-1.65 (6H, m), 1.75-2.05 (4H, m), 3.91 (3H, s), 4.25-4.35 (1H, m), 7.05-7.15 (1H, m), 7.25-7.35 (1H, m), 7.50-7.65 (2H, m)

Reference example 13-2

Reference example 13-2 was prepared in a manner similar to those as described in Reference Example 13-1 using tetrahydropyrane-4-ol instead of cyclohexanol.
$^1$H-NMR ($CDCl_3$) δ ppm: 1.75-1.85 (2H, m), 1.95-2.10 (2H, m), 3.55-3.65 (2H, m), 3.92 (3H, s), 3.95-4.05 (2H, m), 4.50-4.60 (1H, m), 7.05-7.15 (1H, m), 7.25-7.40 (1H, m), 7.55-7.70 (2H, m)

Reference example 14

3-(1-Benzylpiperidin-4-yloxy)benzoic acid

To a mixture of 1-benzylpiperidin-4-ol (2.29g), triphenylphosphine (3.14g) and tetrahydrofuran (20mL) were added diisopropyl azodicarboxylate (2.40mL) and methyl 3-hydroxybenzoate (1.52g) successively under ice-cooling, and the resulting mixture was stirred at room temperature under argon atmosphere overnight. Diethyl ether and 2mol/L

hydrochloric acid were added to the reaction mixture. The aqueous layer was separated and washed with diethyl ether. The aqueous layer was made alkaline with a saturated aqueous solution of sodium carbonate, and diethyl ether was added to the mixture. The organic layer was separated, washed with water and brine successively, and dried over anhydrous magnesium sulfate. The solvent was removed under reduced pressure to give methyl 3-(1-benzylpiperidin-4-yloxy)benzoate. A 2mol/L aqueous solution of sodium hydroxide (20mL) was added to a mixture of methyl 3-(1-benzylpiperidin-4-yloxy)benzoate and methanol (20mL), and the mixture was stirred at 60° C for 1 hour. After methanol was removed under reduced pressure, water and diethyl ether were added to the residue. The aqueous layer was separated, and washed with diethyl ether. Then, the aqueous layer was neutralized with 2mol/L hydrochloric acid, and the resulting precipitate was collected by filtration to give the title compound (2.56g).
$^1$H-NMR ($CDCl_3$) δ ppm: 1.90-2.20 (4H, m), 2.60-3.00 (4H, m), 3.79 (2H, s), 4.45-4.55 (1H, m), 7.00-7.10 (1H, m), 7.25-7.45 (6H, m), 7.55-7.70 (2H, m)

Reference example 15-1

Ethyl 6-(3-hydroxymethylphenoxy)nicotinate

A mixture of ethyl 6-chloronicotinate (1.0g), 3-hydroxymethylphenol (0.736g), potassium carbonate (1.49g) and 1-methyl-2-pyrrolidone (5mL) was stirred at 70° C for 2 days. Water and ethyl acetate were added to the reaction mixture. The organic layer was separated and washed with water, a saturated aqueous solution of sodium carbonate, water and brine successively. The organic layer was dried over anhydrous magnesium sulfate, and the solvent was removed under reduced pressure. The residue was purified by column chromatography on aminopropylated silica gel (eluent: hexane/ethyl acetate = 10/1 - 0/1) to give the title compound (0.44g).
$^1$H-NMR ($CDCl_3$) δ ppm: 1.38 (3H, t, J=7.2Hz), 1.83 (1H, t, J=6.0Hz), 4.38 (2H, q, J=7.2Hz), 4.73 (2H, d, J=6.0Hz), 6.90-7.00 (1H, m), 7.05-7.10 (1H, m), 7.15-7.30 (2H, m), 7.35-7.45 (1H, m), 8.25-8.35 (1H, m), 8.75-8.85 (1H, m)

Reference examples 15-2 to 15-14 were prepared in a manner similar to those as described in Reference Example 15-1 using the corresponding phenols or pyridinols instead of 3-hydroxymethylphenol.

Reference example 15-2

Ethyl 6-(2-fluorophenoxy)nicotinate

$^1$H-NMR ($CDCl_3$) δ ppm: 1. 38 (3H, t, J=7.3Hz), 4.37 (2H, q, J=7.3Hz), 7.00-7.10 (1H, m), 7.15-7.30 (4H, m), 8.25-8.35 (1H, m), 8.78 (1H, d, J=1.8Hz)

Reference example 15-3

Ethyl 6-(3-fluorophenoxy)nicotinate

$^1$H-NMR ($CDCl_3$) δ ppm: 1.39 (3H, t, J=7.0Hz), 4.39 (2H, q, J=7.0Hz), 6.85-7.05 (4H, m), 7.30-7.45 (1H, m), 8.25-8.35 (1H, m), 8.83 (1H, d, J=2.3Hz)

Reference example 15-4

Ethyl 6-(4-fluorophenoxy)nicotinate

$^1$H-NMR ($CDCl_3$) δ ppm: 1.38 (3H, t, J=7.2Hz), 4.38 (2H, q, J=7.2Hz), 6.90-7.00 (1H, m), 7.05-7.15 (4H, m), 8.25-8.35 (1H, m), 8.75-8.85 (1H, m)

Reference example 15-5

Ethyl 6-(2,4-difluorophenoxy)nicotinate

$^1$H-NMR ($CDCl_3$) δ ppm: 1.30-1.45 (3H, m), 4.30-4.45 (2H, m), 6.85-7.10 (3H, m), 7.10-7.25 (1H, m), 8.25-8.35 (1H, m), 8.77 (1H, d, J=2.3Hz)

Reference example 15-6

Ethyl 6-(2,4,6-trifluorophenoxy)nicotinate

$^1$H-NMR ($CDCl_3$) δ ppm: 1.38 (3H, t, J=7.2Hz), 4.38 (2H, q, J=7.2Hz), 6.75-6.85 (2H, m), 7.05-7.20 (1H, m), 8.30-8.40 (1H, m), 8.75 (1H, d, J=2.2Hz)

Reference example 15-7

Ethyl 6-(2-isopropylphenoxy)nicotinate

$^1$H-NMR ($CDCl_3$) δ ppm: 1. 18 (6H, d, J=6.9Hz), 1.38 (3H, t, J=7.2Hz), 3.00-3.15 (1H, m), 4.37 (2H, q, J=7.2Hz), 6.85-6.95 (1H, m), 7.00-7.10 (1H, m), 7.20-7.30 (2H, m), 7.35-7.45 (1H, m), 8.20-8.30 (1H, m), 8.80-8.90 (1H, m)

Reference example 15-8

Ethyl 6-(4-isopropylphenoxy)nicotinate

$^1$H-NMR ($CDCl_3$) δ ppm: 1.27 (6H, d, J=6.9Hz), 1.38 (3H, t, J=7.3Hz), 2.85-3.00 (1H, m), 4.30-4.45 (2H, m), 6.91 (1H, d, J=8.5Hz), 7.00-7.15 (2H, m), 7.20-7.35 (2H, m), 8.20-8.30 (1H, m), 8.80-8.90 (1H, m)

Reference example 15-9

Ethyl 6-(4-fluoro-2-methylphenoxy)nicotinate

$^1$H-NMR ($CDCl_3$) δ ppm: 1.38 (3H, t, J=7.1Hz), 2.14 (3H, s), 4.30-4.45 (2H, m), 6.85-7.10 (4H, m), 8.25-8.35 (1H, m), 8.75-8.85 (1H, m),

Reference example 15-10

Ethyl 6-(2-methoxyphenoxy)nicotinate

$^1$H-NMR ($CDCl_3$) δ ppm: 1.37 (3H, t, J=7.2Hz), 3.75 (3H, s), 4.36 (2H, q, J=7.2Hz), 6.90-6.97 (1H, m), 6.98-7.10 (2H, m), 7.10-7.20 (1H, m), 7.20-7.30 (1H, m), 8.20-8.30 (1H, m), 8.79 (1H, d, J=2.7Hz)

Reference example 15-11

Ethyl 6-(3-methoxyphenoxy)nicotinate

$^1$H-NMR ($CDCl_3$) δ ppm: 1.38 (3H, t, J=7.1Hz). 3.81 (3H, s), 4.38 (2H, q, J=7.1Hz), 6.65-6.85 (3H, m), 6.92 (1H, d, J=8.7Hz), 7.32 (1H, t, J=8.2Hz), 8.27 (1H, dd, J=2.3, 8.7Hz), 8.84 (1H, d, J=2.3Hz)

Reference example 15-12

Ethyl 6-(4-fluoro-2-methoxyphenoxy)nicotinate

$^1$H-NMR ($CDCl_3$) δ ppm: 1.37 (3H, t, J=7.2Hz), 3.74 (3H, s), 4.37 (2H, m), 6.65-6.80 (2H, m), 6.90-7.00 (1H, m), 7.05-7.15 (1H, m), 8.20-8.30 (1H, m), 8.77 (1H, d, J=2.1Hz)

Reference example 15-13

Ethyl 6-(pyridin-3-yloxy)nicotinate

$^1$H-NMR ($CDCl_3$) δ ppm: 1. 39 (3H, t, J=7.2Hz), 4.39 (2H, q, J=7.2Hz), 7.00-7.10 (1H, m), 7.35-7.45 (1H, m), 7.50-7.60 (1H, m), 8.30-8.40 (1H, m), 8.45-8.60 (2H, m), 8.75-8.85 (1H, m).

Reference example 15-14

Ethyl 6-phenethyloxynicotinate

$^1$H-NMR ($CDCl_3$) δ ppm: 1.35-1.45 (3H, m), 3.00-3.15 (2H, m), 4.30-4.65 (4H, m), 6.65-6.80 (1H, m), 7.15-7.40 (5H, m), 8.05-8.20 (1H, m), 8.75-8.90 (1H, m)

Reference example 16

Methyl 4-hydroxy-3-morpholin-4-ylmethylbenzoate

Concentrated sulfuric acid (1.2mL) was added to a solution of 4-hydroxy-3-morpholin-4-ylmethylbenzoic acid mono hydrate (5.11g) in methanol (50mL), and the resulting mixture was heated under reflux overnight. A saturated aqueous solution of sodium carbonate and ethyl acetate were added to the reaction mixture. The organic layer was separated, washed with brine, and dried over anhydrous magnesium sulfate. The solvent was removed under reduced pressure to give the title compound (3.86g).
$^1$H-NMR ($CDCl_3$) δ ppm: 2.40-2.80 (4H, m), 3.60-3.85 (6H, m), 3.87 (3H, s), 6.80-6.90 (1H, m), 7.70-7.80 (1H, m), 7.85-7.95 (1H, m)

Reference example 17-1

Methyl 4-benzyloxy-3-morpholin-4-ylmethylbenzoate

To a mixture of methyl 4-hydroxy-3-morpholin-4-ylmethylbenzoate (3.85g), potassium carbonate (4.24g) and N,N-dimethylformamide (15mL) was added benzyl bromide (1.85mL), and the resulting mixture was stirred at room temperature for 3 days. Water was added to the reaction mixture, and the resulting precipitate was collected by filtration to give the title compound (4.69g).
$^1$H-NMR ($CDCl_3$) δ ppm: 2.40-2.60 (4H, m), 3.61 (2H, brs), 3.65-3.80 (4H, m), 3.89 (3H, s), 5.15 (2H, s), 6.90-7.00 (1H, m), 7.30-7.50 (5H, m), 7.90-8.10 (2H, m)

Reference Example 17-2

Ethyl 4-ethoxy-3-morpholin-4-ylmethylbenzoate

Reference Example 17-2 was prepared in a manner similar to those as described in Reference Example 17-1 using 4-hydroxy-3-morpholin-4-ylmethylbenzoic acid mono hydrate and ethyl iodide instead of methyl 4-hydroxy-3-morpholin-4-ylmethylbenzoate and benzyl bromide.
$^1$H-NMR ($CDCl_3$) δ ppm: 1.39 (3H, t, J=7.1Hz), 1.45 (3H, t, J=7.0Hz), 2.45-2.60 (4H, m), 3.57 (2H, s), 3.65-3.80 (4H, m), 4.10 (2H, q, J=7.0Hz), 4.35 (2H, q, J=7.1Hz), 6.80-6.90 (1H, m), 7.90-8.10 (2H, m)

Reference Example 18-1

6-(3-Chloromethylphenoxy)nicotinoyl chloride hydrochloride

To a mixture of 6-(3-hydroxymethylphenoxy)nicotinic acid (0.4g) and toluene (10mL) were added thionyl chloride (0.715mL) and N,N-dimethylformamide (0.040mL) successively, and the resulting mixture was stirred at 80° C for 2 hours. The reaction mixture was concentrated under reduced pressure to give the title compound (0.42g).
$^1$H-NMR (DMSO-$d_6$) δ ppm: 4.63 (2H, s), 6.95-7.00 (1H, m), 7.05-7.45 (4H, m), 7.62 (1H, s), 8.25-8.35 (1H, m), 8.75-8.80 (1H, m)
Reference Examples 18-2 to 18-19 were prepared in a manner similar to those as described in Reference Example 18-1 using the corresponding carboxylic acids instead of 6-(3-hydroxymethylphenoxy)nicotinic acid.

Reference Example 18-2

6-(2-Fluorophenoxy)nicotinoyl chloride

$^1$H-NMR ($CDCl_3$) δ ppm: 7.11 (1H, d, J=8.8Hz), 7.15-7.35 (4H, m), 8.30-8.40 (1H, m), 8.88 (1H, d, J=2.0Hz)

Reference example 18-3

6-(3-Fluorophenoxy)nicotinoyl chloride

$^1$H-NMR ($CDCl_3$) δ ppm: 6.85-7.10 (4H, m), 7.35-7.45 (1H, m), 8.30-8.40 (1H, m), 8.92 (1H, d, J=2.6Hz)

Reference example 18-4

6-(4-Fluorophenoxy)nicotinoyl chloride

$^1$H-NMR ($CDCl_3$) δ ppm: 7.03 (1H, d, J=8.6Hz), 7.10-7.20 (4H, m), 8.30-8.40 (1H, m), 8.90 (1H, d, J=2.1Hz)

Reference example 18-5

6-(2,4-Difluorophenoxy)nicotinoyl chloride

$^1$H-NMR ($CDCl_3$) δ ppm: 6.90-7.05 (2H, m), 7.10-7.15 (1H, m), 7.15-7.25 (1H, m), 8.30-8.40 (1H, m), 8.85-8.90 (1H, m)

Reference example 18-6

6-(2,4-Trifluorophenoxy)nicotinoyl chloride

$^1$H-NMR ($CDCl_3$) δ ppm: 6.75-6.90 (2H, m), 7.20 (1H, d, J=8.6Hz), 8.35-8.45 (1H, m), 8.85 (1H, d, J=2.2Hz)

Reference example 18-7

6-(2-Isopropyl-phenoxy)nicotinoyl chloride

$^1$H-NMR ($CDCl_3$) δ ppm: 1.19 (6H, m), 2.95-3.10 (1H, m), 6.95-7.10 (2H, m), 7.20-7.30 (2H, m), 7.35-7.45 (1H, m), 8.25-8.40 (1H, m), 8.91 (1H, d, J=2.5Hz)

Reference example 18-8

6-(4-Isopropyl-phenoxy)nicotinoyl chloride

$^1$H-NMR ($CDCl_3$) δ ppm: 1.28 (6H, d, J=6.9Hz), 2.90-3.05 (1H, m), 6.99 (1H, d, J=8.9Hz), 7.00-7.15 (2H, m), 7.25-7.35 (2H, m), 8.25-8.35 (1H, m), 8.94 (1H, d, J=2.5Hz)

Reference example 18-9

6-(4-Fluoro-2-methylphenoxy)nicotinoyl chloride

$^1$H-NMR ($CDCl_3$) δ ppm: 2.14 (3H, s), 6.90-7.10 (4H, m), 8.30-8.40 (1H, m), 8.89 (1H, d, J=2.4Hz)

Reference example 18-10

6-(2-Methoxyphenoxy)nicotinoyl chloride

$^1$H-NMR ($CDCl_3$) δ ppm: 3.76 (3H, s), 6.95-7.10 (3H, m), 7.10-7.20 (1H, m), 7.20-7.35 (1H, m), 8.25-8.35 (1H, m), 8.85-8.95 (1H, m)

Reference example 18-11

6-(3-Methoxyphenoxy)nicotinoyl chloride

$^1$H-NMR ($CDCl_3$) δ ppm: 3.82 (3H, s), 6.65-6.80 (2H, m), 6.80-6.90 (1H, m), 7.00 (1H, d, J=8.5Hz), 7.35 (1H, t, J=8.2Hz), 8.25-8.40 (1H, m), 8.93 (1H, d, J=2.5Hz)

Reference example 18-12

6-(4-Fluoro-2-methoxyphenoxy)nicotinoyl chloride

$^1$H-NMR ($CDCl_3$) δ ppm: 3.74 (3H, s), 6.60-6.80 (2H, m), 7.00-7.15 (2H, m), 8.25-8.35 (1H, m), 8.85-8.90 (2H, m)

Reference example 18-13

5-(3-Chloromethylphenyl)furan-2-carbonyl chloride

$^1$H-NMR ($CDCl_3$) δ ppm: 7.18 (1H, d, J=3.5Hz), 7.33 (1H, d, J=3.5Hz), 7.40-7.55 (2H, m), 7.75-7.85 (1H, m), 7.85-7.95 (1H, m)

Reference example 18-14

5-Pyridin-2-ylfuran-2-carbonyl chloride

$^1$H-NMR ($CDCl_3$) δ ppm: 7.60-7.70 (1H, m), 7.85-7.95 (1H, m), 8.35-8.55 (2H, m), 8.75-8.90 (2H, m)

Reference example 18-15

5-Pyridin-3-ylfuran-2-carbonyl chloride

$^1$H-NMR ($CDCl_3$) δ ppm: 7.35-7.50 (2H, m), 7.75-7.85 (1H, m), 8.45-8.55 (1H, m), 8.65-8.75 (1H, m), 9.10-9.20 (1H, m)

Reference example 18-16

5-Pyridin-4-ylfuran-2-carbonyl chloride

$^1$H-NMR ($CDCl_3$) δ ppm: 7.45-7.55 (1H, m), 7.85-7.90 (1H, m), 8.25-8.35 (2H, m), 8.85-8.95 (2H, m)

Reference example 18-17

5-(Azepane-1-sulfonyl)furan-2-carbonyl chloride

$^1$H-NMR ($CDCl_3$) δ ppm: 1.55-1.85 (8H, m), 3.35-3.50 (4H, m), 7.00-7.10 (1H, m), 7.45-7.50 (1H, m)

Reference example 18-18

3-(4-Methylpiperazine-1-sulfonyl)benzoyl chloride hydrochloride

$^1$H-NMR (DMSO-$d_6$) δ ppm: 2.60-2.85 (5H, m), 3.05-3.25 (2H, m), 3.35-3.50 (2H, m), 3.70-3.90 (2H, m), 7.80-7.90 (1H, m), 8.00-8.10 (1H, m), 8.20-8.35 (2H, m), 10.55 (1H, brs)

Reference example 18-19

4-Benzyloxy-3-morpholin-4-ylmethylbenzoyl chloride

$^1$H-NMR (DMSO-$d_6$) δ ppm: 2.80-2.95 (2H, m), 3.25-3.40 (2H, m), 3.80-3.95 (2H, m), 4.20-4.40 (2H, m), 4.31 (2H, s), 5.27 (2H, brs), 7.10-7.20 (1H, m), 7.35-7.50 (5H, m), 8.15-8.25 (1H, m), 8.45-8.50 (1H, m), 13.38 (1H, brs)

Reference example 19-1

5-Pyridin-2-ylfuran-2-carboxylic acid

To a solution of silver nitrate (0.888g) in water (5mL) was added a 2mol/L aqueous solution of sodium hydroxide (5mL) under ice-cooling. Then, 5-pyridin-2-ylfuran-2-carbaldehyde (0.376g) and tetrahydrofuran (1mL) were added to

the mixture, and the resulting mixture was stirred at room temperature for 1 hour. Insoluble materials were removed by filtration. To the filtrate was added 2mol/L hydrochloric acid (5mL) under ice-cooling, and the mixture was concentrated under reduced pressure. The resulting precipitate was collected by filtration to give the title compound (0.289g).
$^1$H-NMR (DMSO-$d_6$) δ ppm: 7.25-7.50 (3H, m), 7.85-8.05 (2H, m), 8.60-8.70 (1H, m)

Reference Examples 19 - 2 and 19-3 were prepared in a manner similar to those as described in Reference Example 19-1 using the corresponding aldehydes instead of 5-pyridin-2-ylfuran-2-carbaldehyde.

Reference example 19-2

5-(3-Hydroxymethylphenyl)furan-2-carboxylic acid

$^1$H-NMR (DMSO-$d_6$) δ ppm: 4.50-4.60 (2H, m), 5.20-5.40 (1H, m), 7.12 (1H, d, J=3.9Hz), 7.25-7.40 (2H, m), 7.43 (1H, t, J=7.8Hz), 7.68 (1H, d, J=7.8Hz), 7.77 (1H, s), 13.11 (1H, brs)

Reference example 19-3

5-Cyclopropylfuran-2-carboxylic acid

$^1$H-NMR ($CDCl_3$) δ ppm: 0.85-1.05 (4H, m), 1.95-2.05 (1H, m), 6.10 (1H, d, J=3.5Hz), 7.22 (1H, d, J=3.5Hz)

Example 1

3-Cyclopropanecarbonylamino-5-fluorobenzofuran-2-carboxamide (compound 1-1)

3-Amino-5-fluorobenzofuran-2-carboxamide (0.1g) was dissolved in tetrahydrofuran (3mL), and the solution was cooled at 0˚C. Then, triethylamine (0.144mL) and cyclopropanecarbonyl chloride (0.051mL) was added to the mixture successively, and the resulting mixture was stirred at room temperature for 18 hours. Water (6mL) was added to the reaction mixture, and the mixture was stirred for another 1 hour. The resulting precipitate was collected by filtration to give the title compound (0.063g). $^1$H-NMR (DMSO-$d_6$) δ ppm: 0.80-0.98 (4H, m), 1.91-2.06 (1H, m), 7.28-7.40 (1H, m), 7.55-7.70 (2H, m), 7.89 (1H, brs), 8.13 (1H, brs), 10.34 (1H, brs)

Example 2

3-Cyclopropanecarbonylamino-5-chlorobenzofuran-2-carboxamide (compound 1-2)

Cyclopropanecarboxylic acid (0.018g) and triphosgen (0.032g) were dissolved in tetrahydrofuran (1.5mL), and N-methylmorpholine (0.047mL) was added thereto at room temperature. The mixture was stirred for 15 minutes. Then, a solution of 3-amino-5-chlorobenzofuran-2-carboxamide (0.042g) in tetrahydrofuran (1.5mL) was added to the mixture, and the resulting mixture was stirred for 14 hours. The reaction mixture was extracted with diethyl ether, washed with a 10% aqueous solution of sodium hydroxide and brine successively. The organic solvent was removed under reduced pressure, and the residue was purified by column chromatography on silica gel (eluent: hexane/tetrahydrofuran = 7/3) to give the title compound (0.013g).
$^1$H-NMR (DMSO-$d_6$) δ ppm: 0.80-0.95 (4H, m), 1.95-2.05 (1H, m), 7.45-7.65 (2H, m), 7.92 (1H, brs), 7.95-8.00 (1H, m), 8.15 (1H, brs), 10.37 (1H, brs)

Compounds 1-3 to 1-370 were prepared in a manner similar to those as described in Example 1 or Example 2 using the corresponding 3-aminobenzofuran-2-carboxamides and the corresponding acid chlorides or carboxylic acids instead of 3-amino-5-fluorobenzofuran-2-carboxamide and cyclopropanecarbonylchloride. These were illustrated in table 2.

Table 2

| Compound No. | Structure | Compound No. | Structure |
|---|---|---|---|
| 1-1 | 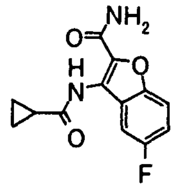 | 1-9 | 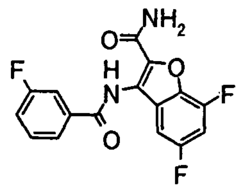 |
| 1-2 | 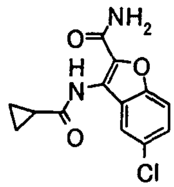 | 1-10 | 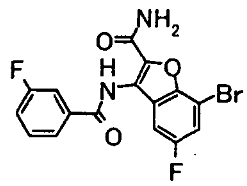 |
| 1-3 | 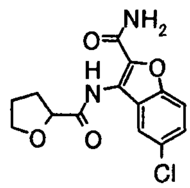 | 1-11 | 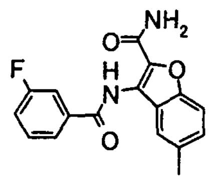 |
| 1-4 | 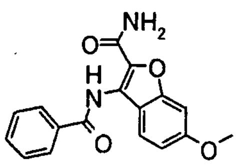 | 1-12 | 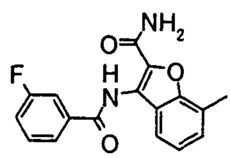 |
| 1-5 | 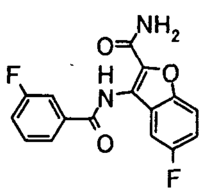 | 1-13 | 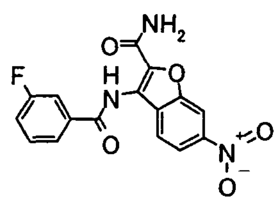 |
| 1-6 | 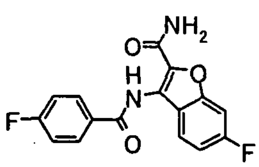 | 1-14 | 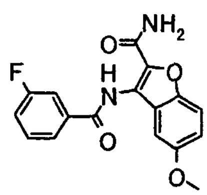 |
| 1-7 | 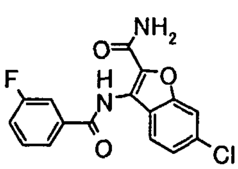 | 1-15 | 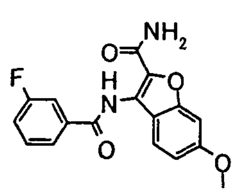 |

(continued)

| Compound No. | Structure | Compound No. | Structure |
|---|---|---|---|
| 1-8 | 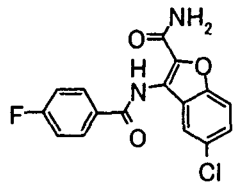 | 1-16 | 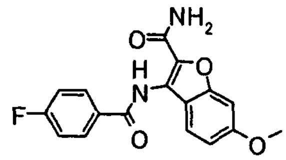 |
| 1-17 | 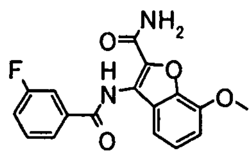 | 1-25 | 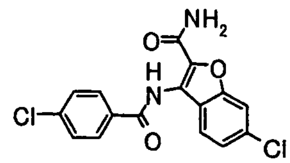 |
| 1-18 | 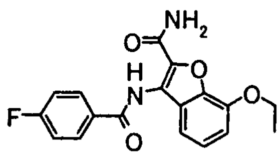 | 1-26 | 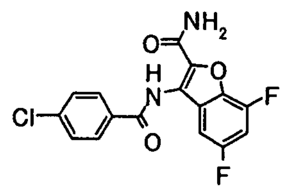 |
| 1-19 | 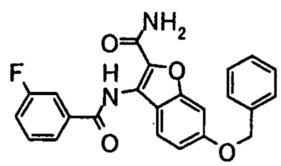 | 1-27 | 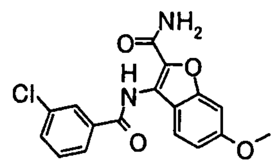 |
| 1-20 | 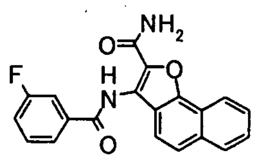 | 1-28 | 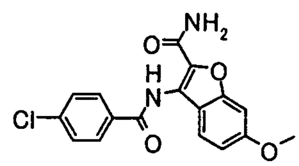 |
| 1-21 | 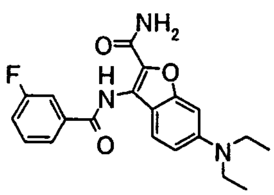 | 1-29 | 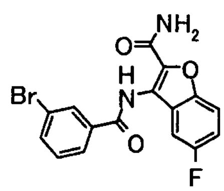 |
| 1-22 | 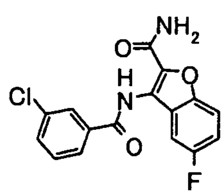 | 1-30 | 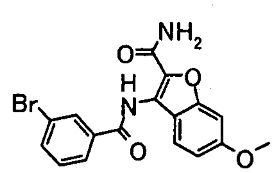 |
| 1-23 | 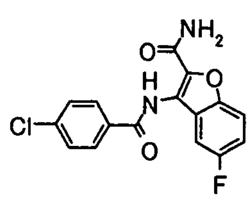 | 1-31 | 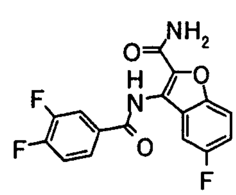 |

(continued)

| Compound No. | Structure | Compound No. | Structure |
|---|---|---|---|
| 1-24 | 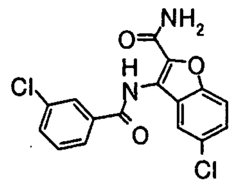 | 1-32 | 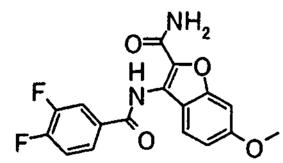 |
| 1-33 | 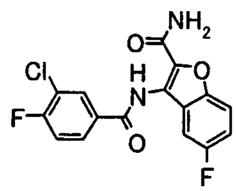 | 1-41 | 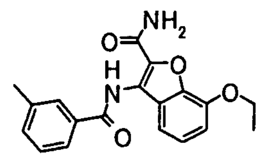 |
| 1-34 | 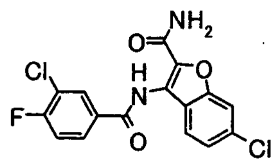 | 1-42 | 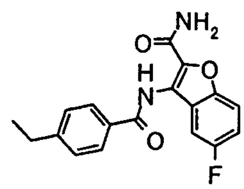 |
| 1-35 | 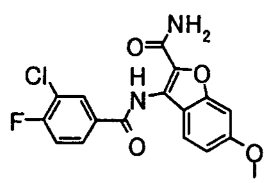 | 1-43 | 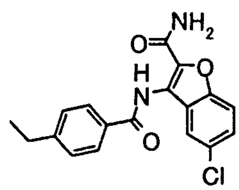 |
| 1-36 | 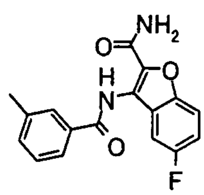 | 1-44 | 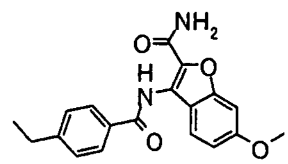 |
| 1-37 | 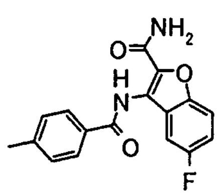 | 1-45 | 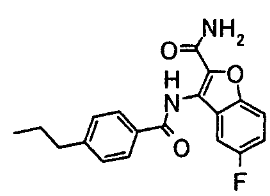 |
| 1-38 | 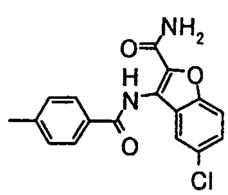 | 1-46 | 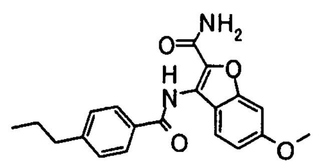 |

(continued)

| Compound No. | Structure | Compound No. | Structure |
|---|---|---|---|
| 1-39 | 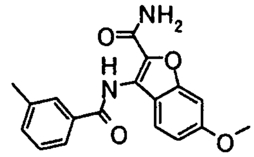 | 1-47 | 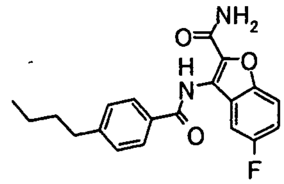 |
| 1-40 | 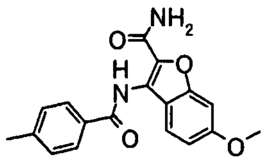 | 1-48 | 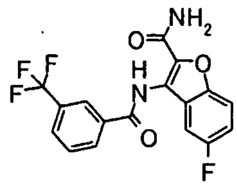 |
| 1-49 | 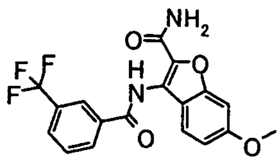 | 1-57 | 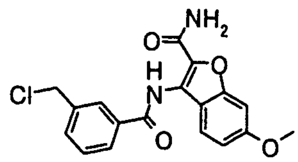 |
| 1-50 | 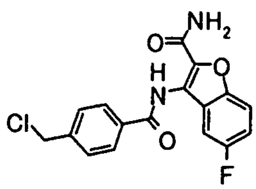 | 1-58 | 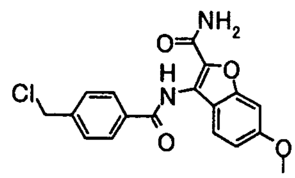 |
| 1-51 | 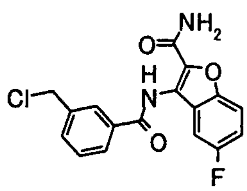 | 1-59 | 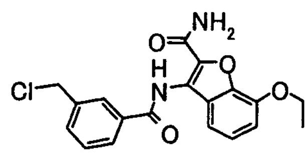 |
| 1-52 | 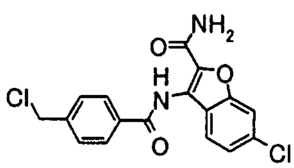 | 1-60 | 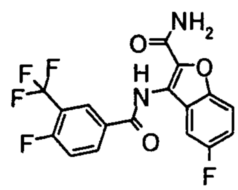 |
| 1-53 | 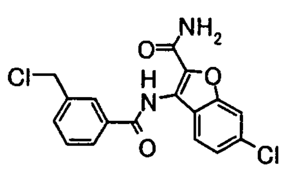 | 1-61 | 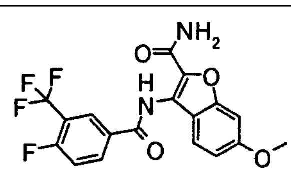 |
| 1-54 | 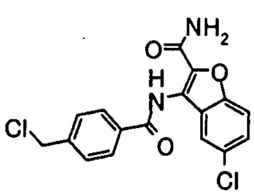 | 1-62 | 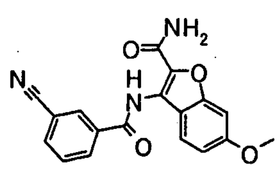 |

(continued)

| Compound No. | Structure | Compound No. | Structure |
|---|---|---|---|
| 1-55 | 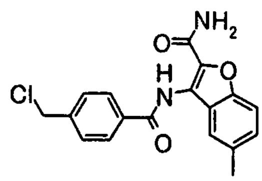 | 1-63 | 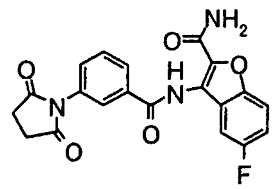 |
| 1-56 | 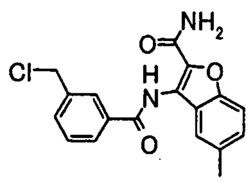 | 1-64 | 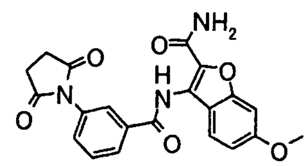 |
| 1-65 | 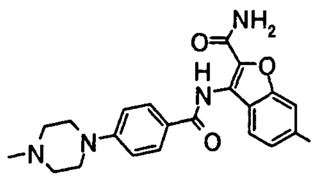 | 1-73 | 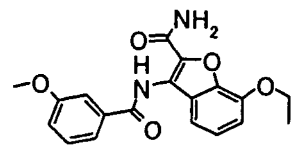 |
| 1-66 | 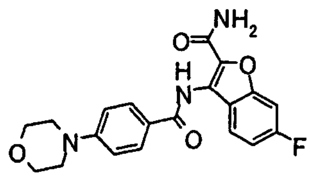 | 1-74 | 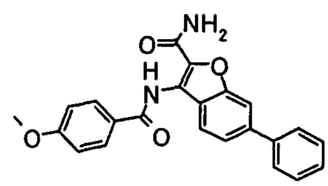 |
| 1-67 | 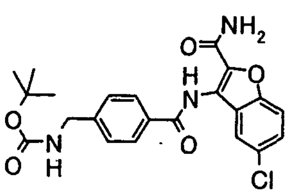 | 1-75 | 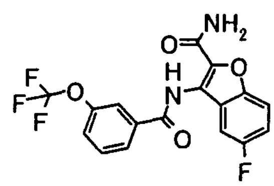 |
| 1-68 | 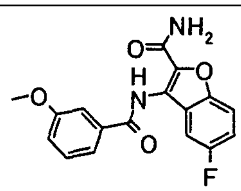 | 1-76 | 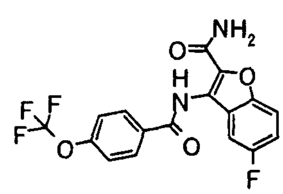 |
| 1-69 | 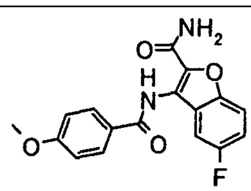 | 1-77 | 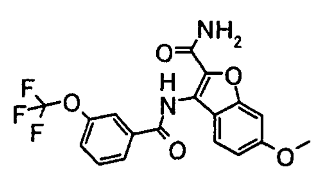 |
| 1-70 | 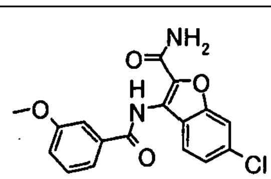 | 1-78 | 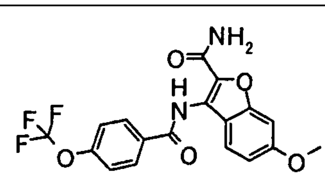 |

(continued)

| Compound No. | Structure | Compound No. | Structure |
|---|---|---|---|
| 1-71 | 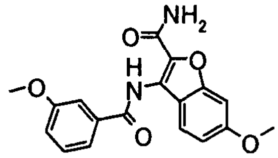 | 1-79 | 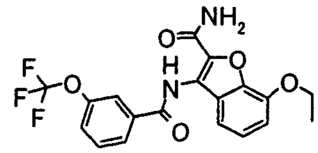 |
| 1-72 | 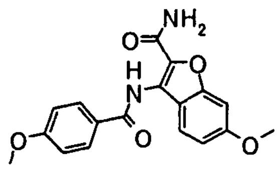 | 1-80 | 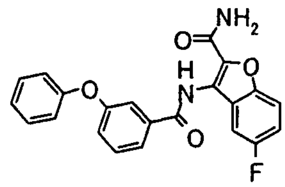 |
| 1-81 | 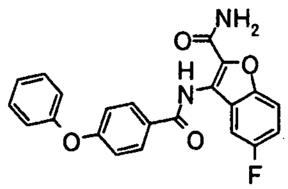 | 1-89 | 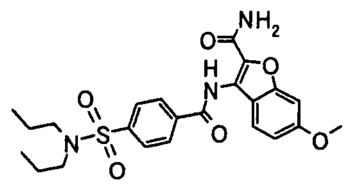 |
| 1-82 | 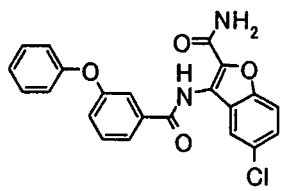 | 1-90 | 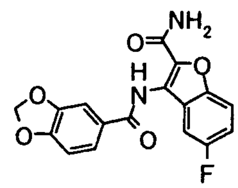 |
| 1-83 | 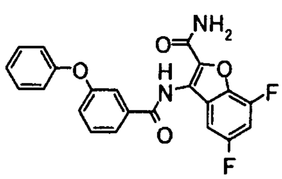 | 1-91 | 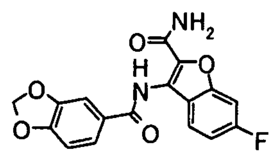 |
| 1-84 | 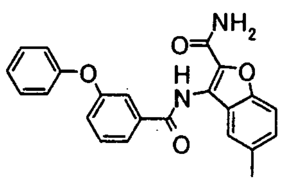 | 1-92 | 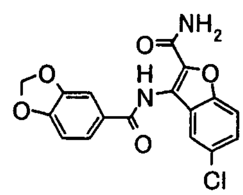 |
| 1-85 | 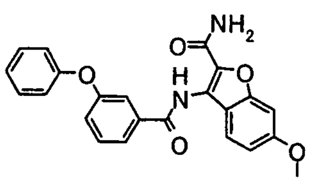 | 1-93 | 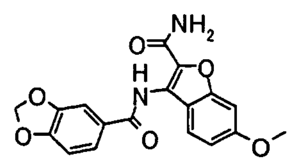 |
| 1-86 | 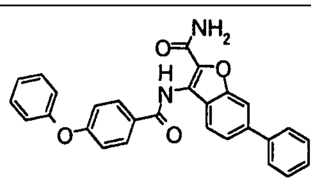 | 1-94 | 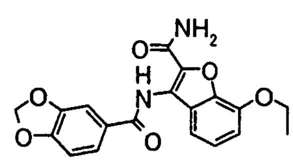 |

(continued)

| Compound No. | Structure | Compound No. | Structure |
|---|---|---|---|
| 1-87 | 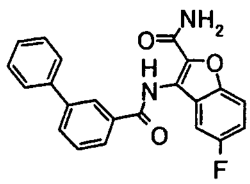 | 1-95 | 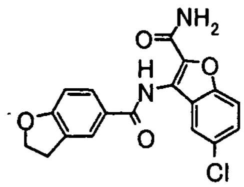 |
| 1-88 | 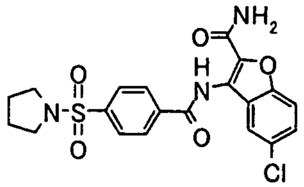 | 1-96 | 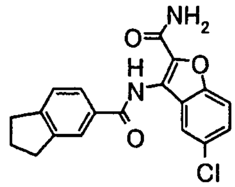 |
| 1-97 | 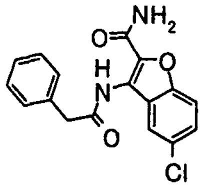 | 1-105 | 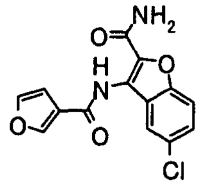 |
| 1-98 | 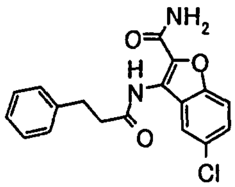 | 1-106 | 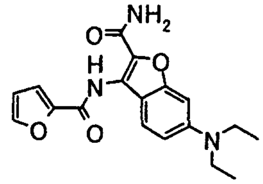 |
| 1-99 | 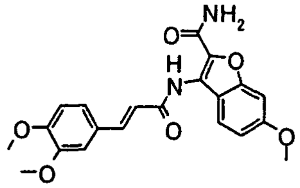 | 1-107 | 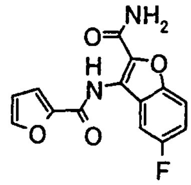 |
| 1-100 | 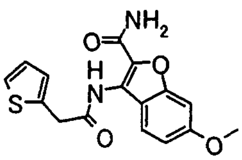 | 1-108 | 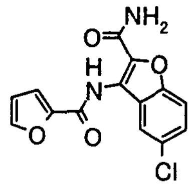 |
| 1-101 | 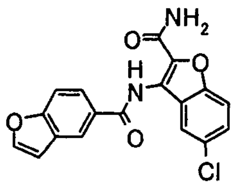 | 1-109 | 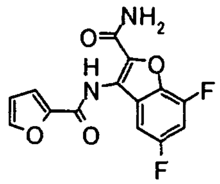 |

(continued)

| Compound No. | Structure | Compound No. | Structure |
|---|---|---|---|
| 1-102 | 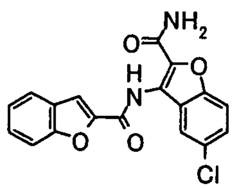 | 1-110 | 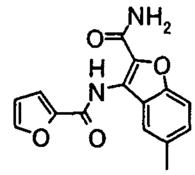 |
| 1-103 | 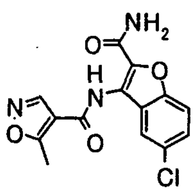 | 1-111 | 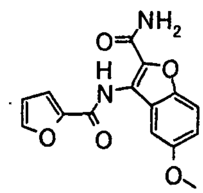 |
| 1-104 | 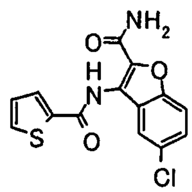 | 1-112 | 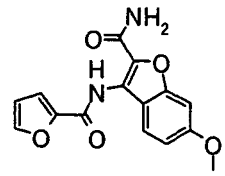 |
| 1-113 | 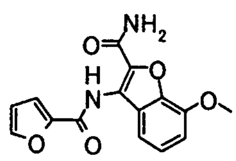 | 1-121 | 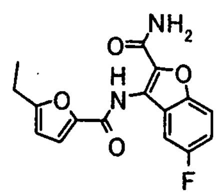 |
| 1-114 | 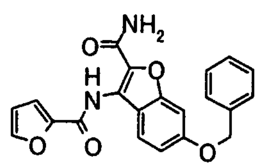 | 1-122 | 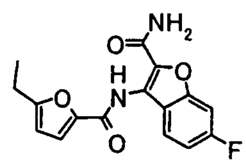 |
| 1-115 | 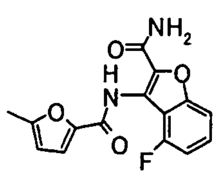 | 1-123 | 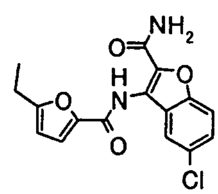 |
| 1-116 | 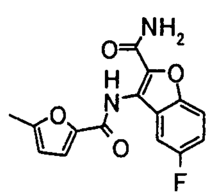 | 1-124 | 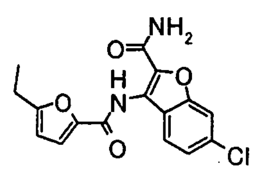 |

(continued)

| Compound No. | Structure | Compound No. | Structure |
|---|---|---|---|
| 1-117 | 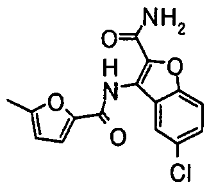 | 1-125 | 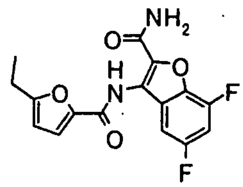 |
| 1-118 | 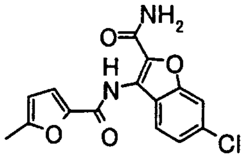 | 1-126 | 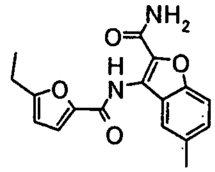 |
| 1-119 | 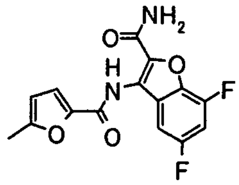 | 1-127 | 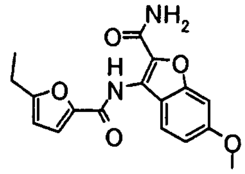 |
| 1-120 | 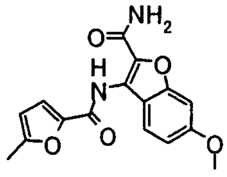 | 1-128 | 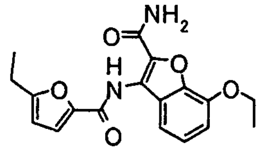 |
| 1-129 | 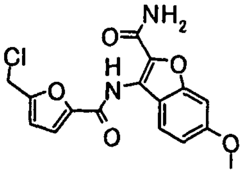 | 1-137 | 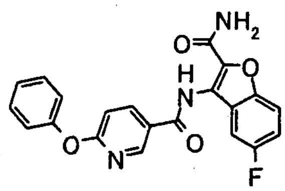 |
| 1-130 | 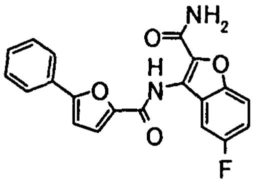 | 1-138 | 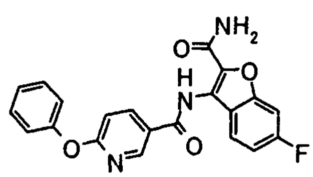 |
| 1-131 | 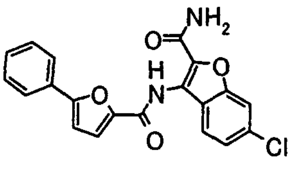 | 1-139 | 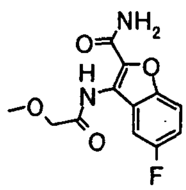 |

(continued)

| Compound No. | Structure | Compound No. | Structure |
|---|---|---|---|
| 1-132 | 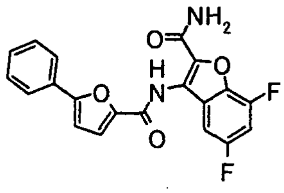 | 1-140 | 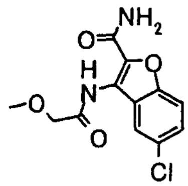 |
| 1-133 | 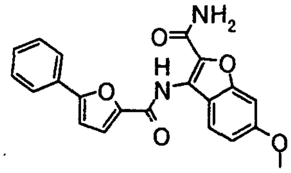 | 1-141 | 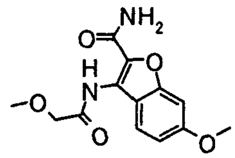 |
| 1-134 | 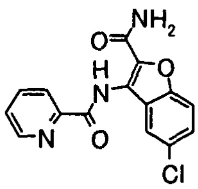 | 1-142 | 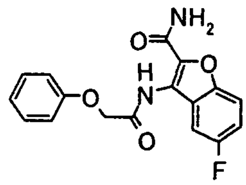 |
| 1-135 | 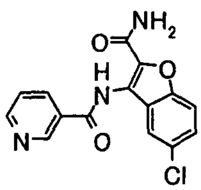 | 1-143 | 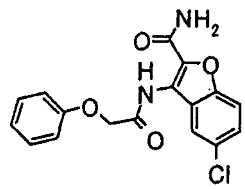 |
| 1-136 | 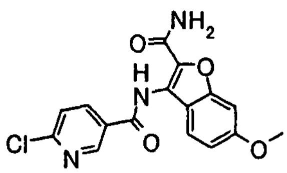 | 1-144 | 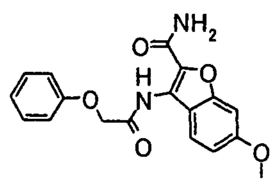 |
| 1-145 | 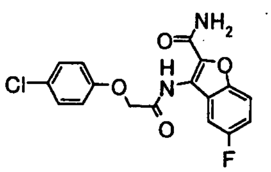 | 1-154 | 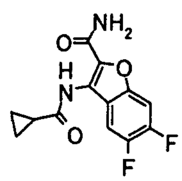 |
| 1-146 | 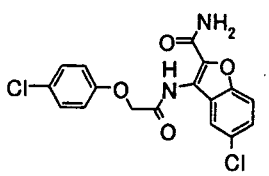 | 1-155 | 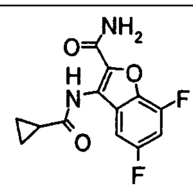 |
| 1-147 | 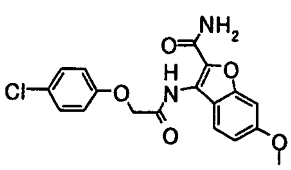 | 1-156 | 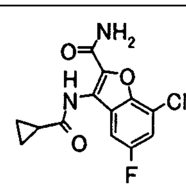 |

(continued)

| Compound No. | Structure | Compound No. | Structure |
|---|---|---|---|
| 1-148 | 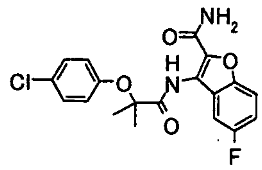 | 1-157 | 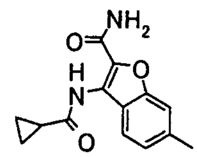 |
| 1-149 | 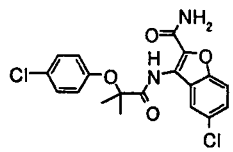 | 1-158 | 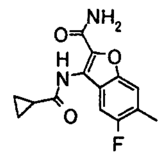 |
| 1-150 | 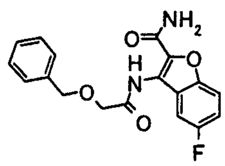 | 1-159 | 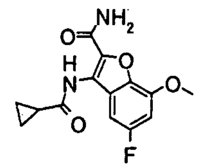 |
| 1-151 | 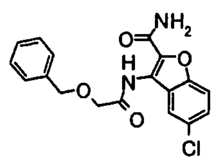 | 1-160 | 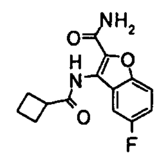 |
| 1-152 | 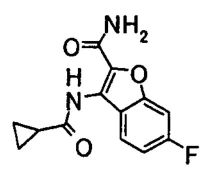 | 1-161 | 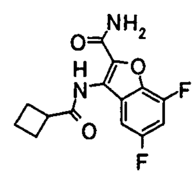 |
| 1-153 | 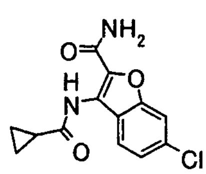 | 1-162 | 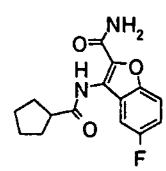 |
| 1-163 | 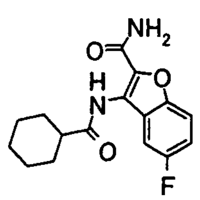 | 1-171 | 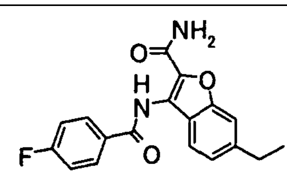 |
| 1-164 | 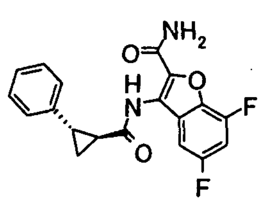 | 1-172 | 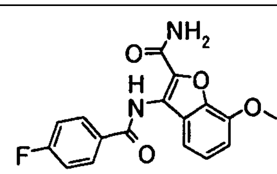 |

(continued)

| Compound No. | Structure | Compound No. | Structure |
|---|---|---|---|
| 1-165 | 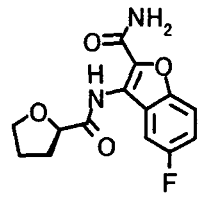 | 1-173 | 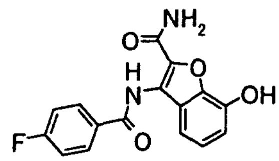 |
| 1-166 | 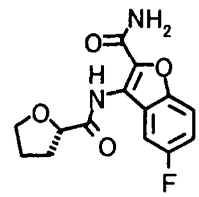 | 1-174 | 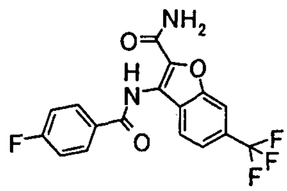 |
| 1-167 | 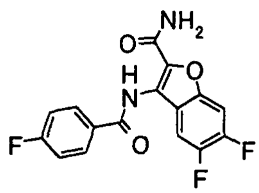 | 1-175 | 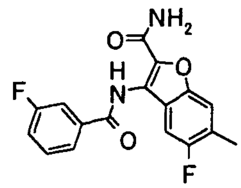 |
| 1-168 | 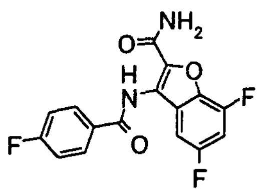 | 1-176 | 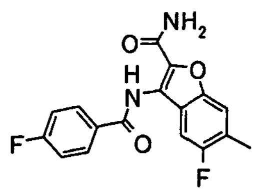 |
| 1-169 | 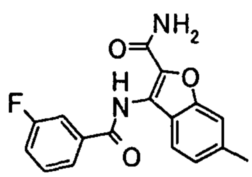 | 1-177 | 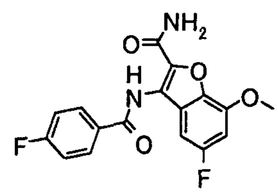 |
| 1-170 | 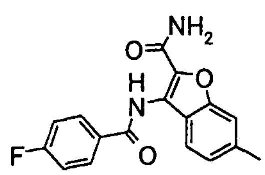 | 1-178 | 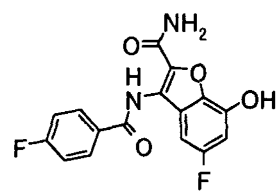 |
| 1-179 | 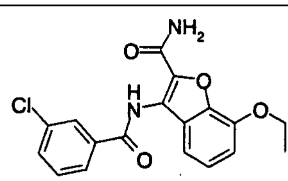 | 1-187 | 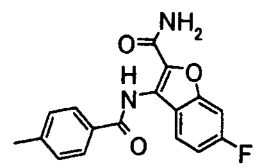 |
| 1-180 | 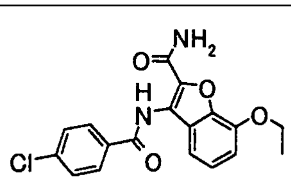 | 1-188 | 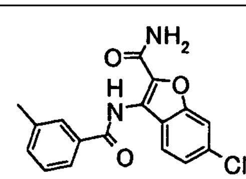 |

(continued)

| Compound No. | Structure | Compound No. | Structure |
|---|---|---|---|
| 1-181 | 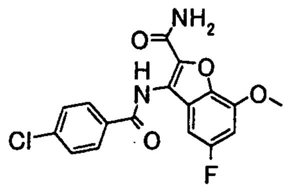 | 1-189 | 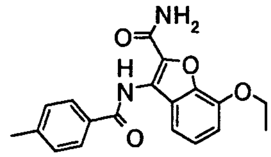 |
| 1-182 | 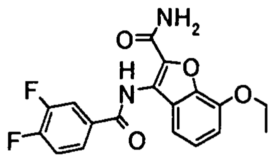 | 1-190 | 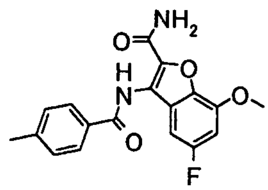 |
| 1-183 | 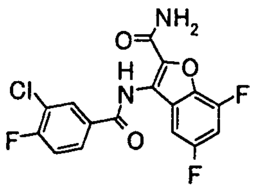 | 1-191 | 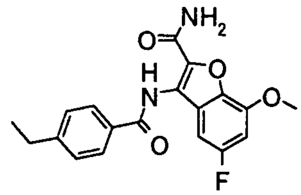 |
| 1-184 | 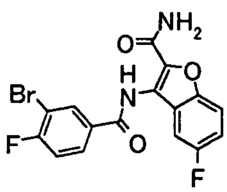 | 1-192 | 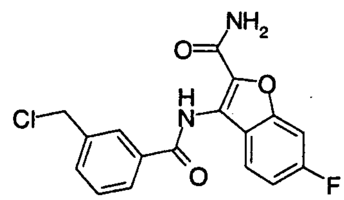 |
| 1-185 | 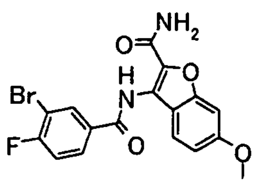 | 1-193 | 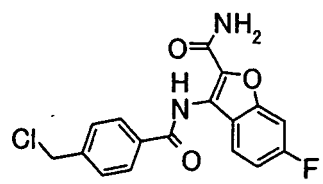 |
| 1-186 | 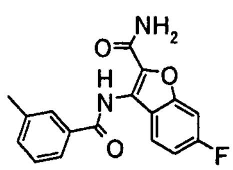 | 1-194 | 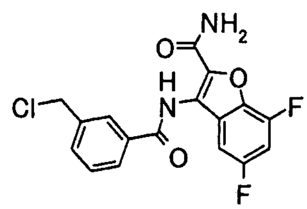 |
| 1-195 | 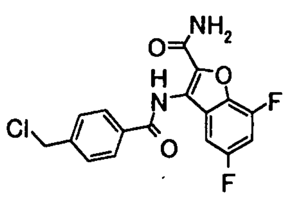 | 1-203 | 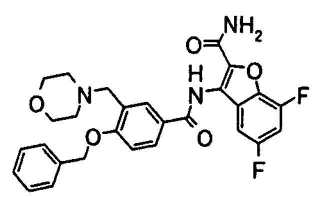 |

(continued)

| Compound No. | Structure | Compound No. | Structure |
|---|---|---|---|
| 1-196 | 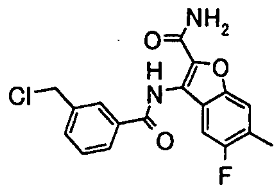 | 1-204 | 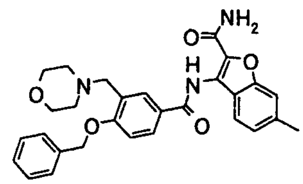 |
| 1-197 | 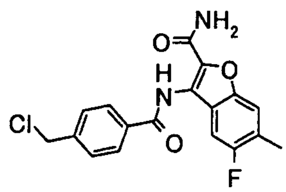 | 1-205 | 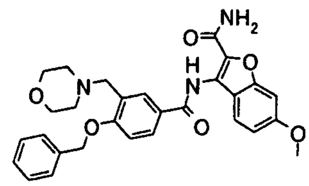 |
| 1-198 | 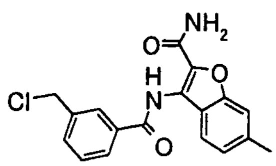 | 1-206 | 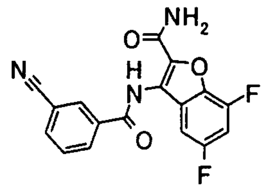 |
| 1-199 | 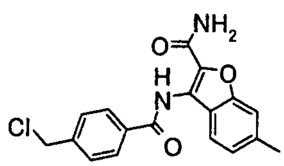 | 1-207 | 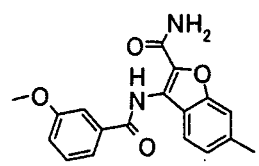 |
| 1-200 | 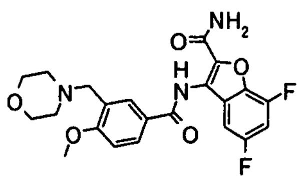 | 1-208 | 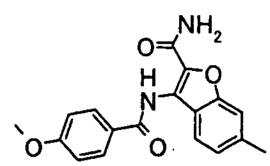 |
| 1-201 | 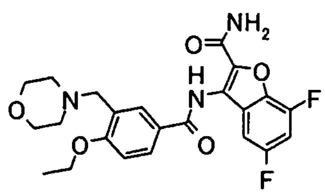 | 1-209 | 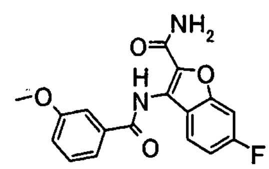 |
| 1-202 | 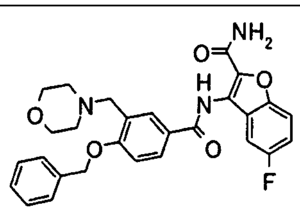 | 1-210 | 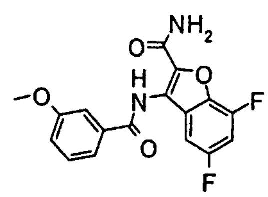 |
| 1-211 | 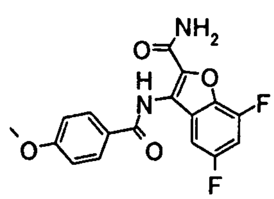 | 1-219 | 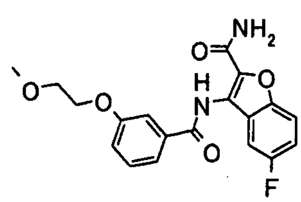 |

(continued)

| Compound No. | Structure | Compound No. | Structure |
|---|---|---|---|
| 1-212 | 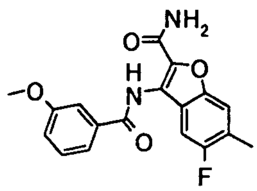 | 1-220 | 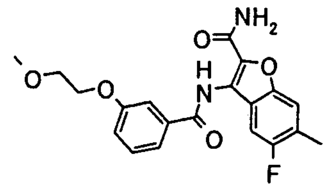 |
| 1-213 | 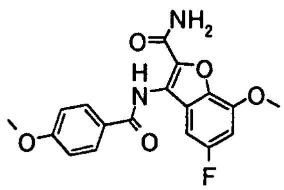 | 1-221 | 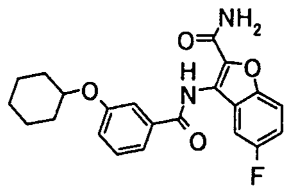 |
| 1-214 | 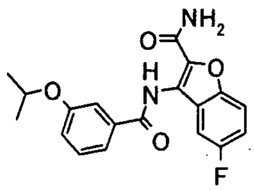 | 1-222 | 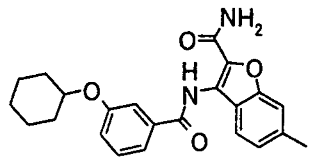 |
| 1-215 | 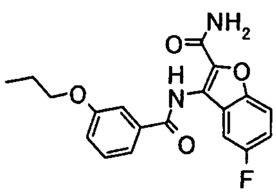 | 1-223 | 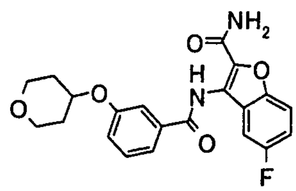 |
| 1-216 | 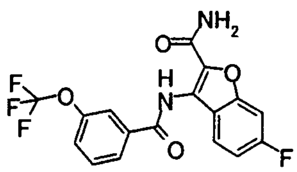 | 1-224 | 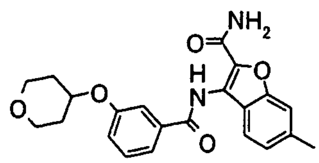 |
| 1-217 | 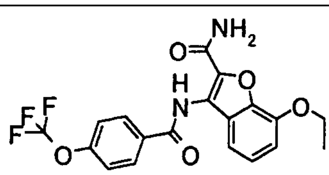 | 1-225 | 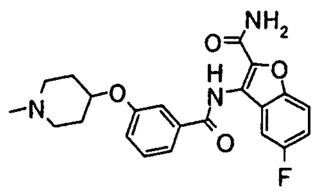 |
| 1-218 | 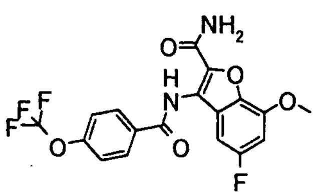 | 1-226 | 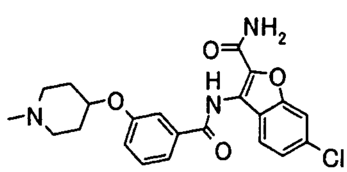 |
| 1-227 | 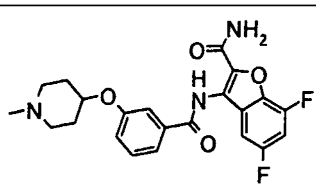 | 1-235 | 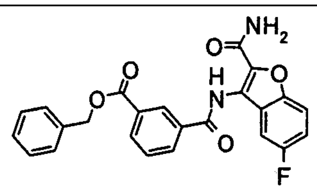 |

(continued)

| Compound No. | Structure | Compound No. | Structure |
|---|---|---|---|
| 1-228 | 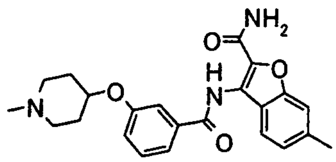 | 1-236 | 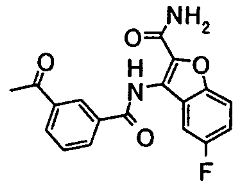 |
| 1-229 | 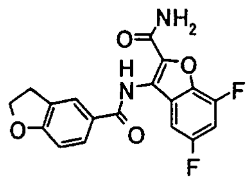 | 1-237 | 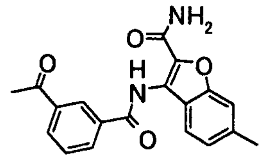 |
| 1-230 | 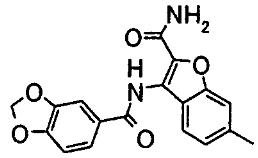 | 1-238 | 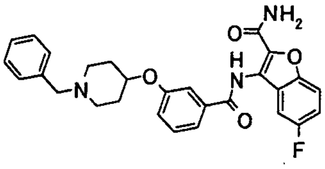 |
| 1-231 | 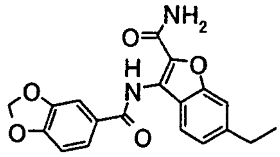 | 1-239 | 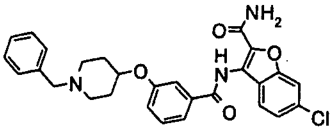 |
| 1-232 | 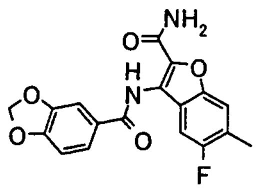 | 1-240 | 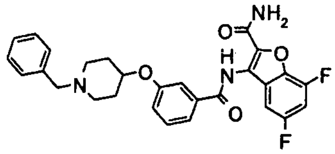 |
| 1-233 | 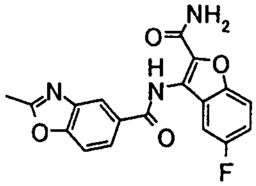 | 1-241 | 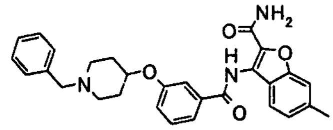 |
| 1-234 | 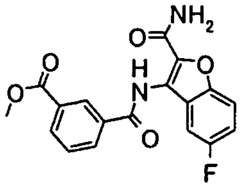 | 1-242 | 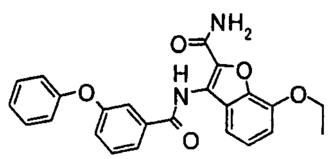 |

(continued)

| Compound No. | Structure | Compound No. | Structure |
|---|---|---|---|
| 1-243 | 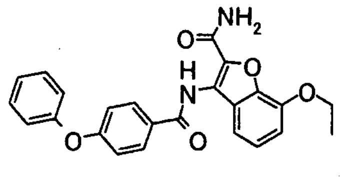 | 1-251 | 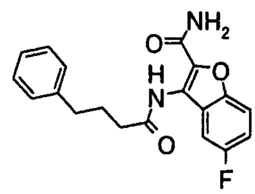 |
| 1-244 | 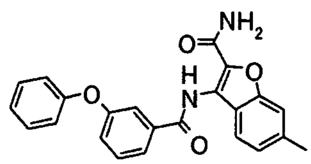 | 1-252 | 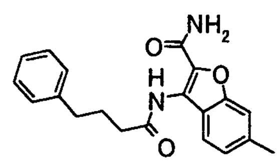 |
| 1-245 | 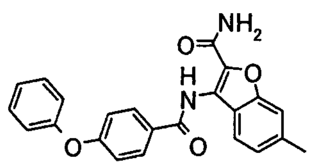 | 1-253 | 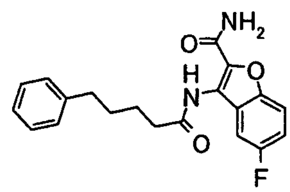 |
| 1-246 | 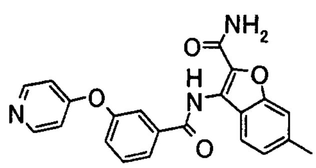 | 1-254 | 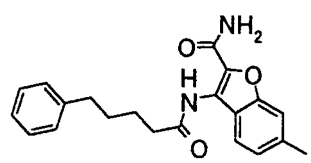 |
| 1-247 | 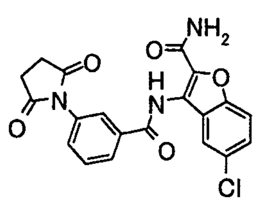 | 1-255 | 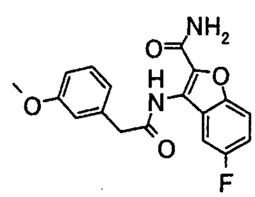 |
| 1-248 | 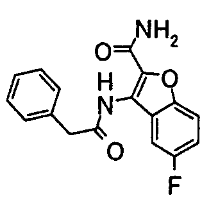 | 1-256 | 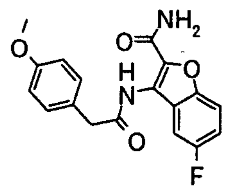 |
| 1-249 | 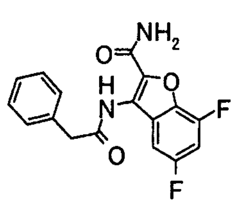 | 1-257 | 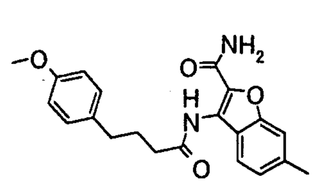 |
| 1-250 | 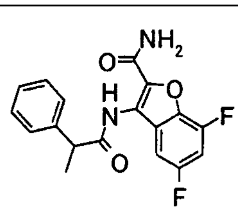 | 1-258 | 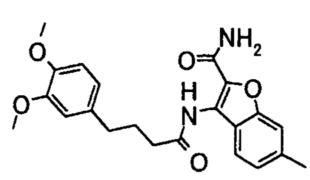 |

(continued)

| Compound No. | Structure | Compound No. | Structure |
|---|---|---|---|
| 1-259 | 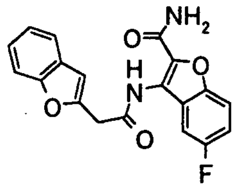 | 1-267 | 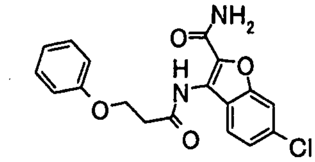 |
| 1-260 | 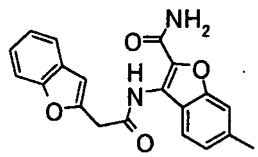 | 1-268 | 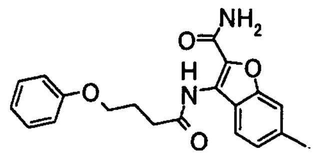 |
| 1-261 | 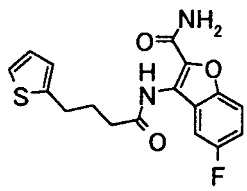 | 1-269 | 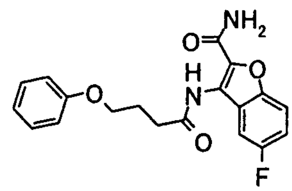 |
| 1-262 | 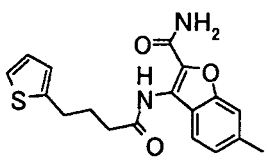 | 1-270 | 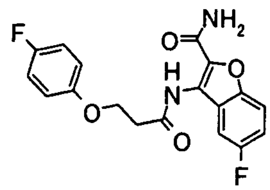 |
| 1-263 | 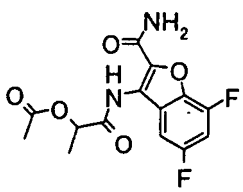 | 1-271 | 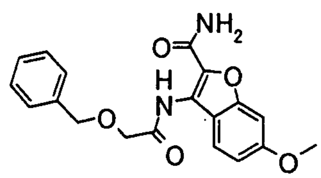 |
| 1-264 | 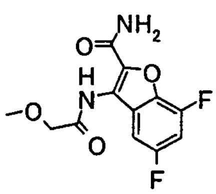 | 1-272 | 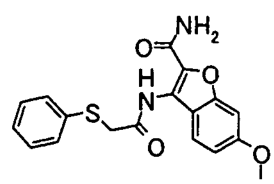 |
| 1-265 | 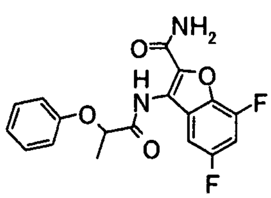 | 1-273 | 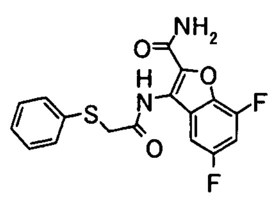 |

(continued)

| Compound No. | Structure | Compound No. | Structure |
|---|---|---|---|
| 1-266 | 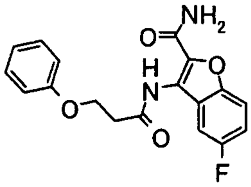 | 1-274 | 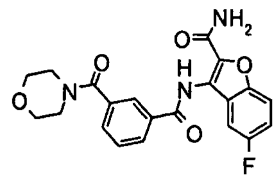 |
| 1-275 | 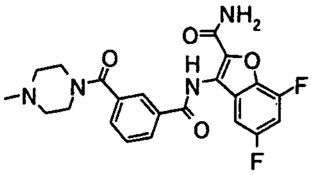 | 1-283 | 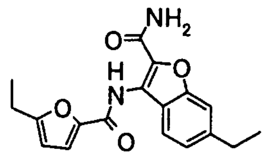 |
| 1-276 | 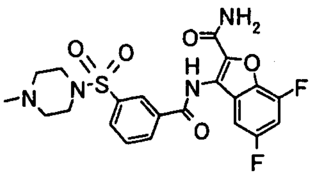 | 1-284 | 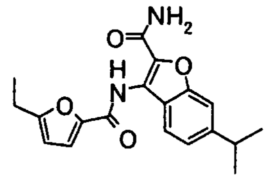 |
| 1-277 | 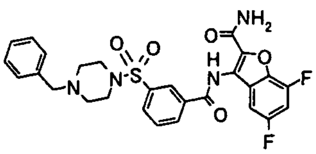 | 1-285 | 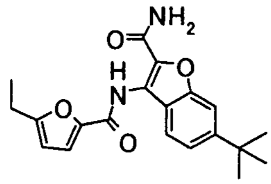 |
| 1-278 | 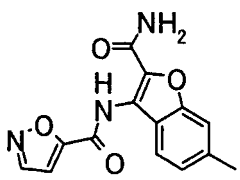 | 1-286 | 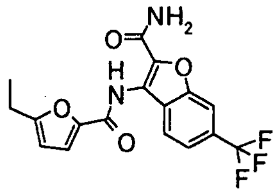 |
| 1-279 | 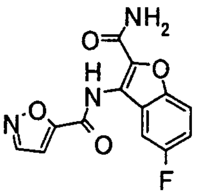 | 1-287 | 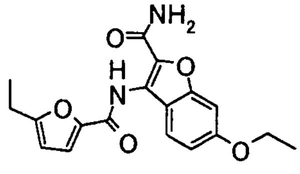 |
| 1-280 | 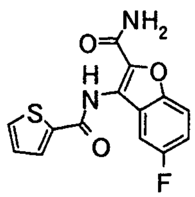 | 1-288 | 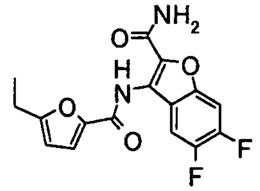 |

(continued)

| Compound No. | Structure | Compound No. | Structure |
|---|---|---|---|
| 1-281 | 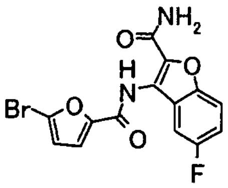 | 1-289 | 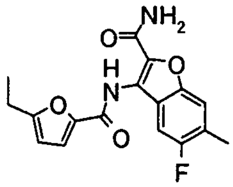 |
| 1-282 | 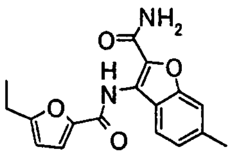 | 1-290 | 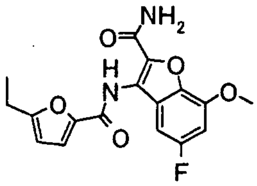 |
| 1-291 | 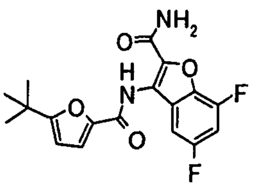 | 1-299 | 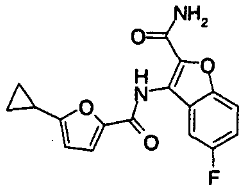 |
| 1-292 | 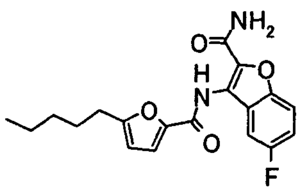 | 1-300 | 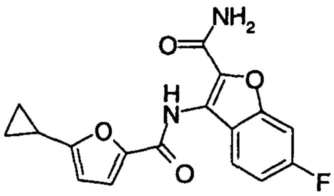 |
| 1-293 | 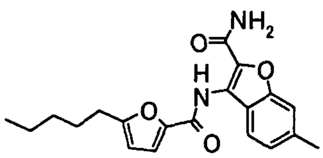 | 1-301 | 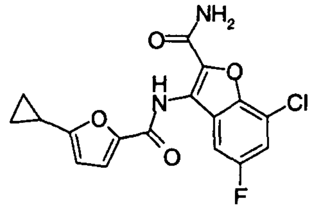 |
| 1-294 | 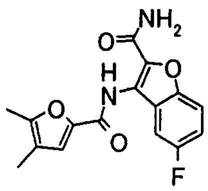 | 1-302 | 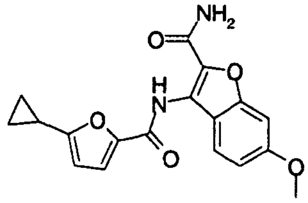 |
| 1-295 | 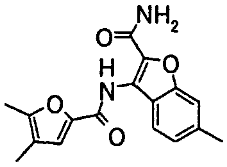 | 1-303 | 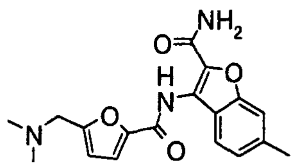 |

(continued)

| Compound No. | Structure | Compound No. | Structure |
|---|---|---|---|
| 1-296 | 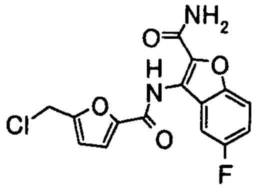 | 1-304 | 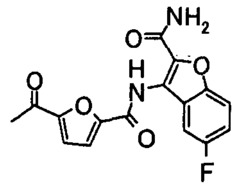 |
| 1-297 | 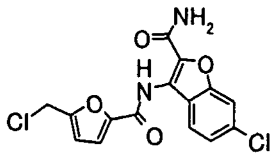 | 1-305 | 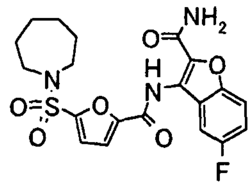 |
| 1-298 | 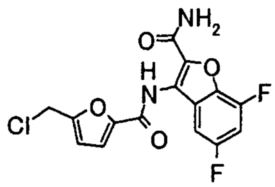 | 1-306 | 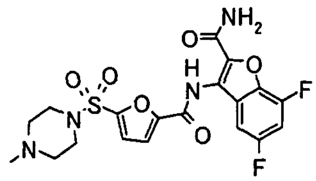 |
| 1-307 | 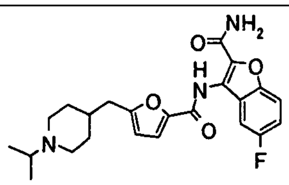 | 1-315 | 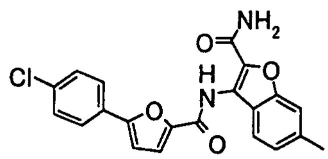 |
| 1-308 | 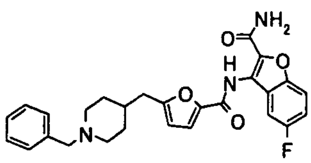 | 1-316 | 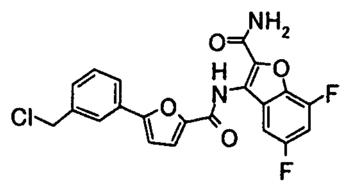 |
| 1-309 | 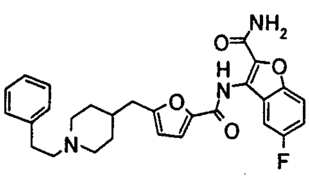 | 1-317 | 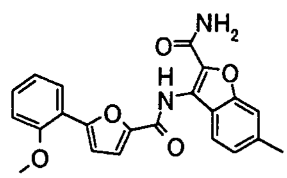 |
| 1-310 | 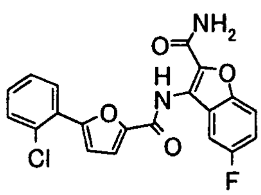 | 1-318 | 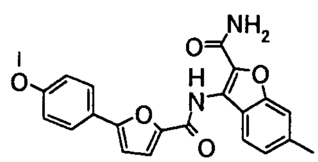 |
| 1-311 | 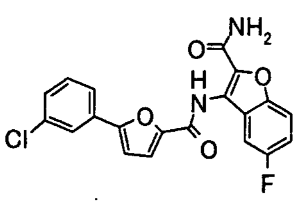 | 1-319 | 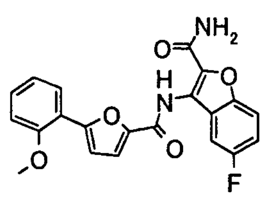 |

(continued)

| Compound No. | Structure | Compound No. | Structure |
|---|---|---|---|
| 1-312 | 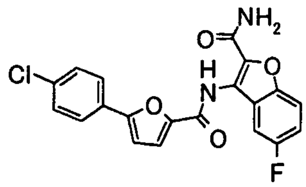 | 1-320 | 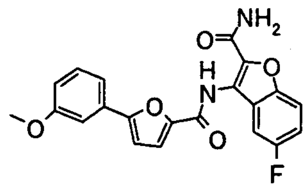 |
| 1-313 | 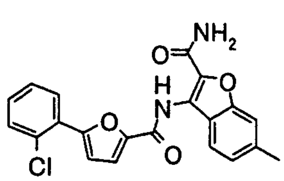 | 1-321 | 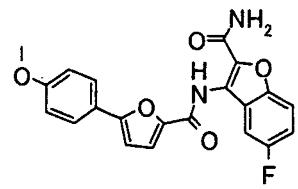 |
| 1-314 | 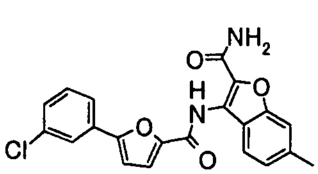 | 1-322 | 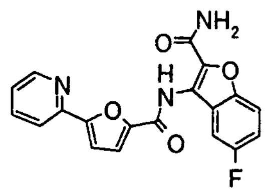 |
| 1-323 | 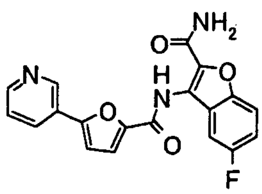 | 1-331 | 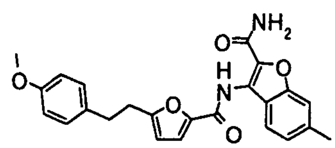 |
| 1-324 | 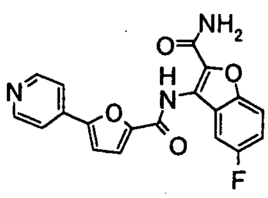 | 1-332 | 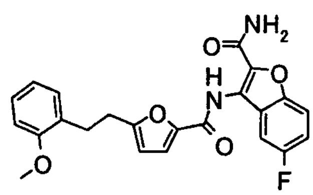 |
| 1-325 | 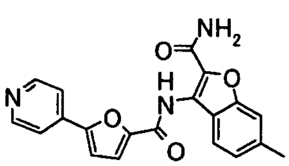 | 1-333 | 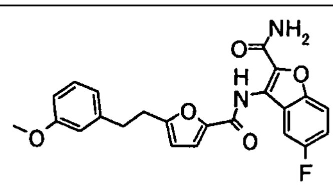 |
| 1-326 | 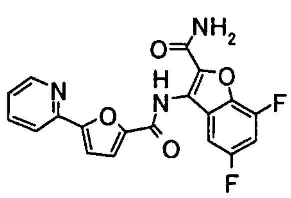 | 1-334 | 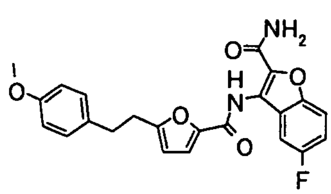 |

(continued)

| Compound No. | Structure | Compound No. | Structure |
|---|---|---|---|
| 1-327 | 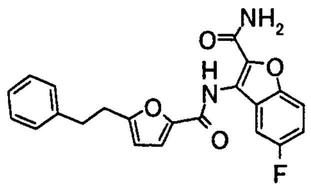 | 1-335 | 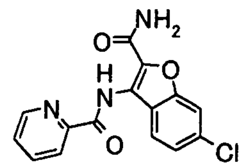 |
| 1-328 | 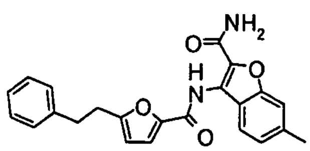 | 1-336 | 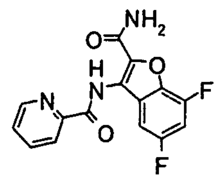 |
| 1-329 | 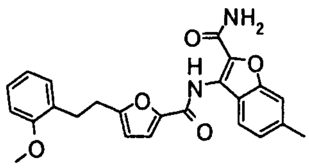 | 1-337 | 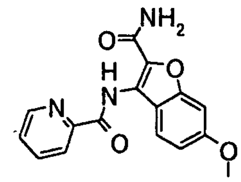 |
| 1-330 | 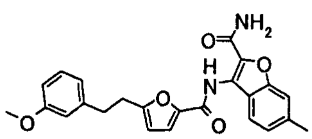 | 1-338 | 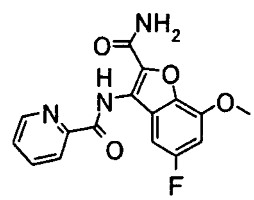 |
| 1-339 | 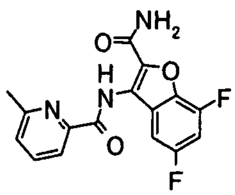 | 1-347 | 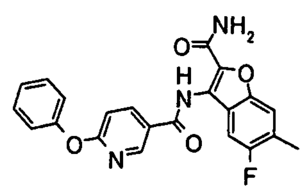 |
| 1-340 | 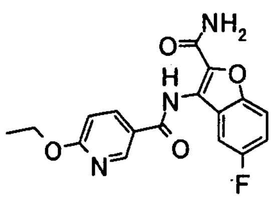 | 1-348 | 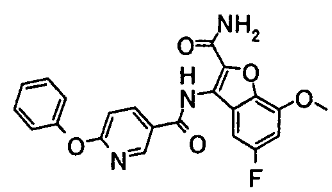 |
| 1-341 | 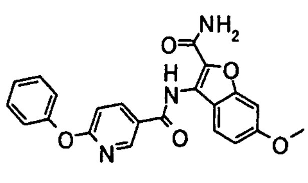 | 1-349 | 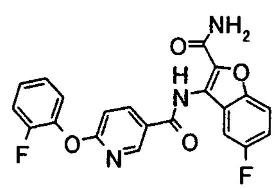 |

(continued)

| Compound No. | Structure | Compound No. | Structure |
|---|---|---|---|
| 1-342 | 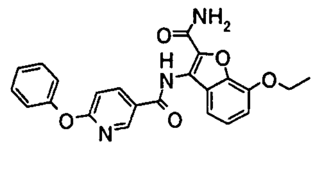 | 1-350 | 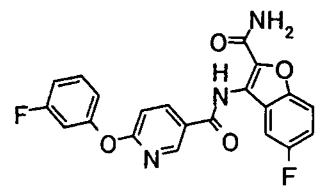 |
| 1-343 | 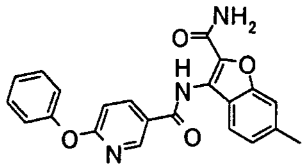 | 1-351 | 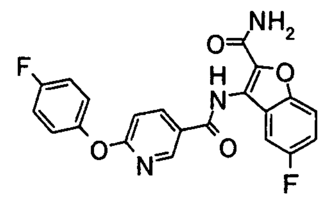 |
| 1-344 | 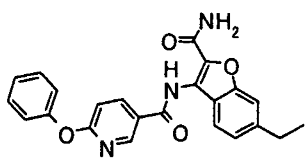 | 1-352 | 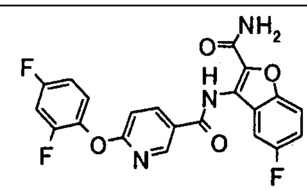 |
| 1-345 | 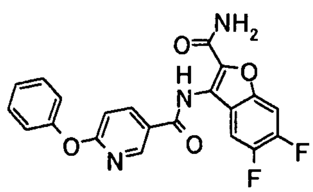 | 1-353 | 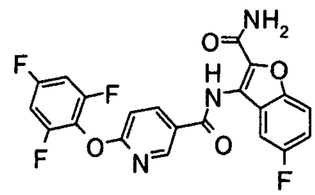 |
| 1-346 | 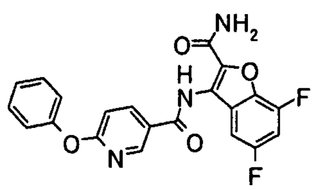 | 1-354 | 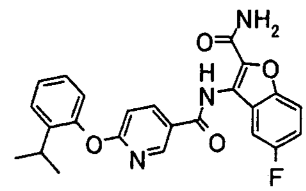 |
| 1-355 | 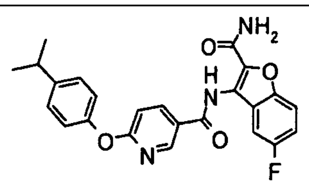 | 1-363 | 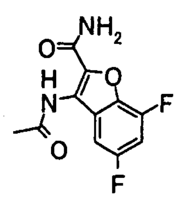 |
| 1-356 | 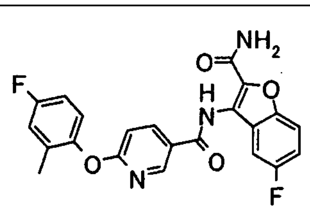 | 1-364 | 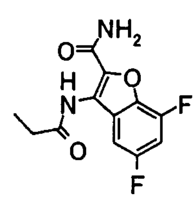 |

(continued)

| Compound No. | Structure | Compound No. | Structure |
| --- | --- | --- | --- |
| 1-357 | 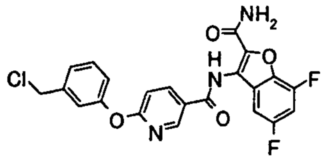 | 1-365 | 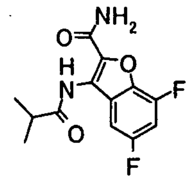 |
| 1-358 | 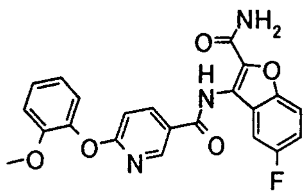 | 1-366 | 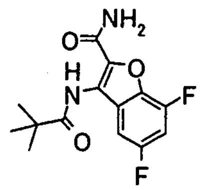 |
| 1-359 | 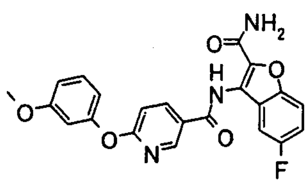 | 1-367 | 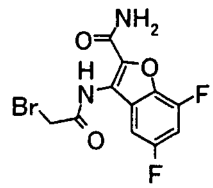 |
| 1-360 | 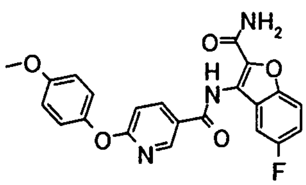 | 1-368 | 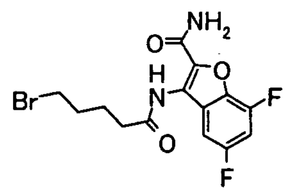 |
| 1-361 | 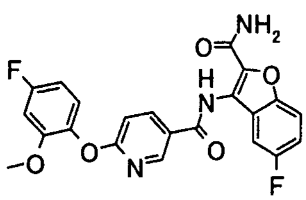 | 1-369 | 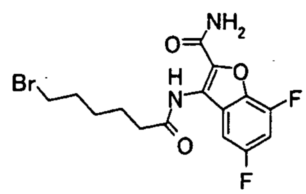 |
| 1-362 | 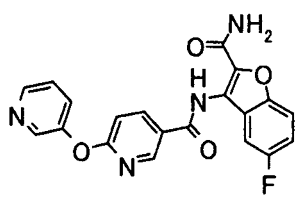 | 1-370 | 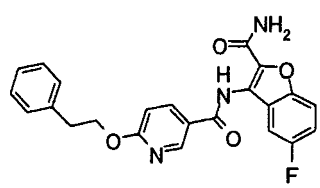 |

The physical data of compounds 1-3 to 1-370 were described below.

Compound 1-3

$^1$H-NMR ($CDCl_3$) δ ppm: 1.65-2.45 (4H, m), 3.95-4.10 (1H, m), 4.15-4.25 (1H, m), 4.50-4.65 (1H, m), 6.47 (1H, brs), 6.70 (1H, brs), 7.30-7.50 (2H, m), 8.55-8.65 (1H, m), 10.89 (1H, brs)

Compound 1-4

$^1$H-NMR (DMSO-$d_6$) δ ppm: 3.87 (3H, s), 7.00 (1H, dd, J=2.2, 8.8Hz), 7.10 (1H, d, J=2.2Hz), 7.55-7.75 (3H, m), 7.76-8.20 (4H, m), 8.26 (1H, d, J=8.8Hz), 11.18 (1H, s)

Compound 1-5

$^1$H-NMR (DMSO-$d_6$) δ ppm: 7.36-7.95 (7H, m), 8. 01 (1H, brs), 8.28 (1H, brs), 10.98 (1H, s)

Compound 1-6

$^1$H-NMR (DMSO-$d_6$) δ ppm: 7.20-7.35 (1H, m), 7.40-7.50 (2H, m), 7.55 (1H, dd, J=2.2, 9.1Hz), 7.95 (1H, brs), 8.00-8.13 (2H, m), 8.15-8.30 (2H, m), 11.03 (1H, s)

Compound 1-7

$^1$H-NMR (DMSO-$d_6$) δ ppm: 7.38-7.90 (6H, m), 7.99 (1H, brs), 8.15-8.32 (2H, m), 11.03 (1H, s)

Compound 1-8

$^1$H-NMR (MeOD-$d_4$) δ ppm: 7.20-7.35 (2H, m), 7.40-7.55 (2H, m), 8.05-8.15 (2H, m), 8.45-8.55 (1H, m)

Compound 1-9

$^1$H-NMR (DMSO-$d_6$) δ ppm: 7.45-7.95 (6H, m), 8.06 (1H, brs), 8.33 (1H, brs), 10.94 (1H, s)

Compound 1-10

$^1$H-NMR (DMSO-$d_6$) δ ppm: 7.48-7.94 (6H, m), 8.07 (1H, brs), 8.18 (1H, brs), 10.91 (1H, s)

Compound 1-11

$^1$H-NMR (DMSO-$d_6$) δ ppm: 2.43 (3H, s), 7.30-7.58 (3H, m), 7.62-7.96 (5H, m), 8.19 (1H, brs), 10.95 (1H, s)

Compound 1-12

$^1$H-NMR (DMSO-$d_6$) δ ppm: 2.54 (3H, s), 7.20-7.38 (2H, m), 7.48-7.72 (2H, m), 7.76-8.00 (4H, m), 8.17 (1H, brs), 10.94 (1H, s)

Compound 1-13

$^1$H-NMR (DMSO-$d_6$) δ ppm: 7.50-7.93 (4H, m), 8.10-8.50 (5H, m), 10.96 (1H, s)

Compound 1-14

$^1$H-NMR (DMSO-$d_6$) δ ppm: 3.81 (3H, s), 7.09-7.19 (1H, m), 7.45-7.72 (4H, m), 7.77-8.00 (3H, m), 8.18 (1H, brs), 10.94 (1H, s)

Compound 1-15

$^1$H-NMR (DMSO-$d_6$) δ ppm: 3. 99 (3H, s), 7.08-7.32 (2H, m), 7.48-7.72 (3H, m), 7.76-8.00 (3H, m), 8.13 (1H, brs), 10.93 (1H, s)

Compound 1-16

$^1$H-NMR (DMSO-$d_6$) δ ppm: 3.99 (3H, s), 7.13 (1H, d, J=7.9Hz), 7.20-7.30 (1H, m), 7.35-7.50 (2H, m), 7.70 (1H, d, J=7.9Hz), 7.89 (1H, brs), 8.00-8.20 (3H, m), 10.91 (1H, s)

Compound 1-17

$^1$H-NMR (DMSO-$d_6$) δ ppm: 3.87 (3H, s), 6.92-7.20 (2H, m), 7.45-8.28 (7H, m), 11.14 (1H, s)

Compound 1-18

$^1$H-NMR (DMSO-$d_6$) δ ppm: 1.43 (3H, t, J=6.9Hz), 4.30 (2H, q, J=6.9Hz), 7.05-7.15 (1H, m), 7.20-7.30 (1H, m), 7.35-7.50 (2H, m), 7.66 (1H, dd, J=0.9, 8.2Hz), 7.90 (1H, brs), 8.00-8.20 (3H, m), 10.89 (1H, s)

Compound 1-19

$^1$H-NMR (DMSO-$d_6$) δ ppm: 5.24 (2H, s), 7.00-7.20 (2H, m), 7.30-7.95 (10H, m), 8.11 (1H, brs), 8.17 (1H, d, J=9.0Hz), 11.14 (1H, s)

Compound 1-20

$^1$H-NMR (DMSO-$d_6$) δ ppm: 7.49-8.24 (10H, m), 8.37 (1H, brs), 8.50 (1H, d, J=8.2Hz), 11.03 (1H, s)

Compound 1-21

$^1$H-NMR (DMSO-$d_6$) δ ppm: 1.14 (6H, t, J=7.1Hz), 3.43 (4H, q, J=7.1Hz), 6.58-6.64 (1H, m), 6.76-6.86 (1H, m), 7.48-7.92 (6H, m), 8.11 (1H, d, J=9.2Hz), 11.27 (1H, s)

Compound 1-22

$^1$H-NMR ($CDCl_3$) δ ppm: 5.64 (1H, brs), 6.43 (1H, brs), 7.20-7.65 (4H, m), 7.85-7.95 (1H, m), 8.00-8.10 (1H, m), 8.30-8.40 (1H, m), 10.88 (1H, brs)

Compound 1-23

$^1$H-NMR ($CDCl_3$) δ ppm: 5.99 (1H, brs), 6.52 (1H, brs), 7.15-7.30 (1H, m), 7.35-7.55 (3H, m), 7.95-8.05 (2H, m), 8.30-8.40 (1H, m), 10.93 (1H, brs)

Compound 1-24

$^1$H-NMR (DMSO-$d_6$) δ ppm: 7.57 (1H, dd, J=2.2, 8.8Hz), 7.60-7.70 (2H, m), 7.72-7.80 (1H, m), 7.90-8.10 (3H, m), 8.19 (1H, d, J=2.2Hz), 8.27 (1H, brs), 10.99 (1H, s)

Compound 1-25

$^1$H-NMR (DMSO-$d_6$) δ ppm: 7.36-7.48 (1H, m), 7.64-7.80 (3H, m), 7.92-8.08 (3H, m), 8.16-8.32 (2H, m), 11.04 (1H, s)

Compound 1-26

$^1$H-NMR (DMSO-$d_6$) δ ppm: 7.52-7.80 (4H, m), 7.96-8.12 (3H, m), 8.33 (1H, brs), 10.95 (1H, s)

Compound 1-27

$^1$H-NMR (DMSO-$d_6$) δ ppm: 3.87 (3H, s), 6.95-7.05 (1H, m), 7.07-7.15 (1H, m), 7.55-8.25 (7H, m), 11.13 (1H, s)

Compound 1-28

$^1$H-NMR (DMSO-$d_6$) δ ppm: 3.87 (3H, s), 7.00 (1H, dd, J=2.2, 8.8Hz), 7.11 (1H, d, J=2.2Hz), 7.65-7.75 (2H, m), 7.76-8.25 (5H, m), 11.15 (1H, s)

Compound 1-29

$^1$H-NMR (DMSO-$d_6$) δ ppm: 7.35-7.45 (1H, m), 7.50-7.75 (2H, m), 7.80-8.05 (4H, m), 8.15-8.30 (2H, m), 10.96 (1H, s)

Compound 1-30

$^1$H-NMR (DMSO-$d_6$) δ ppm: 3.87 (3H, s), 7.00 (1H, dd, J=2.2, 8.8Hz), 7.11 (1H, d, J=2.2Hz), 7.50-7.65 (1H, m), 7.75-8.25 (6H, m), 11.12 (1H, s)

Compound 1-31

$^1$H-NMR (DMSO-$d_6$) δ ppm: 7.35-7.45 (1H, m), 7.60-7.75 (2H, m), 7.80-8.10 (4H, m), 8.24 (1H, brs), 10.91 (1H, s)

Compound 1-32

$^1$H-NMR (DMSO-$d_6$) δ ppm: 3.87 (3H, s), 6.99 (1H, dd, J=2.2, 8.8Hz), 7.11 (1H, d, J=2.2Hz), 7.60-8.20 (6H, m), 11.06 (1H, s)

Compound 1-33

$^1$H-NMR (DMSO-$d_6$) δ ppm: 7.35-7.45 (1H, m), 7.60-7.70 (2H, m), 7.75-7.85 (1H, m), 7.96 (1H, brs), 8.00-8.10 (1H, m), 8.15-8.30 (2H, m), 10.90 (1H, brs)

Compound 1-34

$^1$H-NMR (DMSO-$d_6$) δ ppm: 7.40-7.45 (1H, m), 7.60-7.70 (1H, m), 7.75-7.80 (1H, m), 7.97 (1H, brs), 8.00-8.30 (4H, m), 10.95 (1H, brs)

Compound 1-35

$^1$H-NMR (DMSO-$d_6$) δ ppm: 3.87 (3H, s), 6.95-7.05 (1H, m), 7.10-7.15 (1H, m), 7.60-7.70 (1H, m), 7.85 (1H, brs), 7.95-8.25 (4H, m), 11.06 (1H, brs)

Compound 1-36

$^1$H-NMR (DMSO-$d_6$) δ ppm: 2.43 (3H, s), 7.35-7.55 (3H, m), 7.60-7.70 (1H, m), 7.75-7.90 (2H, m), 7.95-8.10 (2H, m), 8.27 (1H, brs), 11.00 (1H, s)

Compound 1-37

$^1$H-NMR (DMSO-$d_6$) δ ppm: 2.42 (3H, s), 7.35-7.45 (3H, m), 7.66 (1H, m), 7.91 (2H, d, J=8.2Hz), 7.95-8.10 (2H, m), 8.27 (1H, brs), 11.01 (1H, s)

Compound 1-38

$^1$H-NMR (DMSO-$d_6$) δ ppm: 2.42 (3H, s), 7.35-7.70 (4H, m), 7.85-8.00 (2H, m), 8.03 (1H, brs), 8.30-8.40 (1H, m), 8.32 (1H, brs), 11.04 (1H, brs)

Compound 1-39

$^1$H-NMR (DMSO-$d_6$) δ ppm: 2.42 (3H, s), 3.87 (3H, s), 7.00 (1H, dd, J=2.2, 9.1Hz), 7.10 (1H, d, J=2.2Hz), 7.45-7.55 (2H, m), 7.74-8.20 (4H, m), 8.25 (1H, d, J=8.8Hz), 11.15 (1H, s)

Compound 1-40

$^1$H-NMR (DMSO-$d_6$) δ ppm: 2.41 (3H, s), 3.87 (3H, s), 6.99 (1H, dd, J=2.5, 9.1Hz), 7.10 (1H, d, J=2.2Hz), 7.41 (2H, d, J=7.9Hz), 7.70-8.20 (4H, m), 8.27 (1H, d, J=9.1Hz), 11.15 (1H, s)

Compound 1-41

$^1$H-NMR (DMSO-$d_6$) δ ppm: 1.43 (3H, t, J=6.9Hz), 2.42 (3H, s), 4.29 (2H, d, J=7.3Hz), 7.10-7.15 (1H, m), 7.20-7.30 (1H, m), 7.40-7.55 (2H, m), 7.73 (1H, dd, J=0.9, 8.2Hz), 7.76-7.85 (2H, m), 7.90 (1H, brs), 8.08 (1H, brs),

10.93 (1H, s)

Compound 1-42

$^1$H-NMR (DMSO-$d_6$) δ ppm: 1.23 (3H, t, J=7.6Hz), 2.72 (2H, q, J=7.6Hz), 7.35-7.50 (3H, m), 7.66 (1H, m), 7.85-8.10 (4H, m), 8.27 (1H, brs), 11.01 (1H, s)

Compound 1-43

$^1$H-NMR (DMSO-$d_6$) δ ppm: 1.23 (3H, t, J=7.6Hz), 2.72 (2H, q, J=7.6Hz), 7.45 (2H, d, J=8.2Hz), 7.57 (1H, dd, J=2.5, 9.1Hz), 7.67 (1H, d, J=8.8Hz), 7.93 (2H, d, J=8.2Hz), 8.02 (1H, brs), 8.29 (1H, brs), 8.35 (1H, d, J=2.2Hz), 11.03 (1H, s)

Compound 1-44

$^1$H-NMR (DMSO-$d_6$) δ ppm: 1.23 (3H, t, J=7.6Hz), 2.71 (2H, q, J=7.6Hz), 3.87 (3H, s), 6.99 (1H, dd, J=2.2, 9.1Hz), 7.10 (1H, d, J=2.2Hz), 7.44 (2H, d, J=8.2Hz), 7.70-8.20 (4H, m), 8.27 (1H, d, J=8.8Hz), 11.15 (1H, s)

Compound 1-45

$^1$H-NMR (DMSO-$d_6$) δ ppm: 0.92 (3H, t, J=7.3Hz), 1.55-1.75 (2H, m), 2.66 (2H, t, J=7.3Hz), 7.35-7.50 (3H, m), 7.66 (1H, dd, J=4.1, 9.1Hz), 7.85-8.10 (4H, m), 8.27 (1H, brs), 11.00 (1H, s)

Compound 1-46

$^1$H-NMR (DMSO-$d_6$) δ ppm: 0.92 (3H, t, J=7.3Hz), 1.55-1.70 (2H, m), 2.66 (2H, t, J=7.3Hz), 3.87 (3H, s), 6.99 (1H, dd, J=2.2, 8.8Hz), 7.10 (1H, d, J=2.2Hz), 7.42 (2H, d, J=8.2Hz), 7.70-8.20 (4H, m), 8.27 (1H, d, J=8.8Hz), 11.14 (1H, s)

Compound 1-47

$^1$H-NMR (DMSO-$d_6$) δ ppm: 0.91 (3H, t, J=7.3Hz), 1.25-1.40 (2H, m), 1.55-1.65 (2H, m), 2.69 (2H, t, J=7.6Hz), 7.35-7.50 (3H, m), 7.60-7.70 (1H, m), 7.85-8.10 (4H, m), 8.26 (1H, brs), 11.00 (1H, s)

Compound 1-48

$^1$H-NMR (DMSO-$d_6$) δ ppm: 7.35-7.45 (1H, m), 7.60-7.75 (1H, m), 7.80-7.90 (2H, m), 7.95-8.10 (2H, m), 8.20-8.40 (3H, m), 11.07 (1H, s)

Compound 1-49

$^1$H-NMR (DMSO-$d_6$) δ ppm: 3.87 (3H, s), 7.00 (1H, dd, J=2.2, 8.8Hz), 7.12 (1H, d, J=2.2Hz), 7.70-8.20 (5H, m), 8.25-8.35 (2H, m), 11.23 (1H, s)

Compound 1-50

$^1$H-NMR (DMSO-$d_6$) δ ppm: 4.87 (2H, s), 7.30-7.50 (1H, m), 7.60-7.75 (3H, m), 7.90-8.10 (4H, m), 8.27 (1H, brs), 11.02 (1H, s)

Compound 1-51

$^1$H-NMR ($CDCl_3$) δ ppm: 4.68 (2H, s), 5.61 (1H, brs), 6.41 (1H, brs), 7.20-7.30 (1H, m), 7.35-7.45 (1H, m), 7.50-7.70 (2H, m), 7.95-8.10 (2H, m), 8.35-8.45 (1H, m), 10.89 (1H, brs)

Compound 1-52

$^1$H-NMR (DMSO-$d_6$) δ ppm: 4.87 (2H, s), 7.40-7.50 (1H, m), 7.60-7.80 (3H, m), 7.95-8.05 (3H, m), 8.20-8.30 (2H, m), 11.05 (1H, brs)

Compound 1-53

$^1$H-NMR (DMSO-$d_6$) δ ppm: 4.89(2H, s), 7.40-7.50 (1H, m), 7.60-7.65 (1H, m), 7.70-7.80 (2H, m), 7.95-8.15 (3H, m), 8.20-8.30 (2H, m), 11.06 (1H, brs)

Compound 1-54

1H-NMR (DMSO-$d_6$) δ ppm: 4.87 (2H, s), 7.57 (1H, dd, J=2.2, 8.8Hz), 7.60-7.75 (3H, m), 7.95-8.10 (3H, m), 8.25-8.35 (2H, m), 11.04 (1H, s)

Compound 1-55

$^1$H-NMR (DMSO-$d_6$) δ ppm: 2.44 (3H, s), 4.87 (2H, s), 7.30-7.40 (1H, m), 7.45-7.55 (1H, m), 7.60-7.70 (2H, m), 7.90 (1H, brs), 7.95-8.05 (3H, m), 8.17 (1H, brs), 10.98 (1H, brs)

Compound 1-56

$^1$H-NMR (DMSO-$d_6$) δ ppm: 2.44 (3H, s), 4.89 (2H, s), 7.30-7.40 (1H, m), 7.45-7.80 (3H, m), 7.90 (1H, brs), 7.95-8.15 (3H, m), 8.17 (1H, brs), 10.97 (1H, brs)

Compound 1-57

$^1$H-NMR (DMSO-$d_6$) δ ppm: 3.87 (3H, s), 4.89 (2H, s), 7.00 (1H, dd, J=2.2, 8.8Hz), 7.11 (1H, d, J=2.2Hz), 7.55-8.15 (6H, m), 8.21 (1H, d, J=8.8Hz), 11.17 (1H, s)

Compound 1-58

$^1$H-NMR (DMSO-$d_6$) δ ppm: 3.87 (3H, s), 4.87 (2H, s), 7.00 (1H, dd, J=2.2, 9.1Hz), 7.11 (1H, d, J=2.2Hz), 7.66 (2H, d, J=8.5Hz), 7.75-8.15 (4H, m), 8.24 (1H, d, J=8.8Hz), 11.17 (1H, s)

Compound 1-59

$^1$H-NMR (DMSO-$d_6$) δ ppm: 1.43 (3H, t, J=6.9Hz), 4.30 (2H, q, J=6.9Hz), 4.89 (2H, s), 7.10-7.15 (1H, m), 7.20-7.30 (1H, m), 7.55-7.75 (3H, m), 7.91 (1H, brs), 7.95-8.00 (1H, m), 8.05-8.15 (2H, m), 10.95 (1H, s)

Compound 1-60

$^1$H-NMR (DMSO-$d_6$) δ ppm: 7.35-7.45 (1H, m), 7.60-7.85 (3H, m), 7.97 (1H, brs), 8.23 (1H, brs), 8.30-8.45 (2H, m), 11.01 (1H, s)

Compound 1-61

$^1$H-NMR (DMSO-$d_6$) δ ppm: 3.87 (3H, s), 7.00 (1H, dd, J=2.2, 8.8Hz), 7.12 (1H, d, J=2.2Hz), 7.70-7.95 (2H, m), 8.05 (2H, d, J=8.8Hz), 8.30-8.45 (2H, m), 11.15 (1H, s)

Compound 1-62

$^1$H-NMR (DMSO-$d_6$) δ ppm: 3.87 (3H, s), 7.00 (1H, dd, J=2.2, 8.8Hz), 7.12 (1H, d, J=2.2Hz), 7.75-8.20 (5H, m), 8.29 (1H, d, J=8.2Hz), 8.42 (1H, s), 11.11 (1H, s)

Compound 1-63

$^1$H-NMR (DMSO-$d_6$) δ ppm: 2.83 (4H, s), 7.35-7.45 (1H, m), 7.55-7.60 (1H, m), 7.65-7.80 (2H, m), 7.90-8.10 (4H, m), 8.27 (1H, brs), 11.02 (1H, s)

Compound 1-64

$^1$H-NMR (DMSO-$d_6$) δ ppm: 2.70-2.95 (4H, m), 3.87 (3H, s), 6.90-7.30 (2H, m), 7.40-8.30 (7H, m), 11.16 (1H, s)

Compound 1-65

$^1$H-NMR (DMSO-$d_6$) δ ppm: 2.23 (3H, s), 2.45 (4H, t, J=4.7Hz), 3.25-3.40 (4H, m), 7.08 (2H, d, J=8.8Hz), 7.20-7.30 (1H, m), 7.52 (1H, dd, J=2.2, 8.8Hz), 7.84 (2H, d, J=8.8Hz), 7.94 (1H, brs), 8.21 (1H, brs), 8.35-8.45 (1H, m), 10.99 (1H, s)

Compound 1-66

$^1$H-NMR (DMSO-$d_6$) δ ppm: 3.20-3.40 (4H, m), 3.65-3.85 (4H, m), 7.10 (2H, d, J=9.1Hz), 7.20-7.30 (1H, m), 7.53 (1H, dd, J=2.2, 8.8Hz), 7.80-8.00 (3H, m), 8.22 (1H, brs), 8.35-8.45 (1H, m), 11.00 (1H, s)

Compound 1-67

$^1$H-NMR ($CDCl_3$) δ ppm: 1.40-1.55 (9H, m), 4.35-4.50 (2H, m), 4.98 (1H, brs), 5.92 (1H, brs), 6.43 (1H, brs), 7.30-7.50 (4H, m), 7.95-8.05 (2H, m), 8.65-8.75 (1H, m), 10.85 (1H, brs)

Compound 1-68

$^1$H-NMR (DMSO-$d_6$) δ ppm: 3.86 (3H, s), 7.20-7.30 (1H, m), 7.35-7.45 (1H, m), 7.48-7.60 (3H, m), 7.67 (1H, dd, J=4.4, 9.1Hz), 7.90-8.10 (2H, m), 8.27 (1H, brs), 11.02 (1H, s)

Compound 1-69

$^1$H-NMR (DMSO-$d_6$) δ ppm: 3.87 (3H, s), 7.10-7.20 (2H, m), 7.35-7.45 (1H, m), 7.65 (1H, dd, J=4.1, 9.1Hz), 7.90-8.10 (4H, m), 8.26 (1H, brs), 10.95 (1H, s)

Compound 1-70

$^1$H-NMR (DMSO-$d_6$) δ ppm: 3.86 (3H, s), 7.24 (1H, dd, J=2.5, 7.9Hz), 7.43 (1H, dd, J=1.9, 8.8Hz), 7.48-7.60 (3H, m), 7.76 (1H, d, J=1.6Hz), 8.02 (1H, brs), 8.20-8.35 (2H, m), 11.06 (1H, s)

Compound 1-71

$^1$H-NMR (DMSO-$d_6$) δ ppm: 3.86 (3H, s), 3.87 (3H, s), 6.99 (1H, dd, J=2.2, 8.8Hz), 7.10 (1H, d, J=2.2Hz), 7.20-7.30 (1H, m), 7.45-7.60 (3H, m), 7.87 (1H, brs), 8.06 (1H, brs), 8.25 (1H, d, J=9.1Hz), 11.17 (1H, s)

Compound 1-72

$^1$H-NMR (DMSO-$d_6$) δ ppm: 3.863 (3H, s), 3.866 (3H, s), 6.98 (1H, dd, J=2.2, 8.8Hz), 7.09 (1H, d, J=2.2Hz), 7.15-7.20 (2H, m), 7.75-8.15 (4H, m), 8.27 (1H, d, J=8.8Hz), 11.09 (1H, s)

Compound 1-73

$^1$H-NMR (DMSO-$d_6$) δ ppm: 1.43 (3H, t, J=6.9Hz), 3.86 (3H, s), 4.30 (2H, q, J=6.9Hz), 7.12 (1H, d, J=7.9Hz), 7.20-7.30 (2H, m), 7.45-7.65 (3H, m), 7.73 (1H, d, J=8.2Hz), 7.92 (1H, brs), 8.09 (1H, brs), 10.95 (1H, s)

Compound 1-74

$^1$H-NMR (DMSO-$d_6$) δ ppm: 3.87 (3H, s), 7.10-7.20 (2H, m), 7.35-7.60 (3H, m), 7.69 (1H, dd, J=1.6, 8.5Hz), 7.75-7.85 (3H, m), 7.90-8.05 (3H, m), 8.21 (1H, brs), 8.39 (1H, d, J=8.5Hz), 11.03 (1H, s)

Compound 1-75

$^1$H-NMR (DMSO-$d_6$) δ ppm: 7.35-7.45 (1H, m), 7.60-7.80 (3H, m), 7.85-8.10 (4H, m), 8.26 (1H, brs), 11.03 (1H, s)

Compound 1-76

$^1$H-NMR (DMSO-$d_6$) δ ppm: 7.35-7.45 (1H, m), 7. 61 (2H, d, J=7.9Hz), 7.67 (1H, dd, J=4.1, 9.1Hz), 7.85-8.05

(2H, m), 8.10-8.35 (3H, m), 11.00 (1H, s)

Compound 1-77

$^1$H-NMR (DMSO-$d_6$) δ ppm: 3.87 (3H, s), 7.00 (1H, dd, J=2.2, 8.8Hz), 7.12 (1H, d, J=2.2Hz), 7.60-8.25 (7H, m), 11.20 (1H, s)

Compound 1-78

$^1$H-NMR (DMSO-$d_6$) δ ppm: 3.87 (3H, s), 7.00 (1H, dd, J=2.2, 8.8Hz), 7.11 (1H, d, J=2.2Hz), 7.61 (2H, d, J=8.5Hz), 7.70-8.30 (5H, m), 11.15 (1H, s)

Compound 1-79

$^1$H-NMR (DMSO-$d_6$) δ ppm: 1.43 (3H, t, J=7.3Hz), 4.30 (2H, q, J=7.3Hz), 7.12 (1H, d, J=7.3Hz), 7.20-7.30 (1H, m), 7.62 (1H, dd, J=0.9, 8.2Hz), 7.68 (1H, d, J=8.2Hz), 7.72-7.80 (1H, m), 7.91 (1H, brs), 7.95 (1H, brs), 8.00-8.15 (2H, m), 10.97 (1H, s)

Compound 1-80

$^1$H-NMR (DMSO-$d_6$) δ ppm: 7.05-7.50 (7H, m), 7.55-7.80 (4H, m), 7.90-7.95 (1H, m), 7.98 (1H, brs), 8.26 (1H, brs), 11.00 (1H, brs)

Compound 1-81

$^1$H-NMR (DMSO-$d_6$) δ ppm: 7.05-7.30 (5H, m), 7.35-7.55 (3H, m), 7.60-7.70 (1H, m), 7.90-8.10 (4H, m), 8.26 (1H, brs), 10.96 (1H, s)

Compound 1-82

$^1$H-NMR (DMSO-$d_6$) δ ppm: 7.05-7.35 (4H, m), 7.40-7.80 (7H, m), 8.01 (1H, brs), 8.20-8.30 (1H, m), 8.28 (1H, brs), 11.02 (1H, brs)

Compound 1-83

$^1$H-NMR (DMSO-$d_6$) δ ppm: 7.05-7.35 (4H, m), 7.40-7.65 (5H, m), 7.70-7.80 (2H, m), 8.05 (1H, brs), 8.33 (1H, brs), 10.95 (1H, brs)

Compound 1-84

$^1$H-NMR (DMSO-$d_6$) δ ppm: 2.42 (3H, s), 7.05-7.65 (10H, m), 7.75-7.80 (1H, m), 7.88 (1H, brs), 7.90-8.00 (1H, m), 8.16 (1H, brs), 10.97 (1H, brs)

Compound 1-85

$^1$H-NMR ($CDCl_3$) δ ppm: 3.88 (3H, s), 5.69 (1H, brs), 6.26 (1H, brs), 6.85-7.50 (9H, m), 7.65-7.80 (2H, m), 8.50-8.60 (1H, m), 10.95 (1H, brs)

Compound 1-86

$^1$H-NMR (DMSO-$d_6$) δ ppm: 7.10-7.30 (5H, m), 7.35-7.55 (5H, m), 7.65-7.85 (4H, m), 7.97 (1H, brs), 8.00-8.10 (2H, m), 8.22 (1H, brs), 8.35 (1H, d, J=8.2Hz), 11.04 (1H, s)

Compound 1-87

$^1$H-NMR (DMSO-$d_6$) δ ppm: 7.35-7.60 (4H, m), 7.65-7.85 (4H, m), 7.90-8.10 (4H, m), 8.20-8.35 (2H, m), 11.11 (1H, s)

Compound 1-88

$^1$H-NMR ($CDCl_3$+MeOD-$d_4$) δ ppm: 1.60-1.90 (4H, m), 3.00-3.50 (4H, m), 7.25-7.50 (2H, m), 7.80-8.20 (4H, m), 8.45-8.60 (1H, m)

Compound 1-89

$^1$H-NMR (DMSO-$d_6$) δ ppm: 0.75-0.95 (6H, m), 1.40-1.60 (4H, m), 3.00-3.15 (4H, m), 3.87 (3H, s), 7.00 (1H, dd, J=2.5, 9.1Hz), 7.12 (1H, d, J=2.2Hz), 7.87 (1H, brs), 7.95-8.25 (6H, m), 11.21 (1H, s)

Compound 1-90

$^1$H-NMR (DMSO-$d_6$) δ ppm: 6.17 (2H, s), 7.12 (1H, d, J=8.2Hz), 7.35-7.45 (1H, m), 7.50 (1H, d, J=1.9Hz), 7.55-7.70 (2H, m), 7.90-8.05 (2H, m), 8.25 (1H, brs), 10.87 (1H, s)

Compound 1-91

$^1$H-NMR (DMSO-$d_6$) δ ppm: 6.17 (2H, s), 7.12 (1H, d, J=8.2Hz), 7.20-7.30 (1H, m), 7.49 (1H, d, J=1.6Hz), 7.50-7.60 (1H, m), 7.59 (1H, dd, J=1.6, 8.2Hz), 7.94 (1H, brs), 8.20 (1H, brs), 8.25-8.35 (1H, m), 10.94 (1H, s)

Compound 1-92

$^1$H-NMR (DMSO-$d_6$) δ ppm: 6.18 (2H, s), 7.10-7.15 (1H, m), 7.45-7.70 (4H, m), 8.02 (1H, brs), 8.25-8.30 (1H, m), 8.30 (1H, brs), 10.89 (1H, brs)

Compound 1-93

$^1$H-NMR (DMSO-$d_6$) δ ppm: 3.87 (3H, s), 6.17 (2H, s), 6.90-7.20 (3H, m), 7.40-7.65 (2H, m), 7.84 (1H, brs), 8.03 (1H, brs), 8.15-8.30 (1H, m), 11.02 (1H, s)

Compound 1-94

$^1$H-NMR (DMSO-$d_6$) δ ppm: 1.43 (3H, t, J=6.9Hz), 4.29 (2H, q, J=6.9Hz), 6.16 (2H, s), 7.05-7.25 (3H, m), 7.50 (1H, d, J=1.6Hz), 7.59 (1H, dd, J=1.9, 8.2Hz), 7.69 (1H, dd, J=0.9, 7.9Hz), 7.89 (1H, brs), 8.06 (1H, brs), 10.79 (1H, s)

Compound 1-95

$^1$H-NMR (DMSO-$d_6$) δ ppm: 3.25-3.35 (2H, m), 4.60-4.70 (2H, m), 6.90-7.00 (1H, m), 7.50-7.70 (2H, m), 7.75-7.95 (2H, m), 8.01 (1H, brs), 8.30 (1H, brs), 8.30-8.40 (1H, m), 10.93 (1H, brs)

Compound 1-96

$^1$H-NMR ($CDCl_3$) δ ppm: 2.05-2.25 (2H, m), 2.90-3.10 (4H, m), 7.30-7.50 (3H, m), 7.75-7.95 (2H, m), 8.65-8.75 (1H, m)

Compound 1-97

$^1$H-NMR ($CDCl_3$) δ ppm: 3.82 (2H, s),6.11 (1H, brs), 6.46 (1H, brs), 7.10-7.50 (6H, m), 7.55-7.60 (1H, m), 8.50-8.55 (1H, m), 9.94 (1H, brs)

Compound 1-98

$^1$H-NMR ($CDCl_3$) δ ppm: 2.82 (2H, t, J=7.9Hz), 3.11 (2H, t, J=7.9Hz), 5.68 (1H, brs), 6.35 (1H, brs), 7.15-7.55 (7H, m), 8.45-8.55 (1H, m), 9.82 (1H, brs)

Compound 1-99

$^1$H-NMR (DMSO-$d_6$) δ ppm: 3.81 (3H, s), 3.84 (3H, s), 3.86 (3H, s), 6.90-7.10 (4H, m), 7.20-7.30 (1H, m),

7.35-7.40 (1H, m), 7.55-7.65 (1H, m), 7.76 (1H, brs), 7.94 (1H, brs), 8.05-8.15 (1H, m), 10.26 (1H, brs)

Compound 1-100

$^1$H-NMR (DMSO-$d_6$) δ ppm: 3.84 (3H, s), 4.05 (2H, s), 6.93 (1H, dd, J=2.5, 9.1Hz), 6.98-7.15 (3H, m), 7.43 (1H, dd, J=1.3, 5.0Hz), 7.74 (1H, brs), 7.90 (1H, brs), 7.93 (1H, d, J=8.8Hz), 10.34 (1H, s)

Compound 1-101

$^1$H-NMR ($CDCl_3$+MeOD-$d_4$) δ ppm: 6.75-6.90 (1H, m), 7.25-7.75 (4H, m), 7.85-8.00 (1H, m), 8.20-8.30 (1H, m), 8.50-8.65 (1H, m)

Compound 1-102

$^1$H-NMR (DMSO-$d_6$) δ ppm: 7.35-7.45 (1H, m), 7.50-7.90 (6H, m), 8.08 (1H, brs), 8.38 (1H, brs), 8.40-8.45 (1H, m), 11.36 (1H, brs)

Compound 1-103

$^1$H-NMR ($CDCl_3$+MeOD-$d_4$) δ ppm: 2.74 (3H, s), 7.25-7.50 (2H, m), 8.30-8.70 (2H, m)

Compound 1-104

$^1$H-NMR ($CDCl_3$) δ ppm: 5.64 (1H, brs), 6.41 (1H, brs), 7.10-7.20 (1H, m), 7.35-7.50 (2H, m), 7.60-7.70 (1H, m), 7.75-7.85 (1H, m), 8.65-8.75 (1H, m), 10.84 (1H, brs)

Compound 1-105

$^1$H-NMR ($CDCl_3$) δ ppm: 5.64 (1H, brs), 6.40 (1H, brs), 6.80-6.90 (1H, m), 7.30-7.55 (3H, m), 8.10-8.20 (1H, m), 8.65-8.75 (1H, m), 10.54 (1H, brs)

Compound 1-106

$^1$H-NMR (DMSO-$d_6$) δ ppm: 1.14 (6H, t, J=7.1Hz), 3.43 (4H, q, J=7.1Hz), 6.56-6.62 (1H, m), 6.72-6.84 (2H, m), 7.30-7.35 (1H, m), 7.50-8.03 (3H, m), 8.19 (1H, d, J=9.1Hz), 11.21 (1H, s)

Compound 1-107

$^1$H-NMR (DMSO-$d_6$) δ ppm: 6.74-6.82 (1H, m), 7.34-7.47 (2H, m), 7.62-7.70 (1H, m), 7.96-8.12 (3H, m), 8.31 (1H, brs), 11.04 (1H, s)

Compound 1-108

$^1$H-NMR (DMSO-$d_6$) δ ppm: 6.72-6.84 (1H, m), 7.32-7.72 (3H, m), 7.96-8.11 (2H, m), 8.31 (1H, brs), 8.37-8.47 (1H, m), 11. 04 (1H, s)

Compound 1-109

$^1$H-NMR (DMSO-$d_6$) δ ppm: 6.70-6.90 (1H, m), 7.30-7.70 (2H, m), 7.85-8.20 (3H, m), 8.36 (1H, brs), 10.99 (1H, s)

Compound 1-110

$^1$H-NMR (DMSO-$d_6$) δ ppm: 2.43 (3H, s), 6.75-6.80 (1H, m), 7.32-7.39 (2H, m), 7.49 (1H, d, J=8.5Hz), 7.92 (1H, brs), 8.00-8.12 (2H, m), 8.21 (1H, brs), 10.99 (1H, s)

Compound 1-111

$^1$H-NMR (DMSO-$d_6$) δ ppm: 3.81 (3H, s), 6.70-6.85 (1H, m), 7.05-7.60 (3H, m), 7.74-8.10 (3H, m), 8.20 (1H,

brs), 11.02 (1H, s)

Compound 1-112

$^1$H-NMR (DMSO-$d_6$) δ ppm: 3.98 (3H, s), 6.72-6.82 (1H, m), 7.08-7.40 (3H, m), 7.78-8.24 (4H, m), 10.98 (1H, s)

Compound 1-113

$^1$H-NMR (DMSO-$d_6$) δ ppm: 3.86 (3H, s), 6.72-7.40 (4H, m), 7.86 (1H, brs), 7.96-8.35 (3H, m), 11.14 (1H, s)

Compound 1-114

$^1$H-NMR (DMSO-$d_6$) δ ppm: 5.24 (2H, s), 6.72-6.83 (1H, m), 7.00-7.58 (8H, m), 7.85 (1H, brs), 7.97-8.20 (2H, m), 8.30 (1H, d, J=9.0Hz), 11.14 (1H, s)

Compound 1-115

$^1$H-NMR (DMSO-$d_6$) δ ppm: 2.40 (3H, s), 6.30-6.40 (1H, m), 7.05-7.20 (1H, m), 7.28 (1H, d, J=3.2Hz), 7.45-7.55 (2H, m), 7.86 (1H, brs), 8.06 (1H, brs), 9.98 (1H, s)

Compound 1-116

$^1$H-NMR ($CDCl_3$) δ ppm: 2.45 (3H, s), 5.59 (1H, brs), 6.15-6.25 (1H, m), 6.39 (1H, brs), 7.15-7.25 (2H, m), 7.35-7.45 (1H, m), 8.35-8.40 (1H, m), 10.72 (1H, brs)

Compound 1-117

$^1$H-NMR ($CDCl_3$) δ ppm: 2.45 (3H, s), 5.59 (1H, brs), 6.15-6.25 (1H, m), 6.38 (1H, brs), 7.15-7.25 (1H, m), 7.35-7.45 (2H, m), 8.65-8.75 (1H, m), 10.73 (1H, brs)

Compound 1-118

$^1$H-NMR (DMSO-$d_6$) δ ppm: 2.41 (3H, s), 6.32-6.47 (1H, m), 7.20-7.48 (2H, m), 7.70-7.80 (1H, m), 7.97 (1H, brs), 8.24 (1H, brs), 8.36 (1H, d, J=8.7Hz), 10.99 (1H, s)

Compound 1-119

$^1$H-NMR (DMSO-$d_6$) δ ppm: 2.41 (3H, s), 6.35-6.47 (1H, m), 7.22-7.33 (1H, m), 7.54-7.64 (1H, m), 7.88-7.96 (1H, m), 8.05 (1H, brs), 8.35 (1H, brs), 10.93 (1H, s)

Compound 1-120

$^1$H-NMR (DMSO-$d_6$) δ ppm: 2.41 (3H, s), 3.30 (3H, s), 6.35-6.45 (1H, m), 6.95-7.00 (1H, m), 7.05-7.10 (1H, m), 7.20-7.25 (1H, m), 7.82 (1H, brs), 8.03 (1H, brs), 8.25-8.35 (1H, m), 11.07 (1H, brs)

Compound 1-121

$^1$H-NMR (DMSO-$d_6$) δ ppm: 1.27 (3H, t, J=7.6Hz), 2.76 (2H, q, J=7.6Hz), 6.40-6.45 (1H, m), 7.25-7.30 (1H, m), 7.35-7.45 (1H, m), 7.60-7.70 (1H, m), 8.00 (1H, brs), 8.10-8.15 (1H, m), 8.27 (1H, brs), 11.06 (1H, brs)

Compound 1-122

$^1$H-NMR (DMSO-$d_6$) δ ppm: 1.27 (3H, t, J=7.6Hz), 2.75 (2H, q, J=7.6Hz), 6.35-6.45 (1H, m), 7.20-7.30 (2H, m), 7.53 (1H, dd, J=2.2, 9.1Hz), 7.95 (1H, brs), 8.21 (1H, brs), 8.35-8.50 (1H, m), 11.12 (1H, s)

Compound 1-123

$^1$H-NMR (DMSO-$d_6$) δ ppm: 1.27 (3H, t, J=7.6Hz), 2.76 (2H, q, J=7.6Hz), 6.40-6.45 (1H, m), 7.25-7.30 (1H,

m), 7.50-7.70 (2H, m), 8.02 (1H, brs), 8.29 (1H, brs), 8.40-8.50 (1H, m), 11.08 (1H, brs)

Compound 1-124

$^1$H-NMR (DMSO-$d_6$) δ ppm: 1.26 (3H, t, J=7.5Hz), 2.75 (2H, q, J=7.5Hz), 6.40-6.45 (1H, m), 7.20-7.30 (1H, m), 7.35-7.45 (1H, m), 7.70-7.80 (1H, m), 7.99 (1H, brs), 8.25 (1H, brs), 8.35-8.45 (1H, m), 11.09 (1H, brs)

Compound 1-125

$^1$H-NMR (DMSO-$d_6$) δ ppm: 1.26 (3H, t, J=7.8Hz), 2.76 (2H, q, J=7.8Hz), 6.40-6.45 (1H, m), 7.25-7.30 (1H, m), 7.55-7.65 (1H, m), 7.90-8.00 (1H, m), 8.08 (1H, brs), 8.35 (1H, brs), 11.03 (1H, brs)

Compound 1-126

$^1$H-NMR (DMSO-$d_6$) δ ppm: 1.27 (3H, t, J=7.6Hz), 2.43 (3H, s), 2.75 (2H, q, J=7.6Hz), 6.40-6.45 (1H, m), 7.20-7.40 (2H, m), 7.45-7.50 (1H, m), 7.90 (1H, brs), 8.10-8.20 (1H, m), 8.17 (1H, brs), 11.01 (1H, brs)

Compound 1-127

$^1$H-NMR (DMSO-$d_6$) δ ppm: 1.26 (3H, t, J=7.6Hz), 2.75 (2H, q, J=7.6Hz), 3.86 (3H, s), 6.35-6.45 (1H, m), 6.95-7.00 (1H, m), 7.05-7.10 (1H, m), 7.20-7.30 (1H, m), 7.85 (1H, brs), 8.03 (1H, brs), 8.25-8.35 (1H, m), 11.17 (1H, brs)

Compound 1-128

$^1$H-NMR (DMSO-$d_6$) δ ppm: 1.26 (3H, t, J=7.6Hz), 1.42 (3H, t, J=6.9Hz), 2.75 (2H, q, J=7.6Hz), 4.29 (2H, q, J=6.9Hz), 6.41 (1H, d, J=3.5Hz), 7.12 (1H, d, J=8.2Hz), 7.15-7.30 (2H, m), 7.85 (1H, dd, J=0.9, 8.2Hz), 7.92 (1H, brs), 8.09 (1H, brs), 11.00 (1H, s)

Compound 1-129

$^1$H-NMR (DMSO-$d_6$) δ ppm: 3.86 (3H, s), 4.93 (2H, s), 6.75-6.85 (1H, m), 6.95-7.15 (2H, m), 7.25-7.35 (1H, m), 7.86 (1H, brs), 8.06 (1H, brs), 8.15-8.30 (1H, m), 11.16 (1H, brs)

Compound 1-130

$^1$H-NMR (DMSO-$d_6$) δ ppm: 7.25-7.30 (1H, m), 7.40-7.70 (6H, m), 7.90-8.00 (2H, m), 8.15-8.25 (1H, m), 8.16 (1H, brs), 8.36 (1H, brs), 11.48 (1H, brs)

Compound 1-131

$^1$H-NMR (DMSO-$d_6$) δ ppm: 7.20-7.30 (1H, m), 7.35-7.55 (5H, m), 7.70-7.80 (1H, m), 7.90-8.00 (2H, m), 8.15 (1H, brs), 8.34 (1H, brs), 8.45-8.55 (1H, m), 11.50 (1H, brs)

Compound 1-132

$^1$H-NMR (DMSO-$d_6$) δ ppm: 7.25-7.65 (6H, m), 7.85-8.10 (3H, m), 8.23 (1H, brs), 8.44 (1H, brs), 11.42 (1H, brs)

Compound 1-133

$^1$H-NMR (DMSO-$d_6$) δ ppm: 3.87 (3H, s), 6.95-7.15 (2H, m), 7.20-7.30 (1H, m), 7.35-7.55 (4H, m), 7.85-8.15 (4H, m), 8.35-8.45 (1H, m), 11.55 (1H, brs)

Compound 1-134

$^1$H-NMR ($CDCl_3$+MeOD-$d_4$) δ ppm: 7.20-7.60 (3H, m), 7.80-8.00 (1H, m), 8.15-8.40 (1H, m), 8.60-8.80 (2H, m)

Compound 1-135

$^1$H-NMR ($CDCl_3$+MeOD-$d_4$) δ ppm: 7.30-7.60 (3H, m), 8.20-8.80 (3H, m), 9.10-9.30 (1H, m)

Compound 1-136

$^1$H-NMR (DMSO-$d_6$) δ ppm: 3.87 (3H, s), 7.00 (1H, dd, J=2.2, 8.8Hz), 7.12 (1H, d, J=2.2Hz), 7.74-8.20 (4H, m), 8.38 (1H, dd, J=2.5, 8.2Hz), 8.99 (1H, d, J=2.2Hz), 11.12 (1H, s)

Compound 1-137

$^1$H-NMR (DMSO-$d_6$) δ ppm: 7.15-7.33 (4H, m), 7.35-7.55 (3H, m), 7.67 (1H, dd, J=4.1, 9.1Hz), 7.88 (1H, dd, J=2.8, 9.5Hz), 7.98 (1H, brs), 8.24 (1H, brs), 8.40 (1H, dd, J=2.5, 8.5Hz), 8.78 (1H, d, J=2.5Hz), 10.90 (1H, s)

Compound 1-138

$^1$H-NMR (DMSO-$d_6$) δ ppm: 7.15-7.35 (4H, m), 7.40-7.60 (4H, m), 7.95 (1H, brs), 8.10-8.30 (2H, m), 8.39 (1H, dd, J=2.5, 8.5Hz), 8.77 (1H, d, J=2.5Hz), 10.97 (1H, s)

Compound 1-139

$^1$H-NMR (DMSO-$d_6$) δ ppm: 3.45 (3H, s), 4.10 (2H, s), 7.35-7.45 (1H, m), 7.55-7.70 (1H, m), 7.91 (1H, brs), 8.05-8.15 (1H, m), 8.20 (1H, brs), 10.76 (1H, s)

Compound 1-140

$^1$H-NMR (DMSO-$d_6$) δ ppm: 3.45 (3H, s), 4.11 (2H, s), 7.54 (1H, dd, J=2.2, 8.8Hz), 7.63 (1H, d, J=8.8Hz), 7.93 (1H, brs), 8.22 (1H, brs), 8.40 (1H, d, J=2.2Hz), 10.76 (1H, s)

Compound 1-141

$^1$H-NMR (DMSO-$d_6$) δ ppm: 3.45 (3H, s), 3.96 (3H, s), 4.09 (2H, s), 7.08-7.15 (1H, d, J=7.9Hz), 7.18-7.25 (1H, m), 7.75-7.95 (2H, d, J=8.2Hz), 8.07 (1H, brs), 10.73 (1H, s)

Compound 1-142

$^1$H-NMR (DMSO-$d_6$) δ ppm: 4.82 (2H, s), 6.95-7.15 (3H, m), 7.30-7.45 (3H, m), 7.60-7.70 (1H, m), 8.00 (1H, brs), 8.10-8.20 (1H, m), 8.24 (1H, brs), 11.16 (1H, brs)

Compound 1-143

$^1$H-NMR ($CDCl_3$) δ ppm: 4.70 (2H, s), 6.90-7.15 (5H, m), 7.30-7.50 (4H, m), 8.55-8.65 (1H, m), 11.17 (1H, brs)

Compound 1-144

$^1$H-NMR ($CDCl_3$) δ ppm: 3.88 (3H, s), 4.69 (2H, s), 5.63 (1H, brs), 6.24 (1H, brs), 6.85-7.15 (5H, m), 7.30-7.40 (2H, m), 8.45-8.55 (1H, m), 11.11 (1H, brs)

Compound 1-145

$^1$H-NMR (DMSO-$d_6$) δ ppm: 4.83 (2H, s), 7.05-7.15 (2H, m), 7.35-7.45 (3H, m), 7.60-7.70 (1H, m), 8.00 (1H, brs), 8.05-8.15 (1H, m), 8.25 (1H, brs), 11.13 (1H, brs)

Compound 1-146

$^1$H-NMR (DMSO-$d_6$) δ ppm: 4.84 (2H, s), 7.10-7.15 (2H, m), 7.35-7.45 (2H, m), 7.50-7.70 (2H, m), 8.02 (1H, brs), 8.27 (1H, brs), 8.40-8.45 (1H, m), 11.13 (1H, brs)

Compound 1-147

$^1$H-NMR ($CDCl_3$) δ ppm: 3.88 (3H, s), 4.66 (2H, s), 5.54 (1H, brs), 6.26 (1H, brs), 6.80-7.10 (4H, m), 7.20-7.40 (2H, m), 8.45-8.55 (1H, m), 11.09 (1H, brs)

Compound 1-148

$^1$H-NMR (DMSO-$d_6$) δ ppm: 1.58 (6H, s), 7.05-7.20 (2H, m), 7.30-7.45 (3H, m), 7.60-7.70 (1H, m), 7.95 (1H, brs), 8.05-8.15 (1H, m), 8.22 (1H, brs), 11.24 (1H, brs)

Compound 1-149

$^1$H-NMR (DMSO-$d_6$) δ ppm: 1.58 (6H, s), 7.05-7.20 (2H, m), 7.30-7.45 (2H, m), 7.50-7.70 (2H, m), 7.97 (1H, brs), 8.24 (1H, brs), 8.40-8.50 (1H, m), 11.28 (1H, brs)

Compound 1-150

$^1$H-NMR (DMSO-$d_6$) δ ppm: 4.23 (2H, s), 4.70 (2H, s), 7.25-7.45 (4H, m), 7.49 (2H, d, J=7.3Hz), 7.60-7.65 (1H, m), 7.98 (1H, brs), 8.05-8.15 (1H, m), 8.22 (1H, brs), 10.90 (1H, s)

Compound 1-151

$^1$H-NMR (DMSO-$d_6$) δ ppm: 4.23 (2H, s), 4.70 (2H, s), 7.25-7.45 (3H, m), 7.49 (2H, d, J=7.3Hz), 7.55 (1H, dd, J=2.2, 8.8Hz), 7.63 (1H, d, J=8.8Hz), 8.00 (1H, brs), 8.24 (1H, brs), 8.41 (1H, d, J=2.5Hz), 10.90 (1H, s)

Compound 1-152

$^1$H-NMR (DMSO-$d_6$) δ ppm: 0.80-0.95 (4H, m), 1.91-2.05 (1H, m), 7.15-7.25 (1H, m), 7.45-7.53 (1H, m), 7.85 (1H, brs), 7.93-8.02 (1H, m), 8.07 (1H, brs), 10.37 (1H, s)

Compound 1-153

$^1$H-NMR (DMSO-$d_6$) δ ppm: 0.82-0.93 (4H, m), 1.94-2.05 (1H, m), 7.31-7.40 (1H, m), 7.67-7.74 (1H, m), 7.82-7.98 (2H, m), 8.11 (1H, s), 10.36 (1H, s)

Compound 1-154

$^1$H-NMR (DMSO-$d_6$) δ ppm: 0.80-0.95 (4H, m), 1.90-2.00 (1H, m), 7.75-7.95 (1H, m), 7.89 (1H, brs), 8.10 (1H, brs), 10.38 (1H, brs)

Compound 1-155

$^1$H-NMR (DMSO-$d_6$) δ ppm: 0.80-1.00 (4H, m), 1.90-2.05 (1H, m), 7.45-7.60 (2H, m), 7.95 (1H, brs), 8.18 (1H, brs), 10.35 (1H, s)
MS (ESI, m/z): 281 (M+H)+

Compound 1-156

$^1$H-NMR (DMSO-$d_6$) δ ppm: 0.78-0.96 (4H, m), 1.93-2.05 (1H, m), 7.55-7.74 (2H, m), 7.99 (1H, brs), 8.10 (1H, brs), 10.37 (1H, s)

Compound 1-157

$^1$H-NMR (DMSO-$d_6$) δ ppm: 0.81-0.94 (4H, m), 1.90-2.00 (1H, m), 2.44 (3H, s), 7.05-7.15 (1H, m), 7.30-7.40 (1H, m), 7.67-8.10 (3H, m), 10.32 (1H, s)

Compound 1-158

$^1$H-NMR (DMSO-$d_6$) δ ppm: 0.80-0.95 (4H, m), 1.90-2.00 (1H, m), 3.31 (3H, s), 7.45-7.55 (1H, m), 7.60-7.70 (1H, m), 7.83 (1H, brs), 8.06 (1H, brs), 10.34 (1H, brs)

Compound 1-159

$^1$H-NMR (DMSO-$d_6$) δ ppm: 0.80-0.90 (4H, m), 1.90-2.00 (1H, m), 3.97 (3H, s), 7.00-7.10 (1H, m), 7.10-7.20 (1H, m), 7.85 (1H, brs), 8.04 (1H, brs), 10.30 (1H, brs)

Compound 1-160

$^1$H-NMR (DMSO-$d_6$) δ ppm: 1.75-1.90 (1H, m), 1.90-2.05 (1H, m), 2.10-2.35 (4H, m), 3.20-3.45 (1H, m), 7.30-7.45 (1H, m), 7.55-7.70 (1H, m), 7.75-8.00 (2H, m), 8.15 (1H, brs), 10.05 (1H, s)
MS (ESI, m/z): 277 (M+H)+

Compound 1-161

$^1$H-NMR (DMSO-$d_6$) δ ppm: 1.75-1.90 (1H, m), 1.90-2.05 (1H, m), 2.10-2.35 (4H, m), 3.30-3.45 (1H, m), 7.50-7.60 (1H, m), 7.60-7.75 (1H, m), 7.95 (1H, brs), 8.19 (1H, brs),10.03 (1H, s)
MS (ESI, m/z): 295 (M+H)+

Compound 1-162

$^1$H-NMR (DMSO-$d_6$) δ ppm: 1.50-2.00 (8H, m), 2.85-3.00 (1H, m), 7.30-7.40 (1H, m), 7.55-7.65 (1H, m), 7.75-7.85 (1H, m), 7.87 (1H, brs), 8.11 (1H, brs), 10.14 (1H, brs)

Compound 1-163

$^1$H-NMR (DMSO-$d_6$) δ ppm: 1.15-1.50 (5H, m), 1.60-2.00 (5H, m), 2.40-2.55 (1H, m), 7.30-7.40 (1H, m), 7.55-7.65 (1H, m), 7.75-7.85 (1H, m), 7.87 (1H, brs), 8.11 (1H, brs), 10.13 (1H, brs)

Compound 1-164

$^1$H-NMR (DMSO-$d_6$) δ ppm: 1.35-1.45 (1H, m), 1.50-1.60 (1H, m), 2.30-2.55 (2H, m), 7.15-7.40 (5H, m), 7.45-7.60 (2H, m), 7.93 (1H, brs), 8.17 (1H, brs), 10.40 (1H, brs)

Compound 1-165

$^1$H-NMR (DMSO-$d_6$) δ ppm: 1.80-2.00 (2H, m), 2.00-2.15 (1H, m), 2.20-2.35 (1H, m), 3.80-3.95 (1H, m), 3.95-4.10 (1H, m), 4.45-4.60 (1H, m), 7.30-7.45 (1H, m), 7.55-7.70 (1H, m), 7.91 (1H, brs), 8.05-8.20 (1H, m), 8.21 (1H, brs), 10.90 (1H, s)

Compound 1-166

$^1$H-NMR (DMSO-$d_6$) δ ppm: 1.80-2.00 (2H, m), 2.00-2.15 (1H, m), 2.20-2.35 (1H, m), 3.80-3.95 (1H, m), 3.95-4.10 (1H, m), 4.45-4.60 (1H, m), 7.30-7.45 (1H, m), 7.55-7.70 (1H, m), 7.91 (1H, brs), 8.05-8.20 (1H, m), 8.21 (1H. brs), 10.90 (1H, s)

Compound 1-167

$^1$H-NMR (DMSO-$d_6$) δ ppm: 7.40-7.50 (2H, m), 7.80-7.90 (1H, m), 8.00 (1H, brs), 8.05-8.25 (3H, m), 8.25 (1H, brs), 11.00 (1H, brs)

Compound 1-168

$^1$H-NMR (DMSO-$d_6$) δ ppm: 7.40-7.50 (2H, m), 7.55-7.65 (1H, m), 7.70-7.80 (1H, m), 8.05-8.15 (3H, m), 8.37 (1H, brs), 10.92 (1H, brs)

Compound 1-169

$^1$H-NMR (DMSO-$d_6$) δ ppm: 2.48 (3H, s), 7.19 (1H, d, J=8.2Hz), 7.42 (1H, s), 7.43-7.60 (1H, m), 7.60-7.73 (1H, m), 7.75-8.00 (3H, m), 8.11 (1H, d, J=8.2Hz), 8.17 (1H, brs), 11.07 (1H, s)

Compound 1-170

$^1$H-NMR (DMSO-$d_6$) δ ppm: 2.48 (3H, s), 7.19 (1H, d, J=8.5Hz), 7.35-7.50 (3H, m), 7.89 (1H, brs), 8.00-8.30 (4H, m), 11.04 (1H, s)

Compound 1-171

$^1$H-NMR (DMSO-$d_6$) δ ppm: 1.25 (3H, t, J=7.6Hz), 2.77 (2H, q, J=7.6Hz), 7.22 (1H, d, J=8.2Hz), 7.35-7.50 (3H, m), 7.89 (1H, brs), 8.00-8.25 (4H, m), 11.02 (1H, s)

Compound 1-172

$^1$H-MMR (DMSO-$d_6$) δ ppm: 3.87 (3H, s), 6.95-7.05 (1H, m), 7.05-7.15 (1H, m), 7.40-7.50 (2H, m), 7.87 (1H, brs), 8.00-8.15 (3H, m), 8.19 (1H, d, J=8.6Hz), 11.12 (1H, s)

Compound 1-173

$^1$H-NMR (DMSO-$d_6$) δ ppm: 6.80-6.95 (2H, m), 7.40-7.50 (2H, m), 7.81 (1H, brs), 8.00-8.10 (3H, m), 8.10-8.15 (1H, m), 10.09 (1H, s), 11.14 (1H, brs)

Compound 1-174

$^1$H-NMR (DMSO-$d_6$) δ ppm: 7.40-7.50 (2H, m), 7.71 (1H, dd, J=0.9, 8.5Hz), 8.00 (1H, s), 8.03-8.20 (3H, m), 8.31 (1H, brs), 8.36 (1H, d, J=8.5Hz), 10.94 (1H, s)

Compound 1-175

$^1$H-NMR (DMSO-$d_6$) δ ppm: 2.40 (3H, s), 7.45-7.70 (3H, m), 7.75-8.05 (4H, m), 8.25 (1H, brs), 11.06 (1H, s)

Compound 1-176

$^1$H-NMR (DMSO-$d_6$) δ ppm: 2.40 (3H, s), 7.35-7.60 (3H, m), 7.85-8.15 (4H, m), 8.25 (1H, brs), 11.04 (1H, s)

Compound 1-177

$^1$H-NMR (DMSO-$d_6$) δ ppm: 4.00 (3H, s), 7.10-7.15 (1H, m), 7.40-7.50 (3H, m), 7.96 (1H, brs), 8.05-8.15 (2H, m), 8.20 (1H, brs), 10.90 (1H, brs)

Compound 1-178

$^1$H-NMR (DMSO-$d_6$) δ ppm: 6.67-6.84 (1H, m), 7.19-7.53 (3H, m), 7.84-8.15 (4H, m), 10.84 (1H, s), 10.87 (1H, s)

Compound 1-179

$^1$H-NMR (DMSO-$d_6$) δ ppm: 1.43 (3H, t, J=7.0Hz), 4.30 (2H, q, J=7.0Hz), 7.07-7.15 (1H, m), 7.19-7.28 (1H, m), 7.56-7.67 (2H, m), 7.70-7.77 (1H, m), 7.89 (1H, brs), 7.93-8.00 (1H, m), 8.00-8.15 (2H, m), 10.91 (1H, s)

Compound 1-180

$^1$H-NMR (DMSO-$d_6$) δ ppm: 1.43 (3H, t, J=7.0Hz), 4.30 (2H, q, J=7.0Hz), 7.08-7.28 (2H, m), 7.60-7.72 (3H, m), 7.90 (1H, brs), 8.00-8.15 (3H, m), 10.93 (1H, s)

Compound 1-181

$^1$H-NMR (DMSO-$d_6$) δ ppm: 4.00 (3H, s), 7.05-7.15 (1H, m), 7.40-7.45 (1H, m), 7.68 (2H, d, J=8.5Hz), 7.97 (1H, brs), 8.02 (2H, d, J=8.5Hz), 8.21 (1H, brs), 10.94 (1H, brs)

Compound 1-182

$^1$H-NMR (DMSO-$d_6$) δ ppm: 1.43 (3H, t, J=7.0Hz), 4.30 (2H, q, J=7.0Hz), 7.05-7.30 (2H, m), 7.50-7.75 (2H, m), 7.80-7.95 (2H, m), 8.00-8.15 (2H, m), 10.85 (1H, s)

Compound 1-183

$^1$H-NMR (DMSO-$d_6$) δ ppm: 7.50-7.75 (3H, m), 8.00-8.10 (2H, m), 8.20-8.30 (1H, m), 8.32 (1H, brs), 10.88 (1H, brs)

Compound 1-184

$^1$H-NMR (DMSO-$d_6$) δ ppm: 7.35-7.45 (1H, m), 7.55-7.85 (3H, m), 7.97 (1H, brs), 8.00-8.10 (1H, m), 8.23 (1H, brs), 8.30-8.40 (1H, m), 10.90 (1H, brs)

Compound 1-185

$^1$H-NMR (DMSO-$d_6$) δ ppm: 3.87 (3H, s), 6.95-7.15 (2H, m), 7.55-7.65 (1H, m), 7.70-7.90 (2H, m), 8.00-8.15 (2H, m), 8.25-8.35 (1H, m), 11.03 (1H, brs)

Compound 1-186

$^1$H-NMR (DMSO-$d_6$) δ ppm: 2.43 (3H, s), 7.22-7.33 (1H, m), 7.45-7.60 (3H, m), 7.75-7.86 (2H, m), 7.95 (1H, brs), 8.22 (1H, brs), 8.30-8.38 (1H, m), 11.07 (1H, s)

Compound 1-187

$^1$H-NMR (DMSO-$d_6$) δ ppm: 2.41 (3H, s), 7.22-7.35 (1H, m), 7.35-7.45 (2H, m), 7.50-7.60 (1H, m), 7.85-8.05 (3H, m), 8.22 (1H, brs), 8.32-8.40 (1H, m), 11.08 (1H, s)

Compound 1-188

$^1$H-NMR (DMSO-$d_6$) δ ppm: 2.42 (3H, s), 7.40-7.55 (3H, m), 7.75-7.85 (3H, m), 8.02 (1H, brs), 8.25-8.35 (2H, m), 11.04 (1H, brs)

Compound 1-189

$^1$H-NMR (DMSO-$d_6$) δ ppm: 1.43 (3H, t, J=7.0Hz), 2.41 (3H, s), 4.29 (2H, q, J=7.0Hz), 7.05-7.30 (2H, m), 7.35-7.45 (2H, m), 7.70-7.80 (1H, m), 7.85-8.00 (3H, m), 8.08 (1H, brs), 10.93 (1H, s)

Compound 1-190

$^1$H-NMR (DMSO-$d_6$) δ ppm: 2.41 (3H, s), 4.00 (3H, s), 7.10-7.15 (1H, m), 7.41 (2H, d, J=8.0Hz), 7.50-7.55 (1H, m), 7.90 (2H, d, J=8.0Hz), 7.97 (1H, brs), 8.22 (1H, brs), 10.95 (1H, brs)

Compound 1-191

$^1$H-NMR (DMSO-$d_6$) δ ppm: 1.23 (3H, t, J=7.6Hz), 2.71 (2H, q, J=7.6Hz), 4.00 (3H, s), 7.05-7.20 (1H, m), 7.44 (2H, d, J=8.2Hz), 7.50-7.60 (1H, m), 7.92 (2H, d, J=8.2Hz), 7.97 (1H, brs), 8.21 (1H, brs), 10.95 (1H, brs)

Compound 1-192

$^1$H-NMR (DMSO-$d_6$) δ ppm: 4.89 (2H, s), 7.20-7.35 (1H, m), 7.50-7.70 (2H, m), 7.74 (1H, d, J=7.9Hz), 7.85-8.05

(2H, m), 8.09 (1H, s), 8.15-8.35 (2H, m), 11.09 (1H, s)

Compound 1-193

$^1$H-NMR (DMSO-$d_6$) δ ppm: 4.87 (2H, s), 7.20-7.35 (1H, m), 7.50-7.60 (1H, m), 7.60-7.75 (2H, m), 7.90-8.05 (3H, m), 8.22 (1H, brs), 8.27-8.35 (1H, m), 11.09 (1H, s)

Compound 1-194

$^1$H-NMR (DMSO-$d_6$) δ ppm: 4.89 (2H, s), 7.55-7.85 (4H, m), 7.90-8.15 (3H, m), 8.36 (1H, brs), 10.98 (1H, brs)

Compound 1-195

$^1$H-NMR (DMSO-$d_6$) δ ppm: 4.87 (2H, s), 7.50-7.85 (4H, m), 7.95-8.05 (2H, m), 8.09 (1H, brs), 8.36 (1H, brs), 10.98 (1H, brs)

Compound 1-196

$^1$H-NMR (DMSO-$d_6$) δ ppm: 2.40 (3H, s), 4.90 (2H, s), 7.50-7.80 (3H, m), 7.90-8.05 (3H, m), 8.08 (1H, s), 8.26 (1H, brs), 11.10 (1H, s)

Compound 1-197

$^1$H-NMR (DMSO-$d_6$) δ ppm: 2.40 (3H, s), 4.88 (2H, s), 7.50-7.75 (3H, m), 7.90-8.10 (4H, m), 8.26 (1H, brs), 11.10 (1H, s)

Compound 1-198

$^1$H-NMR (DMSO-$d_6$) δ ppm: 2.48 (3H, s), 4.89 (2H, s), 7.20 (1H, d, J=8.5Hz), 7.42 (1H, s), 7.55-7.80 (2H, m), 7.91 (1H, brs), 7.97 (1H, d, J=7.9Hz), 8.09 (1H, s), 8.15 (1H, d, J=7.9Hz), 8.18 (1H, brs), 11.10 (1H, s)

Compound 1-199

$^1$H-NMR (DMSO-$d_6$) δ ppm: 2.48 (3H, s), 4.87 (2H, s), 7.19 (1H, d, J=8.2Hz), 7.41 (1H, s), 7.66 (2H, d, J=8.2Hz), 7.91 (1H, brs), 8.01 (2H, d, J=8.2Hz), 8.10-8.30 (2H, m), 11.10 (1H, s)

Compound 1-200

$^1$H-NMR ($CDCl_3$) δ ppm: 2.50-2.60 (4H, m), 3.61 (2H, s), 3.75-3.85 (4H, m), 3.92 (3H, s), 5.73 (1H, brs), 6.50 (1H, brs), 6.95-7.10 (2H, m), 7.90-8.00 (1H, m), 8.05-8.25 (2H, m), 10.78 (1H, brs)

Compound 1-201

$^1$H-NMR (DMSO-$d_6$) δ ppm: 1.38 (3H, t, J=6.9Hz), 2.35-2.50 (4H, m), 3.54 (2H, s), 3.55-3.70 (4H, m), 4.16 (2H, q, J=6.9Hz), 7.10-7.25 (1H, m), 7.55-7.65 (1H, m), 7.85-8.05 (3H, m), 8.11 (1H, brs), 8.38 (1H, brs), 10.98 (1H, brs)

Compound 1-202

$^1$H-NMR (DMSO-$d_6$) δ ppm: 2.35-2.50 (4H, m), 3.55-3.70 (4H, m), 3.58 (2H, s), 5.27 (2H, brs), 7.25-7.55 (7H, m), 7.60-7.70 (1H, m), 7.85-8.10 (4H, m), 8.26 (1H, brs), 11.01 (1H, brs)

Compound 1-203

$^1$H-NMR (DMSO-$d_6$) δ ppm: 2.35-2.50 (4H, m), 3.57 (2H, s), 3.55-3.70 (4H, m), 5.27 (2H, s), 7.25-7.65 (7H, m), 7.85-8.05 (3H, m), 8.08 (1H, brs), 8.34 (1H, brs), 10.96 (1H, brs)

Compound 1-204

$^1$H-NMR (DMSO-$d_6$) δ ppm: 2.40-2.55 (4H, m), 2.48 (3H, s), 3.50-3.75 (6H, m), 5.28 (2H, brs), 7.10-7.60 (8H, m), 7.80-8.35 (5H, m), 11.08 (1H, brs)

Compound 1-205

$^1$H-NMR (DMSO-$d_6$) δ ppm: 2.40-2.50 (4H, m), 3.55-3.70 (4H, m), 3.58 (2H, s), 3.87 (3H, s), 5.26 (2H, brs), 6.95-7.15 (2H, m), 7.25-7.55 (6H, m), 7.75-8.15 (4H, m), 8.25-8.35 (1H, m), 11.15 (1H, brs)

Compound 1-206

$^1$H-NMR (DMSO-$d_6$) δ ppm: 7.50-8.50 (8H, m), 10.92 (1H, brs)

Compound 1-207

$^1$H-NMR (DMSO-$d_6$) δ ppm: 2.48 (3H, s), 3.86 (3H, s), 7.10-7.30 (2H, m), 7.41 (1H, s), 7.45-7.60 (3H, m), 7.90 (1H, brs), 8.10-8.25 (2H, m), 11.10 (1H, s)

Compound 1-208

$^1$H-NMR (DMSO-$d_6$) δ ppm: 2.48 (3H, s), 3.86 (3H, s), 7.14 (2H, d, J=8.8Hz), 7.18 (1H, d, J=8.2Hz), 7.40 (1H, s), 7.88 (1H, brs), 7.97 (2H, d, J=8.8Hz), 8.15 (1H, brs), 8.21 (1H, d, J=8.2Hz), 11.02 (1H, s)

Compound 1-209

$^1$H-NMR (DMSO-$d_6$) δ ppm: 3.86 (3H, s), 7.20-7.35 (2H, m), 7.45-7.65 (4H, m), 7.96 (1H, brs), 8.22 (1H, brs), 8.29-8.39 (1H, m), 11.09 (1H, s)

Compound 1-210

$^1$H-NMR (DMSO-$d_6$) δ ppm: 3.86 (3H, s), 7.20-7.30 (1H, m), 7.45-7.65 (4H, m), 7.75-7.90 (1H, m), 8.09 (1H, brs), 8.37 (1H, brs), 10.97 (1H, brs)

Compound 1-211

$^1$H-NMR (DMSO-$d_6$) δ ppm: 3.87 (3H, s), 7.10-7.20 (2H, m), 7.50-7.65 (1H, m), 7.80-8.05 (3H, m), 8.06 (1H, brs), 8.33 (1H, brs), 10.90 (1H, brs)

Compound 1-212

$^1$H-NMR (DMSO-$d_6$) δ ppm: 2.40 (3H, s), 3.86 (3H, s), 7.20-7.30 (1H, m), 7.45-7.65 (4H, m), 7.90-8.10 (2H, m), 8.26 (1H, brs), 11.10 (1H, s)

Compound 1-213

$^1$H-NMR (DMSO-$d_6$) δ ppm: 3.86 (3H, s), 3.99 (3H, s), 7.05-7.15 (3H, m), 7.50-7.55 (1H, m), 7.90-8.00 (3H, m), 8.20 (1H, brs), 10.89 (1H, brs)

Compound 1-214

$^1$H-NMR (DMSO-$d_6$) δ ppm: 1.32 (6H, d, J=6.0Hz), 4.65-4.80 (1H, m), 7.15-7.25 (1H, m), 7.35-7.60 (4H, m), 7.63-7.70 (1H, m), 7.90-8.10 (2H, m), 8.27 (1H, brs), 11.01 (1H, s)

Compound 1-215

$^1$H-NMR (DMSO-$d_6$) δ ppm: 1.01 (3H, t, J=7.6Hz), 1.70-1.85 (2H, dt, J=6.6, 7.6Hz), 4.03 (2H, t, J=6.6Hz), 7.20-7.25 (1H, m), 7.35-7.60 (4H, m), 7.63-7.70 (1H, m), 7.90-8.10 (2H, m), 8.27 (1H, brs), 11.01 (1H, s)

Compound 1-216

$^1$H-NMR (DMSO-$d_6$) δ ppm: 7.23-7.33 (1H, m), 7.50-7.60 (1H, m), 7.65-7.80 (2H, m), 7.90-8.08 (3H, m), 8.15-8.30 (2H, m), 11.11 (1H, s)

Compound 1-217

$^1$H-NMR (DMSO-$d_6$) δ ppm: 1.43 (3H, t, J=7.0Hz), 4.30 (2H, q, J=7.0Hz), 7.09-7.28 (2H, m), 7.55-7.68 (3H, m), 7.90 (1H, brs), 8.00-8.20 (3H, m), 10.94 (1H, s)

Compound 1-218

$^1$H-NMR (DMSO-$d_6$) δ ppm: 4.00 (3H, s), 7.05-7.15 (1H, m), 7.35-7.45 (1H, m), 7.60 (2H, d, J=8.5Hz), 7.96 (1H, brs), 8.14 (2H, d, J=8.5Hz), 8.20 (1H, brs), 10.94 (1H, brs)

Compound 1-219

$^1$H-NMR (DMSO-$d_6$) δ ppm: 3.33 (3H, s), 3.65-3.75 (2H, m), 4.15-4.25 (2H, m), 7.20-7.30 (1H, m), 7.35-7.70 (5H, m), 7.90-8.10 (2H, m), 8.27 (1H, brs), 11.00 (1H, s)

Compound 1-220

$^1$H-NMR (DMSO-$d_6$) δ ppm: 3.31 (3H, s), 3.33 (3H, s), 3.65-3.75 (2H, m), 4.10-4.30 (2H, m), 7.25 (1H, dd, J=2.2, 7.6Hz), 7.35-7.75 (4H, m), 7.90-8.05 (2H, m), 8.26 (1H, brs), 11.00 (1H, s)

Compound 1-221

$^1$H-NMR ($CDCl_3$) δ ppm: 1.20-1.65 (6H, m), 1.75-1.90 (2H, m), 1.95-2.10 (2H, m), 4.30-4.45 (1H, m), 5.69 (1H, brs), 6.41 (1H, brs), 7.05-7.65 (6H, m), 8.35-8.45 (1H, m), 10.82 (1H, brs)

Compound 1-222

$^1$H-NMR ($CDCl_3$) δ ppm: 1.20-1.65 (6H, m), 1.75-1.90 (2H, m), 1.95-2.10 (2H, m), 2.51 (3H, s), 4.30-4.45 (1H, m), 5.55 (1H, brs), 6.36 (1H, brs), 7.05-7.30 (3H, m), 7.35-7.45 (1H, m), 7.50-7.65 (2H, m), 8.50-8.60 (1H, m), 10.90 (1H, brs)

Compound 1-223

$^1$H-NMR ($CDCl_3$) δ ppm: 1.75-1.95 (2H, m), 2.00-2.15 (2H, m), 3.55-3.70 (2H, m), 3.95-4.05 (2H, m), 4.55-4.70 (1H, m), 6.08 (1H, brs), 6.48 (1H, brs), 7.10-7.65 (6H, m), 8.30-8.45 (1H, m), 10.86 (1H, brs)

Compound 1-224

$^1$H-NMR ($CDCl_3$) δ ppm: 1.75-1.90 (2H, m), 2.00-2.15 (2H, m), 2.51 (3H, s), 3.55-3.65 (2H, m), 3.95-4.05 (2H, m), 4.55-4.70 (1H, m), 5.60 (1H, brs), 6.39 (1H, brs), 7.10-7.30 (3H, m), 7.35-7.50 (1H, m), 7.55-7.65 (2H, m), 8.45-8.55 (1H, m), 10.94 (1H, brs)

Compound 1-225

$^1$H-NMR ($CDCl_3$) δ ppm: 1.80-2.15 (4H, m), 2.25-2.45 (2H, m), 2.33 (3H, s), 2.65-2.80 (2H, m), 4.40-4.55 (1H, m), 6.00 (1H, brs), 6.48 (1H, brs), 7.05-7.30 (2H, m), 7.35-7.65 (4H, m), 8.30-8.45 (1H, m), 10.85 (1H, brs)

Compound 1-226

$^1$H-NMR (DMSO-$d_6$) δ ppm: 1.60-1.75 (2H, m), 1.90-2.05 (2H, m), 2.10-2.30 (5H, m), 2.55-2.75 (2H, m), 4.40-4.55 (1H, m), 7.20-7.30 (1H, m), 7.40-7.60 (4H, m), 7.70-7.80 (1H, m), 8.00 (1H, brs), 8.15-8.35 (2H, m), 11.02 (1H, s)
MS (ESI, m/z): 428 (M+H)+

Compound 1-227

$^1$H-NMR ($CDCl_3$) δ ppm: 1.80-2.15 (4H, m), 2.20-2.45 (2H, m), 2.33 (3H, s), 2.60-2.80 (2H, m), 4.40-4.50 (1H, m), 5.96 (1H, brs), 6.56 (1H, brs), 7.00-7.20 (2H, m), 7.35-7.65 (3H, m), 8.15-8.25 (1H, m), 10.81 (1H, brs)

Compound 1-228

$^1$H-NMR ($CDCl_3$) δ ppm: 1.80-2.10 (4H, m), 2.25-2.40 (2H, m), 2.32 (3H, s), 2.50 (3H, s), 2.65-2.80 (2H, m), 4.40-4.50 (1H, m), 5.81 (1H, brs), 6.41 (1H, brs), 7.00-7.30 (3H, m), 7.35-7.65 (3H, m), 8.45-8.55 (1H, m), 10.92 (1H, brs)

Compound 1-229

$^1$H-NMR (DMSO-$d_6$) δ ppm: 3.23-3.36 (2H, m), 4.60-4.70 (2H, m), 6.95 (1H, d, J=8.2Hz), 7.53-7.63 (1H, m), 7.77-7.92 (3H, m), 8.05 (1H, brs), 8.33 (1H, brs), 10.85 (1H, s)

Compound 1-230

$^1$H-NMR (DMSO-$d_6$) δ ppm: 2.47 (3H, s), 6.17 (2H, s), 7.12 (1H, d, J=8.2Hz), 7.18 (1H, d, J=8.2Hz), 7.40 (1H, s). 7.48 (1H, d, J=1.6Hz), 7.58 (1H, dd, J=1.6, 8.2Hz), 7.88 (1H, brs), 8.05-8.25 (2H, m), 10.95 (1H, s)

Compound 1-231

$^1$H-NMR (DMSO-$d_6$) δ ppm: 1.25 (3H, t, J=7.6Hz), 2.77 (2H, q, J=7.6Hz), 6.17 (2H, s), 7.05-7.15 (1H, m), 7.22 (1H, d, J=8.2Hz), 7.41 (1H, s), 7.49 (1H, s), 7.58 (1H, d, J=8.2Hz), 7.88 (1H, brs), 8.13 (1H, brs), 8.17 (1H, d, J=8.2Hz), 10.93 (1H, s)

Compound 1-232

$^1$H-NMR (DMSO-$d_6$) δ ppm: 2.39 (3H, s), 6.17 (2H, s), 7.05-7.20 (1H, m), 7.48 (1H, d, J=1.9Hz), 7.52-7.60 (2H, m), 7.90-8.05 (2H, m), 8.24 (1H, brs), 10.95 (1H, s)

Compound 1-233

$^1$H-NMR (DMSO-$d_6$) δ ppm: 2.68 (3H, s), 7.35-7.45 (1H, m), 7.67 (1H, dd, J=4.1, 9.1Hz), 7.87 (1H, d, J=8.5Hz), 7.93-8.10 (3H, m), 8.26 (1H, brs), 8.30 (1H, d, J=1.6Hz), 11.03 (1H, s)

Compound 1-234

$^1$H-NMR (DMSO-$d_6$) δ ppm: 3.92 (3H, s), 7.35-7.48 (1H, m), 7.64-7.95 (3H, m), 8.02 (1H, brs), 8.20-8.35 (3H, m), 8.55-8.65 (1H, m), 11.11 (1H, s)

Compound 1-235

$^1$H-NMR (DMSO-$d_6$) δ ppm: 5.43 (2H, s), 7.32-8.08 (10H, m) 8.22-8.35 (3H, m), 8.59-8.65 (1H, m), 11.12 (1H, s)

Compound 1-236

$^1$H-NMR (DMSO-$d_6$) δ ppm: 2.68 (3H, s), 7.35-7.45 (1H, m), 7.65-7.80 (1H, m), 7.90-8.00 (1H, m), 8.01 (1H, brs), 8.20-8.30 (3H, m), 8.27 (1H, brs), 8.55-8.60 (1H, m), 11.12 (1H, brs)

Compound 1-237

$^1$H-NMR (DMSO-$d_6$) δ ppm: 2.48 (3H, s), 2.68 (3H, s), 7.15-7.25 (1H, m), 7.40-7.45 (1H, m), 7.70-7.80 (1H, m), 7.91 (1H, brs), 8.10-8.15 (1H, m), 8.17 (1H, brs), 8.20-8.30 (2H, m), 8.50-8.60 (1H, m), 11.19 (1H, brs)

Compound 1-238

$^1$H-NMR (DMSO-$d_6$) δ ppm: 1.60-1.75 (2H, m), 1.90-2.05 (2H, m), 2.20-2.35 (2H, m), 2.60-2.75 (2H, m), 3.50

(2H, s), 4.45-4.60 (1H, m), 7.20-7.70 (11H, m), 7.95-8.05 (1H, m), 8.02 (1H, brs), 8.30 (1H, brs), 10.98 (1H, brs)

Compound 1-239

$^1$H-NMR (DMSO-$d_6$) δ ppm: 1.60-1.75 (2H, m), 1.90-2.05 (2H, m), 2.20-2.35 (2H, m), 2.60-2.75 (2H, m), 3.50 (2H, s), 4.45-4.60 (1H, m), 7.20-7.60 (10H, m), 7.70-7.80 (1H, m), 7.99 (1H, brs), 8.15-8.35 (2H, m), 11.01(1H, s)
MS (ESI, m/z): 504 (M+H)+

Compound 1-240

$^1$H-NMR (DMSO-$d_6$) δ ppm: 1.60-1.80 (2H, m), 1.90-2.05 (2H, m), 2.20-2.35 (2H, m), 2.60-2.80 (2H, m), 3.50 (2H, s), 4.45-4.60 (1H, m), 7.20-7.35 (6H, m), 7.40-7.65 (4H, m), 7.75-7.85 (1H, m), 8.07 (1H, brs), 8.33 (1H, brs), 10.92 (1H, brs)

Compound 1-241

$^1$H-NMR (DMSO-$d_6$) δ ppm: 1.60-1.75 (2H, m), 1.90-2.05 (2H, m), 2.20-2.35 (2H, m), 2.48 (3H, s), 2.60-2.75 (2H, m), 3.50 (2H, s), 4.45-4.60 (1H, m), 7.15-7.60 (10H, m), 7.91 (1H, brs), 8.15-8.20 (1H, m), 8.19 (1H, brs), 11.06 (1H, brs)

Compound 1-242

$^1$H-NMR (DMSO-$d_6$) δ ppm: 1.43 (3H, t, J=7.0Hz), 4.29 (2H, q, J=7.0Hz), 7.05-7.50 (8H, m), 7.55-7.82 (4H, m), 7.90 (1H, brs), 8.08 (1H, brs), 10.93 (1H, s)

Compound 1-243

$^1$H-NMR (DMSO-$d_6$) δ ppm: 1.43 (3H, t, J=7.0Hz), 4.29 (2H, q, J=7.0Hz), 7.05-7.30 (7H, m), 7.40-7.52 (2H, m), 7.65-7.75 (1H, m), 7.90 (1H, brs), 8.00-8.15 (3H, m), 10.88 (1H, s)

Compound 1-244

$^1$H-NMR (DMSO-$d_6$) δ ppm: 2.47 (3H, s), 7.05-7.25 (4H, m), 7.31 (1H, dd, J=2.2, 8.2Hz), 7.35-7.50 (3H, m), 7.53-7.65 (2H, m), 7.70-7.80 (1H, m), 7.88 (1H, brs), 8.12 (1H, d, J=8.2Hz), 8.16 (1H, brs), 11.08 (1H, s)

Compound 1-245

$^1$H-NMR (DMSO-$d_6$) δ ppm: 2.48(3H, s), 7.10-7.30(6H,m), 7.35-7.55 (3H, m), 7.89 (1H, brs), 7.95-8.07 (2H, m), 8.10-8.25 (2H, m), 11.03 (1H, s)

Compound 1-246

$^1$H-NMR (DMSO-$d_6$) δ ppm: 2.48 (3H, s), 7.00-7.10 (2H, m), 7.15-7.25 (1H, m), 7.35-7.55 (2H, m), 7.65-7.80 (2H, m), 7.90 (1H, brs), 7.90-8.00 (1H, m), 8.05-8.15 (1H, m), 8.18 (1H, brs), 8.45-8.55 (2H, m), 11.08 (1H, brs)

Compound 1-247

$^1$H-NMR (DMSO-$d_6$) δ ppm: 2.83 (4H, s), 7.50-7.80 (4H, m), 7.90-8.10 (3H, m), 8.20-8.35 (2H, m), 11.03 (1H, s)

Compound 1-248

$^1$H-NMR (DMSO-$d_6$) δ ppm: 3.82 (2H, s), 7.25-7.45 (6H, m),7.55-7.75 (2H, m), 7.86 (1H, brs), 8.08 (1H, brs), 10.25 (1H, brs)

Compound 1-249

$^1$H-NMR (DMSO-$d_6$) δ ppm: 3.82 (2H, s), 7.20-7.60 (7H, m), 7.94 (1H, brs), 8.16 (1H, brs), 10.27 (1H, s)

Compound 1-250

$^1$H-NMR (DMSO-$d_6$) δ ppm: 1.40-1.55 (3H, m), 3.95-4.10 (1H, m), 7.15-7.60 (7H, m), 7.93 (1H, brs), 8.15 (1H, brs), 10.26 (1H, brs)

Compound 1-251

$^1$H-NMR (DMSO-$d_6$) δ ppm: 1.85-2.00 (2H, m), 2.40-2.73 (4H, m), 7.15-7.40 (6H, m), 7.56-7.75 (2H, m), 7.88 (1H, brs), 8.11 (1H, brs), 10.10 (1H, s)

Compound 1-252

$^1$H-NMR (DMSO-$d_6$) δ ppm: 1.87-2.02 (2H, m), 2.40-2.73 (7H, m), 7.08-7.44 (7H, m), 7.78 (1H, brs), 7.88-7.96 (1H, m), 8. 00 (1H, brs), 10.11(1H, s)

Compound 1-253

$^1$H-NMR (DMSO-$d_6$) δ ppm: 1.60-1.70 (4H, m), 2.40-2.67 (4H, m), 7.11-7.41 (6H, m), 7.56-7.75 (2H, m), 7.88 (1H, brs), 8.11 (1H, brs), 10.10 (1H, s)

Compound 1-254

$^1$H-NMR (DMSO-$d_6$) δ ppm: 1.60-1.70 (4H, m), 2.40-2.67 (7H, m), 7.08-7.40 (7H, m), 7.77 (1H, brs), 7.86-7.94 (1H, m), 8.00 (1H, brs), 10.10 (1H, s)

Compound 1-255

$^1$H-NMR (DMSO-$d_6$) δ ppm: 3.77 (3H, s), 3.78 (2H, s), 6.80-7.05 (3H, m), 7.20-7.35 (2H, m), 7.55-7.75 (2H, m), 7.87 (1H, brs), 8.09 (1H, brs), 10.24 (1H, brs)

Compound 1-256

$^1$H-NMR (DMSO-$d_6$) δ ppm: 3.73 (2H, s), 3.74 (3H, s), 6.85-6.95 (2H, m), 7.25-7.40 (3H, m), 7.55-7.75 (2H, m), 7.86 (1H, brs), 8.08 (1H, brs), 10.20 (1H, brs)

Compound 1-257

$^1$H-NMR (DMSO-$d_6$) δ ppm: 1.84-1.95 (2H, m), 2.35-2.55 (5H, m), 2.59 (2H, t, J=7.7Hz), 3.71 (3H,s), 6.80-6.90 (2H, m), 7.10-7.20 (3H, m), 7.36 (1H, s), 7.77 (1H, brs), 7.90 (1H, d, J=8.2Hz), 8.00 (1H, brs), 10.09 (1H, s)

Compound 1-258

$^1$H-NMR (DMSO-$d_6$) δ ppm: 1.86-1.98 (2H, m), 2.40-2.47 (5H, m), 2.59 (2H, t, J=7.5Hz), 3.71 (3H, s), 3.74 (3H, s), 6.70-6.90 (3H, m), 7.10-7.20 (1H, m), 7.36 (1H, s), 7.77 (1H, brs), 7.92 (1H, d, J=8.1Hz), 8.01 (1H, brs), 10.10 (1H, s)

Compound 1-259

$^1$H-NMR (DMSO-$d_6$) δ ppm: 4.13 (2H, s), 6.89 (1H, s), 7.10-7.80 (7H, m), 7.92 (1H, brs), 8.14 (1H, brs), 10.44 (1H, brs)

Compound 1-260

$^1$H-NMR (DMSO-$d_6$) δ ppm: 2.45 (3H, s), 4.12 (2H, s), 6.88 (1H, s), 7.10-7.65 (6H, m), 7.80 (1H, brs), 7.88-7.96 (1H, m), 8.04 (1H, brs), 10.43(1H, brs)

Compound 1-261

$^1$H-NMR (DMSO-$d_6$) δ ppm: 1.92-2.03 (2H, m), 2.47-2.55 (2H, m), 2.89 (2H, t, J=7.5Hz), 6.85-7.00 (2H, m), 7.30-7.40 (2H, m), 7.58-7.75 (2H, m), 7.89 (1H, brs), 8.11 (1H, brs), 10.11 (1H, s)

Compound 1-262

$^1$H-NMR (DMSO-$d_6$) δ ppm: 1.90-2.04(2H,m), 2.45(3H, s), 2.47-2.53 (2H, m), 2.89 (2H, t, J=7.6Hz), 6.86-7.00 (2H, m), 7.13 (1H, d, J=8.1Hz), 7.30-7.40 (2H, m), 7.77 (1H, brs), 7.91 (1H, d, J=8.3Hz), 8.00 (1H, brs), 10.11 (1H, s)

Compound 1-263

$^1$H-NMR (DMSO-$d_6$) δ ppm: 1.50 (3H, d, J=6.9Hz), 2.18 (3H, s), 5.22 (1H, q, J=6.9Hz), 7.50-7.65 (1H, m), 7.65-7.80 (1H, m), 8.07 (1H, brs), 8.29 (1H, brs), 10.71 (1H, s)

Compound 1-264

$^1$H-NMR (DMSO-$d_6$) δ ppm: 3.45 (3H, s), 4.11 (2H, s), 7.50-7.65 (1H, m), 7.90-8.10 (2H, m), 8.29 (1H, brs), 10.74 (1H, s)

Compound 1-265

$^1$H-NMR (DMSO-$d_6$) δ ppm: 1.60 (3H, d, J=6.7Hz), 5.10 (1H, q, J=6.7Hz), 6.95-7.15 (3H, m), 7.25-7.40 (2H, m), 7.50-7.65 (1H, m), 7.80-7.95 (1H, m), 8.05 (1H, brs), 8.30 (1H, brs), 11.10 (1H, brs)

Compound 1-266

$^1$H-NMR (DMSO-$d_6$) δ ppm: 2.85-3.00 (2H, m), 4.25-4.40 (2H, m), 6.90-7.05 (2H, m), 7.20-7.70 (6H, m), 7.89 (1H, brs), 8.08 (1H, brs), 10.27 (1H, brs)

Compound 1-267

$^1$H-NMR (DMSO-$d_6$) δ ppm: 2.85-3.00 (2H, m), 4.20-4.40 (2H, m), 6.90-7.05 (3H, m), 7.20-7.45 (3H, m), 7.65-7.80 (1H, m), 7.90-8.05 (1H, m), 7.91 (1H, brs), 8.09 (1H, brs), 10.31 (1H, brs)

Compound 1-268

$^1$H-NMR (DMSO-$d_6$) δ ppm: 2.02-2.14 (2H, m), 2.45 (3H, s), 2.58-2.65 (2H, m), 3.98-4.10 (2H, m), 6.86-6.98 (3H, m), 7.08-7.38 (4H, m), 7.78 (1H, brs), 7.91 (1H, d, J=8.1Hz), 7.91 (1H, brs), 10.15 (1H, s)

Compound 1-269

$^1$H-NMR (DMSO-$d_6$) δ ppm: 2.03-2.13 (2H, m), 2.63 (2H, t, J=7.4Hz), 4.05 (2H, t, J=6.4Hz), 6.87-7.00 (3H, m), 7.22-7.40 (3H, m), 7.57-7.77 (2H, m), 7.89 (1H, brs), 8.11 (1H, brs), 10.15 (1H, s)

Compound 1-270

$^1$H-NMR (DMSO-$d_6$) δ ppm: 2.92 (2H, t, J=6.0Hz), 4.28 (2H, t, J=6.0Hz), 6.95-7.05 (2H, m), 7.08-7.18 (2H, m), 7.30-7.40 (1H, m), 7.55-7.70 (2H, m), 7.91 (1H, brs), 8.11 (1H, brs), 10.28 (1H, s)

Compound 1-271

$^1$H-NMR (DMSO-$d_6$) δ ppm: 3.85 (3H, s), 4.22 (2H, s), 4.69 (2H, s), 6.90-7.00 (1H, m), 7.02-7.10 (1H, m), 7.28-7.42 (3H, m), 7.46-7.54 (2H, m), 7.82 (1H, brs), 7.97 (1H, brs), 8.30 (1H, d, J=9.0Hz), 10.98 (1H, s)

Compound 1-272

$^1$H-NMR (DMSO-$d_6$) δ ppm: 3.83 (3H, s), 4.05 (2H, s), 6.85-6.95 (1H, m), 7.00-7.10 (1H, m), 7.15-7.50 (5H,

m), 7.77 (1H, brs), 7.85-8.00 (2H, m), 10.84 (1H, brs)

Compound 1-273

$^1$H-NMR (DMSO-$d_6$) δ ppm: 4.06 (2H, s), 7.15-7.60 (7H, m), 8.00 (1H, brs), 8.23 (1H, brs), 10.74 (1H, brs)

Compound 1-274

$^1$H-NMR (DMSO-$d_6$) δ ppm: 3.19-3.88 (8H, m), 7.36-7.48 (1H, m), 7.63-7.77 (3H, m), 7.88-8.16 (4H, m), 8.30 (1H, s), 11.03 (1H, s)

Compound 1-275

$^1$H-NMR (DMSO-$d_6$) δ ppm: 2.10-2.60 (7H, m), 3.10-3.80 (4H, m), 7.52-7.80(4H,m), 7.95-8.15 (3H, m), 8.32 (1H, brs), 10. 97 (1H, s)
MS (ESI, m/z): 443 (M+H)+

Compound 1-276

$^1$H-NMR (DMSO-$d_6$) δ ppm: 2.15 (3H, s), 2.30-2.45 (4H, m), 2.90-3.05 (4H, m), 7.55-7.65 (1H, m), 7.70-7.80 (1H, m), 7.85-8.05 (2H, m), 8.06 (1H, brs), 8.25-8.40 (3H, m), 11.09 (1H, brs)

Compound 1-277

$^1$H-NMR (DMSO-$d_6$) δ ppm: 2.40-2.50 (4H, m), 2.90-3.05 (4H, m), 3.47 (2H, s), 7.15-7.35 (5H, m); 7.50-7.65 (1H, m), 7.70-7.80 (1H, m), 7.85-8.05 (2H, m), 8.06 (1H, brs), 8.25-8.40 (3H, m), 11.09 (1H, brs)

Compound 1-278

$^1$H-NMR (DMSO-$d_6$) δ ppm: 2.48 (3H, s), 7.15-7.45 (3H, m), 7.95 (1H, brs), 8.05-8.15 (1H, m), 8.22 (1H, brs), 8.80-8.90 (1H, m), 11.34 (1H, brs)

Compound 1-279

$^1$H-NMR (DMSO-$d_6$) δ ppm: 7.25-7.50 (2H, m), 7.60-7.75 (1H, m), 7.90-8.00 (1H, m), 8.02 (1H, brs), 8.35 (1H, brs), 8.80-8.95 (1H, m), 11.27 (1H, brs)

Compound 1-280

$^1$H-NMR (DMSO-$d_6$) δ ppm: 7.25-7.45 (2H, m), 7.60-7.70 (1H, m), 7.85-8.00 (3H, m), 8.00 (1H, brs), 8.26 (1H, brs), 10.94 (1H, brs)

Compound 1-281

$^1$H-NMR (DMSO-$d_6$) δ ppm: 6.85-6.95 (1H, m), 7.30-7.50 (2H, m), 7.60-7.70 (1H, m), 7.95-8.05 (2H, m), 8.28 (1H, brs), 11.00 (1H, brs)

Compound 1-282

$^1$H-NMR (DMSO-$d_6$) δ ppm: 1.26 (3H, t, J=7.6Hz), 2.47 (3H, s), 2.75 (2H, q, J=7.6Hz), 6.35-6.45 (1H,m), 7.18 (1H, d, J=8.2Hz), 7.24 (1H, d, J=3.5Hz), 7.39 (1H, s), 7.89 (1H, brs), 8.16 (1H, brs), 8.27 (1H, d, J=8.2Hz), 11.11 (1H, s)

Compound 1-283

$^1$H-NMR (DMSO-$d_6$) δ ppm: 1.20-1.30 (6H, m), 2.70-2.82 (4H, m), 6.41 (1H, d, J=3.2Hz), 7.21 (1H, dd, J=1.6, 8.2Hz), 7.25 (1H, d, J=3.2Hz), 7.40 (1H, s), 7.89 (1H, brs), 8.14 (1H, brs), 8.29 (1H, d, J=8.2Hz), 11.10 (1H, s)

Compound 1-284

$^1$H-NMR (DMSO-$d_6$) δ ppm: 1.20-1.30 (9H, m), 2.75 (2H, q, J=7.6Hz), 3.00-3.15 (1H, m), 6.35-6.45 (1H, m), 7.20-7.30 (2H, m), 7.35-7.45 (1H, m), 7.90 (1H, brs), 8.12 (1H, brs), 8.29 (1H, d, J=8.5Hz), 11.08 (1H, s)

Compound 1-285

$^1$H-NMR (DMSO-$d_6$) δ ppm: 1.27 (3H, t, J=7.6Hz), 1.36 (9H, s), 2.76 (2H, q, J=7.6Hz), 6.42 (1H, d, J=3.2Hz), 7.26 (1H, d, J=3.2Hz), 7.44 (1H, dd, J=1.9, 8.5Hz), 7.48 (1H, d, J=1.9Hz), 7.93 (1H, brs), 8.13 (1H, brs), 8.30 (1H, d, J=8.5Hz), 11.10 (1H, s)

Compound 1-286

$^1$H-NMR (DMSO-$d_6$) δ ppm: 1.27 (3H, t, J=7.6Hz), 2.76 (2H, q, J=7.6Hz), 6.40-6.45 (1H, m), 7.29 (1H, d, J=3.5Hz), 7.70 (1H, dd, J=1.3, 8.5Hz), 7.97 (1H, s), 8.10 (1H, brs), 8.33 (1H, brs), 8.55 (1H, d, J=8.5Hz), 11.02 (1H, s)

Compound 1-287

$^1$H-NMR (DMSO-$d_6$) δ ppm: 1.26 (3H, t, J=7.6Hz), 1.37 (3H, t, J=6.9Hz), 2.75 (2H, q, J=7.6Hz), 4.13 (2H, q, J=6.9Hz), 6.41 (1H, d, J=3.2Hz), 6.96 (1H, dd, J=2.2, 8.8Hz), 7.06 (1H, d, J=2.2Hz), 7.24 (1H, d, J=3.2Hz), 7.84 (1H, brs), 8.03 (1H, brs), 8.31 (1H, d, J=8.8Hz), 11.18 (1H, s)

Compound 1-288

$^1$H-NMR ($CDCl_3$) δ ppm: 1. 34 (3H, t, J=7.6Hz), 2.79 (2H, q, J=7.6Hz), 5.67 (1H, brs), 6.15-6.25 (1H, m), 6.34 (1H, brs), 7.20-7.30 (2H, m), 8.55-8.65 (1H, m), 10.80 (1H, brs)

Compound 1-289

$^1$H-NMR (DMSO-$d_6$) δ ppm: 1.26 (3H, t, J=7.6Hz), 2.30-2.45 (3H, m), 2.75 (2H, q, J=7.6Hz), 6.42 (1H, d, J=3.5Hz), 7.26 (1H, d, J=3.5Hz), 7.54 (1H, d, J=3.5Hz), 7.97 (1H, brs), 8.10 (1H, d, J=10.4Hz), 8.25 (1H, brs), 11.13 (1H, s)

Compound 1-290

$^1$H-NMR (DMSO-$d_6$) δ ppm: 1.26 (3H, t, J=7.5Hz), 2.75 (2H, q, J=7.5Hz), 3.99 (3H, s), 6.35-6.45 (1H, m), 7.05-7.30 (1H, m), 7.60-7.70 (1H, m), 7.99 (1H, brs), 8.23 (1H, brs), 11.03 (1H, brs)

Compound 1-291

$^1$H-NMR (DMSO-$d_6$) δ ppm: 1.34 (9H, s), 6.41 (1H, d, J=3.5Hz), 7.26 (1H, d, J=3.5Hz), 7.55-7.70 (1H, m), 8.04-8.10 (1H, m), 8.19 (1H, brs), 8.41 (1H, brs), 11.30 (1H, s)

Compound 1-292

$^1$H-NMR (DMSO-$d_6$) δ ppm: 0.82-0.95 (3H, m), 1.27-1.39 (4H, m), 1.62-1.75 (2H, m), 2.73 (2H, t, J=7.6Hz), 6.43 (1H, d, J=3.2Hz), 7.22-7.46 (2H,m), 7.60-7.70 (1H, m), 8.00 (1H, brs), 8.08-8.16 (1H, m), 8.27 (1H, brs), 11.03 (1H, s)

Compound 1-293

$^1$H-NMR (DMSO-$d_6$) δ ppm: 0.82-0.95 (3H, m), 1.25-1.40 (4H, m), 1.60-1.80 (2H, m), 2.47 (3H, s), 2.72 (2H, t, J=7.6Hz), 6.42 (1H, d, J=3.5Hz), 7.13-7.30 (2H, m), 7.39 (1H, s), 7.88 (1H, brs), 8.15 (1H, brs), 8.27 (1H, d, J=8.6Hz), 11.07 (1H, s)

Compound 1-294

$^1$H-NMR (DMSO-$d_6$) δ ppm: 2.00 (3H, s), 2.32 (3H, s), 7.09-7.72 (3H, m), 7.92-8.40 (3H, m), 10.95 (1H, s)

Compound 1-295

$^1$H-NMR (DMSO-$d_6$) δ ppm: 2.00 (3H, s), 2.32 (3H, s), 2.47 (3H, s), 7.11-7.20(2H, m), 7.38 (1H, s), 7.85 (1H, brs), 8.14 (1H, brs), 8.26 (1H, d, J=8.1Hz), 10.98 (1H, s)

Compound 1-296

$^1$H-NMR ($CDCl_3$+MeOD-$d_4$) δ ppm: 4.67 (2H, s), 6.55-6.60 (1H, m), 7.15-7.30 (2H, m), 7.35-7.45 (1H, m), 8.25-8.35 (1H, m)

Compound 1-297

$^1$H-NMR (DMSO-$d_6$) δ ppm: 4.93 (2H, s), 6.81 (1H, d, J=3.5Hz), 7.34 (1H, d, J=3.5Hz), 7.43 (1H, dd, J=1.9, 9.1Hz), 7.75 (1H, d, J=1.9Hz), 8.00 (1H, brs), 8.20-8.35 (2H, m), 11.07 (1H, s)

Compound 1-298

$^1$H-NMR (DMSO-$d_6$) δ ppm: 4.93 (2H, s), 6.82 (1H, d, J=3.5Hz), 7.36 (1H, d, J=3.5Hz), 7.55-7.65 (1H, m), 7.80-7.90 (1H, m), 8.08 (1H, brs), 8.36 (1H, brs), 11.00 (1H, s)

Compound 1-299

$^1$H-NMR (DMSO-$d_6$) δ ppm: 0.85-1.07 (4H, m), 2.04-2.15 (1H, m), 6.35-6.46 (1H, m), 7.18-7.28 (1H, m), 7.33-7.49 (1H, m), 7.57-7.70 (1H, m), 7.94-8.39 (3H, m), 11.08 (1H, s)

Compound 1-300

$^1$H-NMR (DMSO-$d_6$) δ ppm: 0.90-1.09 (4H, m), 2.05-2.17 (1H, m), 6.39-6.46 (1H, m), 7.20-7.33 (2H, m), 7.48-7.60 (1H, m), 8.00 (1H, brs), 8.24 (1H, brs), 8.43-8.50 (1H, m), 11.14 (1H, s)

Compound 1-301

$^1$H-NMR (DMSO-$d_6$) δ ppm: 0.90-1.09 (4H, m), 2.06-2.18 (1H, m), 6.38-6.47 (1H, m), 7.21-7.30 (1H, m), 7.66-7.76 (1H, m), 8.06-8.33 (3H, m), 11.03 (1H, s)

Compound 1-302

$^1$H-NMR (DMSO-$d_6$) δ ppm: 0.90-1.05 (4H, m), 2.05-2.15 (1H, m), 3.86 (3H, s), 6.42 (1H, d, J=3.4Hz), 6.95-7.10 (2H, m), 7.22 (1H, d, J=3.4Hz), 7.90 (1H, brs), 8.06 (1H, brs), 8.34 (1H, d, J=9.0Hz), 11.19 (1H, s)

Compound 1-303

$^1$H-NMR (DMSO-$d_6$) δ ppm: 2.25 (6H, s), 2.47 (3H, s), 3.60 (2H, s), 6.55-6.65 (1H, m), 7.15-7.20 (1H, m), 7.25-7.45 (2H, m), 7.90 (1H, brs), 8.16 (1H, brs), 8.20-8.30 (1H, m), 11.08 (1H, brs)

Compound 1-304

$^1$H-NMR (DMSO-$d_6$) δ ppm: 2.56 (3H, s), 7.35-7.70 (4H, m), 8.00-8.10 (1H, m), 8.11 (1H, brs), 8.35 (1H, brs), 11.38 (1H, brs)

Compound 1-305

$^1$H-NMR (DMSO-$d_6$) δ ppm: 1.40-1.75 (8H, m), 3.35-3.45 (4H, m), 7.30-7.75 (4H, m), 8.00-8.10 (1H, m), 8.10 (1H, brs), 8.35 (1H, brs), 11.27 (1H, brs)

Compound 1-306

$^1$H-NMR ($CDCl_3$+MeOD-$d_4$) δ ppm: 2.32 (3H, s), 2.50-2.60 (4H, m), 3.35-3.45 (4H, m), 7.00-7.10 (1H, m), 7.15

(1H, d, J=3.7Hz), 7.36 (1H, d, J=3.7Hz), 8.05-8.15 (1H, m)

Compound 1-307

MS (ESI, m/z): 428 (M+H)+

Compound 1-308

MS (ESI, m/z): 476 (M+H)+

Compound 1-309

MS (ESI, m/z): 490 (M+H)+

Compound 1-310

$^1$H-NMR (DMSO-$d_6$) δ ppm: 7.35-7.70 (7H, m), 8.05-8.20 (2H, m), 8.13 (1H, brs), 8.36 (1H, brs), 11.39 (1H, brs)

Compound 1-311

$^1$H-NMR (DMSO-$d_6$) δ ppm: 7.35-7.70 (6H, m), 7.85-7.95 (1H, m), 8.00-8.20 (3H, m), 8.35 (1H, brs), 11.39 (1H, brs)

Compound 1-312

$^1$H-NMR (DMSO-$d_6$) δ ppm: 7.05-7.25 (3H, m), 7.30-7.70 (3H, m), 7.90-8.00 (2H, m), 8.10-8.20 (2H, m), 8.36 (1H, brs), 11. 40 (1H, brs)

Compound 1-313

$^1$H-NMR (DMSO-$d_6$) δ ppm: 2.48 (3H, s), 7.15-7.75 (7H, m), 7.85-8.45 (4H, m), 11.45 (1H, brs)

Compound 1-314

$^1$H-NMR (DMSO-$d_6$) δ ppm: 2.48 (3H, s), 7.15-7.25 (1H, m), 7.35-7.60 (5H, m), 7.85-8.05 (3H, m), 8.24 (1H, brs), 8.25-8.35 (1H, m), 11.45 (1H, brs)

Compound 1-315

$^1$H-NMR (DMSO-$d_6$) δ ppm: 2.48 (3H, s), 7.15-7.25 (1H, m), 7.30-7.35 (1H, m), 7.40-7.50 (2H, m), 7.55-7.65 (2H, m), 7.90-8.00 (2H, m), 8.03 (1H, brs), 8.25 (1H, brs), 8.25-8.35 (1H, m), 11.46 (1H, brs)

Compound 1-316

$^1$H-NMR (DMSO-$d_6$) δ ppm: 4.83 (2H, s), 7.30 (1H, d, J=4.0Hz), 7.45-7.70 (4H, m), 7.91 (1H, d, J=7.6Hz), 7.95-8.05 (2H, m), 8.17 (1H, brs), 8.40 (1H, brs), 11.31 (1H, s)
MS (ESI, m/z): 431 (M+H)+

Compound 1-317

$^1$H-NMR (DMSO-$d_6$) δ ppm: 2.48 (3H, s), 3.97 (3H, s), 7.05-7.25 (4H, m), 7.35-7.50 (3H, m), 8.00-8.10 (2H, m), 8.34 (1H, brs), 8.30-8.40 (1H, m), 11.52 (1H, brs)

Compound 1-318

$^1$H-NMR (DMSO-$d_6$) δ ppm: 2.48 (3H, s), 3.84 (3H, s), 7.00-7.25 (4H, m), 7.35-7.45 (2H, m), 7.80-7.95 (2H, m), 8.02 (1H, brs), 8.24 (1H, brs), 8.30-8.40 (1H, m), 11.48 (1H, brs)

Compound 1-319

$^1$H-NMR (DMSO-$d_6$) δ ppm: 3.98 (3H, s), 7.05-7.25 (3H, m), 7.35-7.50 (3H, m), 7.60-7.70 (1H, m), 8.00-8.10 (1H, m), 8.17 (1H, brs), 8.20-8.25 (1H, m), 8.37 (1H, brs), 11.47 (1H, brs)

Compound 1-320

$^1$H-NMR (DMSO-$d_6$) δ ppm: 3.87 (3H, s), 6.95-7.05 (1H, m), 7.25-7.70 (7H, m), 8.14 (1H, brs), 8.20-8.30 (1H, m), 8.37 (1H, brs), 11.51 (1H, brs)

Compound 1-321

$^1$H-NMR (DMSO-$d_6$) δ ppm: 3.84 (3H, s), 7.05-7.15 (3H, m), 7.40-7.50 (1H, m), 7.60-7.70 (1H, m), 7.80-7.90 (3H, m), 8.14 (1H, brs), 8.20-8.25 (1H, m), 8.36 (1H, brs), 11.42 (1H, brs)

Compound 1-322

$^1$H-NMR (DMSO-$d_6$) δ ppm: 7.30-7.70 (5H, m), 7.90-8.05 (2H, m), 8.10-8.20 (1H, m), 8.12 (1H, brs), 8.34 (1H, brs), 8.65-8.70 (1H, m), 11.43 (1H, brs)

Compound 1-323

$^1$H-NMR (DMSO-$d_6$) δ ppm: 7.35-7.70 (5H, m), 8.15 (1H, brs), 8.15-8.35 (1H, m), 8.35 (1H, brs), 8.55-8.65 (1H, m), 9.15-9.25 (1H, m), 11.52 (1H, brs)

Compound 1-324

$^1$H-NMR (DMSO-$d_6$) δ ppm: 7.35-7.90 (7H, m), 8.10-8.20 (1H, m), 8.35 (1H, brs), 8.65-8.75 (2H, m), 11.51 (1H, brs)

Compound 1-325

$^1$H-NMR (DMSO-$d_6$) δ ppm: 2.48 (3H, s), 7.15-7.25 (1H, m), 7.35-7.60 (3H, m), 7.80-7.90 (2H, m), 8.05 (1H, brs), 8.24 (1H, brs), 8.25-8.40 (1H, m), 8.65-8.75 (2H, m), 11.58 (1H, brs)

Compound 1-326

MS (ESI, m/z): 382 (M-H)-

Compound 1-327

$^1$H-NMR (DMSO-$d_6$) δ ppm: 3.00-3.13 (4H, m), 6.39 (1H, d, J=3.5Hz), 7.14-7.70 (8H, m), 8.00-8.16 (2H, m), 8.32 (1H, brs), 11. 13 (1H, s)

Compound 1-328

$^1$H-NMR (DMSO-$d_6$) δ ppm: 2.47 (3H, s), 3.00-3.10 (4H, m), 6.38 (1H, d, J=3.5Hz), 7.13-7.33 (7H, m), 7.40 (1H, s), 7.93 (1H, brs), 8.14-8.32 (2H, m), 11.17 (1H, s)

Compound 1-329

$^1$H-NMR (DMSO-$d_6$) δ ppm: 2.47 (3H, s), 2.99 (4H, s), 3.80 (3H, s), 6.30-6.44 (1H, m), 6.79-7.02 (2H, m), 7.10-7.45 (5H, m), 7.80-8.35 (3H, m), 11.12 (1H, s)

Compound 1-330

$^1$H-NMR (DMSO-$d_6$) δ ppm: 2.47 (3H, s), 2.95-3.15 (4H, m), 3.71 (3H, s), 6.33-6.44 (1H, m), 6.66-6.90 (3H, m), 7.09-7.45 (4H, m), 7.80-8.35 (3H, m), 11.16 (1H, s)

Compound 1-331

$^1$H-NMR (DMSO-$d_6$) δ ppm: 2.47 (3H, s), 2.90-3.10 (4H, m), 3.70 (3H, s), 6.30-6.45 (1H, m), 6.73-7.45 (7H, m), 7.80-8.35 (3H, m), 11.15 (1H, s)

Compound 1-332

$^1$H-NMR (DMSO-$d_6$) δ ppm: 2.99 (4H, s), 3.81 (3H, s), 6.33-6.42 (1H, m), 6.80-7.03 (2H, m), 7.10-7.70 (5H, m), 8.01 (1H, brs), 8.07-8.16 (1H, m), 8.27 (1H, brs), 11.07 (1H, s)

Compound 1-333

$^1$H-NMR (DMSO-$d_6$) δ ppm: 2.90-3.15 (4H, m), 3.71 (3H, s), 6.39 (1H, d, J=3.5Hz), 6.70-6.90 (3H, m), 7.10-7.73 (4H, m), 8.03 (1H, brs), 8.08-8.17 (1H, m), 8.28 (1H, brs), 11.11 (1H, s)

Compound 1-334

$^1$H-NMR (DMSO-$d_6$) δ ppm: 2.90-3.10 (4H, m), 3.70 (3H, s), 6.37 (1H, d, J=3.3Hz), 6.75-6.90 (2H, m), 7.10-7.73 (5H, m), 8.03 (1H, brs), 8.08-8.17 (1H, m), 8.28 (1H, brs), 11.10 (1H, s)

Compound 1-335

$^1$H-NMR (DMSO-$d_6$) δ ppm: 7.40-7.50 (1H, m), 7.70-7.80 (2H, m), 7.95 (1H, brs), 8.10-8.30 (3H, m), 8.50-8.60 (1H, m), 8.75-8.80 (1H, m), 12.09 (1H, brs)

Compound 1-336

$^1$H-NMR (DMSO-$d_6$) δ ppm: 7.50-7.80 (2H, m), 8.03 (1H, brs), 8.05-8.30 (3H, m), 8.35 (1H, brs), 8.70-8.85 (1H, m), 12.05 (1H, brs)

Compound 1-337

$^1$H-NMR (DMSO-$d_6$) δ ppm: 3.87 (3H, s), 6.95-7.05 (1H, m), 7.09 (1H, d, J=2.3Hz), 7.60-7.90 (2H, m), 8.00 (1H, brs), 8.05-8.20 (1H, m), 8.24 (1H, d, J=8.0Hz), 8.46 (1H, d, J=8.7Hz), 8.77 (1H, d, J=4.3Hz), 12.11 (1H, s)

Compound 1-338

$^1$H-NMR (DMSO-$d_6$) δ ppm: 4.00 (3H, s), 7.10-7.20(1H, m), 7.70-7.75 (1H, m), 7.75-7.85 (1H, m), 7.91 (1H, brs), 8.05-8.15 (1H, m), 8.15-8.30 (2H, m), 8.75-8.80 (1H, m), 12.03 (1H, brs)

Compound 1-339

$^1$H-NMR (DMSO-$d_6$) δ ppm: 2.62 (3H, s), 7.55-7.65 (2H, m), 7.95-8.10 (3H, m), 8.15-8.25 (1H, m), 8.35 (1H, brs), 12.26 (1H, brs)

Compound 1-340

$^1$H-NMR (DMSO-$d_6$) δ ppm: 1.30-1.40 (3H, m), 4.35-4.50 (2H, m), 6.95-7.05 (1H, m), 7.35-7.45 (1H, m), 7.60-7.75 (1H, m), 7.85-7.95 (1H, m), 8.00 (1H, brs), 8.15-8.35 (2H, m), 8.80-8.85 (1H, m), 10.90 (1H, s)

Compound 1-341

$^1$H-NMR (DMSO-$d_6$) δ ppm: 3.87 (3H, s), 6.99 (1H. dd, J=2.2, 8.8Hz), 7.11 (1H, d, J=2.2Hz), 7.15-7.35 (4H, m), 7.40-7.55 (2H, m), 7.88 (1H, brs), 8.07 (1H, brs), 8.12 (1H, d, J=8.8Hz), 8.38 (1H, dd, J=2.5, 8.8Hz), 8.76 (1H, d, J=2.5Hz), 11.05 (1H, s)

Compound 1-342

$^1$H-NMR (DMSO-$d_6$) δ ppm: 1.43 (3H, t, J=6.9Hz), 4.29 (2H, q, J=6.9Hz), 7.05-7.35 (6H, m), 7.40-7.65 (3H, m), 7.91 (1H, brs), 8.08 (1H, brs), 8.40 (1H, dd, J=2.5, 8.5Hz), 8.78 (1H, d, J=2.5Hz), 10.83 (1H, s)

Compound 1-343

$^1$H-NMR (DMSO-$d_6$) δ ppm: 2.48(3H, s), 7.15-7.35 (5H, m), 7.38-7.55 (3H, m), 7.88 (1H, brs), 8.07 (1H, d, J=8.2Hz), 8.14 (1H, brs), 8.39 (1H, dd, J=2.5, 8.2Hz), 8.70-8.80 (1H, m), 10.97 (1H, s)

Compound 1-344

$^1$H-NMR (DMSO-$d_6$) δ ppm: 1.25 (3H, t, J=7.6Hz), 2.77 (2H, q, J=7.6Hz), 7.15-7.35 (5H, m), 7.40-7.50 (3H, m), 7.89(1H, brs), 8.07 (1H, d, J=8.5Hz), 8.12 (1H, brs), 8.39 (1H, dd, J=2.5, 8.5Hz), 8.77 (1H, d, J=2.5Hz), 10.96 (1H, s)

Compound 1-345

$^1$H-NMR (DMSO-$d_6$) δ ppm: 7.15-7.35 (4H, m), 7.40-7.55 (2H, m), 7.80-7.90 (1H, m), 7.99 (1H, brs), 8.10-8.20 (1H, m), 8.23 (1H, brs), 8.35-8.45 (1H, m), 8.70-8.80 (1H, m), 10.95 (1H, brs)

Compound 1-346

$^1$H-NMR (DMSO-$d_6$) δ ppm: 7.15-7.35 (4H, m), 7.40-7.65 (3H, m), 7.70 (1H, dd, J=2.2, 8.8Hz), 8.07 (1H, brs), 8.33 (1H, brs), 8.40 (1H, dd, J=2.5, 8.8Hz), 8.77 (1H, d, J=2.5Hz), 10.87 (1H, s)

Compound 1-347

$^1$H-NMR (DMSO-$d_6$) δ ppm: 2.39 (3H, s), 7.15-7.35 (4H, m), 7.40-7.60 (3H, m), 7.87 (1H, d, J=10.1Hz), 7.97 (1H, brs), 8.23 (1H, brs), 8.39 (1H, dd, J=2.2, 8.5Hz), 8.76 (1H, d, J=2.2Hz), 10.97 (1H, s)

Compound 1-348

$^1$H-NMR (DMSO-$d_6$) δ ppm: 4.00 (3H, s), 7.05-7.55 (8H, m), 7.95 (1H, brs), 8.17 (1H, brs), 8.35-8.45 (1H, m), 8.70-8.80 (1H, m), 10.88 (1H, brs)

Compound 1-349

$^1$H-NMR (DMSO-$d_6$) δ ppm: 7.25-7.50 (6H, m), 7.60-7.75 (1H, m), 7.86 (1H, dd, J=2.8, 9.1Hz), 7.97 (1H, brs), 8.22 (1H, brs), 8.43 (1H, dd, J=2.5, 8.5Hz), 8.74 (1H, d, J=2.5Hz), 10.88 (1H, s)

Compound 1-350

$^1$H-NMR (DMSO-$d_6$) δ ppm: 7.00-7.33 (4H, m), 7.35-7.55 (2H, m), 7.63-7.73 (1H, m), 7.87 (1H, dd, J=2.8, 9.1Hz), 7.98 (1H, brs), 8.24 (1H, brs), 8.42 (1H, brs), 8.79 (1H, d, J=2.2Hz), 10.91 (1H, s)

Compound 1-351

$^1$H-NMR (DMSO-$d_6$) δ ppm: 7.15-7.45 (6H, m), 7.60-7.70 (1H, m), 7.87 (1H, dd, J=2.5, 9.5Hz), 7.98 (1H, brs), 8.23 (1H, brs), 8.35-8.45 (1H, m), 8.76 (1H, d, J=2.5Hz), 10.90(1H, s)

Compound 1-352

$^1$H-NMR (DMSO-$d_6$) δ ppm: 7.10-7.25 (1H, m), 7.30-7.55 (4H, m), 7.67 (1H, dd, J=4.1, 8.8Hz), 7.85 (1H, dd, J=2.5, 9.1Hz), 7.98 (1H, brs), 8.25 (1H, brs), 8.43 (1H, dd, J=2.2, 8.8 Hz), 8.74 (1H, dd, J=2.2 Hz), 10.88 (1H, s)

Compound 1-353

$^1$H-NMR (DMSO-$d_6$) δ ppm: 7.30-7.55 (4H, m), 7.68 (1H, dd, J=4.1, 9.1 Hz), 7.83 (1H, dd, J=2.5, 9.1Hz), 7.98

(1H, brs), 8.24 (1H, brs), 8.48 (1H, dd, J=2.2, 8.5 Hz), 8.74 (1H, d, J=2.2Hz), 10.88 (1H, s)

Compound 1-354

$^1$H-NMR (DMSO-$d_6$) δ ppm: 1.15 (6H, d, J=6.9Hz), 2.95-3.10 (1H, m), 7.05-7.13 (1H, m), 7.15-7.33 (3H, m), 7.35-7.50 (2H, m), 7.67 (1H, dd, J=4.1, 9.1 Hz), 7.86 (1H, dd, J=2.5, 9.1 Hz), 7.97 (1H, brs), 8.22 (1H, brs), 8.39 (1H, dd, J=2.5, 8.5Hz), 8.75 (1H, d, J=2.5Hz), 10.87 (1H, s)

Compound 1-355

$^1$H-NMR (DMSO-$d_6$) δ ppm: 1.24 (6H, d, J=6.9Hz), 2.85-3.05 (1H, m), 7.05-7.25 (3H, m), 7.30-7.45 (3H, m), 7.60-7.70 (1H, m), 7.88 (1H, dd, J=2.8, 9.1Hz), 7.98(1H, brs), 8.24 (1H, brs), 8.38 (1H, dd, J=2.5, 8.8Hz), 8.76 (1H, d, J=2.5Hz), 10.90 (1H, s)

Compound 1-356

$^1$H-NMR (DMSO-$d_6$) δ ppm: 2.10 (3H, s), 7.05-7.30 (4H, m), 7.35-7.45 (1H, m), 7.67 (1H, dd, J=4.1, 9.1Hz), 7.86 (1H, dd, J=2.8, 9.1Hz), 7.99 (1H, brs), 8.25 (1H, brs), 8.40 (1H, dd, J=2.5, 8.8Hz), 8.73 (1H, d, J=2.5Hz), 10.88 (1H, s)

Compound 1-357

$^1$H-NMR (DMSO-$d_6$) δ ppm: 4.80 (2H, s), 7.15-7.75 (7H, m), 8.04 (1H, brs), 8.30 (1H, brs), 8.35-8.45 (1H, m), 8.75-8.85 (1H, m), 10.86 (1H, brs)

Compound 1-358

$^1$H-NMR (DMSO-$d_6$) δ ppm: 3.71 (3H, s), 6.95-7.45 (6H, m), 7.67 (1H, dd, J=4.1, 9.1Hz), 7.87 (1H, dd, J=2.8, 9.1Hz), 7.97 (1H, brs), 8.23 (1H, brs), 8.36 (1H, dd, J=2.2, 8.5Hz), 8.71 (1H, d, J=2.2Hz), 10.87 (1H, s)

Compound 1-359

$^1$H-NMR (DMSO-$d_6$) δ ppm: 3.77 (3H, s), 6.70-7.00 (3H, m), 7.20 ($^1$H, d, J=8.5Hz), 7.30-7.50(2H,m), 7.60-7.75 (1H,m), 7.80-7.92 (1H, m), 7.98 (1H, brs), 8.23 (1H, brs), 8.39 (1H, dd, J=1.9, 8.5Hz), 8.79 (1H, s), 10.90 (1H, s)

Compound 1-360

$^1$H-NMR (DMSO-$d_6$) δ ppm: 3.79 (3H, s), 6.95-7.05 (2H, m), 7.10-7.20 (3H, m), 7.35-7.45 (1H, m), 7.63-7.70 (1H, m), 7.85-7.93 (1H, m), 7.98 (1H, brs), 8.23 (1H, brs), 8.37 (1H, dd, J=2.5, 8.8 Hz), 8.76 (1H, d, J=2.5 Hz), 10.89 (1H, s)

Compound 1-361

$^1$H-NMR (DMSO-$d_6$) δ ppm: 3.73 (3H, s), 6.75-6.90 (1H, m), 7.05-7.30 (3H, m), 7.35-7.45 (1H, m), 7.67 (1H, dd, J=4.1, 9.1Hz), 7.87 (1H, dd, J=2.5, 9.1Hz), 7.97 (1H, brs), 8.23 (1H, brs), 8.36 (1H, dd, J=2.5, 8.8Hz), 8.71 (1H, d, J=2.5Hz), 10.86 (1H, s)

Compound 1-362

$^1$H-NMR (DMSO-$d_6$) δ ppm: 7.30-7.47 (2H, m), 7.48-7.60 (1H, m), 7.60-7.80 (2H, m), 7.86 (1H, dd, J=2.5, 9.1Hz), 7.98 (1H, brs), 8.24 (1H, brs), 8.44 (1H, dd, J=2.5, 8.5Hz), 8.47-8.58 (2H, m), 8.76 (1H, d, J=2.2Hz), 10.91 (1H, s)

Compound 1-363

$^1$H-NMR (DMSO-$d_6$) δ ppm: 2.16 (3H, s), 7.45-7.60 (2H, m), 7.93 (1H, brs), 8.14 (1H, brs), 10.08 (1H, brs)

Compound 1-364

$^1$H-NMR (DMSO-$d_6$) δ ppm: 1.13 (3H, t, J=7.5Hz), 2.46 (2H, q, J=7.5Hz), 7.45-7.65 (2H, m), 7.92 (1H, brs), 8.19 (1H, brs), 10.09 (1H, s)

Compound 1-365

$^1$H-NMR (DMSO-$d_6$) δ ppm: 1.18 (6H, d, J=7.0Hz), 2.65-2.80 (1H, m), 7.45-7.70 (2H, m), 7.95 (1H, brs), 8.19 (1H, brs), 10.15 (1H, s)
MS (ESI, m/z): 283 (M+H)+

Compound 1-366

$^1$H-NMR (DMSO-$d_6$) δ ppm: 1.28 (9H, s), 7.50-7.60 (1H, m), 7.80-7.90 (1H, m), 8.03 (1H, brs), 8.29 (1H, brs), 10.43 (1H, s)

Compound 1-367

$^1$H-NMR (DMSO-$d_6$) δ ppm: 4.29 (2H, s), 7.45-7.62 (2H, m), 8.02 (1H, brs), 8.25 (1H, brs), 10.65 (1H, s)

Compound 1-368

$^1$H-NMR (DMSO-$d_6$) δ ppm: 1.70-1.80 (2H, m), 1.85-1.95 (2H, m), 2.40-2.60 (2H, m), 3.59 (2H, t, J=6.7Hz), 7.45-7.60 (2H, m), 7.94 (1H, brs), 8.16 (1H, brs), 10.10 (1H, s)

Compound 1-369

$^1$H-NMR (DMSO-$d_6$) δ ppm: 1.40-1.55 (2H, m), 1.60-1.75 (2H, m), 1.80-1.95 (2H, m), 2.46 (2H, t, J=7.4Hz), 3.55 (2H, t, J=6.8Hz), 7.45-7.60 (2H, m), 7.93 (1H, brs), 8.15 (1H, brs), 10.09 (1H, s)

Compound 1-370

$^1$H-NMR (DMSO-$d_6$) δ ppm: 3.08 (2H, t, J=7.0Hz), 4.59 (2H, t, J=7.0Hz), 6.90-7.05 (1H, m), 7.10-7.75 (7H, m), 7.80-8.35 (4H, m), 8.75-8.90 (1H, m), 10.90 (1H, s)

Example 3

6-Methoxy-3-[3-(4-phenylpiperazin-1-ylmethyl)benzoylamino]-benzofuran-2-carboxamide (compound 2-1)

Triethylamine (0.035mL) and 4-phenylpiperazine (76mg) were added to a solution of 3-(3-chloromethylbenzoylamino)-6-methoxybenzofuran-2-carboxamide (compound 1-57, 73mg) in tetrahydrofuran (1mL), and the resulting mixture was stirred at 60˚ C overnight. Water was added to the reaction mixture, and the resulting precipitate was collected by filtration to give the title compound (67mg).
$^1$H-NMR (DMSO-$d_6$) δ ppm: 2.50-2.60 (4H, m), 3.10-3.20 (4H, m), 3.30 (2H, s), 3.87 (3H, s), 6.70-6.80 (1H, m), 6.85-7.15 (6H, m), 7.55-7.70 (2H, m), 7.75-8.15 (4H, m), 8.20-8.30 (1H, m), 11.19 (1H, brs)

Compounds 2-2 to 2-145 were prepared in a manner similar to those as described in Example 3 using the corresponding benzylhalides or 2-chloromethylfurans, and amines or thiols instead of 3-(3-chloromethylbenzoylamino)-6-methoxybenzofuran-2-carboxamide (compound 1-57) and 4-phenylpiperazine. These were illustrated in table 3.

Table 3

| Compound No. | Structure | Compound No. | Structure |
|---|---|---|---|
| 2-1 | 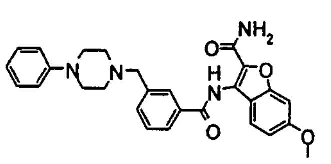 | 2-9 | 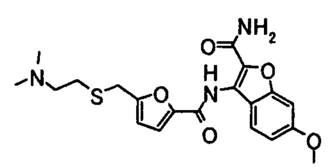 |

(continued)

| Compound No. | Structure | Compound No. | Structure |
|---|---|---|---|
| 2-2 | 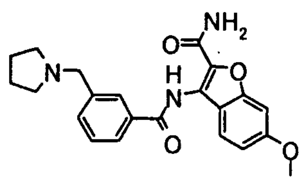 | 2-10 | 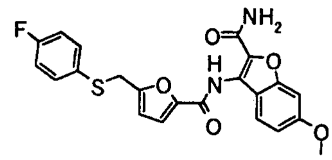 |
| 2-3 | 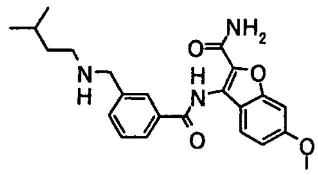 | 2-11 | 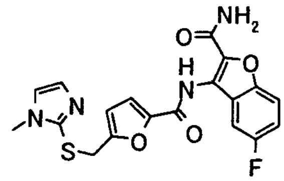 |
| 2-4 | 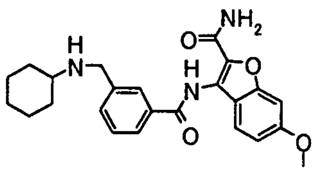 | 2-12 | 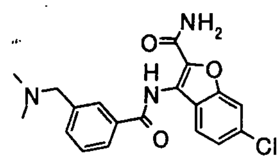 |
| 2-5 | 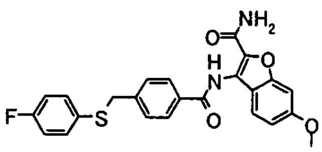 | 2-13 | 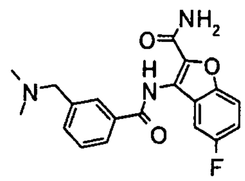 |
| 2-6 | 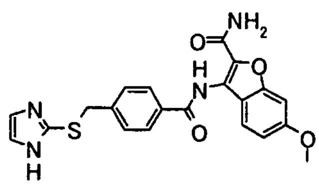 | 2-14 | 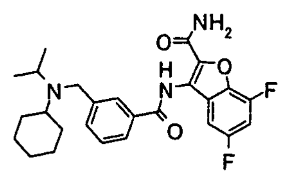 |
| 2-7 | 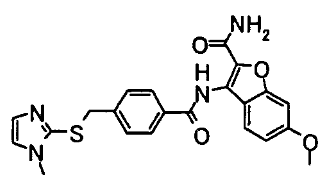 | 2-15 | 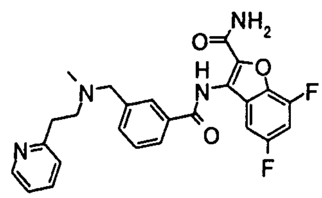 |
| 2-8 | 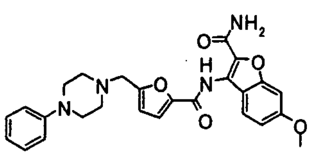 | 2-16 | 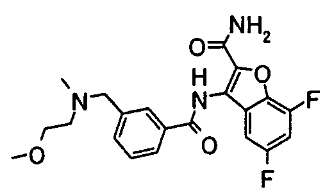 |
| 2-17 | 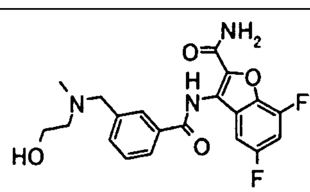 | 2-25 | 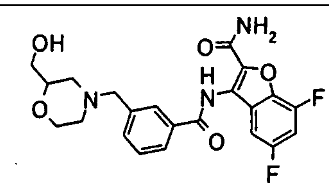 |

(continued)

| Compound No. | Structure | Compound No. | Structure |
|---|---|---|---|
| 2-18 | 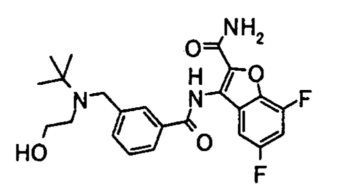 | 2-26 | 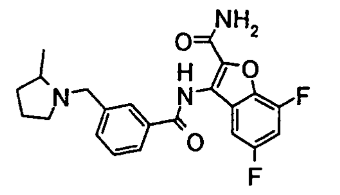 |
| 2-19 | 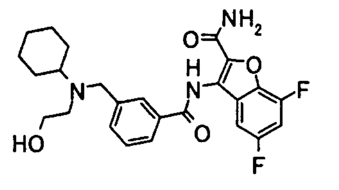 | 2-27 | 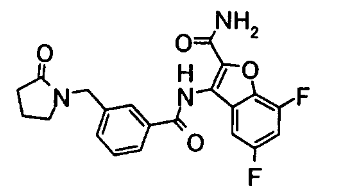 |
| 2-20 | 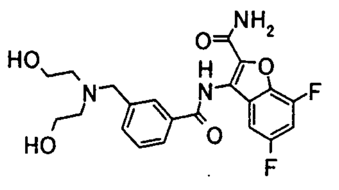 | 2-28 | 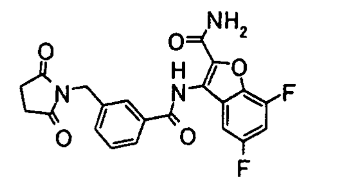 |
| 2-21 | 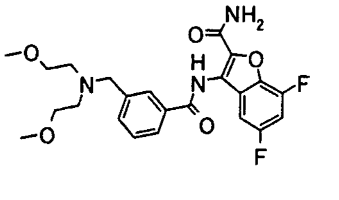 | 2-29 | 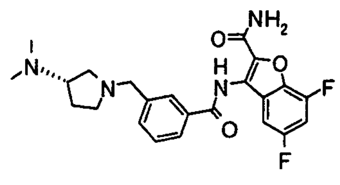 |
| 2-22 | 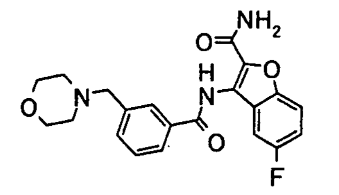 | 2-30 | 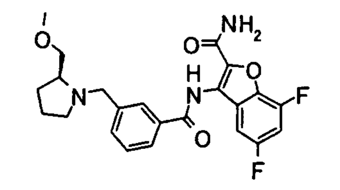 |
| 2-23 | 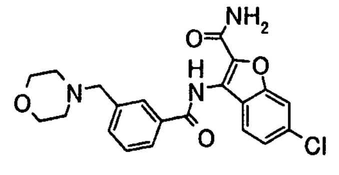 | 2-31 | 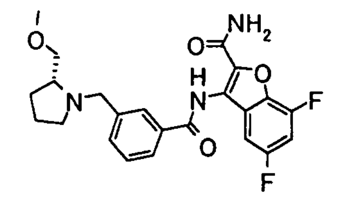 |
| 2-24 | 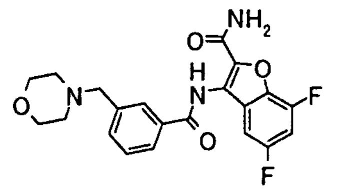 | 2-32 | 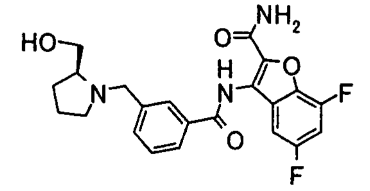 |

(continued)

| Compound No. | Structure | Compound No. | Structure |
|---|---|---|---|
| 2-33 | 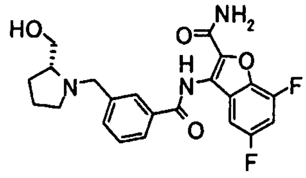 | 2-41 | 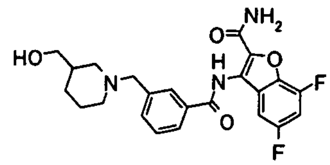 |
| 2-34 | 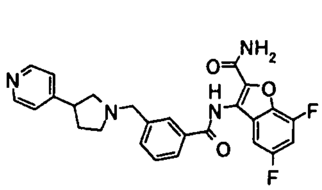 | 2-42 | 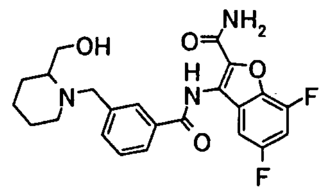 |
| 2-35 | 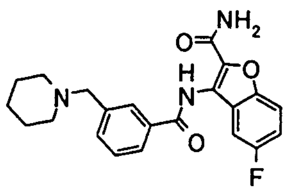 | 2-43 | 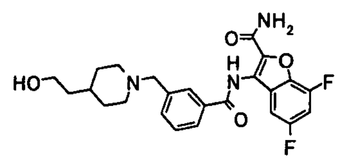 |
| 2-36 | 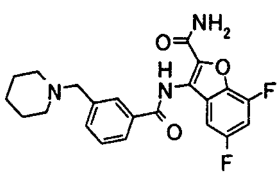 | 2-44 | 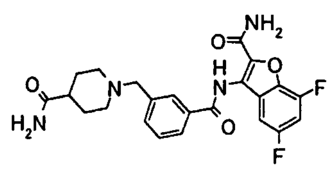 |
| 2-37 | 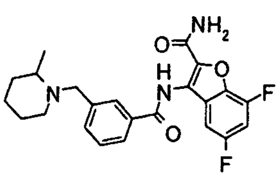 | 2-45 | 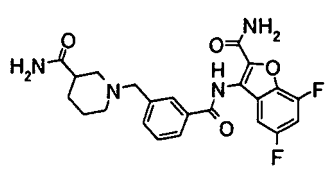 |
| 2-38 | 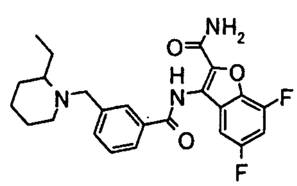 | 2-46 | 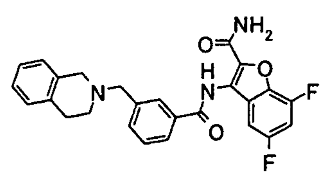 |
| 2-39 | 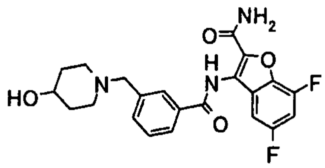 | 2-47 | 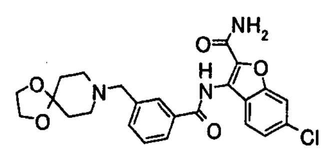 |
| 2-40 | 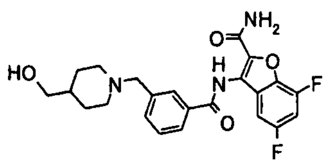 | 2-48 | 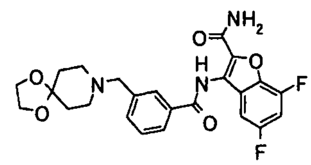 |

(continued)

| Compound No. | Structure | Compound No. | Structure |
|---|---|---|---|
| 2-49 | 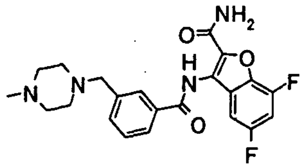 | 2-57 | 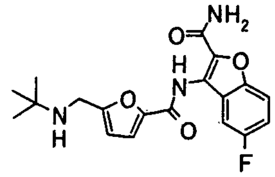 |
| 2-50 | 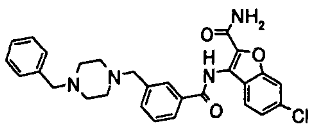 | 2-58 | 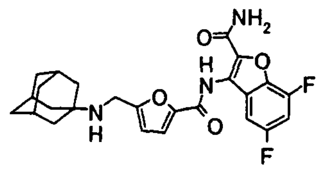 |
| 2-51 | 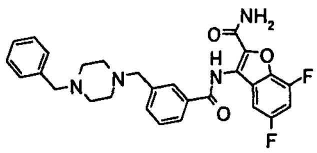 | 2-59 | 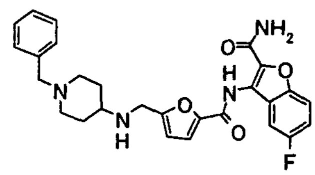 |
| 2-52 | 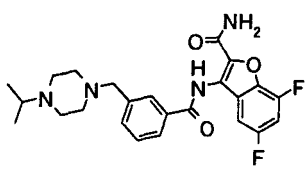 | 2-60 | 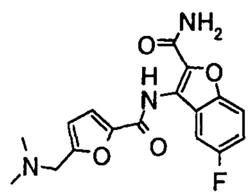 |
| 2-53 | 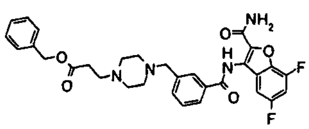 | 2-61 | 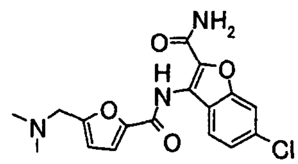 |
| 2-54 | 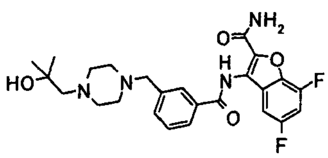 | 2-62 | 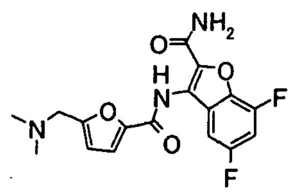 |
| 2-55 | 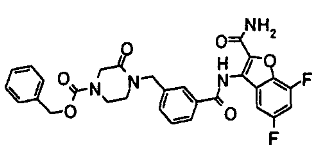 | 2-63 | 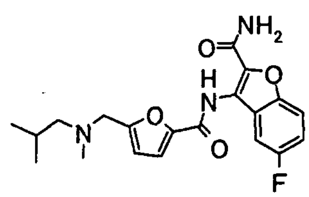 |
| 2-56 | 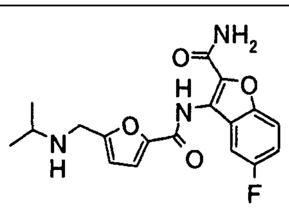 | 2-64 | 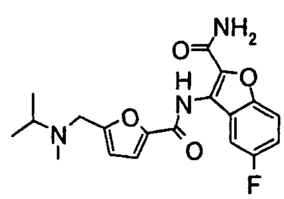 |

(continued)

| Compound No. | Structure | Compound No. | Structure |
|---|---|---|---|
| 2-65 | 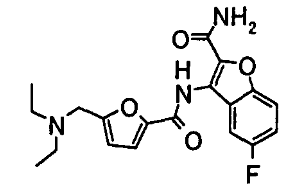 | 2-73 | 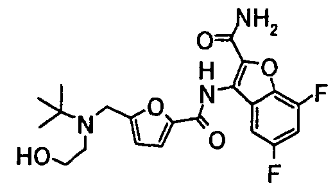 |
| 2-66 | 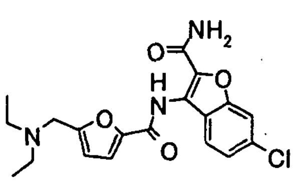 | 2-74 | 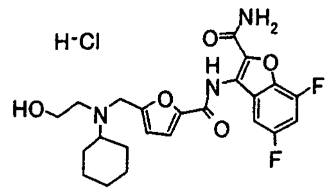 |
| 2-67 | 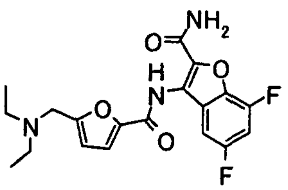 | 2-75 | 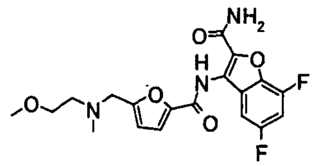 |
| 2-68 | 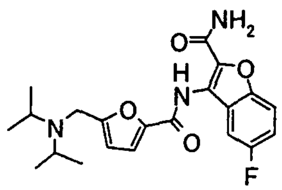 | 2-76 | 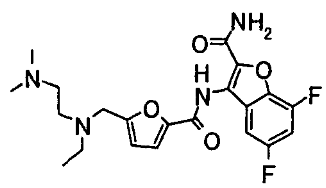 |
| 2-69 | 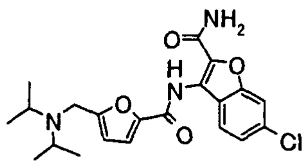 | 2-77 | 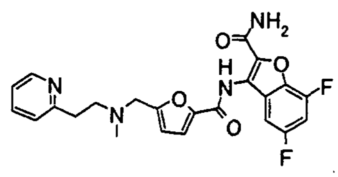 |
| 2-70 | 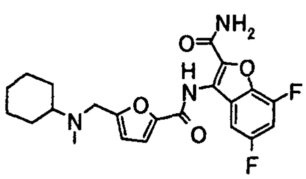 | 2-78 | 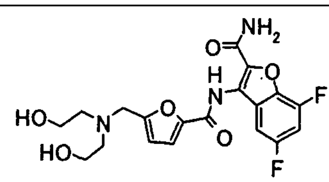 |
| 2-71 | 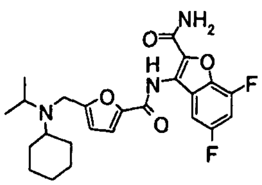 | 2-79 | 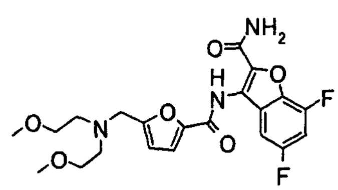 |

(continued)

| Compound No. | Structure | Compound No. | Structure |
|---|---|---|---|
| 2-72 | 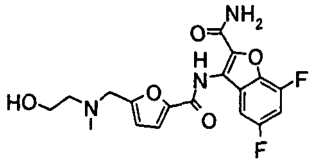 | 2-80 | 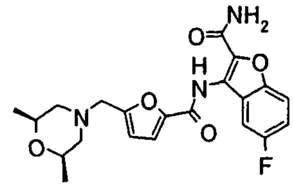 |
| 2-81 | 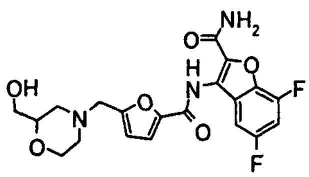 | 2-89 | 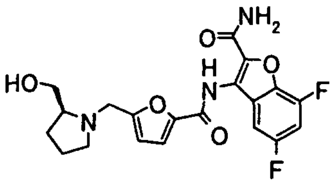 |
| 2-82 | 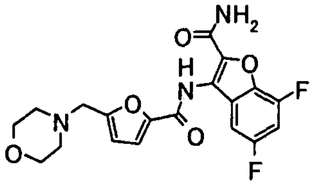 | 2-90 | 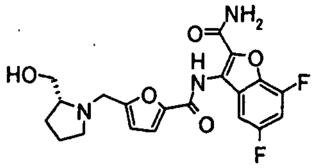 |
| 2-83 | 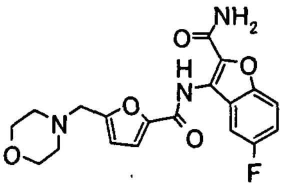 | 2-91 | 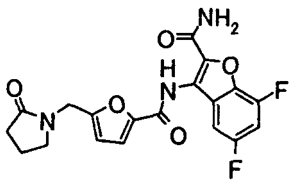 |
| 2-84 | 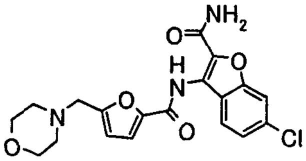 | 2-92 | 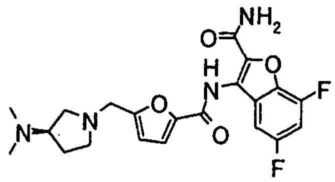 |
| 2-85 | 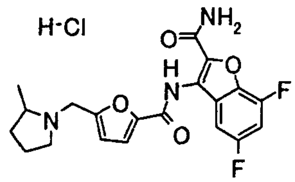 | 2-93 | 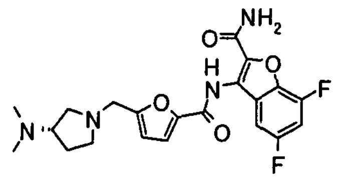 |
| 2-86 | 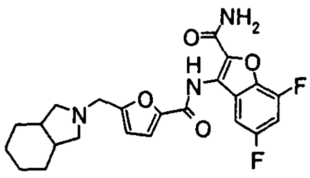 | 2-94 | 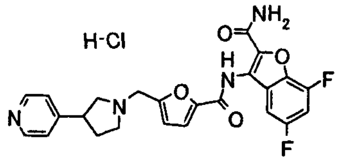 |

(continued)

| Compound No. | Structure | Compound No. | Structure |
|---|---|---|---|
| 2-87 | 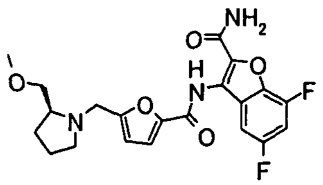 | 2-95 | 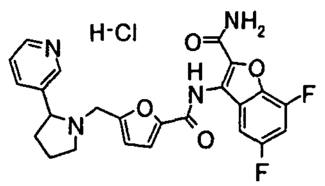 |
| 2-88 | 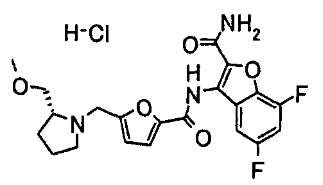 | 2-96 | 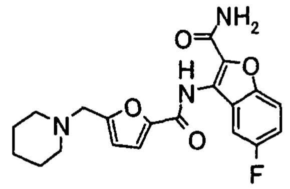 |
| 2-97 | 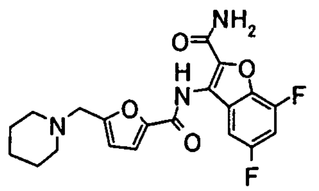 | 2-105 | 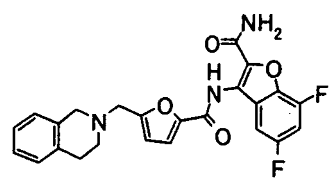 |
| 2-98 | 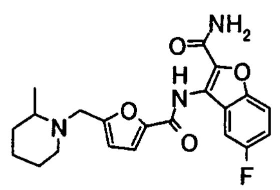 | 2-106 | 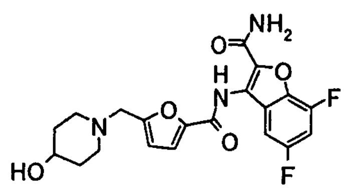 |
| 2-99 | 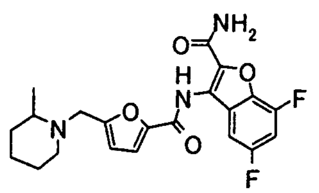 | 2-107 | 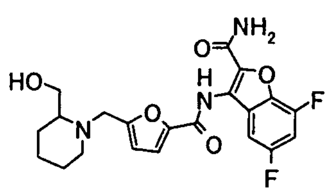 |
| 2-100 | 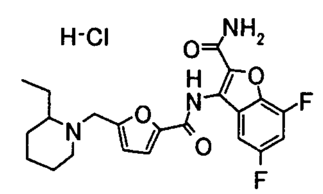 | 2-108 | 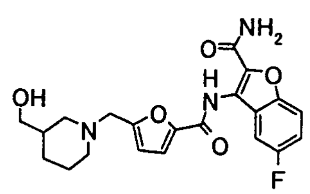 |
| 2-101 | 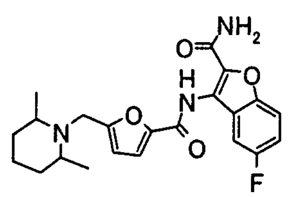 | 2-109 | 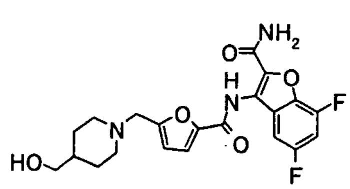 |

(continued)

| Compound No. | Structure | Compound No. | Structure |
|---|---|---|---|
| 2-102 | 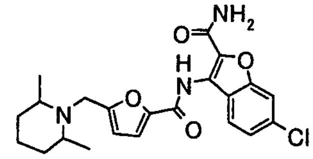 | 2-110 | 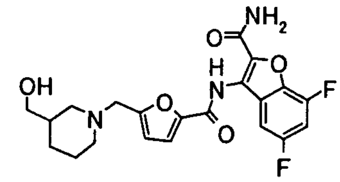 |
| 2-103 | 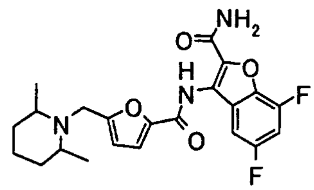 | 2-111 | 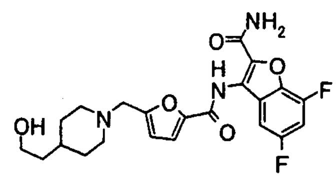 |
| 2-104 | 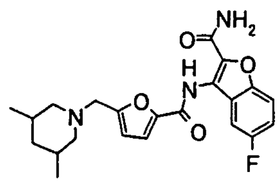 | 2-112 | 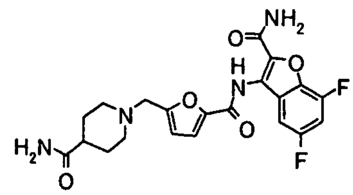 |
| 2-113 | 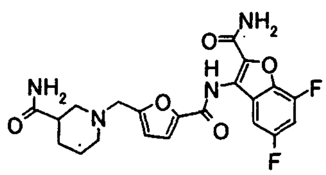 | 2-121 | 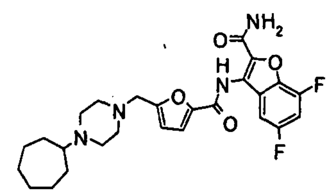 |
| 2-114 | 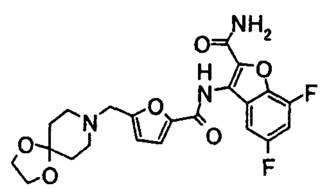 | 2-122 | 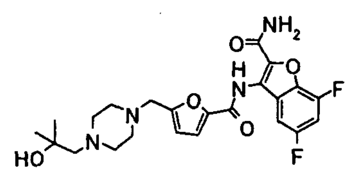 |
| 2-115 | 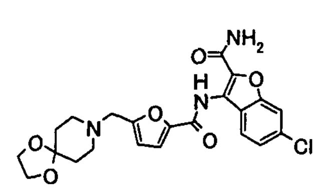 | 2-123 | 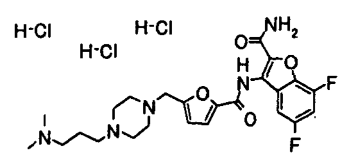 |
| 2-116 | 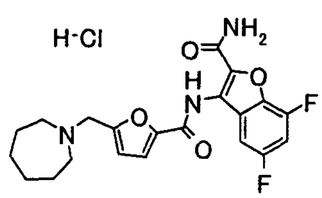 | 2-124 | 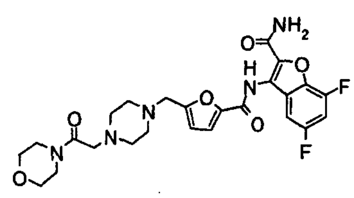 |

(continued)

| Compound No. | Structure | Compound No. | Structure |
|---|---|---|---|
| 2-117 | 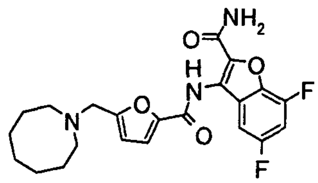 | 2-125 | 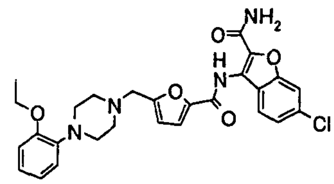 |
| 2-118 | 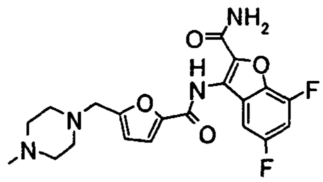 | 2-126 | 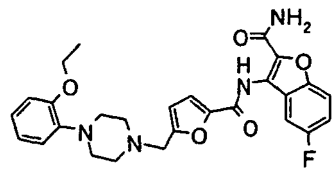 |
| 2-119 | 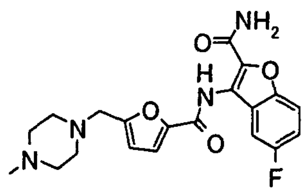 | 2-127 | 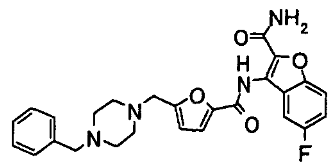 |
| 2-120 | 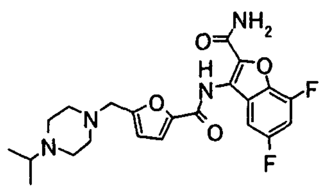 | 2-128 | 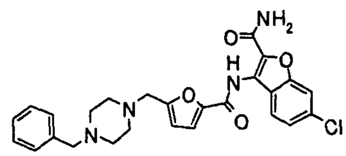 |
| 2-129 | 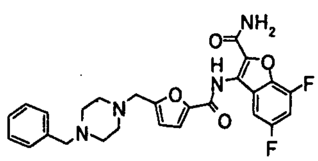 | 2-138 | 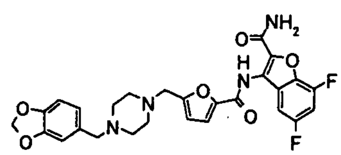 |
| 2-130 | 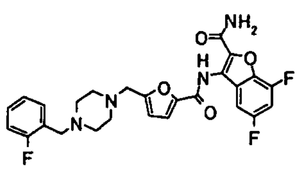 | 2-139 | 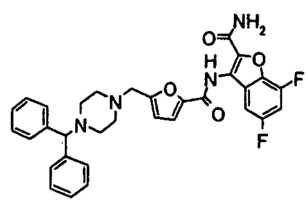 |
| 2-131 | 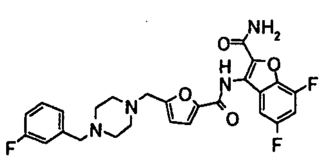 | 2-140 | 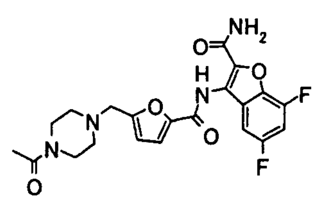 |
| 2-132 | 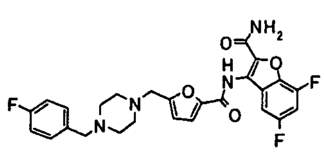 | 2-141 | 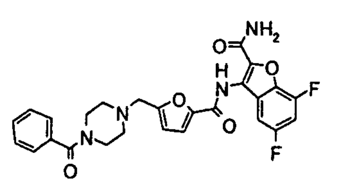 |

(continued)

| Compound No. | Structure | Compound No. | Structure |
|---|---|---|---|
| 2-133 | 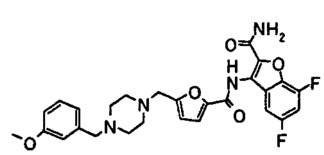 | 2-142 | 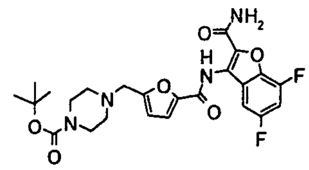 |
| 2-134 | 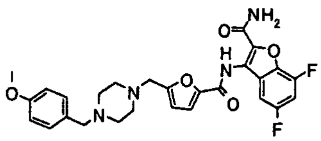 | 2-143 | 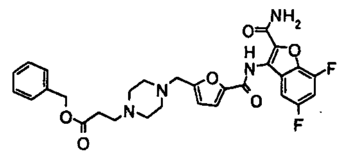 |
| 2-135 | 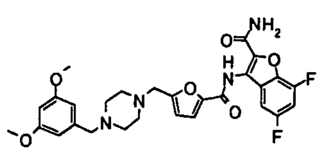 | 2-144 | 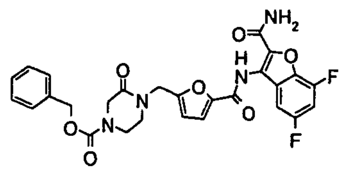 |
| 2-136 | 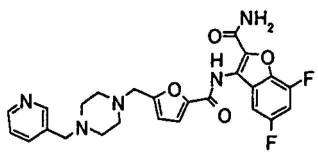 | 2-145 | 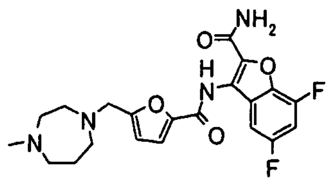 |
| 2-137 | 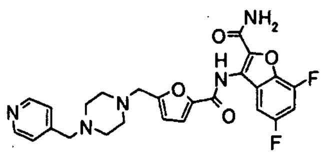 | | |

The physical data of compounds 2-2 to 2-145 were described below.

Compound 2-2

$^1$H-NMR (DMSO-$d_6$) δ ppm: 1.65-1.75 (4H, m), 2.40-2.50 (4H, m), 3.68 (2H, s), 3.87 (3H, s), 6.95-7.15 (2H, m), 7.50-7.65 (2H, m), 7.80-7.95 (3H, m), 8.06 (1H, brs), 8.20-8.30 (1H, m), 11.18 (1H, brs)

Compound 2-3

$^1$H-NMR (DMSO-$d_6$) δ ppm: 0.85 (6H, d, J=6.6Hz), 1.30-1.40 (2H, m), 1.64 (1H, heptet, J=6.6Hz), 2.08 (1H, brs), 2.45-2.55 (2H, m), 3.78 (2H, s), 3.87 (3H, s), 6.95-7.15 (2H, m), 7.50-7.65 (2H, m), 7.80-8.15 (4H, m), 8.20-8.30 (1H, m), 11.14 (1H, brs)

Compound 2-4

$^1$H-NMR (DMSO-$d_6$) δ ppm: 1.00-1.25 (5H, m), 1.50-2.05 (6H, m), 2.30-2.45 (1H, m), 3.82 (2H, s), 3.87 (3H, s), 6.95-7.15 (2H, m), 7.45-7.70 (2H, m), 7.80-8.10 (3H, m), 8.05 (1H, brs), 8.20-8.30 (1H, m), 11.14 (1H, brs)

Compound 2-5

$^1$H-NMR (DMSO-$d_6$) δ ppm: 3.86 (3H, s), 4.30 (2H, s), 6.90-7.25 (4H, m), 7.35-7.55 (4H, m), 7.80-7.95 (3H, m), 8.04 (1H, brs), 8.15-8.25 (1H, m), 11.11 (1H, brs)

Compound 2-6

$^1$H-NMR (DMSO-$d_6$) δ ppm: 3.87 (3H, s), 4.32 (2H, s), 6.90-7.20 (4H, m), 7.40-7.50 (2H, m), 7.80-7.95 (3H, m), 8.05 (1H, brs), 8.20-8.30 (1H, m), 11.11 (1H, brs), 12.25 (1H, brs)

Compound 2-7

$^1$H-NMR (DMSO-$d_6$) δ ppm: 3.39 (3H, s), 3.87 (3H, s), 4.27 (2H, s), 6.95-7.15 (3H, m), 7.20-7.25 (1H, m), 7.35-7.45 (2H, m), 7.80-7.90 (3H, m), 8.05 (1H, brs), 8.20-8.30 (1H, m), 11.13 (1H, brs)

Compound 2-8

$^1$H-NMR (DMSO-$d_6$) δ ppm: 2.55-2.70 (4H, m), 3.05-3.25 (4H, m), 3.70 (2H, s), 3.86 (3H, s), 6.60-6.65 (1H, m), 6.70-6.80 (1H, m), 6.85-7.25 (6H, m), 7.30-7.35 (1H, m), 7.88 (1H, brs), 8.08 (1H, brs), 8.25-8.30 (1H, m), 11.13 (1H, brs)

Compound 2-9

$^1$H-NMR (DMSO-$d_6$) δ ppm: 2.10-2.25 (6H, m), 2.65-2.75 (2H, m), 3.80-3.95 (5H, m), 6.55-6.65 (1H, m), 6.95-7.15 (2H, m), 7.25-7.40 (1H, m), 7.86 (1H, brs), 8.08 (1H, brs), 8.20-8.30 (1H, m), 11.11 (1H, brs)

Compound 2-10

$^1$H-NMR (DMSO-$d_6$) δ ppm: 3.86 (3H, s), 4.35 (2H, s), 6.40-6.50 (1H, m), 6.90-7.25 (5H, m), 7.40-7.55 (2H, m), 7.89 (1H, brs), 8.08 (1H, brs), 8.20-8.30 (1H, m), 11.08 (1H, brs)

Compound 2-11

$^1$H-NMR (DMSO-$d_6$) δ ppm: 3.53 (3H, s), 4.31 (2H, s), 6.35-6.40 (1H, m), 6.95-7.00 (1H, m), 7.20-7.30 (2H, m), 7.35-7.45 (1H, m), 7.60-7.70 (1H, m), 7.95-8.10 (1H, m), 8.01 (1H, brs), 8.27 (1H, brs), 10.95 (1H, brs)

Compound 2-12

$^1$H-NMR ($CDCl_3$) δ ppm: 2.28 (6H, s), 3.53 (2H, s), 5.65 (1H, brs), 6.40 (1H, brs), 7.25-7.40 (1H, m), 7.45-7.65 (3H, m), 7.90-8.00 (2H, m), 8.55-8.65 (1H, m), 10.88 (1H, brs)

Compound 2-13

$^1$H-NMR (DMSO-$d_6$) δ ppm: 2.19 (6H, s), 3.51 (2H, s), 7.35-7.50 (1H, m), 7.50-7.75 (3H, m), 7.85-7.95 (1H, m), 7.95 (1H, s), 7.95-8.10 (2H, m), 8.31 (1H, brs), 11.05 (1H, s)
MS (ESI, m/z): 356 (M+H)+

Compound 2-14

MS (ESI, m/z): 470 (M+H)+

Compound 2-15

$^1$H-NMR (DMSO-$d_6$) δ ppm: 2.22 (3H, s), 2.70-2.80 (2H, m), 2.90-3.00 (2H, m), 3.63 (2H, s), 7.10-7.20 (1H, m), 7.28 (1H, d, J=7.6Hz), 7.45-7.70 (4H, m), 7.80-7.95 (3H, m), 8.09 (1H, brs), 8.36 (1H, brs), 8.44 (1H, dd, J=0.9, 4.7Hz), 10.96 (1H, s)

Compound 2-16

$^1$H-NMR (DMSO-$d_6$) δ ppm: 2.20 (3H, s), 2.50-2.65 (2H, m), 3.24 (3H, s), 3.42-3.70 (4H, m), 7.48-7.70 (3H, m), 7.76-8.00 (3H, m), 8.09 (1H, s), 8.36 (1H, s), 10.98 (1H, s)

Compound 2-17

MS (ESI, m/z): 404 (M+H)+

Compound 2-18

$^1$H-NMR ($CDCl_3$) δ ppm: 1.16 (9H, s), 2.82 (2H, t, J=5.5Hz), 3.63 (2H, t, J=5.5Hz), 3.82 (2H, s), 6.68 (1H, brs), 6.95-7.10 (1H, m), 7.40-7.60 (2H, m), 7.85-7.95 (1H, m), 8.10-8.30 (2H, m), 10.99 (1H, brs)

Compound 2-19

$^1$H-NMR (DMSO-$d_6$) δ ppm: 0.90-1.35 (5H, m), 1.45-1.90 (7H, m), 2.30-2.70 (3H, m), 3.73 (2H, s), 4.20-4.35 (1H, m), 7.45-7.70 (3H, m), 7.80-7.90 (2H, m), 7.97 (1H, s), 8.10 (1H, brs), 8.40 (1H, brs), 11.00 (1H, brs)

Compound 2-20

$^1$H-NMR ($CDCl_3$+MeOD-$d_4$) δ ppm: 2.65-2.75 (4H, m), 3.60-3.70 (4H, m), 3.78 (2H, s), 7.00-7.10 (1H, m), 7.45-7.55 (2H, m), 7.90-8.00 (1H, m), 8.15-8.25 (1H, m)

Compound 2-21

$^1$H-NMR (DMSO-$d_6$) δ ppm: 2.67 (4H, t, J=5.9Hz), 3.21 (6H, s), 3.43 (4H, t, J=5.9Hz), 3.76 (2H, s), 7.50-7.65 (3H, m), 7.80-7.95 (2H, m), 7.96 (1H, s), 8.10 (1H, brs), 8.38 (1H, brs), 10.98 (1H, s)
MS (ESI, m/z): 462 (M+H)+

Compound 2-22

$^1$H-NMR (DMSO-$d_6$) δ ppm: 2.30-2.45 (4H, m), 3.50-3.65 (6H, m), 7.35-7.45 (1H, m), 7.50-7.70 (3H, m), 7.85-8.10 (4H, m), 8.27 (1H, brs), 11.04 (1H, s)
MS (ESI, m/z): 398 (M+H)+

Compound 2-23

$^1$H-NMR (DMSO-$d_6$) δ ppm: 2.30-2.45 (4H, m), 3.50-3.65 (6H, m), 7.40-7.50 (1H, m), 7.50-7.65 (2H, m), 7.70-7.80 (1H, m), 7.90 (1H, d, J=7.8Hz), 7.90-8.10 (2H, m), 8.20-8.35 (2H, m), 11.07 (1H, s)
MS (ESI, m/z): 414 (M+H)+

Compound 2-24

$^1$H-NMR (DMSO-$d_6$) δ ppm: 2.40 (4H, brs), 3.50-3.70 (6H, m), 7.50-7.70 (3H, m), 7.80-7.90 (1H, m), 7.90 (1H, d, J=7.5Hz), 7.96 (1H, s), 8.10 (1H, brs), 8.38 (1H, brs), 11.00 (1H, s)
MS (ESI, m/z): 416 (M+H)+

Compound 2-25

$^1$H-NMR (DMSO-$d_6$) δ ppm: 1.82 (1H, t, J=10.5Hz), 2.00-2.15 (1H, m), 2.64 (1H, d, J=11.0Hz), 2.78 (1H, d, J=11.0Hz), 3.20-3.65 (6H, m), 3.77(1H, d, J=11.1Hz), 4.65(1H, t, J=6.0Hz), 7.50-7.65 (3H, m), 7.83 (1H, d, J=7.8Hz), 7.91 (1H, d, J=7.3Hz), 7.96 (1H, s), 8.11 (1H, brs), 8.38 (1H, brs), 10.99 (1H, s)
MS (ESI, m/z): 446 (M+H)+

Compound 2-26

$^1$H-NMR (DMSO-$d_6$) δ ppm: 1.13 (3H, d, J=6.0Hz), 1.30-1.45 (1H, m), 1.50-1.75 (2H, m), 1.85-2.20 (2H, m), 2.35-2.50 (1H, m), 2.75-2.85 (1H, m), 3.23 (1H, d, J=13.2Hz), 4.07 (1H, d, J=13.2Hz), 7.45-7.65 (3H, m), 7.80-8.00 (3H, m), 8.10 (1H, brs), 8.37 (1H, brs), 11.00 (1H, s)

Compound 2-27

$^1$H-NMR (DMSO-$d_6$) δ ppm: 1.90-2.05 (2H, m), 2.34 (2H, t, J=8.1Hz), 3.29 (2H,t, J=7.1Hz), 4.49 (2H, s), 7.52 (1H, d, J=7.8Hz), 7.55-7.65 (2H, m), 7.75-7.90 (2H, m), 7.92 (1H, d, J=7.8Hz), 8.10 (1H, brs), 8.38 (1H, brs), 11.00 (1H, s)
MS (ESI, m/z): 414 (M+H)+

Compound 2-28

$^1$H-NMR (DMSO-$d_6$) δ ppm: 2.74 (4H, s), 4.66 (2H, s), 7.50-7.70 (3H, m), 7.80-7.95 (3H, m), 8.11 (1H, brs), 8.39 (1H, brs), 11.00 (1H, brs)

Compound 2-29

MS (ESI, m/z): 443 (M+H)+

Compound 2-30

$^1$H-NMR (DMSO-$d_6$) δ ppm: 1.40-2.00 (4H, m), 2.10-2.30 (1H, m), 2.60-2.90 (2H, m), 3.20-3.50 (6H, m), 4.10-4.30 (1H, m), 7.50-7.65 (3H, m), 7.80-8.00 (3H, m), 8.09 (1H, brs), 8.37 (1H, brs), 10.99 (1H, s)
MS (ESI, m/z): 444 (M+H)+

Compound 2-31

$^1$H-MMR (DMSO-$d_6$) δ ppm: 1.40-2.00 (4H, m), 2.10-2.30 (1H, m), 2.60-2.90 (2H, m), 3.20-3.50 (6H, m), 4.10-4.30 (1H, m), 7.50-7.65 (3H, m), 7.80-8.00 (3H, m), 8.09 (1H, brs), 8.37 (1H, brs), 10.99 (1H, s)

Compound 2-32

MS (ESI, m/z): 430 (M+H)+

Compound 2-33

MS (ESI, m/z): 430 (M+H)+

Compound 2-34

$^1$H-NMR (DMSO-$d_6$) δ ppm: 1.70-1.90 (1H, m), 2.20-3.05 (5H, m), 3.25-3.45 (1H, m), 3.77 (2H, brs), 7.25-7.40 (2H, m), 7.50-7.70 (3H, m), 7.80-7.95 (2H, m), 8.00 (1H, s), 8.10 (1H, brs), 8.39 (1H, brs), 8.42-8.50 (2H, m), 11.01 (1H, s)

Compound 2-35

$^1$H-NMR (DMSO-$d_6$) δ ppm: 1.30-1.45 (2H, m), 1.45-1.60 (4H, m), 2.35 (4H, brs), 3.53 (2H, s), 7.35-7.45 (1H, m), 7.50-7.75 (3H, m), 7.80-8.10 (4H, m), 8.27 (1H, brs), 11.03 (1H, s)
MS (ESI, m/z): 396 (M+H)+

Compound 2-36

$^1$H-NMR (DMSO-$d_6$) δ ppm: 1.30-1.60 (6H, m), 2.36 (4H, brs), 3.54 (2H, s), 7.45-7.65 (3H, m), 7.75-8.00 (3H, m), 8.09 (1H, brs), 8.37 (1H, brs), 10.99 (1H, brs)
MS (ESI, m/z): 414 (M+H)+

Compound 2-37

$^1$H-NMR (DMSO-$d_6$) δ ppm: 1.12 (3H, d, J=6.0Hz), 1.15-1.70 (6H, m), 1.90-2.05 (1H, m), 2.25-2.45 (1H, m), 2.55-2.70 (1H, m), 3.25 (1H, d, J=14Hz), 4.01 (1H, d, J=14Hz), 7.50-7.65 (3H, m), 7.80-7.90 (2H, m), 7.94 (1H, s), 8.11 (1H, brs), 8.38 (1H, brs), 11.00 (1H, brs)
MS (ESI, m/z): 428 (M+H)+

Compound 2-38

$^1$H-NMR (DMSO-$d_6$) δ ppm: 0.88 (3H, t, J=7.6Hz), 1.00-1.80 (8H, m), 1.90-2.80 (3H, m), 3.20-3.40 (1H, m), 3.90-4.10 (1H, m), 7.45-7.70 (3H, m), 7.80-8.00 (3H, m), 8.09 (1H, brs), 8.36 (1H, brs), 10.99 (1H, s)

Compound 2-39

$^1$H-NMR (DMSO-$d_6$) δ ppm: 1.30-1.50 (2H, m), 1.65-1.80 (2H, m), 2.00-2.15 (2H, m), 2.60-2.75 (2H, m), 3.40-3.60 (3H, m), 4.50-4.60 (1H, m), 7.50-7.70 (3H, m), 7.80-8.00 (3H, m), 8.10 (1H, brs), 8.38 (1H, brs), 10.98 (1H, s)
MS (ESI, m/z): 430 (M+H)+

Compound 2-40

$^1$H-NMR ($CDCl_3$+MeOD-$d_4$) δ ppm: 1.20-1.80 (5H, m), 1.90-2.15 (2H, m),2.85-3.00(2H,m),3.30-3.50(2H,m), 3.61(2H,s),7.00-7.10 (1H, m), 7.45-7.60 (2H, m), 7.85-8.00 (2H, m), 8.10-8.20 (1H, m)

Compound 2-41

$^1$H-NMR (DMSO-$d_6$) δ ppm: 0.80-2.00 (7H, m), 2.60-2.95 (2H, m), 3.10-3.60 (4H, m), 4.30-4.45 (1H, m), 7.50-7.65 (3H, m), 7.80-7.95 (3H, m), 8.08 (1H, brs), 8.36 (1H, brs), 10.98 (1H, s)

Compound 2-42

MS (ESI, m/z): 444 (M+H)+

Compound 2-43

MS (ESI, m/z): 458 (M+H)+

Compound 2-44

MS (ESI, m/z): 457 (M+H)+

Compound 2-45

$^1$H-NMR (DMSO-$d_6$) δ ppm: 1.25-1.40 (1H, m), 1.40-1.55 (1H, m), 1.55-1.80 (2H, m), 1.85-2.10 (2H, m), 2.25-2.40 (1H, m), 2.65-2.85 (2H, m), 3.50-3.65 (2H, m), 6.76 (1H, brs), 7.28 (1H, brs), 7.50-7.65 (3H, m), 7.80-8.00 (3H, m), 8.10 (1H, brs), 8.39 (1H, brs), 10.99 (1H, s)
MS (ESI, m/z): 457 (M+H)+

Compound 2-46

MS (ESI, m/z): 462 (M+H)+

Compound 2-47

$^1$H-NMR (DMSO-$d_6$) δ ppm: 1.50-1.70 (4H, m), 3.62 (2H, s), 3.80-3.90 (4H, m), 7.35-7.50 (1H, m), 7.50-7.65 (2H, m), 7.70-7.80 (1H, m), 7.85-8.10 (3H, m), 8.15-8.40 (2H, m), 11.05 (1H, s)
MS (ESI, m/z): 470 (M+H)+

Compound 2-48

$^1$H-NMR (DMSO-$d_6$) δ ppm: 1.55-1.70 (4H, m), 2.35-2.60 (4H, m), 3.59 (2H, s), 3.85 (4H, s), 7.50-7.70 (3H, m), 7.75-8.00 (3H, m), 8.10 (1H, brs), 8.38 (1H, brs), 10.97 (1H, brs)
MS (ESI, m/z): 472 (M+H)+

Compound 2-49

$^1$H-NMR (DMSO-$d_6$) δ ppm: 2.05-2.60 (10H, m), 3.56 (2H, s), 7.50-7.70 (3H, m), 7.80-8.00 (3H, m), 8.10 (1H, brs), 8.38 (1H, brs) 10.98 (1H, s)
MS (ESI, m/z): 429 (M+H)+

Compound 2-50

$^1$H-NMR (DMSO-$d_6$) δ ppm: 2.20-2.60 (8H, m), 3.47 (2H, s), 3.58 (2H, s), 7.20-7.40 (5H, m), 7.40-7.50 (1H, m), 7.50-7.65 (2H, m), 7.70-7.80 (1H, m), 7.85-8.15 (3H, m), 8.15-8.40 (2H, m), 11.05 (1H, s)
MS (ESI, m/z): 503 (M+H)+

Compound 2-51

$^1$H-NMR (DMSO-$d_6$) δ ppm: 2.20-2.60 (8H, m), 3.46 (2H, s), 3.57 (2H, s), 7.15-7.35 (5H, m), 7.50-7.65 (3H, m), 7.80-8.00 (3H, m), 8.07 (1H, brs), 8.34 (1H, brs), 10.97 (1H, s)
MS (ESI, m/z): 505 (M+H)+

Compound 2-52

$^1$H-NMR (DMSO-$d_6$) δ ppm: 0.85-1.10 (6H, m), 2.20-2.75 (9H, m), 3.55 (2H, s), 7.45-7.65 (3H, m), 7.75-8.00 (3H, m), 8.06 (1H, brs), 8.34 (1H, brs), 10.97 (1H, s)
MS (ESI, m/z): 457 (M+H)+

Compound 2-53

$^1$H-NMR ($CDCl_3$) δ ppm: 2.30-2.70 (8H, m), 2.56 (2H, t, J=7.4Hz), 2.74 (2H, t, J=7.4Hz), 3.60 (2H, s), 5.13 (2H, s), 5.96 (1H, brs), 6.56 (1H, brs), 7.00-7.10 (1H, m), 7.25-7.60 (7H, m), 7.85-8.05 (2H, m), 8.15-8.25 (1H, m), 10.82 (1H, brs)

Compound 2-54

MS (ESI, m/z): 487 (M+H)+

Compound 2-55

$^1$H-NMR (DMSO-$d_6$) δ ppm: 3.25-3.55 (2H, m), 3.60-3.75 (2H, m), 4.05-4.25 (2H, m), 4.66 (2H, s), 5.11 (2H, s), 7.25-7.45 (5H, m), 7.50-7.70 (3H, m), 7.75-8.00 (3H, m), 8.10 (1H, brs), 8.38 (1H, brs), 11.01 (1H, s)
MS (ESI, m/z): 563 (M+H)+

Compound 2-56

$^1$H-NMR (DMSO-$d_6$) δ ppm: 1.01 (6H, d, J=6.0Hz), 2.70-2.85 (1H, m), 3.79 (2H, s), 6.45-6.60 (1H, m), 7.20-7.35 (1H, m), 7.35-7.50 (1H, m), 7.60-7.70 (1H, m), 7.90-8.15 (2H, m), 8.29 (1H, brs), 10.97 (1H, s)
MS (ESI, m/z): 360 (M+H)+

Compound 2-57

$^1$H-MMR (DMSO-$d_6$) δ ppm: 1.09 (9H, s), 3.78 (2H, s), 6.45-6.60 (1H, m), 7.25-7.35 (1H, m), 7.35-7.50 (1H, m), 7.60-7.75 (1H, m), 7.95-8.20 (2H, m), 8.29 (1H, brs), 10.96 (1H, s)
MS (ESI, m/z): 374 (M+H)+

Compound 2-58

$^1$H-NMR (DMSO-$d_6$) δ ppm: 1.50-1.70 (12H, m), 1.95-2.10 (3H, m), 3.80 (2H, s), 6.45-6.55 (1H, m), 7.25-7.35 (1H, m), 7.50-7.65 (1H, m), 7.85-7.95 (1H, m), 8.05 (1H, brs), 8.35 (1H, brs), 10.89 (1H, brs)

Compound 2-59

$^1$H-NMR (DMSO-$d_6$) δ ppm: 1.20-1.35 (2H, m), 1.75-2.05 (4H, m), 2.35-2.55 (1H, m), 2.65-2.80 (2H, m), 3.43 (2H, s), 3.82 (2H, s), 6.54 (1H, d, J=3.1Hz), 7.20-7.35 (6H, m), 7.35-7.45 (1H, m), 7.60-7.70 (1H, m), 7.95-8.10 (2H, m), 8.29 (1H, brs), 10.96 (1H, s)
MS (ESI, m/z): 491 (M+H)+

Compound 2-60

$^1$H-NMR (DMSO-$d_6$) δ ppm: 2.23 (6H, s), 3.58 (2H, s), 6.55-6.65 (1H, m), 7.30-7.45 (2H, m), 7.60-7.70 (1H, m), 8.02 (1H, brs), 8.05-8.15 (1H, m), 8.27 (1H, brs), 11.03 (1H, brs)

Compound 2-61

$^1$H-NMR (DMSO-$d_6$) δ ppm: 2.23 (6H, s), 3.57 (2H, s), 6.55-6.60 (1H, m), 7.25-7.35 (1H, m), 7.40-7.50 (1H, m), 7.70-7.80 (1H, m), 8.01 (1H, brs), 8.25 (1H, brs), 8.36 (1H, d, J=9.1Hz), 11.05 (1H, s)
MS (ESI, m/z): 362 (M+H)+

Compound 2-62

$^1$H-NMR ($CDCl_3$) δ ppm: 2.36 (6H, s), 3.62 (2H, s), 6.40-6.45 (1H, m), 6.50 (1H, brs), 6.95 (1H, brs), 6.95-7.05 (1H, m), 7.20-7.25 (1H, m), 8.05-8.15 (1H, m), 10.80 (1H, brs)

Compound 2-63

$^1$H-NMR ($CDCl_3$) δ ppm: 0.85-0.95 (6H, m), 1.70-1.90 (1H, m), 2.15-2.25 (2H, m), 2.32 (3H, s), 3.68 (2H, s), 5.99 (1H, brs), 6.20-6.60 (2H, m), 7.15-7.40 (3H, m), 8.30-8.40 (1H, m), 10.76 (1H, brs)

Compound 2-64

$^1$H-NMR ($CDCl_3$) δ ppm: 1.10 (6H, d, J=6.5Hz), 2.33 (3H, s), 2.90 (1H, heptet, J=6.5Hz), 3.72 (2H, s), 5.99 (1H, brs), 6.30-6.60 (2H, m), 7.10-7.40 (3H, m), 8.25-8.40 (1H, m), 10.74 (1H, brs)

Compound 2-65

$^1$H-NMR ($CDCl_3$) δ ppm: 1. 18 (6H,t, J=7.2Hz), 2.67 (4H, q, J=7.2Hz), 3.90 (2H, s), 6.40-6.50 (1H, m), 6.75 (1H, brs), 6.91 (1H, brs), 7.10-7.40 (3H, m), 8.25-8.40 (1H, m), 10.84 (1H, brs)

Compound 2-66

$^1$H-NMR (DMSO-$d_6$) δ ppm: 0.90-1.10 (6H, m), 3.73 (2H, s), 6.57 (1H, brs), 7.25-7.35 (1H, m), 7.35-7.45 (1H, m), 7.70-7.80 (1H, m), 8.01 (1H, brs), 8.25 (1H, brs), 8.30-8.45 (1H, m), 11.04 (1H, s)
MS (ESI, m/z): 390 (M+H)+

Compound 2-67

$^1$H-NMR (DMSO-$d_6$) δ ppm: 1.04 (6H, t, J=6.9Hz), 2.40-2.60 (4H, m), 3.73 (2H, s), 6.57 (1H, d, J=3.5Hz), 7.32 (1H, d, J=3.5Hz), 7.50-7.65 (1H, m), 7.90-8.00 (1H, m), 8.09 (1H, brs), 8.35 (1H, brs), 10.98 (1H, s)

Compound 2-68

$^1$H-NMR ($CDCl_3$) δ ppm: 1.06 (12H, d, J=6.5Hz), 3.10 (2H, heptet, J=6.5Hz), 3.75 (2H, s), 5.78 (1H, brs), 6.30-6.50 (2H, m), 7.15-7.40 (3H, m), 8.30-8.45 (1H, m), 10.70 (1H, brs)

Compound 2-69

$^1$H-NMR (DMSO-$d_6$) δ ppm: 0.90-1.15 (12H, m), 2.90-3.15 (2H, m), 3.72 (2H, s), 6.45-6.60 (1H, m), 7.20-7.35 (1H, m), 7.35-7.50 (1H, m), 7.70-7.80 (1H, m), 7.99 (1H, brs), 8.24 (1H, brs), 8.30-8.45 (1H, m). 10.98 (1H, s)

MS (ESI, m/z): 418 (M+H)+

Compound 2-70

$^1$H-NMR ($CDCl_3$+MeOD-$d_4$) δ ppm: 1.00-2.00 (10H, m), 2.35-2.45 (1H, m), 2.37 (3H, s), 3.78 (2H, s), 6.40-6.50 (1H, m), 6.95-7.10 (1H, m), 7.20-7.35 (1H, m), 8.05-8.15 (1H, m)

Compound 2-71

MS (ESI, m/z): 460 (M+H)+

compound 2-72

MS (ESI, m/z): 394 (M+H)+

Compound 2-73

$^1$H-NMR ($CDCl_3$) δ ppm: 1.14 (9H, s), 2.92 (2H, t, J=6.0Hz), 3.57 (2H, t, J=6.0Hz), 3.86 (2H, s), 6.30-6.40 (1H, m), 6.69 (1H, brs), 6.95-7.10 (1H, m), 7.20-7.30 (1H, m), 8.15-8.25 (1H, m), 10.88 (1H, brs)

Compound 2-74

$^1$H-NMR (DMSO-$d_6$) δ ppm: 1.00-1.65 (6H, m), 1.70-2.20 (4H, m), 3.15-3.90 (5H, m), 4.61 (2H, d, J=3.8Hz), 5.39 (1H, brs), 7.05 (1H, d, J=3.5Hz), 7.47 (1H, d, J=3.5Hz), 7.55-7.70 (1H, m), 7.85-7.95 (1H, m), 8.13 (1H, brs), 8.44 (1H, brs), 10.15 (1H, brs), 11.11 (1H, s)

Compound 2-75

MS (ESI, m/z): 408 (M+H)+

Compound 2-76

$^1$H-NMR (DMSO-$d_6$) δ ppm: 1.04 (3H, t, J=7.1Hz), 2.16 (6H, s), 2.30-2.45 (2H, m), 2.45-2.60 (4H, m), 3.77 (2H, s), 6.59 (1H, d, J=3.5Hz), 7.34(1H, d, J=3.5Hz), 7.55-7.70(1H, m), 7.90-8.00 (1H, m), 8.11 (1H, brs), 8.39 (1H, brs), 11.00 (1H, brs)
MS (ESI, m/z): 435 (M+H)+

Compound 2-77

$^1$H-NMR (DMSO-$d_6$) δ ppm: 2.30 (3H, s), 2.65-3.00 (4H, m), 3.71 (2H, s), 6.50-6.60 (1H, m), 7.10-7.40 (4H, m), 7.50-7.75 (2H, m), 7.85-8.00 (1H, m), 8.08 (1H, brs), 8.35-8.55 (2H, m)

Compound 2-78

$^1$H-NMR (DMSO-$d_6$) δ ppm: 2.55-2.65 (4H, m), 3.45-3.55 (4H, m), 3.83 (2H, s), 4.35-4.45 (2H, m), 6.55-6.65 (1H, m), 7.30-7.40 (1H, m), 7.55-7.65 (1H, m), 7.90-8.00 (1H, m), 8.14 (1H, brs), 8.41 (1H, brs), 10.99 (1H, brs)

Compound 2-79

$^1$H-NMR (DMSO-$d_6$) δ ppm: 2.70 (4H, t, J=6.0Hz), 3.24 (6H, s), 3.44 (4H, t, J=6.0Hz), 3.83 (2H, s), 6.59 (1H, d, J=3.5Hz), 7.33 (1H, d, J=3.5Hz), 7.55-7.70 (1H, m), 7.90-8.00 (1H, m), 8.10 (1H, brs), 8.38 (1H, brs), 10.99 (1H, s)
MS (ESI, m/z): 452 (M+H)+

Compound 2-80

$^1$H-NMR (DMSO-$d_6$) δ ppm: 1.04 (6H, d, J=6.4Hz), 1.70-1.85 (2H, m), 2.70-2.80 (2H, m), 3.50-3.70 (4H, m), 6.60 (1H, d, J=3.3Hz), 7.32 (1H, d, J=3.3Hz), 7.35-7.50 (1H, m), 7.60-7.70 (1H, m), 7.95-8.10 (2H, m), 8.30 (1H, brs), 10.99 (1H, s)

MS (ESI, m/z): 416 (M+H)+

Compound 2-81

$^1$H-NMR (DMSO-$d_6$) δ ppm: 1.88 (1H, t, J=10.5Hz), 2.10-2.25 (1H, m), 2.65-2.75 (1H, m), 2.82 (1H, d, J=10.9Hz), 3.25-3.60 (4H, m), 3.64 (2H, s), 3.70-3.85 (1H, m), 4.70 (1H, t, J=5.7Hz), 6.61 (1H, d, J=3.5Hz), 7.34 (1H, d, J=3.5Hz), 7.50-7.65 (1H, m), 7.85-7.95 (1H, m), 8.10 (1H, brs), 8.40 (1H, brs), 10.95 (1H, s)
MS (ESI, m/z): 436 (M+H)+

Compound 2-82

$^1$H-NMR (DMSO-$d_6$) δ ppm: 2.40-2.50 (4H, m), 3.50-3.65 (6H, m), 6.61 (1H, d, J=3.5Hz), 7.34 (1H, d, J=3.5Hz), 7.55-7.65 (1H, m), 7.85-7.95 (1H, m), 8.11 (1H, brs), 8.38 (1H, brs), 10.97 (1H, s)
MS (ESI, m/z): 406 (M+H)+

Compound 2-83

$^1$H-NMR (DMSO-$d_6$) δ ppm: 2.40-2.55 (4H, m), 3.55-3.70 (6H, m), 6.61 (1H, d, J=3.5Hz), 7.32 (1H, d, J=3.5Hz), 7.35-7.45 (1H, m), 7.60-7.70 (1H, m), 8.00 (1H, brs), 8.00-8.10 (1H, m), 8.27 (1H, brs), 11.00 (1H, s)
MS (ESI, m/z): 388 (M+H)+

Compound 2-84

$^1$H-NMR (DMSO-$d_6$) δ ppm: 2.40-2.55 (4H, m), 3.50-3.70 (6H, m), 6.55-6.65 (1H, m), 7.25-7.35 (1H, m), 7.40-7.50 (1H, m), 7.70-7.80 (1H, m), 8.00 (1H, brs), 8.25 (1H, brs), 8.34 (1H, d, J=9.1Hz), 11.02 (1H, s)
MS (ESI, m/z): 404 (M+H)+

Compound 2-85

$^1$H-NMR (DMSO-$d_6$) δ ppm: 1.41 (3H, d, J=6.3Hz), 1.55-1.70 (1H, m), 1.80-2.05 (2H, m), 2.15-2.30 (1H, m), 3.20-3.70 (3H, m), 4.40-4.55 (1H, m), 4.60-4.80 (1H, m), 7.01 (1H, d, J=3.5Hz), 7.46 (1H, d, J=3.5Hz), 7.55-7.70 (1H, m), 7.80-7.95 (1H, m), 8.13 (1H, brs), 8.44 (1H, brs), 10.62 (1H, brs), 11.09 (1H, s)

Compound 2-86

$^1$H-NMR ($CDCl_3$+MeOD-$d_4$) δ ppm: 1.15-1.65 (8H, m), 2.00-2.25 (2H, m), 2.55-3.05 (4H, m), 3.84 (2H, s), 6.35-6.45 (1H, m), 6.95-7.05 (1H, m), 7.15-7.30 (1H, m), 8.05-8.15 (1H, m)

Compound 2-87

$^1$H-NMR (DMSO-$d_6$) δ ppm: 1.40-1.55 (1H, m), 1.55-1.75 (2H, m), 1.75-1.95 (1H, m), 2.30-2.60 (1H, m), 2.65-2.80 (1H, m), 2.85-3.05 (1H, m), 3.15-3.45 (5H, m), 3.55-3.75 (1H, m), 4.00-4.15 (1H, m), 6.50-6.60 (1H, m), 7.25-7.35 (1H, m), 7.55-7.65 (1H, m), 7.85-8.00 (1H, m), 8.12 (1H, brs), 8.38 (1H, brs), 10.97 (1H, s)
MS (ESI, m/z): 434 (M+H)+

Compound 2-88

$^1$H-NMR (DMSO-$d_6$) δ ppm: 1.60-2.30 (4H, m), 3.20-3.90 (8H, m), 4.45-4.75 (2H, m), 7.01 (1H, d, J=3.5Hz), 7.46 (1H, d, J=3.5Hz), 7.55-7.70 (1H, m), 7.80-7.90 (1H, m), 8.12 (1H, brs), 8.44 (1H, brs), 10.76 (1H, brs), 11.09 (1H, s)

Compound 2-89

MS (ESI, m/z): 420 (M+H)+

Compound 2-90

MS (ESI, m/z): 420 (M+H)+

Compound 2-91

$^1$H-NMR (DMSO-$d_6$) δ ppm: 1.90-2.05 (2H, m), 2.30 (2H, t, J=8.1Hz), 3.45 (2H, t, J=7.1Hz), 4.51 (2H, s), 6.55-6.65 (1H, m), 7.30-7.40 (1H, m), 7.55-7.70 (1H, m), 7.85-8.00 (1H, m), 8.12 (1H, brs), 8.39 (1H, brs), 10.99 (1H, s)
MS (ESI, m/z): 404 (M+H)+

Compound 2-92

MS (ESI, m/z): 433 (M+H)+

Compound 2-93

MS (ESI, m/z): 433 (M+H)+

Compound 2-94

MS (ESI, m/z): 467 (M+H)+

Compound 2-95

MS (ESI, m/z): 467 (M+H)+

Compound 2-96

$^1$H-NMR (DMSO-$d_6$) δ ppm: 1.30-1.45 (2H, m), 1.45-1.60 (4H, m), 2.35-2.50 (4H, m), 3.58 (2H, s), 6.56 (1H, d, J=3.5Hz), 7.30 (1H, d, J=3.5Hz), 7.35-7.45 (1H, m), 7.60-7.70 (1H, m), 8.00 (1H, brs), 8.00-8.15 (1H, m), 8.27 (1H, brs), 10.98 (1H, brs) MS (ESI, m/z): 386 (M+H)+

Compound 2-97

$^1$H-NMR (DMSO-$d_6$) δ ppm: 1.30-1.45 (2H, m), 1.45-1.60 (4H, m), 2.30-2.50 (4H, m), 3.59 (2H, s), 6.55-6.60 (1H, m), 7.30-7.40 (1H, m), 7.55-7.65 (1H, m), 7.85-7.95 (1H, m), 8.10 (1H, brs), 8.37 (1H, brs), 10.96 (1H, s)
MS (ESI, m/z): 404 (M+H)+

Compound 2-98

$^1$H-NMR (DMSO-$d_6$) δ ppm: 1.05-1.65 (9H, m), 2.10-2.35 (2H, m), 2.75-2.90 (1H, m), 3.81 (2H, brs), 6.55-6.65 (1H, m), 7.30-7.50 (2H, m), 7.60-7.75 (1H, m), 8.00-8.20 (2H, m), 8.31 (1H, brs), 11.05 (1H, s)
MS (ESI, m/z): 400 (M+H)+

Compound 2-99

$^1$H-NMR (DMSO-$d_6$) δ ppm: 1.05-1.30 (5H, m), 1.30-1.65 (4H, m), 2.10-2.30 (2H, m), 2.75-2.85 (1H, m), 3.78 (2H, s), 6.58 (1H, d, J=3.4Hz), 7.33 (1H, d, J=3.4Hz), 7.55-7.70 (1H, m), 7.90-8.00 (1H, m), 8.12 (1H, brs), 8.38 (1H, brs), 11.00 (1H, s) MS (ESI, m/z): 418 (M+H)+

Compound 2-100

$^1$H-NMR (DMSO-$d_6$) δ ppm: 0.85-1.00 (3H, m), 1.30-2.05 (7H, m), 2.10-2.25 (1H, m), 2.90-3.60 (3H, m), 4.40-4.70 (2H, m), 6.95-7.10 (1H, m), 7.40-7.55 (1H, m), 7.55-7.70 (1H, m), 7.80-7.95 (1H, m), 8.05-8.20 (1H, m), 8.35-8.50 (1H, m), 10.43 (1H, brs), 11.00-11.20 (1H, m)

Compound 2-101

$^1$H-NMR (DMSO-$d_6$) δ ppm: 1.10-1.30 (9H, m), 1.45-1.65 (3H, m), 2.25-2.40 (2H, m), 3.97 (2H, s), 6.50-6.65 (1H, m), 7.32 (1H, d, J=3.4Hz), 7.35-7.45 (1H, m), 7.60-7.70 (1H, m), 8.02 (1H, brs), 8.10-8.20 (1H, m), 8.26 (1H, brs), 11.02 (1H, s)
MS (ESI, m/z): 414 (M+H)+

Compound 2-102

$^1$H-NMR (DMSO-$d_6$) δ ppm: 1.00-1.35 (9H, m), 1.45-1.65 (3H, m), 2.25-2.50 (2H, m), 3.98 (2H, brs), 6.50-6.65 (1H, m), 7.25-7.40 (1H, m), 7.40-7.50 (1H, m), 7.70-7.80 (1H, m), 8.01 (1H, brs), 8.25 (1H, brs), 8.35-8.50 (1H, m), 11.05 (1H, s)
MS (ESI, m/z): 430 (M+H)+

Compound 2-103

$^1$H-NMR (DMSO-$d_6$) δ ppm: 1.10-1.30 (9H, m), 1.45-1.65 (3H, m), 2.25-2.40 (2H, m), 3.97 (2H, s), 6.59 (1H, d, J=3.6Hz), 7.34 (1H, d, J=3.6Hz), 7.55-7.70 (1H, m), 7.90-8.05 (1H, m), 8.12 (1H, brs), 8.38 (1H, brs), 11.00 (1H, s)
MS (ESI, m/z): 432 (M+H)+

Compound 2-104

$^1$H-NMR (DMSO-$d_6$) δ ppm: 0.40-0.55 (1H, m), 0.81 (6H, d, J=6.3Hz), 1.45-1.70 (5H, m), 2.75-2.90 (2H, m), 3.59 (2H, s), 6.50-6.60 (1H, m), 7.25-7.35 (1H, m), 7.35-7.45 (1H, m), 7.60-7.70 (1H, m), 7.95-8.10 (2H, m), 8.29 (1H, brs), 10.97 (1H, s)
MS (ESI, m/z): 414 (M+H)+

Compound 2-105

MS (ESI, m/z): 452 (M+H)+

Compound 2-106

$^1$H-NMR (DMSO-$d_6$) δ ppm: 1.30-1.50 (2H, m), 1.65-1.80 (2H, m), 2.05-2.25 (2H, m), 2.65-2.85 (2H, m), 3.35-3.50 (1H, m), 3.60 (2H, s), 4.57 (1H, d, J=4.0Hz), 6.58 (1H, d, J=3.5Hz), 7.33 (1H, d, J=3.5Hz), 7.55-7.65 (1H, m), 7.85-7.95 (1H, m), 8.12 (1H, brs), 8.38 (1H, brs), 10.96 (1H, s)
MS (ESI, m/z): 420 (M+H)+

Compound 2-107

MS (ESI, m/z): 434 (M+H)+

Compound 2-108

$^1$H-NMR (DMSO-$d_6$) δ ppm: 0.75-0.95 (1H, m), 1.35-1.80 (5H, m), 1.95-2.05 (1H, m), 2.75-2.85 (1H, m), 2.85-2.95 (1H, m), 3.15-3.40 (2H, m), 3.59 (2H, s), 4.41 (1H, t, J=5.3Hz), 6.55-6.60 (1H, m), 7.30-7.35 (1H, m), 7.35-7.45 (1H, m), 7.60-7.70 (1H, m), 7.95-8.10 (2H, m), 8.30 (1H, brs), 10.98 (1H, s)
MS (ESI, m/z): 416 (M+H)+

Compound 2-109

$^1$H-NMR ($CDCl_3$+MeOD-$d_4$) δ ppm: 1.25-1.85 (5H, m), 2.10-2.25 (2H, m), 2.95-3.05 (2H, m), 3.30-3.50 (2H, m), 3.69 (2H, s), 6.45 (1H, d, J=3.5Hz), 7.00-7.10 (1H, m), 7.26 (1H, d, J=3.5Hz), 8.05-8.15 (1H, m)

Compound 2-110

$^1$H-NMR (DMSO-$d_6$) δ ppm: 0.75-0.95 (1H, m), 1.35-1.80 (5H, m), 1.90-2.05 (1H, m), 2.70-2.85 (1H, m), 2.85-2.95 (1H, m), 3.10-3.40 (2H, m), 3.59 (2H, s), 4.41 (1H, t, J=5.2Hz), 6.50-6.65 (1H, m), 7.25-7.40 (1H, m), 7.50-7.70 (1H, m), 7.85-7.95 (1H, m), 8.10 (1H, brs), 8.38 (1H, brs), 10.94 (1H, s)
MS (ESI, m/z): 434 (M+H)+

Compound 2-111

MS (ESI, m/z): 448 (M+H)+

Compound 2-112

MS (ESI, m/z): 447 (M+H)+

Compound 2-113

$^1$H-NMR (DMSO-$d_6$) δ ppm: 1.20-1.55 (2H, m), 1.55-1.75 (2H, m), 1.95-2.15 (2H, m), 2.25-2.40 (1H, m), 2.75-2.90 (2H, m), 3.61 (2H, s), 6.59 (1H, d, J=3.2Hz), 6.78 (1H, brs), 7.25-7.40 (2H, m), 7.55-7.65 (1H, m), 7.85-7.95 (1H, m), 8.10 (1H, brs), 8.40 (1H, brs), 10.94 (1H, brs)
MS (ESI, m/z): 447 (M+H)+

Compound 2-114

$^1$H-NMR (DMSO-$d_6$) δ ppm: 1.55-1.70 (4H, m), 3.65 (2H, s), 3.84 (4H, s), 6.55-6.65 (1H, m), 7.30-7.40 (1H, m), 7.55-7.65 (1H, m), 7.85-7.95 (1H, m), 8.12 (1H, brs), 8.38 (1H, brs), 10.97 (1H, brs)
MS (ESI, m/z): 462 (M+H)+

Compound 2-115

$^1$H-NMR (DMSO-$d_6$) δ ppm: 1.55-1.70 (4H, m), 2.45-2.60 (4H, m), 3.65 (2H, s), 3.84 (4H, s), 6.55-6.65 (1H, m), 7.20-7.40 (1H, m), 7.42 (1H, dd, J=1.9, 8.8Hz), 7.70-7.80 (1H, m), 8.01 (1H, brs), 8.25 (1H, brs), 8.34 (1H, d, J=8.8Hz), 11.02 (1H, s)
MS (ESI, m/z): 460 (M+H)+

Compound 2-116

$^1$H-NMR (DMSO-$d_6$) δ ppm: 1.45-2.00 (8H, m), 3.10-3.60 (4H, m), 4.54 (2H, d, J=4.1Hz), 7.02 (1H, d, J=3.8Hz), 7.46 (1H, d, J=3.8Hz), 7.55-7.70 (1H, m), 7.80-7.90 (1H, m), 8.12 (1H, brs), 8.43 (1H, brs), 10.83 (1H, brs), 11.10 (1H, s)

Compound 2-117

$^1$H-NMR (DMSO-$d_6$) δ ppm: 1.40-1.65(10H, m), 2.55-2.70 (4H, m), 3.75 (2H, s), 6.57 (1H, d, J=3.4Hz), 7.33 (1H, d, J=3.4Hz), 7.55-7.70 (1H, m), 7.90-8.00 (1H, m), 8.11 (1H, brs), 8.38 (1H, brs), 10.99 (1H, s)
MS (ESI, m/z): 432 (M+H)+

Compound 2-118

$^1$H-NMR (DMSO-$d_6$) δ ppm: 2.10-2.70 (11H, m), 3.61 (2H, s), 6.55-6.65 (1H, m), 7.25-7.40 (1H, m), 7.55-7.65 (1H, m), 7.85-7.95 (1H, m), 8.10 (1H, brs), 8.38 (1H, brs), 10.96 (1H, brs)
MS (ESI, m/z): 419(M+H)+

Compound 2-119

$^1$H-NMR (DMSO-$d_6$) δ ppm: 2.16 (3H, s), 2.20-2.60 (8H, m), 3.62 (2H, s), 6.58 (1H, d, J=3.5Hz), 7.31 (1H, d, J=3.5Hz), 7.35-7.45 (1H, m), 7.60-7.70 (1H, m), 7.95-8.15 (2H, m), 8.27 (1H, brs), 10.99 (1H, s)
MS (ESI, m/z): 401(M+H)+

Compound 2-120

$^1$H-NMR (DMSO-$d_6$) δ ppm: 0.85-1.05 (6H, m), 2.20-2.70 (9H, m), 3.61 (2H, s), 6.58 (1H, d, J=3.5Hz), 7.33 (1H, d, J=3.5Hz), 7.55-7.65 (1H, m), 7.85-7.95 (1H, m), 8.08 (1H, brs), 8.35 (1H, brs), 10.95 (1H, s)
MS (ESI, m/z): 447 (M+H)+

Compound 2-121

MS (ESI, m/z): 501 (M+H)+

Compound 2-122

MS (ESI, m/z): 477 (M+H)+

Compound 2-123

MS (ESI, m/z): 490 (M+H)+

Compound 2-124

MS (ESI, m/z): 532 (M+H)+

Compound 2-125

$^1$H-NMR (DMSO-$d_6$) δ ppm: 1.31 (3H, t, J=6.9Hz), 2.63 (4H, brs), 3.01 (4H, brs), 3.70 (2H, s), 3.99 (2H, q, J=6.9Hz), 6.63 (1H, d, J=3.1Hz), 6.80-6.95 (4H, m), 7.33 (1H, d, J=3.1Hz), 7.35-7.50 (1H, m), 7.70-7.80 (1H, m), 7.99 (1H, brs), 8.25 (1H, brs), 8.34 (1H, d, J=9.0Hz), 11.04 (1H, s)
MS (ESI, m/z): 523 (M+H)+

Compound 2-126

$^1$H-NMR (DMSO-$d_6$) δ ppm: 1.32 (3H, t, J=7.0Hz), 2.55-2.70 (4H, m), 2.90-3.10 (4H, m), 3.70 (2H, s), 3.99 (2H, q, J=7.0Hz), 6.60-6.70 (1H, m), 6.80-6.95 (4H, m), 7.30-7.45 (2H, m), 7.60-7.70 (1H, m), 8.00 (1H, brs), 8.05-8.15 (1H, m), 8.27 (1H, brs), 11.02 (1H, brs)

Compound 2-127

$^1$H-NMR (DMSO-$d_6$) δ ppm: 2.20-2.60 (8H, m), 3.45 (2H, s), 3.62 (2H, s), 6.50-6.60 (1H, m), 7.15-7.35 (6H, m), 7.35-7.45 (1H, m), 7.60-7.70 (1H, m), 7.90-8.15 (2H, m), 8.26 (1H, brs), 10.98 (1H, s)
MS (ESI, m/z): 477 (M+H)+

Compound 2-128

$^1$H-NMR (DMSO-$d_6$) δ ppm: 2.20-2.60 (4H, m), 3.45 (2H, s), 3.62 (2H, s), 6.50-6.60 (1H, m), 7.20-7.40 (6H, m), 7.40-7.50 (1H, m), 7.70-7.80 (1H, m), 7.99 (1H, brs), 8.24 (1H, brs), 8.30-8.40 (1H, m), 11.01 (1H, s)
MS (ESI, m/z): 493 (M+H)+

Compound 2-129

MS (ESI, m/z): 495 (M+H)+

Compound 2-130

$^1$H-NMR (DMSO-$d_6$) δ ppm: 2.30-2.60 (8H, m), 3.51 (2H, s), 3.62 (2H, s), 6.57 (1H, d, J=3.1Hz), 7.10-7.20 (2H, m), 7.25-7.35 (2H, m), 7.35-7.45 (1H, m), 7.55-7.65 (1H, m), 7.85-7.95 (1H, m), 8.08 (1H, brs), 8.34 (1H, brs), 10.95 (1H, s)
MS (ESI, m/z): 513 (M+H)+

Compound 2-131

$^1$H-NMR (DMSO-$d_6$) δ ppm: 2.25-2.60 (8H, m), 3.48 (2H, s), 3.63 (2H, s), 6.58 (1H, d, J=3.7Hz), 7.00-7.20 (3H, m), 7.30-7.40 (2H, m), 7.50-7.65 (1H, m), 7.85-7.95 (1H, m), 8.07 (1H, brs), 8.34 (1H, brs), 10.95 (1H, s)
MS (ESI, m/z): 513 (M+H)+

Compound 2-132

$^1$H-NMR (DMSO-$d_6$) δ ppm: 2.20-2.60 (8H, m), 3.43 (2H, s), 3.62 (2H, s), 6.50-6.65 (1H, m), 7.05-7.20 (2H, m), 7.20-7.40 (3H, m), 7.55-7.65 (1H, m), 7.85-7.95 (1H, m), 8.07 (1H, brs), 8.34 (1H, brs), 10.94 (1H, s)

MS (ESI, m/z): 513 (M+H)+

Compound 2-133

$^1$H-NMR (DMSO-$d_6$) δ ppm: 2.25-2.60 (8H, m), 3.42 (2H, brs), 3.62 (2H, s), 3.73 (3H, s), 6.58 (1H, d, J=3.5Hz), 6.75-6.95 (3H, m), 7.15-7.30 (1H, m), 7.32 (1H, d, J=3.5Hz), 7.55-7.65 (1H, m), 7.85-7.95 (1H, m), 8.07 (1H, brs), 8.34 (1H, brs), 10.95 (1H, s)
MS (ESI, m/z): 525 (M+H)+

Compound 2-134

$^1$H-NMR (DMSO-$d_6$) δ ppm: 2.20-2.65 (8H, m), 3.25-3.45 (2H, m), 3.62 (2H, s), 3.72 (3H, s), 6.57 (1H, brs), 6.80-6.95 (2H, m), 7.10-7.25 (2H, m), 7.32 (1H, brs), 7.50-7.65 (1H, m), 7.85-8.00 (1H, m), 8.07 (1H, brs), 8.34 (1H, brs), 10.94 (1H, s)
MS (ESI, m/z): 525 (M+H)+

Compound 2-135

$^1$H-NMR (DMSO-$d_6$) δ ppm: 2.25-2.60 (8H, m), 3.38 (2H, s), 3.62 (2H, s), 3.71 (6H, s), 6.30-6.40 (1H, m), 6.40-6.55 (2H, m), 6.58 (1H, d, J=3.4Hz), 7.32 (1H, d, J=3.4Hz), 7.55-7.65 (1H, m), 7.85-7.95 (1H, m), 8.08 (1H, brs), 8.35 (1H, brs), 10.95 (1H, s)
MS (ESI, m/z): 555 (M+H)+

Compound 2-136

$^1$H-NMR (DMSO-$d_6$) δ ppm: 2.25-2.65 (8H, m), 3.49 (2H, s), 3.63 (2H, s), 6.50-6.65 (1H, m), 7.30-7.45 (2H, m), 7.50-7.80 (2H, m), 7.85-8.00 (1H, m), 8.00-8.20 (1H, m), 8.25-8.55 (3H, m), 10.95 (1H, s)
MS (ESI, m/z): 496 (M+H)+

Compound 2-137

$^1$H-NMR (DMSO-$d_6$) δ ppm: 2.30-2.60 (8H, m), 3.50 (2H, s), 3.64 (2H, s), 6.55-6.65 (1H, m), 7.25-7.40 (3H, m), 7.55-7.65 (1H, m), 7.85-8.00 (1H, m), 8.00-8.15 (1H, m), 8.25-8.45 (1H, m), 8.45-8.60 (2H, m), 10.95 (1H, s)
MS (ESI, m/z): 496 (M+H)+

Compound 2-138

MS (ESI, m/z): 539 (M+H)+

Compound 2-139

MS (ESI, m/z): 571 (M+H)+

Compound 2-140

$^1$H-NMR (DMSO-$d_6$) δ ppm: 1.98 (3H, s), 2.35-2.55 (4H, m), 3.35-3.55 (4H, m), 3.68 (2H, s), 6.61 (1H, d, J=3.5Hz), 7.34 (1H, d, J=3.5Hz), 7.55-7.70 (1H, m), 7.85-7.95 (1H, m), 8.11 (1H, brs), 8.38 (1H, brs), 10.97 (1H, s)

Compound 2-141

$^1$H-NMR (DMSO-$d_6$) δ ppm: 2.35-2.65 (4H, m), 3.50-3.80 (4H, m), 6.55-6.70 (1H, m), 7.30-7.50 (6H, m), 7.55-7.70 (1H, m), 7.85-8.00 (1H, m), 8.12 (1H, brs), 8.39 (1H, brs), 10.97 (1H, s)
MS (ESI, m/z): 509 (M+H)+

Compound 2-142

$^1$H-NMR (DMSO-$d_6$) δ ppm: 1.38 (9H, s), 2.35-2.50 (4H, m), 3.66 (2H, s), 6.61 (1H, d, J=3.4Hz), 7.34 (1H, d, J=3.4Hz), 7.55-7.70 (1H, m), 7.85-8.00 (1H, m), 8.11 (1H, brs), 8.38 (1H, brs), 10.97 (1H, s)

MS (ESI, m/z): 505 (M+H)+

Compound 2-143

$^1$H-NMR ($CDCl_3$) δ ppm: 2.35-2.80 (1.2H, m), 3.67 (2H, s), 5.12 (2H, s), 5.70 (1H, brs), 6.35-6.45 (1H, m), 6.48 (1H, brs), 6.95-7.10 (1H, m), 7.20-7.45 (6H, m), 8.10-8.25 (1H, m), 10.72 (1H, brs)

Compound 2-144

$^1$H-NMR (DMSO-$d_6$) δ ppm: 3.45-3.60 (2H, m), 3.60-3.80 (2H, m), 4.00-4.20 (2H, m), 4.67 (2H, s), 5.11 (2H, m), 6.62 (1H, d, J=3.1Hz), 7.25-7.45 (6H, m), 7.50-7.65 (1H, m), 7.85-8.00 (1H, m), 8.08 (1H, brs), 8.36 (1H, brs), 11.01 (1H, s)
MS (ESI, m/z): 553 (M+H)+

Compound 2-145

$^1$H-NMR (DMSO-$d_6$) δ ppm: 1.60-1.80 (3H, m), 2.20-2.80 (10H, m), 3.75 (2H, s), 6.57 (1H, d, J=3.5Hz), 7.32 (1H, d, J=3.5Hz), 7.55-7.65 (1H, m), 7.85-8.00 (1H, m), 8.10 (1H, brs), 8.40 (1H, brs), 11.00 (1H, brs)

Example 4

5-Fluoro-3-(4-hydroxy-3-morpholin-4-ylmethylbenzoylamino)-benzofuran-2-carboxamide (compound 3-1)

3-(4-Benzyloxy-3-morpholin-4-ylmethylbenzoylamino)-5-fluorobenzofuran-2-carboxamide (compound 1-202,0.026g) was dissolved in tetrahydrofuran (3mL) and 10% palladium on carbon (50% wet, 0.025g) was added to the solution, and the mixture was stirred at room temperature under hydrogen atmosphere for 6 hours. After the catalyst was removed by filtration, the filtrate was concentrated under reduced pressure. The residue was purified by a SCX ion-exchanged column conditioned with methanol (argonaut 1g, eluent: methanol) to give the title compound (0.01g).
$^1$H-NMR ($CDCl_3$) δ ppm: 2.45-2.80 (4H, m), 2.96 (1H, brs), 3.65-4.00 (6H, m), 5.78 (1H, brs), 6.43 (1H, brs), 6.90-7.00 (1H, m), 7.15-7.25 (1H, m), 7.35-7.45 (1H, m), 7.70-7.80 (1H, m), 7.85-7.95 (1H, m), 8.30-8.40 (1H, m), 10.73 (1H, brs)

Compounds 3-2 to 3-8 were prepared in a manner similar to those as described in Example 4 using 3-(4-benzyloxy-3-morpholin-4-ylmethylbenzoylamino)-5,7-difluorobenzofuran-2-carboxamide (compound 1-203), 3-(4-benzyloxy-3-morpholin-4-ylmethylbenzoylamino)-6-methyl benzofuran-2-carboxamide (compound 1-204), 3-(4-benzyloxy-3-morpholin-4-ylmethylbenzoylamino)-6-methoxybenzofuran-2-carboxamide (compound 1-205), benzyl N-(2-carbamoyl-5-fluorobenzofuran-3-yl)isophthalamate (compound 1-235), benzyl 3-{4-[3-(2-carbamoyl-5,7-difluorobenzofuran-3-ylcarbamoyl)benzyl]piperazin-1-yl}propionate (compound 2-53), benzyl 4-[3-(2-carbamoyl-5,7-difluorobenzofuran-3-ylcarbamoyl)benzyl]-3-oxopiperazine-1-carboxylate (compound 2-55) and benzyl 4-[5-(2-carbamoyl-5,7-difluorobenzofuran-3-ylcarbamoyl)furan-2-ylmethyl]-3-oxopiperazine-1-carboxylate (compound 2-144) instead of 3-(4-benzyloxy-3-morpholin-4-ylmethylbenzoylamino)-5-fluoro benzofuran-2-carboxamide (compound 1-202). These were illustrated in table 4.

Table 4

| Compound No. | Structure | Compound No. | Structure |
|---|---|---|---|
| 3-1 | 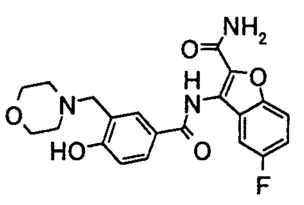 | 3-5 | 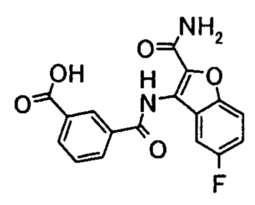 |
| 3-2 | 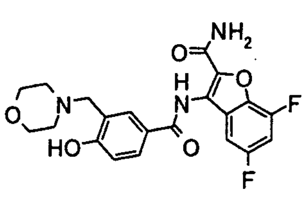 | 3-6 | 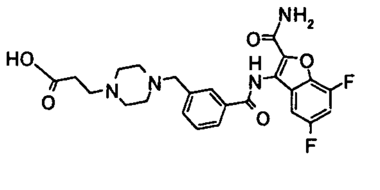 |

(continued)

| Compound No. | Structure | Compound No. | Structure |
|---|---|---|---|
| 3-3 | 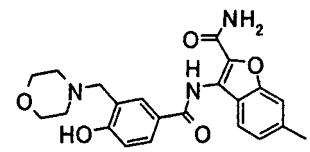 | 3-7 | 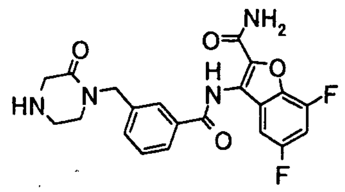 |
| 3-4 | 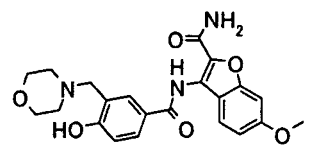 | 3-8 | 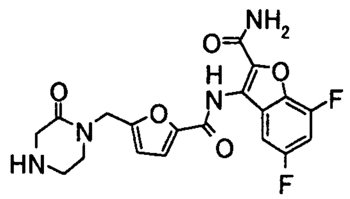 |

The physical data of compounds 3-2 to 3-8 were described below.

Compound 3-2

$^1$H-NMR ($CDCl_3$+MeOD-$d_4$) δ ppm: 2.50-2.80 (4H, m), 3.70-3.85 (4H, m), 3.83 (2H, s), 6.90-7.10 (2H, m), 7.70-7.90 (2H, m), 8.05-8.15 (1H, m)

Compound 3-3

$^1$H-NMR ($CDCl_3$) δ ppm: 2.50 (3H, s), 2.40-2.80 (4H, m), 3.70-3.85 (4H, m), 3.82 (2H, s), 5.54 (1H, brs), 6.36 (1H, brs), 6.90-6.95 (1H, m), 7.10-7.25 (2H, m), 7.75-7.80 (1H, m), 7.85-7.95 (1H, m), 8.45-8.55 (1H, m), 10.80 (1H, brs)

Compound 3-4

$^1$H-NMR ($CDCl_3$) δ ppm: 2.40-2.80 (4H, m), 2.99 (1H, brs), 3.70-3.85 (6H, m), 3.89 (3H, s), 5.52 (1H, brs), 6.30 (1H, brs), 6.85-7.00 (3H, m), 7.70-7.80 (1H, m), 7.85-7.95 (1H, m), 8.50-8.60 (1H, m), 10.86 (1H, brs)

Compound 3-5

$^1$H-NMR (DMSO-$d_6$) δ ppm: 7.35-7.50 (1H, m), 7.62-7.80 (2H, m), 7.90-8.09 (2H, m), 8.15-8.35 (3H, m), 8.60 (1H, s), 11.13 (1H, s), 12.80-13.78 (1H, br)

Compound 3-6

$^1$H-NMR (MeOD-$d_4$) δ ppm: 2.40-2.85 (6H, m), 2.95-3.20 (4H, m), 3.70 (2H, s), 7.15-7.30 (1H, m), 7.45-7.65 (2H, m), 7.85-8.10 (3H, m)

Compound 3-7

$^1$H-NMR (DMSO-$d_6$) δ ppm: 2.85-2.95 (2H, m), 3.15-3.30 (2H, m), 4.62 (2H, s), 7.50-7.70 (3H, m), 7.75-8.00 (3H, m), 8.10 (1H, brs), 8.37 (1H, brs), 10.99 (1H, s)
MS (ESI, m/z): 429 (M+H)+

Compound 3-8

$^1$H-NMR (DMSO-$d_6$) δ ppm: 2.60-2.85 (1H, m), 2.93 (2H, t, J=5.5Hz), 3.29 (2H, s), 3.39 (2H, t, J=5.5Hz), 4.62 (2H, s), 6.60 (1H, d, J=3.4Hz), 7.34 (1H, d, J=3.4Hz), 7.50-7.65 (1H, m), 7.85-8.00 (1H, m), 8.11 (1H, brs), 8.39 (1H, brs), 10.99 (1H, s)
MS (ESI, m/z): 419 (M+H)+

Example 5

5,7-Difluoro-3-[5-(4-methylpiperazine-1-carbonyl)furan-2-carbonyl]aminobenzofuran-2-carboxamide (compound 4-1)

To a solution of furan-2,5-dicarbonyl dichloride(0.073g) in tetrahydrofuran (3mL) were added 3-amino-5,7-difluorobenzofuran-2-carboxamide (0.08g) and N,N-dimethylaniline (0.096mL) successively under ice-cooling, and the resulting mixture was stirred at room temperature overnight. 1-Methylpiperazine (0.085mL) was added to the mixture, and the resulting mixture was stirred for 3 hours. Water and ethyl acetate were added to the reaction mixture. The organic layer was separated and washed with water. The organic layer was extracted with 2mol/L hydrochloric acid, and the aqueous layer was washed with ethyl acetate. Then, the aqueous layer was made alkaline with a saturated aqueous solution of sodium carbonate, and was extracted with ethyl acetate . The organic layer was separated, and washed with water, a saturated aqueous solution of sodium carbonate and brine, and dried over anhydrous magnesium sulfate. The solvent was removed under reduced pressure, and the residue was purified by column chromatography on aminopropylated silica gel (eluent: hexane/ethyl acetate = 9/1 - 0/1) to give the title compound (0.004g). The structural formula was illustrated in table 5.
$^1$H-NMR ($CDCl_3$+MeOD-$d_4$) $\delta$ ppm: 2.30-2.65 (6H, m), 2.37 (3H, s), 3.95-4.20 (2H, m), 7.00-7.10 (1H, m), 7.20 (1H, d, J=3.5Hz), 7.35 (1H, d, J=3.5Hz), 8.10-8.25 (1H, m)
3-[5-(4-Benzylpiperazine-1-carbonyl)furan-2-carbonyl] amino-5,7-difluorobenzofuran-2-carboxamide (compounds 4-2) was prepared in a manner similar to those as described in Example 5 using 1-benzylpiperazine instead of 1-methylpiperazine. The structural formula was illustrated in table 5.
$^1$H-NMR ($CDCl_3$+DMSO-$d_6$) $\delta$ ppm: 2.50-2.70 (4H, m), 2.88 (2H, s), 3.55-3.65 (2H, m), 3.90-4.10 (2H, m), 6.04 (1H, brs), 6.69 (1H, brs), 7.00-7.40 (8H, m), 8.15-8.25 (1H, m)

Example 6

5-(2-Carbamoyl-5,7-difluorobenzofuran-3-ylcarbamoyl)furan-2-carboxylic acid (compound 5-1)

To a solution of furan-2, 5-dicarbonyl dichloride (0.073g) in tetrahydrofuran (3mL) were added 3-amino-5,7-difluorobenzofuran-2-carboxamide (0.08g) and N,N-dimethylaniline (0.096mL) successively under ice-cooling, and the resulting mixture was stirred at room temperature overnight. A saturated aqueous solution of sodium carbonate and ethyl acetate were added to the reaction mixture, and the aqueous layer was separated and washed with ethyl acetate. To the aqueous layer was added 2mol/L hydrochloric acid, and the resulting precipitate was collected by filtration. The precipitate was washed with water and methanol to give the title compound (0.018g). The structural formula was illustrated in table 5.
$^1$H-NMR (DMSO-$d_6$) $\delta$ ppm: 7.40 (1H, d, J=3.8Hz), 7.47 (1H, d, J=3.8Hz), 7.55-7.65 (1H, m), 7.75-7.90 (1H, m), 8.09 (1H, brs), 8.36 (1H, brs), 11.09 (1H, brs), 13.69 (1H, brs)

Example 7

5-Fluoro-3-[3-(1-hydroxyethyl)benzoylamino]benzofuran-2-carboxamide (compound 6-1)

Sodium borohydride (0.02g) was added to a mixture of 3-(3-acetylbenzoylamino)-5-fluorobenzofuran-2-carboxamide (compound 1-236, 0.045g), tetrahydrofuran (5mL) and methanol (3mL), and the resulting mixture was stirred at room temperature for 2 hours. Water was added to the reaction mixture and the mixture was stirred for another 1 hour. The mixture was concentrated under reduced pressure. To the residue were added water and ethyl acetate. The organic layer was separated, washed with brine, and dried over anhydrous magnesium sulfate. The solvent was removed under reduced pressure, and the residue was purified by column chromatography on silica gel (eluent: hexane/ethyl acetate = 3/1 - 0/1) to give the title compound (0.038g). The structural formula was illustrated in the table 5.
$^1$H-NMR ($CDCl_3$) $\delta$ ppm: 1.54 (3H, d, J=6.5Hz), 3.47 (1H, d, J=4.0Hz), 4.95-5.05 (1H, m), 6.48 (1H, brs), 6.65 (1H, brs), 7.15-7.25 (1H, m), 7.35-7.55 (2H, m), 7.60-7.70 (1H, m), 7.85-7.95 (1H, m), 8.00-8.10 (1H, m), 8.30-8.40 (1H, m), 10.96 (1H, brs)
5-Fluoro-3-[5-(1-hydroxyethyl)furan-2-carbonyl]-aminobenzofuran-2-carboxamide (compound 6-2) was prepared in a manner similar to those as described in Example 7-1 using 3-(5-acetylfuran-2-carbonyl)amino-5-fluorobenzofuran-2-carboxamide (compound 1-304) instead of 3-(3-acetylbenzoylamino)-5-fluorobenzofuran-2-carboxamide (compound 1-236). The structural formula was illustrated in table 5.
$^1$H-NMR ($CDCl_3$) $\delta$ ppm: 1.63 (3H, d, J=6.6Hz), 3.34 (1H, d, J=2.5Hz), 4.90-5.05 (1H, m), 6.09 (1H, brs), 6.44 (1H, d, J=3.5Hz), 6.51 (1H, brs), 7.15-7.25 (1H, m), 7.23 (1H, d, J=3.5Hz), 7.30-7.40 (1H, m), 8.30-8.40 (1H, m), 10.88 (1H, brs)

Example 8

5,7-Difluoro-3-[3-(1H-tetrazol-5-yl)benzoylamino]benzofuran -2-carboxamide (compound 7-1)

A mixture of 3-(3-cyanobenzoylamino)-5,7-difluorobenzofuran-2-carboxamide (compound 1-206, 0.12g), sodium azide (0.093g) trimethylamine hydrochloride (0.101g) and 1-methyl-2-pyrrolidone (3mL) was stirred at 130° C overnight. Water (7mL) was added to the reaction mixture, and the mixture was acidified with the addition of concentrated hydrochloric acid. The mixture was stirred at room temperature for 1 hour, and the resulting precipitate was collected by filtration. The crude product was purified by column chromatography on silica gel (eluent: dichloromethane/methanol = 19/1 - 5/1) to give the title compound (0.046g). The structural formula was illustrated in the table 5.
$^1$H-NMR (DMSO-$d_6$) δ ppm: 7.55-7.65 (1H, m), 7.70-7.90 (2H, m), 8.08 (1H, brs), 8.10-8.20 (1H, m), 8.25-8.35 (1H, m), 8.34 (1H, brs), 8.65-8.75 (1H, m), 11.08 (1H, brs)

Example 9

7-Ethoxy-5-fluoro-3-(4-fluorobenzoylamino)benzofuran-2-carboxamide (compound 8-1)

To a mixture of 5-fluoro-3-(4-fluorobenzoylamino)-7-hydroxybenzofuran-2-carboxamide (compound 1-178, 0.08g), potassium carbonate (0.1g) and N,N-dimethylfomamide (2mL) was added ethyl iodide (0.029mL) at room temperature, and the resulting mixture was stirred at 60° C overnight. Water was added to the reaction mixture, and the resulting precipitate was collected by filtration to give the title compound (0.04g). The structural formula was illustrated in table 5.
$^1$H-NMR (DMSO-$d_6$) δ ppm: 1.43 (3H, t, J=7.1Hz), 4.26-4.36 (2H, m), 7.05-7.15 (1H, m), 7.35-7.50 (3H, m), 7.90-8.25 (4H, m), 10.89 (1H, s)

Example 10

5,7-Difluoro-3-(5-piperazin-1-ylmethylfuran-2-carbonyl)-aminobenzofuran-2-carboxamide (compound 9-1)

A solution of tert-butyl 4-[5-(2-carbamoyl-5,7-difluorobenzofuran-3-ylcarbamoyl)furan-2-ylmethyl]piperazine-1-carboxylate (compound 2-142, 0.44g) in 4mol/L hydrogen chloride in ethyl acetate (18mL) was stirred at room temperature for 4 hours. The solvent of the reaction mixture was removed under reduced pressure. The residue was dissolved in ethyl acetate, methanol and a 1mol/L aqueous solution of sodium hydroxide, and the mixture was stirred at room temperature for 20 minutes. Water and ethyl acetate were added to the mixture. The organic layer was separated, and the solvent was removed under reduced pressure to give the title compound (0.166g). The structural formula was illustrated in the table 5.
$^1$H-NMR (DMSO-$d_6$) δ ppm: 2.30-2.50 (4H, m), 2.65-2.80 (4H, m), 3.59 (2H, s), 6.50-6.65 (1H, m), 7.25-7.40 (1H, m), 7.50-7.65 (1H, m), 7.85-8.00 (1H, m), 8.06 (1H, brs), 8.39 (1H, brs)

Example 11

6-Chloro-3-(4-fluoro-3-morpholin-4-ylmethylbenzoylamino)-benzofuran-2-carboxamide (compound 10-1)

A mixture of 4-fluoro-3-methylbenzoic acid (0.1g), N-bromosuccinimide (0.121g), benzoyl peroxide (0.021g, 25%wet) and carbon tetrachloride was heated under reflux for 4 hours. Insoluble materials were removed by filtration. The filtrate was concentrated under reduced pressure, and the residue was purified by column chromatography on silica gel (eluent: hexane: ethyl acetate = 9:1-4:1:1% acetic acid was added) to give 3-bromomethyl-4-fluorobenzoic acid (0.151g).

N,N-Dimethylformamide (1 drop) was added to a mixture of 3-bromomethyl-4-fluorobenzoic acid and thionyl chloride (2mL) at room temperature, and the mixture was stirred at 80°C for 2.5 hours. The reaction mixture was concentrated under reduced pressure. The residue was dissolved in tetrahydrofuran (1mL), and the resulting solution was added to a mixture of 3-amino-6-chlorobenzofuran-2-carboxamide (0.1g), dimethylaniline (0.19mL) and tetrahydrofuran (2mL). The mixture was stirred at room temperature for 2 hours. Water was added to the reaction mixture, and the resulting precipitate was collected by filtration to give 3-(3-bromomethyl-4-fluorobenzoylamino)-6-chlorobenzofuran-2-carboxamide (0.042g).

A mixture of 3-(3-bromomethyl-4-fluorobenzoylamino)-6-chlorobenzofuran-2-carboxamide, morpholine (0.0092g),PS-NMM (registrated mark) resin (argonaut, 100-200mesh, 1% divinylbenzene, 1.98mmol/g resin, 0.152g) and 1-methyl-2-pyrrolidone (2mL) was stirred at 80° C for 1 hour. After cooling the reaction mixture to room temperature,

TMI-isocyanate (registrated mark) (mimotope, D-type, 100micromol/unit, 2units) was added and the mixture was stirred at 80° C for 2 hours. The reaction mixture was purified by a SCX ion-exchanged column conditioned with methanol (argonaut, 2g, washing solvent:N,N-dimethylformamide, eluent: 2mol/L ammonia in methanol) to give the title compound (0.024g). The structural formula was illustrated in the table 5.
$^1$H-NMR (DMSO-$d_6$) δ ppm: 2.35-2.50 (4H, m), 3.50-3.70 (6H, m), 7.35-7.50 (2H, m), 7.70-7.80 (1H, m), 7.90-8.15 (3H, m), 8.15-8.35 (2H, m), 11.05 (1H, s)
MS (ESI, m/z) : 432 (M+H)+

Example 12

3-[5-(3-Dimethylaminomethylphenyl)furan-2-carbonyl]amino-5,7-difluorobenzofuran-2-carboxamide (Compound 11-1)

A mixture of 3-[5-(3-chloromethylphenyl)furan-2-carbonyl]amino-5,7-difluorobenzofuran-2-carboxamide (compound 1-316, 0.041g), dimethylamine hydrochloride (0.0282g), PS-NMM (registered mark) resin (argonauts, 100-200mesh, 1% divinylbenzene, 1.98mmol/g resin, 0.144g) and 1-methyl-2-pyrrolidone (2mL) was stirred at 80°C for 7.5 hours. After cooling the reaction mixture to room temperature, TMI-isocyanate (registered mark) (mimotope, D-type, 100micromol/unit, 3units) was added, the mixture was stirred at 80° C for 4 hours. The mixture was purified by a SCX ion-exchanged column conditioned with methanol (argonaut, 2g, washing solvent:N,N-dimethylformamide, eluent: 2mol/L ammonia in methanol) to give the title compound (0.0045g). The structural formula was illustrated in table 5.
$^1$H-NMR (DMSO-$d_6$) δ ppm: 2.18 (6H, s), 3.47 (2H, s), 7.20-7.40 (2H, m), 7.40-7.55 (2H, m), 7.55-7.65 (1H, m), 7.75-7.90 (2H, m), 7.95-8.10 (1H, m), 8.16 (1H, brs), 8.39 (1H, brs), 11.30 (1H, s)
MS (ESI, m/z) : 440 (M+H)+
N-(2-carbamoyl-5,7-difluorobenzofuran-3-yl)-6-(3-dimethyl aminomethylphenoxy)nicotinamide (compound 11-2) was prepared in a manner similar to those as described in example 12 using N-(2-carbamoyl-5,7-difluorobenzofuran-3-yl)-6-(3-chloromethylphenoxy)nicotinamide(compounds 1-357) instead of 3-[5-(3-chloromethylphenyl)furan-2-carbonyl]-amino-5,7-difluorobenzofuran-2-carboxamide (compound 1-316). The structural formula was illustrated in table 5.
$^1$H-NMR (DMSO-$d_6$) δ ppm: 2.17 (6H, brs), 3.44 (2H, brs), 7.05-7.30 (4H, m), 7.35-7.80 (3H, m), 8.07 (1H, brs), 8.34 (1H, brs), 8.35-8.50 (1H, m), 8.75-8.85 (1H, m), 10.88 (1H, brs)

Table 5

| Compound No. | Structure | Compound No. | Structure |
|---|---|---|---|
| 4-1 | 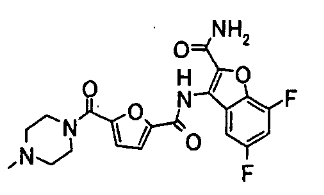 | 8-1 | 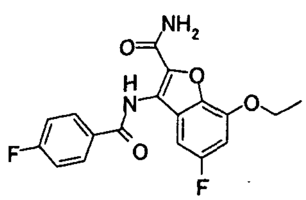 |
| 4-2 | 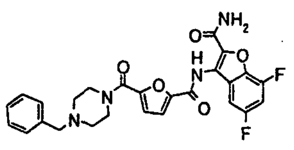 | 9-1 | 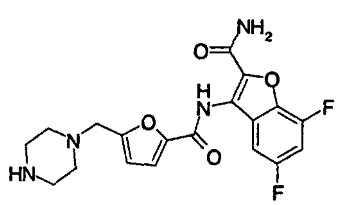 |
| 5-1 | 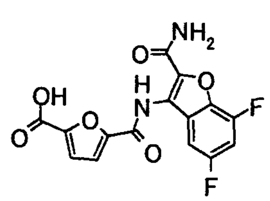 | 10-1 | 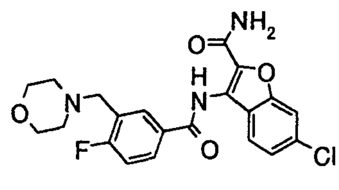 |

(continued)

| Compound No. | Structure | Compound No. | Structure |
|---|---|---|---|
| 6-1 | 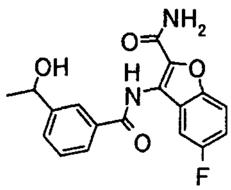 | 11-1 | 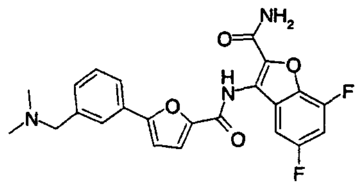 |
| 6-2 | 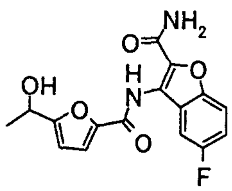 | 11-2 | 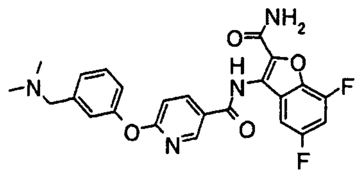 |
| 7-1 | 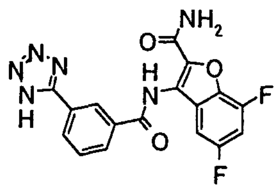 | | |

Test Example 1

Measurement of antagonistic action for human adenosine $A_{2A}$ receptor

1) Preparation of human adenosine $A_{2A}$ receptor-expressed cells

When Chinese hamster ovary-K1 (CHO-K1) cells were grown to subconfluence, they were treated with trypsin and suspended in D-MEM/F-12 medium (Invitrogen) including 10% fetal bovine serum (Sankojunyaku). The expression vector of human adenosine $A_{2A}$ receptor prepared in OPTI-MEM I (Invitrogen) and the transfection reagent of Lipofectamine 2000 (Invitrogen) were added to the cell suspension, and those cells were inoculated in poly-D-lysine-coated 96-well plates (5 x $10^4$ cells/well). After incubating the plates for 24-30 hours at 37QC in a humidified atmosphere with 5% $CO_2$, they were used for the subsequent assays.

2) Measurement of the inhibitory effect on human adenosine $A_{2A}$ receptor activity

A test compound was dissolved in dimethyl sulfoxide (DMSO) and the resulting solution was added to D-MEM/F-12 medium supplemented with 2.5 unit/mL adenosine deaminase (Carbiochem) and 30 pM rolipram (Sigma), a phosphodiesterase inhibitor to prepare a solution of the test compund. Adenosine deaminase was used for the inactivation of adenosine derived from the cells.

The human adenosine $A_{2A}$-expressed cells inoculated in the 96-well plates were washed twice with 150 µL/well of D-MEM/F-12 medium and a 50-µL aliquot of the test compound solution was then added to each well of the plates. After incubating the cells for 10 minutes at 37° C in a humidified atmosphere with 5% $CO_2$, a 50-µL aliquot of 1.2 nM 5'-N-ethylcarboxyamide adenosine (NECA; Sigma) including 2.5 unit/mL adenosine deaminase and 30 µM rolipram was added to each well of the plates and incubated for 25 minutes at 37° C in a humidified atmosphere with 5% $CO_2$. Stopping the reaction and the subsequent measurement of cAMP were performed by use of a cAMP enzyme-linked immunosorbent assay kit (Applied Biosystem). The chemoluminescence was measured by a Microplate Luminometer TR717 (Applied Biosystem). The experiments were carried out in duplicate wells.

Inhibitory rate of a test compound for NECA-induced cAMP production was found by the following formula:

Inhibitory rate (%) = [1-{(cAMP level with a test compound and NECA)-(cAMP level without a test compound and NECA)}/{(cAMP level with NECA)-(cAMP level without a test compound and NECA)}] x 100

Using a Km value for NECA found by the measurement in the same plate as that of test compounds, an inhibitory constant Ki was found by the following formula:

$$Ki = IC_{50} / \{1 + (6 \times 10^{10} / Km)\}$$

The results were shown in Table 6.

Table 6

| Compound NO. | Ki value (nM) | Compound NO. | Ki value (nM) |
|---|---|---|---|
| 1-1 | 8.5 | 1-150 | 2.2 |
| 1-2 | 4.1 | 1-153 | 9.2 |
| 1-15 | 6.5 | 1-155 | 8.1 |
| 1-16 | 0.9 | 1-156 | 9.8 |
| 1-36 | 6.4 | 1-159 | 3.3 |
| 1-91 | 6.5 | 1-161 | 7.0 |
| 1-108 | 9.3 | 1-213 | 2.1 |
| 1-109 | 2.7 | 1-249 | 4.6 |
| 1-119 | 1.9 | 1-275 | 0.5 |
| 1-121 | 0.8 | 2-1 | 8.0 |
| 1-125 | 1.2 | 2-7 | 8.6 |
| 1-133 | 3.2 | 2-129 | 1.6 |
| 1-138 | 4.5 | 2-138 | 2.0 |
| 1-141 | 9.3 | 2-142 | 2.0 |
| 1-145 | 2.7 | 6-2 | 1.1 |

Test example 2

Haloperidol-induced catalepsy in rats

The effect of a compound on drug-induced catalepsy in rats was evaluated. Parkinson's disease is a movement disorder reflecting a decline in striatal dopamine due to the degeneration of dopaminergic neuron arising from the sbstantia nigra. When haloperidol, a blocker of dopamine D2 receptor is administered in rats, the drug induces catalepsy which is considered as one of the Parkinson's disease-like symptoms since haloperidol blocks the neurotransmission of dopaminergic neuron. Male rats (Crj: CD(SD)IGS, body weight: 210-260g, Charles River Japan, Inc.) were used for the experiments. Three to five rats for each group were tested for catalepsy 5 hours after intraperitoneal administration of 1mg/kg haloperidol (Serenace (registered mark); Dainippon Pharma), a dopamine D2 antagonist. Catalepsy was measured using the horizontal bar test (Morelli and Chiara, "Eur. J. Pharmacol.", vol.117, p.179-185 (1985)). A compound (10 mg/kg) was dissolved in PEG400 and administered orally 2 hours prior to the measurement of catalepsy. PEG400 was also administered orally to the rats for the control group. The front feet of the rats were placed on a horizontal bar (3 mm in diameter) suspended 10-12cm above a platform and their rear feet were placed on the platform. The degree of catalepsy produced in the rats was measured by how long it took for their front feet to remove themselves from the bar. A maximum of 180 seconds was allowed. The results were shown in Table 7.

Table 7

| Group | Duration of catalepsy (sec) |
|---|---|
| Control | 180.0 |
| Compound 1-2 | 91.6 |
| Compound 1-16 | 116.4 |
| Compound 1-121 | 48.2 |

(continued)

| Group | Duration of catalepsy (sec) |
|---|---|
| Compound 1-155 | 38.3 |

The compounds of the present invention exhibited superior catalepsy improvement action, and are accordingly useful as a medicament for treating a Parkinson's disease.

INDUSTRIAL APPLICABILITY

Compounds represented by general formula (I) of the present invention exhibit potent adenosine $A_{2A}$ receptor antagonistic activities, and are accordingly useful for treating or preventing diseases mediated by adenosine $A_{2A}$ receptors such as motor function disorders, depression, an anxiety disorder, cognitive function disorders, cerebral ischemia disorder, restless legs syndrome and the like.

**Claims**

**1.** A compound represented by general formula (I):

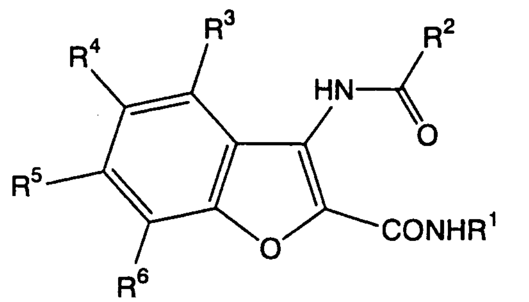

(I)

a prodrug thereof , or a pharmaceutically acceptable salt thereof, wherein
$R^1$ is a hydrogen atom or a lower alkyl group;
$R^2$ is:

a) a lower alkyl group,
b) a halo-lower alkyl group,
c) a hydroxy-lower alkyl group,
d) a cycloalkyl group,
e) an aryl-cycloalkyl group,
f) a heterocycloalkyl group,
g) an aryl group, unsubstituted or substituted with 1 to 5 substituents selected from the group consisting of $X^1$, $X^2$, $X^3$, $X^4$ and $X^5$,
h) an aralkyl group, wherein the ring of the aralkyl group is unsubstituted or substituted with 1 to 5 substituents selected from the group consisting of $X^1$, $X^2$, $X^3$, $X^4$ and $X^5$,
i) an aryl-alkenyl group, wherein the ring of the aryl-alkenyl group is unsubstituted or substituted with 1 to 5 substituents selected from the group consisting of $X^1$, $X^2$, $X^3$, $X^4$ and $X^5$,
j) a lower alkyl group substituted with a group selected from a lower alkoxy group or a lower acyloxy group,
k) an aryloxy-lower alkyl group, wherein the ring of the aryloxy-lower alkyl group is unsubstituted or substituted with 1 to 5 substituents selected from the group consisting of $X^1$, $X^2$, $X^3$, $X^4$ and $X^5$,
1) an aralkyloxy-lower alkyl group, wherein the ring of the aralkyloxy-lower alkyl group is unsubstituted or substituted with 1 to 5 substituents selected from the group consisting of $X^1$, $X^2$, $X^3$, $X^4$ and $X^5$,
m) an arylsulfanyl-lower alkyl group, wherein the ring of the arylsulfanyl-lower alkyl group is unsubstituted or substituted with 1 to 5 substituents selected from the group consisting of $X^1$, $X^2$, $X^3$, $X^4$ and $X^5$,
n) a heteroaryl group, unsubstituted or substituted with 1 to 3 substituents selected from the group consisting of $X^6$, $X^7$ and $X^8$, or

o) a heteroaryl-lower alkyl group, wherein the ring of the heteroaryl-lower alkyl group is unsubstituted or substituted with 1 to 3 substituents selected from the group consisting of $X^6$, $X^7$ and $X^8$;

$X^1$, $X^2$, $X^3$, $X^4$ and $X^5$ are each independently:

a) a halogen atom,
b) a lower alkyl group,
c) a halo-lower alkyl group,
d) a cycloalkyl group,
e) a lower alkoxy group,
f) a halo-lower alkoxy group,
g) a cycloalkyloxy group,
h) a heterocycloalkyloxy group,
i) a lower alkoxy-lower alkoxy group,
j) a hydroxy-lower alkyl group,
k) a hydroxyl group,
1) a carboxy group,
m) a lower alkoxycarbonyl group,
n) an aralkyloxycarbonyl group,
o) a lower acyl group,
p) a cyano group,
q) $-A^1-NR^{20}R^{21}$,
r) $-A^2-SR^{22}$,
s) $-SO_2NR^{23}R^{24}$,
t) a phenyl group, unsubstituted or substituted with 1 to 3 substituents selected independently from the group consisting of a halogen atom, a lower alkyl group, a halo-lower alkyl group, a lower alkoxy group, a halo-lower alkoxy group and a di(lower alkyl)amino-lower alkyl group,
u) a phenoxy group, unsubstituted or substituted with 1 to 3 substituents selected independently from the group consisting of a halogen atom, a lower alkyl group, a halo-lower alkyl group, a lower alkoxy group, a halo-lower alkoxy group and a di(lower alkyl)amino-lower alkyl group,
v) a heteroaryl group, unsubstituted or substituted with 1 to 3 substituents selected independently from the group consisting of a halogen atom, a lower alkyl group, a halo-lower alkyl group, a lower alkoxy group, a halo-lower alkoxy group and a di(lower alkyl)amino-lower alkyl group,
w) a heteroaryloxy group, unsubstituted or substituted with 1 to 3 substituents selected independently from the group consisting of a halogen atom, a lower alkyl group, a halo-lower alkyl group, a lower alkoxy group, a halo-lower alkoxy group and a di(lower alkyl)amino-lower alkyl group, or
x) a lower alkoxy group substituted with a group selected form an aryl group or a heteroaryl group, or

when two of $X^1$, $X^2$, $X^3$, $X^4$ and $X^5$ are adjacent each other, they are bonded together to form a group represented by $-O-(CH_2)_m-O-$, $-O-(CH_2)_n-$ or $-(CH_2)_p-$;
$R^{20}$ and $R^{21}$ are each independently a hydrogen atom, a lower alkyl group, a cycloalkyl group, a heterocycloalkyl group, a bridged cyclic hydrocarbon group,a heteroaryl-lower alkyl group, a hydroxy-lower alkyl group, a lower alkoxy-lower alkyl group, a lower acyl group, a lower alkoxycarbonyl group, an aralkyloxycarbonyl group or a di (lower alkyl) amino - lower alkyl group, or
$R^{20}$ and $R^{21}$, taken together with the nitrogen atom to which they are bonded, form a cyclic amino group, wherein the cyclic amino group is unsubstituted or substituted with one or two substituents selected independently from the group consisting of:

a) a lower alky group,
b) a cycloalkyl group,
c) a phenyl group, unsubstituted or substituted with 1 to 3 substituents selected independently from the group consisting of a halogen atom, a lower alkyl group, a halo-lower alkyl group, a lower alkoxy group and a halo-lower alkoxy group,
d) an aralkyl group, wherein the ring of the aralkyl group is unsubstituted or substituted with 1 to 3 substituents selected independently from the group consisting of a halogen atom, a lower alkyl group, a halo-lower alkyl group, a lower alkoxy group and a halo-lower alkoxy group, or adjacent ring-carbon atoms of the aralkyl group are substituted with $-O-(CH_2)_m-O-$,
e) a heteroaryl group,

f) a heteroaryl-lower alkyl group,
g) a lower alkyl group substituted with a group selected from a hydroxyl group, a lower alkoxy group, a carboxy group, an aralkyloxycarbonyl group, a cyclic aminocarbonyl group or a di(lower alkyl)amino group,
h) a hydroxyl group,
i) an oxo group,
j) a lower alkoxycarbonyl group,
k) an aralkyloxycarbonyl group,
1) a carbamoyl group,
m) a lower acyl group,
n) a benzoyl group,
o) a di(lower alkyl)amino group, and
p) a diphenylmethylene group;

$A^1$ is a bond, a $C_{1-3}$-alkylene group or a carbonyl group;
$A^2$ is a bond or a $C_{1-3}$-alkylene group;
$R^{22}$ is:

a) a lower alkyl group,
b) a phenyl group, unsubstituted or substituted with 1 to 3 substituents selected independently from the group consisting of a halogen atom, a lower alkyl group, a halo-lower alkyl group, a lower alkoxy group and a halo-lower alkoxy group,
c) a heteroaryl group, unsubstituted or substituted with 1 to 3 substituents selected independently from the group consisting of a halogen atom, a lower alkyl group, a halo-lower alkyl group, a lower alkoxy group and a halo-lower alkoxy group, or
d) a di(lower alkyl)amino-lower alkyl group;

$R^{23}$ and $R^{24}$ are each independently a hydrogen atom or a lower alkyl group, or
$R^{23}$ and $R^{24}$, taken together with the nitrogen atom to which they are bonded, form a cyclic amino group, wherein the cyclic amino group is unsubstituted or substituted with a group selected from a lower alkyl group or an aralkyl group;
m is 1 or 2;
n is 2 or 3;
p is 3 or 4;
$X^6$, $X^7$ and $X^8$ are each independently:

a) a halogen atom,
b) a lower alkyl group,
c) a halo-lower alkyl group,
d) a hydroxy-lower alkyl group,
e) a cycloalkyl group,
f) a heterocycloalkyl-lower alkyl group,
g) a lower alkoxy group,
h) a halo-lower alkoxy group,
i) a lower acyl group,
j) a carboxy group,
k) $-A^1-NR^{20}R^{21}$,
1) $-A^2-SR^{22}$,
m) $-SO_2NR^{23}R^{24}$,
n) a phenyl group, unsubstituted or substituted with 1 to 3 substituents selected independently from the group consisting of a halogen atom, a lower alkyl group, a halo-lower alkyl group, a lower alkoxy group, a halo-lower alkoxy group and a di(lower alkyl)amino-lower alkyl group,
o) a phenoxy group, unsubstituted or substituted with 1 to 3 substituents selected independently from the group consisting of a halogen atom, a lower alkyl group, a halo-lower alkyl group, a lower alkoxy group, a halo-lower alkoxy group and a di(lower alkyl)amino-lower alkyl group,
p) an aralkyl group, wherein the ring of the aralkyl group is unsubstituted or substituted with 1 to 3 substituents selected independently from the group consisting of a halogen atom, a lower alkyl group, a halo - lower alkyl group , a lower alkoxy group, a halo-lower alkoxy group and a di(lower alkyl)amino-lower alkyl group,
q) a heteroaryl group, unsubstituted or substituted with 1 to 3 substituents selected independently from the

group consisting of a halogen atom, a lower alkyl group, a halo-lower alkyl group, a lower alkoxy group, a halo-lower alkoxy group and a di(lower alkyl)amino-lower alkyl group,
r) a heteroaryloxy group, unsubstituted or substituted with 1 to 3 substituents selected independently from the group consisting of a halogen atom, a lower alkyl group, a halo-lower alkyl group, a lower alkoxy group, a halo-lower alkoxy group and a di(lower alkyl)amino-lower alkyl group, or
s) an aralkyloxy group;

$R^3$, $R^4$, $R^5$ and $R^6$ are each independently:

a) a hydrogen atom,
b) a halogen atom,
c) a lower alkyl group,
d) a halo-lower alkyl group,
e) a lower alkoxy group,
f) a halo-lower alkoxy group,
g) a hydroxyl group,
h) a cyano group,
i) an aryl group, unsubstituted or substituted with 1 to 3 substituents selected independently from the group consisting of a halogen atom, a lower alkyl group, a halo-lower alkyl group, a lower alkoxy group and a halo-lower alkoxy group,
j) an aralkyloxy group, wherein the ring of the aralkyloxy group is unsubstituted or substituted with 1 to 3 substituents selected independently from the group consisting of a halogen atom, a lower alkyl group, a halo-lower alkyl group, a lower alkoxy group and a halo-lower alkoxy group,
k) a di(lower alkyl)amino group,
1) a lower alkylsulfanyl group, or
m) a nitro group, or

when two of $R^3$, $R^4$, $R^5$ and $R^6$ are adjacent each other, they are bonded together to form a group represented by -CH=CH-CH=CH-, provided that at least one of $R^3$, $R^4$, $R^5$ and $R^6$ is other than a hydrogen atom;
with the proviso that the following compounds are excluded:

(1) 1-acetylaminonaphtho[2,1-b]furan-2-carboxamide,
(2) 1-benzoylaminonaphtho[2,1-b]furan-2-carboxamide,
(3) 3-benzoylamino-5-chlorobenzofuran-2-carboxamide,
(4) 5-chloro-3-[2-(3,4-diethoxyphenyl)acetylamino]-benzofuran-2-carboxamide,
(5) 5-bromo-3-[2-(3,4-diethoxyphenyl)acetylamino]-benzofuran-2-carboxamide,
(6) 5-chloro-3-(2-chloroacetylamino)benzofuran-2-carboxamide, and
(7) 3-acetylamino-5-chlorobenzofuran-2-carboxamide.

**2.** The compound according to claim 1, or a pharmaceutically acceptable salt thereof, wherein $R^1$ is a hydrogen atom.

**3.** The compound according to claim 2, or a pharmaceutically acceptable salt thereof, wherein $R^3$ is a hydrogen atom.

**4.** The compound according to claim 3, or a pharmaceutically acceptable salt thereof, wherein
$R^2$ is:

a) a lower alkyl group,
b) a cycloalkyl group,
c) an aryl group, unsubstituted or substituted with 1 to 5 substituents selected from the group consisting of $X^1$, $X^2$, $X^3$, $X^4$ and $X^5$,
d) an aralkyl group, wherein the ring of the aralkyl group is unsubstituted or substituted with 1 to 5 substituents selected from the group consisting of $X^1$, $X^2$, $X^3$, $X^4$ and $X^5$,
e) a lower alkoxy-lower alkyl group,
f) an aryloxy-lower alkyl group, wherein the ring of the aryloxy-lower alkyl group is unsubstituted or substituted with 1 to 5 substituents selected from the group consisting of $X^1$, $X^2$, $X^3$, $X^4$ and $X^5$,
g) an aralkyloxy- lower alkyl group, wherein the ring of the aralkyloxy-lower alkyl group is unsubstituted or substituted with 1 to 5 substituents selected from the group consisting of $X^1$, $X^2$, $X^3$, $X^4$ and $X^5$, or
h) a heteroaryl group, unsubstituted or substituted with 1 to 3 substituents selected from the group consisting

of $X^6$, $X^7$ and $X^8$; and

$X^1$, $X^2$, $X^3$, $X^4$, $X^5$, $X^6$, $X^7$ and $X^8$ are as defined in claim 1.

**5.** The compound according to claim 4, or a pharmaceutically acceptable salt thereof, wherein $R^4$, $R^5$ and $R^6$ are each independently a hydrogen atom, a halogen atom, a lower alkyl group, a halo-lower alkyl group or a lower alkoxy group, provided that at least one of $R^4$, $R^5$ and $R^6$ is other than a hydrogen atom.

**6.** The compound according to claim 5, or a pharmaceutically acceptable salt thereof, wherein
$R^2$ is:

a) a cycloalkyl group,
b) an aryl group, unsubstituted or substituted with 1 to 5 substituents selected from the group consisting of $X^1$, $X^2$, $X^3$, $X^4$ and $X^5$, or
c) a heteroaryl group, unsubstituted or substituted with 1 to 3 substituents selected from the group consisting of $X^6$, $X^7$ and $X^8$.

**7.** The compound according to claim 6, or a pharmaceutically acceptable salt thereof, wherein
$X^1$, $X^2$, $X^3$, $X^4$ and $X^5$ are each independently:

a) a halogen atom,
b) a lower alkyl group,
c) a lower alkoxy group,
d) a halo-lower alkoxy group,
e) a heterocycloalkyloxy group,
f) a hydroxyl group,
g) $-A^1-NR^{20}R^{21}$,
h) $-A^2-SR^{22}$,
i) $-SO_2NR^{23}R^{24}$,
j) a heteroaryl group, unsubstituted or substituted with 1 to 3 substituents selected independently from the group consisting of a halogen atom, a lower alkyl group, a halo-lower alkyl group, a lower alkoxy group, a halo-lower alkoxy group and di(lower alkyl)amino-lower alkyl group, or

when two of $X^1$, $X^2$, $X^3$, $X^4$ and $X^5$ are adjacent each other, they are bonded together to form $-OCH_2O-$; and
$X^6$, $X^7$ and $X^8$ are each independently:

a) a halogen atom,
b) a lower alkyl group,
c) a hydroxy-lower alkyl group,
d) a cycloalkyl group,
e) a heterocycloalkyl-lower alkyl group,
f) $-A^1-NR^{20}R^{21}$,
g) $-SO_2NR^{23}R^{24}$,
h) a phenyl group, unsubstituted or substituted with 1 to 3 substituents selected independently from the group consisting of a halogen atom, a lower alkyl group, a halo-lower alkyl group, a lower alkoxy group, a halo-lower alkoxy group and a di(lower alkyl)amino-lower alkyl group, or
i) a phenoxy group, unsubstituted or substituted with 1 to 3 substituents selected independently from the group consisting of a halogen atom, a lower alkyl group, a halo-lower alkyl group, a lower alkoxy group, a halo-lower alkoxy group and a di(lower alkyl)amino-lower alkyl group; and

$A^1$, $A^2$, $R^{20}$, $R^{21}$, $R^{22}$, $R^{23}$ and $R^{24}$ are as defined in claim 1.

**8.** The compound according to claim 1, or a pharmaceutically acceptable salt thereof, selected from the group consisting of:

(1) 3-cyclopropanecarbonylamino-5-fluorobenzofuran-2-carboxamide;
(2) 5-chloro-3-cyclopropanecarbonylaminobenzofuran-2-carboxamide;
(3) 3-(3-fluorobenzoylamino)-6-methoxybenzofuran-2-carboxamide;

(4) 3-(4-fluorobenzoylamino)-6-methoxybenzofuran-2-carboxamide;
(5) 5-fluoro-3-(3-methylbenzoylamino)benzofuran-2-carboxamide;
(6) 3-(benzo[1,3]dioxole-5-carbonyl)amino-6-fluorobenzofuran-2-carboxamide;
(7) 5-chloro-3-(furan-2-carbonyl)aminobenzofuran-2-carboxamide;
(8) 5,7-difluoro-3-(furan-2-carbonyl)aminobenzofuran-2-carboxamide;
(9) 5,7-difluoro-3-(5-methylfuran-2-carbonyl)aminobenzofuran-2-carboxamide;
(10) 3-(5-ethylfuran-2-carbonyl)amino-5-fluorobenzofuran-2-carboxamide;
(11) 3-(5-ethylfuran-2-carbonyl)amino-5,7-difluorobenzofuran-2-carboxamide;
(12) 6-methoxy-3-(5-phenylfuran-2-carbonyl)aminobenzofuran-2-carboxamide;
(13) 6-fluoro-3-(6-phenoxypyridine-3-carbonyl)aminobenzofuran-2-carboxamide;
(14) 6-methoxy-3-(2-methoxyacetylamino)benzofuran-2-carboxamide;
(15) 3-[2-(4-chlorophenoxy)acetylamino]-5-fluorobenzofuran-2-carboxamide;
(16) 3-(2-benzyloxyacetylamino)-5-fluorobenzofuran-2-carboxamide;
(17) 6-chloro-3-cyclopropanecarbonylaminobenzofuran-2-carboxamide;
(18) 3-cyclopropanecarbonylamino-5,7-difuorobenzofuran-2-carboxamide;
(19) 7-chloro-3-cyclopropanecarbonylamino-5-fluorobenzofuran-2-carboxamide;
(20) 3-cyclopropanecarbonylamino-5-fluoro-7-methoxybenzofuran-2-carboxamide;
(21) 3-cyclobutanecarbonylamino-5,7-difluorobenzofuran-2-carboxamide;
(22) 5-fluoro-7-methoxy-3-(4-methoxybenzoylamino)-benzofuran-2-carboxamide;
(23) 5,7-difluoro-3-phenylacetylaminobenzofuran-2-carboxamide;
(24) 5,7-difluoro-3-[3-(4-methylpiperazine-l-carbonyl)-benzoylamino]benzofuran-2-carboxamide;
(25) 6-methoxy-3-[3-(4-phenylpiperazin-1-ylmethyl)benzoylamino]benzofuran-2-carboxamide;
(26) 6-methoxy-3-[4-(1-methyl-1H-imidazol-2-ylsulfanylmethyl)benzoylamino]benzofuran-2-carboxamide;
(27) 3-[5-(4-benzylpiperazin-1-ylmethyl)furan-2-carbonyl]-amino-5,7-difluorobenzofuran-2-carboxamide;
(28) 3-[5-(4-benzo[1,3]dioxol-5-ylmethylpiperazin-1-ylmethyl)furan-2-carbonyl]amino-5,7-difluorobenzofuran-2-carboxamide;
(29) tert-butyl 4-[5-(2-carbamoyl-5,7-difluorobenzofuran-3-ylcarbamoyl)furan-2-ylmethyl]piperazine-1-carboxylate, and
(30) 5-fluoro-3-[5-(1-hydroxyethyl)furan-2-carbonyl]amino-benzofuran-2-carboxamide.

**9.** A pharmaceutical composition which comprises, as an active ingredient, a compound according to any one of claims 1 to 8 or a pharmaceutically acceptable salt thereof.

**10.** A therapeutic or prophylactic agent for a disease mediated by adenosine $A_{2A}$ receptors, which comprises, as an active ingredient, a compound according to any one of claims 1 to 8 or a pharmaceutically acceptable salt thereof.

**11.** The therapeutic or prophylactic agent according to claim 10, wherein the disease mediated by adenosine $A_{2A}$ receptors is a motor function disorder.

**12.** The therapeutic or prophylactic agent according to claim 11, wherein the motor function disorder is Parkinson's disease, Huntington's disease or Wilson's disease.

**13.** The therapeutic or prophylactic agent according to claim 10, wherein the disease mediated by adenosine $A_{2A}$ receptors is depression or an anxiety disorder.

**14.** The therapeutic or prophylactic agent according to claim 10, wherein the disease mediated by adenosine $A_{2A}$ receptors is a cognitive function disorder.

**15.** The therapeutic or prophylactic agent according to claim 10, wherein the disease mediated by adenosine $A_{2A}$ receptors is a cerebral ischemia disorder.

**16.** The therapeutic or prophylactic agent according to claim 10, wherein the disease mediated by adenosine $A_{2A}$ receptors is restless legs syndrome.

**17.** A pharmaceutical combination comprising a compound according to any one of claims 1 to 8 or a pharmaceutically acceptable salt thereof and at least one selected from anti-Parkinson drugs, antidepressants, drugs for cognitive function disorders and drugs for cerebral ischemia disorders other than adenosine $A_{2A}$ receptor antagonists.

**18.** A use of a compound according to any one of claims 1 to 8 or a pharmaceutically acceptable salt thereof for the manufacture of a medicament for treating or preventing a disease mediated by adenosine $A_{2A}$ receptors.

**19.** A method for treating or preventing a disease mediated by adenosine $A_{2A}$ receptors, which comprises administering an effective amount of a compound according to any one of claims 1 to 8 or a pharmaceutically acceptable salt thereof.

**INTERNATIONAL SEARCH REPORT**

International application No.
PCT/JP2005/001168

A. CLASSIFICATION OF SUBJECT MATTER
Int.Cl[7] C07D307/85, 307/86, 405/14, 413/14, 405/12, 409/12, 413/12, 491/113, A61K31/343, 31/5377, 31/496, 31/4025, 31/497, 31/41, 31/438, 31/357, 31/381, 31/422, 31/443, 31/35, 31/4525, 31/423, 31/4178, 31/5377,
According to International Patent Classification (IPC) or to both national classification and IPC

B. FIELDS SEARCHED
Minimum documentation searched (classification system followed by classification symbols)
Int.Cl[7] C07D307/85, 307/86, 405/14, 413/14, 405/12, 409/12, 413/12, 491/113, A61K31/343, 31/5377, 31/496, 31/4025, 31/497, 31/41, 31/438, 31/357, 31/381, 31/422, 31/443, 31/35, 31/4525, 31/423, 31/4178, 31/5377,

Documentation searched other than minimum documentation to the extent that such documents are included in the fields searched

Electronic data base consulted during the international search (name of data base and, where practicable, search terms used)
CAPLUS(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | PADMASHALI, Basavaraj et al., Synthesis and pharmacological evaluation of some naphtho [2,1-b] furo [3,2-d] pyrimidines, Indian Journal of Heterocyclic Chemistry, 2002, 12(2), 89-94 | 1-2,4<br>3,5-18 |
| A | SCAMMELLS, Peter J. et al., XH-14 analogs as adenosine antagonists, Bioorganic & Medicinal Chemistry, 1998, 6(9), 1517-1524 | 1-18 |
| A | WO 03/045385 A1 (F. HOFFMANN-LA ROCHE AG.), 05 June, 2003 (05.06.03), & EP 1450796 A1 01 September, 2004 (01.09.04) & US 6596718 B 22 July, 2003 (22.07.03) | 1-18 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
21 February, 2005 (21.02.05)

Date of mailing of the international search report
08 March, 2005 (08.03.05)

Name and mailing address of the ISA/
Japanese Patent Office

Authorized officer

Facsimile No.

Telephone No.

Form PCT/ISA/210 (second sheet) (January 2004)

**INTERNATIONAL SEARCH REPORT**

International application No.
PCT/JP2005/001168

C (Continuation). DOCUMENTS CONSIDERED TO BE RELEVANT

| Category* | Citation of document, with indication, where appropriate, of the relevant passages | Relevant to claim No. |
|---|---|---|
| A | WO 02/074056 A2 (KING PHARMACEUTICALS RESEARCH AND DEVELOPMENT INC.), 26 September, 2002 (26.09.02), & US 2001-47008 A 29 November, 2001 (29.11.01) & JP 2004-536789 A 09 December, 2004 (09.12.04) & EP 1401441 A 31 March, 2004 (31.03.04) | 1-18 |

Form PCT/ISA/210 (continuation of second sheet) (January 2004)

**INTERNATIONAL SEARCH REPORT**

International application No.
PCT/JP2005/001168

Continuation of A. CLASSIFICATION OF SUBJECT MATTER
(International Patent Classification (IPC))

Int.Cl$^7$ 31/4439, 31/4725, 31/4035, 31/55, A61P9/10, 25/14, 25/24, 25/28

(According to International Patent Classification (IPC) or to both national classification and IPC)

Continuation of B. FIELDS SEARCHED
Minimum documentation searched (International Patent Classification (IPC))

Int.Cl$^7$ 31/4439, 31/4725, 31/4035, 31/55, A61P9/10, 25/14, 25/24, 25/28

Minimum documentation searched (classification system followed by classification symbols)

Form PCT/ISA/210 (extra sheet) (January 2004)

**INTERNATIONAL SEARCH REPORT**

International application No.
PCT/JP2005/001168

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 19
because they relate to subject matter not required to be searched by this Authority, namely:
The invention as set forth in claim 19 pertains to methods for treatment of the human body by therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest.
☐ No protest accompanied the payment of additional search fees.

Form PCT/ISA/210 (continuation of first sheet (2)) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004108137 A
- WO 2004019949 A
- JP 7267961 A

### Non-patent literature cited in the description


- **FERRE S. et al.** *Proc. Natl. Acad. Sci. U.S.A.,* 1991, vol. 88, 7238-7241
- **FERRE S. et al.** *Neurosci. Lett.,* 1991, vol. 130, 162-164
- **MANDHANE S.N. et al.** *Eur. J. Pharmacol.,* 1997, vol. 328, 135-141
- **VARANI K. et al.** *The FASEB Journal,* 2003, vol. 17, 2148-2150
- **EL. YACOUBI M. et al.** *British J. Pharmacol.,* 2001, vol. 134, 68-77
- **DALL'LGNA O. et al.** *British J. Pharmacol.,* 2003, vol. 138, 1207-1209
- **PHILLIS J.W. et al.** *Brain Res.,* 1995, vol. 705, 79-84
- **SANGAPURE S. S. et al.** *Indian J. Chem.,* 1978, vol. 16B, 627-629
- **AGASIMUNDIN Y.S. et al.** *Indian J. Chem.,* 1981, vol. 20, 114-117
- **AGASIMUNDIN Y.S. et al.** *Indian J. Chem.,* 1993, vol. 32B, 965-968
- **AGASIMUNDIN Y.S. et al.** *Indian J. Heterocyclic Chem.,* 1994, vol. 3, 247-252
- **BASAVARAJ P. et al.** *Indian J. Heterocyclic Chem.,* 2002, vol. 12, 89-94
- *CHEMICAL ABSTRACTS,* 340017-67-6
- *CHEMICAL ABSTRACTS,* 663931-40-6
- *CHEMICAL ABSTRACTS,* 633288-47-8
- *CHEMICAL ABSTRACTS,* 397881-00-4
- *CHEMICAL ABSTRACTS,* 332375-01-6
- **MORELLI ; CHIARA.** *Eur. J. Pharmacol.,* 1985, vol. 117, 179-185